# NEW EUROPEAN PATENT SPECIFICATION

(11) **EP 0 269 436 B2**
(45) Date of publication and mention of the opposition decision: **02.11.1994**
(45) Mention of the grant of the patent: 06.03.1991
(21) Application number: 87310417.8
(22) Date of filing: 25.11.1987
(51) Int. Cl.: G03C 7/38, C07D 487/04

(54) **Silver halide color photographic light-sensitive material containing pyrazoloazole type cyan coupler**
Farbphotographisches, lichtempfindliches Silberhalogenidmaterial, das einen Pyrazoloazol-Typ-Cyankuppler enthält
Matériau photographique couleur à l'halogénure d'argent sensible à la lumière contenant un coupleur cyan de type pyrazoloazole

(30) Priority: 25.11.1986 JP 280164/86; 27.12.1986 JP 313455/86; 02.03.1987 JP 47323/87; 09.03.1987 JP 53417/87; 17.03.1987 JP 62162/87; 17.03.1987 JP 62163/87; 23.07.1987 JP 184552/87
(43) Date of publication of application: 01.06.1988
(73) Proprietor: KONICA CORPORATION, Tokyo 160 (JP)
(72) Inventor: Tachibana, Kimie, Hino-shi Tokyo (JP); Kaneko, Yutaka, Hino-shi Tokyo (JP); Ishii, Fumio, Hino-shi Tokyo (JP)
(74) Representative: Ellis-Jones, Patrick George Armine

(56) References cited:
- EP-A- 0 119 741
- EP-A- 0 178 794
- EP-A- 0 252 288
- WO-A-86/02467
- GB-A- 1 334 515
- RESEARCH DISCLOSURE, no. 124, August 1974, pages 39-41, abstract no. 12443; "A novel synthesis of pyrazolo[3,2-c]-s-triazole couplers"
- The Theory of the Photographic Process", 4th edition, 1982, pp. 194-207, Macmillan Publishing Co. Inc., New York
- Research Disclosure Sept. 1984, Item 24531, pp. 442-454
- F.M. Hamer "Heterocyclic compounds (Cyanine Dyes and Related Compounds)", 1964, Interscience, pp. 123,286, 525

## Description

This invention relates to a silver halide color photographic light-sensitive material containing a novel cyan dye-forming coupler having excellent spectral absorption characteristics.

A color image is formed by exposing a silver halide photographic light-sensitive material to light and then treating the light-sensitive material in a color development process in which an oxidized aromatic primary amine color developing agent is reacted with a dye-forming coupler (sometimes be referred to simply as a coupler ) to form a dye.

Generally, in this process, the color reproduction method uses a color subtraction technique, so that yellow, magenta and cyan color images are formed.

For the cyan color image dye-forming couplers, phenols or naphthols have popularly been used so far.

From the viewpoint of color reproduction, however, cyan images obtained from phenols or naphthols have serious problems. There is poor spectral absorption sharpness on the short wavelength side and an undesirable absorption, i.e., an irregular absorption, in the green spectral region. Therefore with a light-sensitive negative material, it is necessary to provide a mask or take some other measure to remedy the irregular absorption. With light-sensitive paper material,there is no remedying measure, and the color reproducibility thereof is consequently impaired.

With dye images obtained from phenols and naphthols so far used, there are still several unsolved problems concerning their preservability. For example, the dye images obtained from the 2-acylaminophenol cyan couplers described in U.S. Patent Nos. 2,367,531 and 2,423,730 generally have poor fastness against heat; the dye images obtained from the 2,5-diacylaminophenol cyan couplers described in U.S. Patent Nos. 2,369,929 and 2.772,162 generally have poor fastness against light; and dye images obtained from the 1-hydroxy-2-naphthamide cyan coupler generally have poor fastness against both light and heat. They have therefore been unsatisfactory.

The 2,5-diacylaminophenol cyan couplers described in for example U.S. Patent No. 4,122,369, and Japanese Patent Publication Open to Public Inspection (hereinafter called Japanese Patent O.P.I. Publication) Nos. 155538-1982 and 157246-1982, and the 2,5-diacylamino phenol cyan couplers having a hydroxy group in the ballast group described in U.S. Patent No. 3,880,661, do not provide satisfactory performance, from the viewpoints of the fastness against light and heat and yellow-stain prevention.

The present invention seeks to provide a silver halide color photographic light-sensitive material in which the above-mentioned defects of the cyan coupler are improved, namely, the spectral absorption is sharpened and the absorption in the green spectral region is reduced, so that a dear cyan image having excellent spectral absorption characteristics is formed.

The present invention further seeks to provide a silver halide color photographic light-sensitive material capable of forming a cyan image which does not change in hue due to heat and moisture, and to provide a light-sensitive silver halide photographic material containing a novel cyan dye-forming coupler which has improved dispersion stability especially when stored under cool conditions.

The present invention provides a light-sensitive silver halide color photographic material comprising a support and provided thereon at least one red-light-sensitive silver halide emulsion layer containing a pyrazoloazole type cyan dye-forming coupler as defined below, said coupler being such that, following reaction with an oxidized product of color developing agent, the resultant compound has a spectral absorption maximum within the visible wavelengths of from 600mm to 700mm.

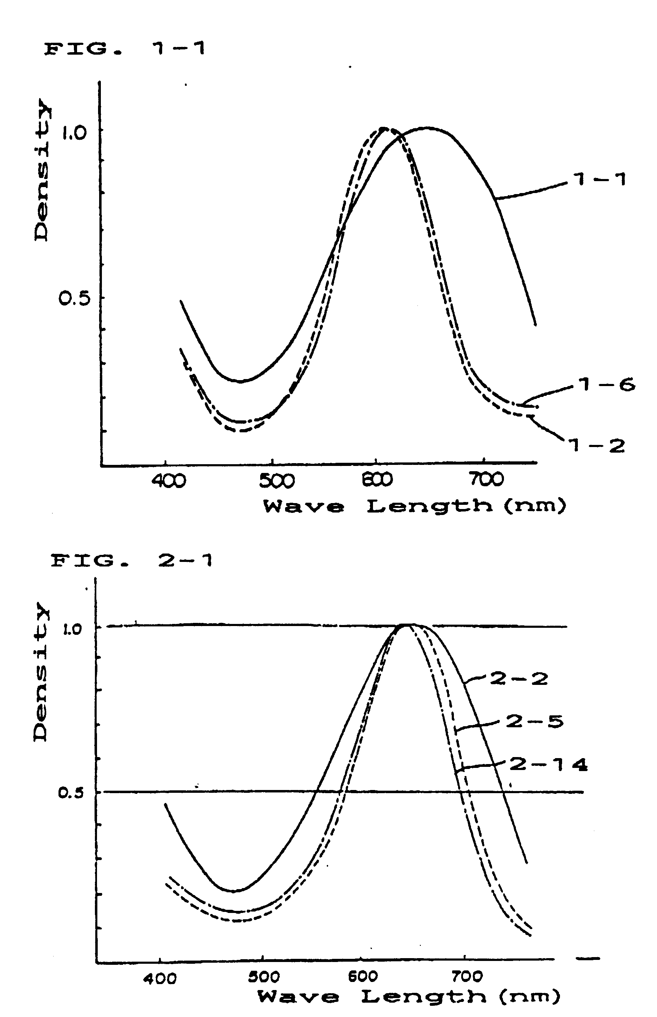

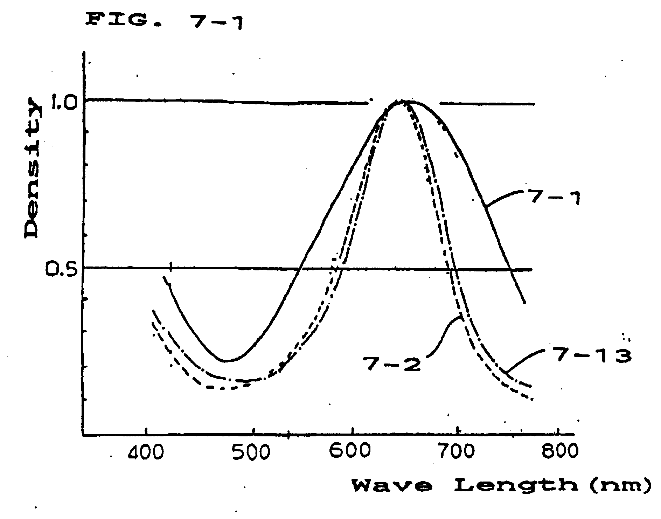

Figs. 1-1 to 7-1 show the absorption spectra of processed samples of the present invention and comparison samples.

The cyan dye-forming coupler of the present invention is of Formula [)]-1: 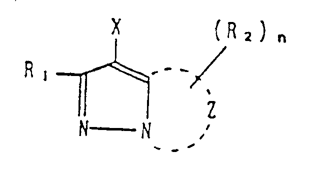 Wherein at least one of R₁ and the or each R₂ is an electron withdrawing group, and the remainder are hyrogen atoms or substituents;Z is a non-metallic group which completes together with the atoms to which it is attached, a nitrogen-containing heterocyclic ring; each R₂ is bonded to a carbon atom of the heterocyclic ring; X is a hydrogen atom or a substituent capable of splitting off upon reaction with an oxidized product of a color developing agent; and n is 1 or 2.

In Formula [I]-I, the electron withdrawing groups, represented by at least one of R₁ and the or each R₂, preferably have a substituent constant αp (defined by Hammett) of not less than +0.20. They include sulfonyl, sulfinyl, sulfonyloxy, sulfonylmethyl. sulfamoyl, phosphonyl, tetrazolyl. pyrrolyl,nitro, carbamoyl. acyl, acyloxy, oxycarbonyl, halogenated alkoxy, halogen atomes.

The carbamoyl groups may be substituted, for example by an alkyl group, or an aryl group (preferably, a phenyl group).

The acyl groups include, for example, an alkylcarbonyl group, and an arylcarbonyl group.

The acyloxy groups include, for example an alkylcarbonyloxy group.

The oxycarbonyl groups include, for example, an alkoxycarbonyl group, and an aryloxycarbonyl group.

The halogenated alkoxy groups include, preferably, a 1-halogenated alkoxy group.

The halogenated aryloxy groups include, preferably, a tetrafluoroaryloxy group and a pentafluoroaryloxy group.

The halogenated alkyl groups include, for example, a trifluoromethyl group, a heptafluoroisopropyl group, and a nonylfluoro(t)butyl group.

The halogenated aryl groups include, for example, a tetrafluorophenyl group, and a pentafluorophenyl group.

Besides the above-mentioned groups, an alkylsulfonylmethyl group, or an arylsulfonylmethyl group may, for example, also preferably be used.

The above-mentioned groups may have a further substituent, including ballast groups such as a long-chained hydrocarbon group, and a polymer residual group; and substituents such as an electron withdrawing group.

When R₁ or R₂ is a other than an electron withdrawing group, R₁ or R₂ may be a hydrogen atom or any other substituent including, typically, alkyl, aryl, anilino, acylamino, sulfonamido. alkylthio, arylthio, alkenyl, cycloalkyl, cycloalkenyl, alkynyl, heterocyclic ring, alkoxy, aryloxy, heterocyclicoxy, siloxy, carbamoyloxy, amino, alkylamino, imido, ureido, sulfamoylamino, carbonylamino and heterocyclicthio groups; a spiro compound residue; and a cross-linked hydrocarbon compound residue.

The alkyl groups represented by R₁ preferably have 1 to 32 carbon atoms, and may be either straight chained or branched.

The aryl groups represented by R₁ include, preferably, a phenyl group.

The acylamino groups represented by R₁ include, for example, an alkylcarbonylamino group, and an arylcarbonylamino group.

The sulfonamido groups represented by R₁ include, for example, an alkylsulfonylamino group and an arylsulfonylamino group.

In the alkylthio groups and the arylthio groups represented by R₁, the alkyl component and aryl component include, for example, the alkyl groups and aryl groups represented by R₁, respectively.

The alkenyl groups represented by R₁ preferably have 2 to 32 carbon atoms, and the cycloalkyl groups preferably have 3 to 12 carbon atoms more preferably 5 to 7 carbon atoms. The alkenyl groups may be either straight chained or branched.

The cycloalkenyl groups represented by R₁ preferably have 3 to 12 carbon atoms, more preferably 5 to 7 carbon atoms.

The carbamoyloxy groups include, for example, an alkylcarbamoyloxy group, and an arylcarbamoyloxy group.

The ureido groups include, for example, an alkylureido group and an arylureido group.

The sulfamoylamino groups include, for example, an alkylsulfamoylamino group and an arylsulfamoylamino group.

The heterocyclic groups are preferably 5- to 7-membered and include a 2-furyl group, a 2-thienyl group, a 2-pyrimidinyl group and a 2-benzothiazolyl group.

The heterocyclicoxy groups preferably have a 5- to 7-membered heterocyclic ring and include, for example, a 3,4,5,6-tetrahydropyranyl-2-oxy group and a 1-phenyltetrazole-5-oxy group.

The heterocyclicthio groups preferably have a 5- to 7-membered heterocyclicthio group and include, for example, a 2-pyridylthio group, a 2-benzothiazolylthio group and a 2,4-diphenoxy-1,3,5-triazole-6-thio group.

The siloxy groups include, for example, a trimethylsiloxy group, a triethylsiloxy group and a dimethylbu- tylsiloxy group.

The imido groups include, for example, a succinimido group, a 3-heptadecylsuccinimido group, a phthalimido group and a glutarimido group.

The spiro compound residue includes, for example, a spiro [3,3] heptane-1-yl residue.

The cross-linked hydrocarbon compound residue includes, for example, a bicyclo [2.2.1] heptane-1-yl, a tricyclo [3.3.1.1³⁷] decane-1-yl and a 7,7-dimethyl-bicyclo [2.2.1] heptane-1-yl residue.

The carbonylamino groups include, for example, an alkoxycarbonylamino group and an aryloxycarbonylamino group.

The above mentioned groups can have a ballast group such as a long-chained hydrocarbon group or a polymer residue as a substituent.

The substituents represented byX include, forexample, a halogen atom such as a chlorine atom, a bromine atom and a fluorine atom, and the groups alkoxy, aryloxy, heterocyclicoxy, acyloxy, sulfonyloxy. alkoxycarbo- nyloxy, aryloxycarbonyl, alkyloxalyloxy, alkoxyoxalyloxy, alkylthio, arylthio, heterocyclic-thio, alkyloxythiocar- bonylthio, acylamino, sulfonamido, nitrogen-containing heterocyclic ring bonded to an N atom, alkyloxycarbonylamino, aryloxycarbonylamino, carboxyl, and 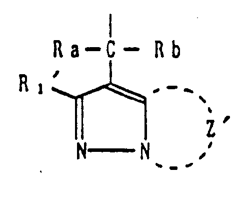
wherein R₁' and Z' are as defined for R₁ and Z, respectively; and Ra and Rb each indepenently represents a hydrogen atom, an aryl group, an alkcyl group or a heterocyclic group. Among these a halogen atom,and particularly a chlorine atom, are preferred.

The nitrogen-containing heterocyclic rings completed by either Z orZ' include, for example, a pyrazole ring. an imidazole ring and a triazole ring.

According to another preferred embodiment of the present invention. the cyan dye-forming coupler is of Formula [1]-2: 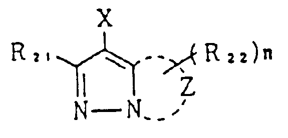
wherein Z is a non-metallic group which completes, together with the atoms to which is attached a nitrogen-containing heterocyclic; X is a hydrogen atom or a substituent capable of splitting off upon reaction with an oxidized product of a color developing agent; R₂₁ is an electron withdrawing group; R₂₂ is a hydrogen atom or a substituent; and n is 1 or 2.

The couplers of Formula [1]-2 have an electron withdrawing group in the pyrazolo portion of the pyrazoloazole nucleus of the coupler.

The electron withdrawing groups, R₂₁ preferably have a 8p value of +0.20 in the quantitative structural active relation defined in the Hansch method. They include, typically, a halogen atom and nitro, cyano, acyloxy, halogenated alkyl, halogenated alkoxy, sulfonyl, carboxyl, sulfonyloxy, sulfinyl, sulfamoyl, phosphonyl, pyrrole, tetrazolyl, acyl, carbamoyl and oxycarbonyl groups.

The halogen atoms represented by R₂₁ include, a fluorine atom, a chlorine atom and a bromine atom.

The acyloxy groups represented by R₂₁ include acetyloxy, 2-chloroacetyloxy and benzoyloxy groups.

The halogenated alkyl groups represented by R₂₁ include, trifluoromethyl and 2-chloroethyl groups.

The sulfonyl groups represented by R₂₁ include methyl sulfonyl, trifluoromethyl sulfonyl, benzene sulfonyl and p-toluene sulfonyl groups.

The sulfonyloxy groups include methyl sulfonyloxy, trifluoromethyl sulfonyloxy and benzene sulfonyloxy groups.

The sulfinyl groups include methyl sulfinyl, octyl sulfinyl, 3-phenoxybutyl sulfinyl and m-pentadecylphenyl sulfinyl groups.

The sulfamoyl groups include N,N-dipropyl sulfamoyl, N-phenyl-N-methyl sulfamoyl, N,N-diethyl sulfamoyl and N-ethyl-N-dodecyl sulfamoyl groups.

The phoshonyl groups include ethoxy phosphonyl, butoxy phosphonyl, phenoxy phosphonyl groups.

The tetrazolyl groups include 1-tetrazolyl and 5-chloro-1-tetrazolyl groups.

The halogenated alkoxy groups include trifluoromethoxy groups.

The acyl groups include acetyl, dodecanoyl, benzoyl and p-chlorobenzoyl groups.

The carbamoyl groups include N,N-dibutyl carbamoyl and N-ethyl-N-dodecyl carbamoyl groups.

The oxycarbonyl groups include alkoxycarbonyl groups such as ethoxycarbonyl and aryloxycarbonyl groups such as phenoxycarbonyl.

Besides the above-mentioned electron withdrawing groups, fluoroalkylamido, trifluoropropynyl, carbox- yethenyl, dicyanoethenyl, trifluoromethane sulfenyl, thiocyanate and isothiocyanate may also be mentioned.

The above-mentioned groups may have a substituent such as a ballast group or an electron withdrawing group.

The groups represented by R₂₂ include, typically, alkyl, aryl, anilino, acylamino, sulfonamido, alkylthio, arylthio, alkenyl, cycloalkyl, cycloalkenyl, alkynyl, heterocyclic, alkoxy, aryloxy, heterocyclic-oxy, siloxy, carbamoyloxy, amino, alkylamino, imido, ureido, sulfamoylamino, alkoxycarbonylamino, aryloxycarbonylamino, alkoxycarbonyl, aryloxycarbonyl and heterocyclicthio groups, and spiro-compound residues and cross-linked hydrocarbon compound residues.

The alkyl groups represented by R₂₂ preferably have 1 to 32 carbon atoms. They may be straight-chained or branched.

The aryl groups represented by R₂₂ include a phenyl group.

The acylamino groups represented by R₁ include alkylcarbonylamino and arylcarbonylamino groups.

The sulfonamido groups represented by R₂₂ include alkylsulfonylamino and arylsulfonylamino groups.

The alkyl components and aryl components of the alkylthio groups and arylthio groups represented by R₂₂ include the alkyl groups and aryl groups represented by the R₂₁,respectively.

The alkenyl groups represented by R₂₂ preferably, have 2 to 32 carbon atoms. The cycloalkyl groups represented thereby include preferably have 3 to 12 carbon atoms, and, more preferably 5 to 7 carbon atoms. The alkenyl groups may be straight-chained or branched.

The cycloalkenyl groups represented by R₂₂ preferably, have 3 to 12 carbon atoms, more preferably 5 to 7 carbon atoms.

The carbamoyloxy groups include, for example, an alkylcarbamoyloxy group and an arylcarbamoyloxy group.

The ureido groups include, for example, an alkylureido group and an arylureido group.

The sulfamoylamino groups include, for example, an alkylsulfamoylamino group and an arylsul-famoyla- mino group.

The heterocyclic groups are preferably 5- to 7-membered and include a 2-furyl group, a 2-thienyl group, a 2-pyrimidinyl group and a 2-benzothiazolyl group.

The heterocyclicoxy groups preferably have a 5- to 7-membered heterocyclic ring and include, for example, a 3,4,5,6-tetrahydropyranyl-2-oxy group and a 1-phenyltetrazole-5-oxy group.

The heterocyclicthio groups preferably have a 5- to 7-membered heterocyclicthio group and include, for example, a 2-pyridylthio group, a 2-benzothiazolylthio group and a 2,4-diphenoxy-1,3,5-triazole-6-thio group.

The siloxy groups include, for example, a trimethylsiloxy group, a triethylsiloxy group and a dimethylbu- tylsiloxy group.

As regards X, and Z the same definitions as in formula (I]-1 respectively apply.

According to a further embodiment of the invention, the cyan dye-forming coupler has the following Formula [1]-3: 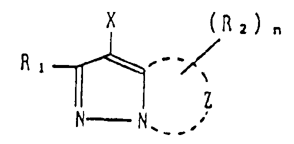
wherein R₁ is a sulfonyl, sulfinyl, sulfonyloxy, sulfonylmethyl. sulfamoyl. phosphoryl, tetrazolyl, pyrrolyl. halogenated alkoxy, halogenated aryloxy, acyl, halogen, nitro or carboxy group; X is a hydrogen atorn or a substituent capable of splitting off upon reaction with oxidized product of a color developing agent; Z represents a non-metallic group which completes, together with the atoms to which it is attached, a nitrogen containing heterocyclic ring; R₂ is a hydrogen atom or a substituent and is bonded to a carbon atom of the above-mentioned nitrogen-containing heterocyclic ring; and n is 1 or 2, provided that each R₂ may be the same or different if n is 2.

In Formula [I]-3, at least R₁ is a substituent selected from sulfonyl, sulfinyl, sulfonyloxy, sulfonylmethyl, sulfamoyl, phosphoryl, halogenated alkoxy, halogenated aryloxy, acyl, halogen, nitro, tetrazolyl, pyrrolyl and carboxyl groups.

The sulfonyl groups include alkylsulfonyl, arylsulfonyl, halogenated alkylsulfonyl and halogenated arylsulfonyl groups.

The sulfinyl groups include alkylsulfinyl, arylsulfinyl, halogenated alkylsufinyl and halogenated arylsulfinyl groups.

The sulfonyloxy groups include alkylsulfonyloxy and arylsulfonyloxy groups.

The sulfamoyl groups may be substituted with, for example, an alkyl or aryl group.

The phosphoryl groups include alkoxyphosphoryl, alkylphosphoryl and arylphosphoryl groups.

The halogenated alkoxy groups preferably include a 1-halogenated alkoxy group such as a trifluoromethoxy group.

The halogenated aryloxy groups preferably include a tetrafluoroaryloxy group and a pentafluoroaryloxy group.

The halogen atoms include a bromine atom and a chlorine atom.

The tetrazolyl groups include a 1-tetrazolyl group.

The pyrrolyl groups include a 1-pyrrolyl group.

The acyl groups include an alkylcarbonyl and arylcarbonyl group.

The sulfonylmethyl groups include an alkylsulfonylmethyl and arylsulfonylmethyl group.

The particularly preferred above-mentioned groups include sulfonyl, sulfinyl, sulfonyloxy, sulfamoyl, phosphoryl, tetrazolyl, pyrrolyl, halogenated alkoxy, halogenated aryloxy and acyl groups. In Formula [I]-3, at least R₁ provides the particularly desirable spectral absorption characteristics.

Among the above-mentioned groups, the more preferred ones include sulfonyl, sulfinyl, sulfonyloxy, sulfamoyl and acyl groups. These provide more desirable spectral absorption characteristics.

The above-mentioned groups may have a substituent, including a ballast group such as a long-chained hydrocarbon group and a polymer residue and an elecron withdrawing group.

In Formula [I]-3, R₂ for example, any of the above mentioned may be groups or a hydrogen atom.

The groups represented by R₂ include, typically, alkyl, aryl, anilino, acylamino, sulfonamido, alkylthio, arylthio, alkenyl, cycloalkyl, cycloalkenyl, alkynyl, heterocyclic, alkoxy, aryloxy, heterocyclicoxy, siloxy, carbamoyloxy, amino, alkylamino, imido, ureido, sulfamoylamino, carbonylamino and heterocyclicthio groups, a spiro-compound residue and a cross-linked hydrocarbon compound residue.

The alkyl groups represented by R₂ preferably, have 1 to 32 carbon atoms and may be straight-chained or branched.

The aryl groups represented by R₂ include, preferably, a phenyl group.

The acylamino groups represented by R₂ include an alkylcarbonylamino and arylcarbonylamino group.

The sulfonamido groups represented by R₂ include an alkylsulfonylamino and arylsulfonylamino group.

The alkyl components and aryl components of the alkylthio groups and arylthio groups represented by R₂ include the alkyl groups and aryl groups represented by R₂.

The alkenyl groups represented by R₂ preferably have 2 to 32 carbon atoms. The cycloalkyl groups represented thereby preferably have 3 to 12 carbon atoms, more preferably 5 to 7 carbon atoms. The above-mentioned alkenyl groups may be straight-chained or branched.

The cycloalkenyl groups represented by R₂ preferably have 3 to 12 carbon atoms, more preferably 5 to 7 carbon atoms.

The carbamoyloxy groups include, for example, an alkylcarbamoyloxy group and an arylcarbamoyloxy group.

The ureido groups include, for example, an alkylureido group and an arylureido group.

The sulfamoylamino groups include, for example, an alkylsulfamoylamino group and an arylsulfamoylamino group.

The heterocyclic groups are preferably 5- to 7-membered and include typically a 2-furyl group, a 2-thienyl group, a 2-pyrimidinyl group and a 2-benzothiazolyl group.

The heterocyclicoxy groups preferably have a 5- to 7-membered heterocyclic ring and include, for example, a 3,4,5,6-tetrahydropyranyl-2-oxy group and a 1-phenyltetrazole-5-oxy group.

The heterocyclicthio groups are preferably a 5- to 7-membered heterocyclicthio group and include, for example, a 2-pyridylthio group, a 2-benzothiazolylthio group, and a 2,4-diphenoxy-1,3,5-triazole-6-thio group.

The siloxy groups include, for example, a trimethylsiloxy group, a triethylsiloxy group and a dimethylbu- tylsiloxy group.

The imido groups include succinimido, 3-heptadecyl succinimido, phthalimido and glutarimido groups.

The spiro-compound residue includes a spiro [3.3] heptane-1-yl residue.

The cross-linked hydrocarbon compound residue includes a bicyclo [2.2.1] heptane-1-yl, a tricyclo [3.3.1.1³⁷] decane-1-yl and a 7,7-dimethyl-bicyclo [2.2.1] heptane-1-yl residue.

The carbonylamino groups include alkoxycarbonylamino and aryloxycarbonylamino groups.

The above-mentioned groups may have a substituent including a ballast group such as a long-chained hydrocarbon group or polymer residue, or an electron withdrawing group.

As regards X and Z, the same definitions as in formula [I]-1 respectively apply.

According to a still further embodiment of the invention, the cyan dye-forming coupler is of Formula [1]-4: 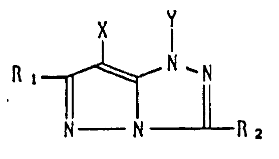
wherein R₁ is a cyano group, an acyloxy group. an oxycarbonyl group or a carbamoyl group; R₂ and y each is a hydrogen atom or a substituent; and X is a hydrogen atom or a group capable of splitting off upon reaction with an oxidized product of a color developing agent.

The cyan dye-forming couplers of Formula (1]-4 are pyrazoloazole type compounds having an electron withdrawing group.

In Formula [I]-4, the electron withdrawing groups Preferably have a 8p value (defined by Hammett) of not less than + 0.20.

The acyloxy groups include an alkylcarbonyloxy group.

The oxycarbonyl groups include, for example. an alkoxycarbonyl group and an aryloxycarbonyl group.

The carbamoyl groups may be substituted with for example, alkyl group or an aryl group preferably a phenyl group.

There is be no special limitation regarding the groups. represented by R₂ in Formula [1]-4. They they include a halogen atom and alkyl, aryl, anilino, acylamino, sulfonamido, alkylthio, arylthio, alkenyl, cycloalkyl, cycloalkenyl, alkynyl, heterocyclic, sulfonyl, sulfinyl, phosphonyl, acyl, sulfamoyl, sulfonyloxy, alkoxy, aryloxy, heterocyclicoxy, siloxy, carbamoyloxy, amino, alkylamino, imido, ureido, sulfamoylamino, alkoxycarbonylamino, aryloxycarbonylamino, heterocyclicthio, thioureido, carboxy, hydroxy, mercapto, nitro and sulfonic acid groups, a spiro-compound residue and a cross-linked hydrocarbon compound residue.

The alkyl groups represented by R₂ preferably have 1 to 32 carbon atoms, and may be either straight chained or branched.

The aryl groups include, preferably, a phenyl group.

The acylamino groups include, for example, an alkylcarbonylamino group and an arylcarbonylamino group.

The sulfonamido groups include, for example, an alkylsulfonylamino group and an arylsulfonylamino group.

In the alkylthio groups and the arylthio groups, the alkyl component and aryl component include the alkyl groups and aryl groups represented by R₂, respectively.

The alkenyl groups preferably have 2 to 32 carbon atoms, and the cycloalkyl groups preferably have 3 to 12 carbon atoms, more preferably 5 to 7 carbon atoms. The alkenyl groups may be either straight chained or branched;

The cycloalkenyl groups preferably have 3 to 12 carbon atoms, more preferably 5 to 7 carbon atoms.

The sulfonyl groups include alkylsulfonyl and arylsulfonyl groups.

The sulfinyl groups include alkylsulfinyl and arylsulfinyl groups.

The phosphonyl groups include alkylphosphonyl, alkoxyphosphonyl, aryloxyphosphonyl and arylphosphonyl groups.

The acyl groups include alkylcarbonyl and arylcarbonyl groups.

The sulfamoyl groups include alkylsulfamoyl and arylsulfamoyl groups.

The carbamoyloxy groups include alkylcarbamoyloxy and arylcarbamoyloxy group.

The ureido groups include alkylureido, and arylureido groups.

The sulfamoylamino groups include alkylsulfamoylamino and arylsulfamoylamino groups.

The heterocyclic groups preferably are 5- to 7-membered and include typically, a 2-furyl group, a 2-thienyl group, a 2-pyrimidinyl group, a 2-benzothiazolyl group, a 1-pyrrolyl group and a 1-tetrazolyl group.

The heterocyclicoxy groups preferably have a 5- to 7-membered heterocyclic ring and include, for example, a 3,4,5,6-tetrahydropyranyl-2-oxy group and a 1-phenyltetrazole-5-oxy group and so forth;

The heterocyclicthio groups preferably are a 5- to 7-membered heterocyclicthio group and include, for example, a 2-pyridylthio group, a 2-benzothiazolylthio group and a 2,4-diphenoxy-1,3,5-triazole-6-thio group.

The siloxy groups include, for example, a trimethylsiloxy group, a triethylsiloxy group and a dimethylbu- tylsiloxy group.

The imido groups include, for example, a succinimido group, a 3-heptadecylsuccinimido group, a phthalimido group and a glutarimido group.

The spiro compound residues include, for example, a spiro [3,3] heptane-1-yl residue.

The cross-linked hydrocarbon compound residues include, for example, a bicyclo [2.2.1] heptane-1-yl, a tricyclo [3.3.1.1³⁷] decane-1-yl and a 7,7-dimethyl-bicyclo [2.2.1] heptane-1-yl.

The aforementioned groups represented by R₁ and R₂ may have a substituent, including a ballast group such as a long-chained hydrocarbon group or a polymer residue.

As regards X, the same definition given in formula [1]-1 applies.

Advantageous Y groups include those which are split off from the resulting compound after the reaction of the cyan dye-forming couplerwith an oxidation product of a color developing agent. For example, those which can be split off under alkaline condition are disclosed in Japanese Patent O.P.I. publication No. 228444/1986 and those which couple-off upon reaction with the oxidation product of a color developing agent are disclosed in Japanese Patent O.P.I. Publication No. 133734/1981. A hydrogen atom is most preferred.

According to yet still another embodiment of the invention, the cyan dye-forming coupler is of Formula [I]-5: 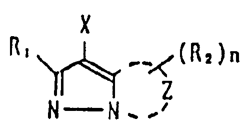
wherein Z is a non-metallic group which completes, together with the atoms to which it is attached, a nitrogen-containing heterocyclic ring; X is a hydrogen atom or a substituent capable of splitting off upon reaction with an oxidized product of a color developing agent; R₁ is a hydrogen atom or a substituent; R₂ is an electron withdrawing group; and n is 1 or 2.

The cyan dye-forming couplers of Formula (1]-5 have at least one electron withdrawing group R₂ in the azole portion of the pyrazoloazole nucleus of the coupler.

The substituents represented by R₁ include typically, a haloge atom and alkyl, aryl, anilino, acylamino, sulfonamido, alkylthio, arylthio, alkenyl, cycloalkyl, cycloalkenyl, alkynyl, heterocyclic, alkoxy, aryloxy, heterocyclicoxy, siloxy, carbamoyloxy, amino, alkylamino, imido, ureido, sulfamoylamino, alkoxycarbonylamino, aryloxycarbonylamino, alkoxycarbonyl, aryloxycarbonyl and heterocyclicthio groups, a spiro-compound residue and a cross-linked hydrocarbon compound residue.

The alkyl groups represented by R₁ preferably have 1 to 32 carbon atoms They may be straight-chained or branched.

The aryl groups represented by R₁ include a phenyl group.

The acylamino groups represented by R₁ include alkylcarbonylamino and arylcarbonylamino groups.

The sulfonamido groups represented by R₁ include alkylsulfonylamino and arylsulfonylamino groups.

The alkyl components and aryl components of the alkylthio groups and arylthio groups represented by R₁ include the alkyl groups and aryl groups represented by R₁, respectively.

The alkenyl groups represented by R₁ preferably have those having 2 to 32 carbon atoms. The cycloalkyl groups represented thereby preferably have 3 to 12 carbon atoms, more preferably 5 to 7 carbon atoms. The alkenyl groups may be straight-chained or branched.

The cycloalkenyl groups represented by R₁, preferably have 3 to 12 carbon atoms, more preferably have 5 to 7 carbon atoms.

The sulfonyl groups represented by R₁ include alkylsulfonyl and arylsulfonyl groups.

The sulfinyl groups include alkylsulfinyl and arylsulfinyl groups.

The phosphonyl groups include alkylphosphonyl, alkoxyphosphonyl, aryloxyphosphonyl and arylphosphonyl groups.

The acyl groups include alkylcarbonyl and arylcarbonyl groups.

The carbamoyl groups include alkylcarbamoyl and arylcarbamoyl groups.

The sulfamoyl groups include alkylsulfamoyl and arylsulfamoyl groups.

The acyloxy groups include alkylcarbonyloxy and arylcarbonyloxy groups.

The carbamoyloxy groups include alkylcarbamoyloxy and arylcarbamoyloxy groups.

The ureido groups include alkylureido and arylureido groups.

The sulfamoylamino groups include alkylsulfamoylamino and arylsulfamoylamino groups.

The heterocyclic groups are preferably 5- to 7-membered and include typically a 2-furyl group, a 2-thienyl group, a 2-pyrimidinyl group and a 2-benzothiazolyl group.

The heterocyclicoxy groups preferably have a 5- to 7-membered heterocyclic ring and include, for example, a 3,4,5,6-tetrahydropyranyl-2-oxy group and a 1-phenyltetrazole-5-oxy group.

The heterocyclicthio groups are preferably a 5- to 7-membered heterocyclicthio group and include, for example, a 2-pyridylthio group, a 2-benzothiazolylthio group and a 2,4-diphenoxy-1,3,5-triazole-6-thio group.

The siloxy groups include, for example, a trimethylsiloxy group, a triethylsiloxy group and a dimethylbu- tylsiloxy group.

The imido groups include, for example, a succinimido group, a 3-heptadecylsuccinimido group, a phthalimido group and a glutarimido group.

The spiro compound residue includes, for example, a spiro [3.3] heptane-1-yl residue.

The cross-linked hydrocarbon compound residues include, for example, bicyclo [2.2.1] heptane-1-yl, tricyclo [3.3.1.1³⁷] decane-1-yl and 7,7-dimethyl-bicyclo [2.2.1] heptane-1-yl residues.

The electron withdrawing groups represented by R₂ are substituents having an F value of not less than +0.30 in the quantitative structural active relation according to the Hansch Method. They include, typically, a halogen atom and nitro, cyano, acylamino, acyloxy, halogenated alkyl, sulfonyl, carboxyl, sulfonyloxy, sulfinyl, sulfamoyl, phosphonyl, pyrrole and tetrazolyl groups.

The halogen atoms represented by R₂ include a fluorine atom, a chlorine atom and a bromine atom.

The acylamino groups represented by R₂ include acetylamino, trifluoroacetylamino and benzoylamino groups.

The acyloxy groups represented by R₂ include acetyloxy, 2-chloroacetyloxy and benzoyloxy groups.

The halogenated alkyl groups represented by R₂ include trifluoromethyl and 2-chloroethyl groups.

The sulfonyl groups represented by R₂ include methyl sulfonyl, trifluoromethyl sulfonyl, benzene sulfonyl and p-toluene sulfonyl groups.

The sulfonyloxy groups include methyl sulfonyloxy, trufluoromethyl sulfonyloxy and benzene sulfonyloxy groups.

The sulfinyl groups include methyl sulfinyl, octyl sulfinyl, 3-phenoxybutyl sulfinyl and m-pentadecylphenyl sulfinyl groups.

The sulfamoyl groups include N-propyl sulfamoyl, N-phenyl sulfamoyl, N,N-diethyl sulfamoyl and N-ethyl-N-dodecyl sulfamoyl groups.

The phosphonyl groups include ethoxy phosphonyl, butoxy phosphonyl and phenoxy phosphonyl groups.

The tetrazolyl groups include 1-tetraazolyl and 5-chloro-1-tetrazolyl groups.

Among the above-mentioned groups, the preferred groups are cyano, acyloxy, sulfonyl, sulfinyl, sulfamoyl, phosphonyl and tetrazolyl groups.

The above-mentioned groups may have a substituent such as a ballast group or an electron withdrawing group.

As for X and Z, the same definitions apply as in formula [I]-1.

Among substituents for R₁ electron withdrawing groups are preferred, more preferably those having an F-value of not less than +0.30. can be mentioned. Example of such groups are halogen atom, such as a chlorine, bromine or fluorine atom, an alkylchloride group such as a trifluoromethyl group, an acyloxy group such as an acetyloxy group or a y-chloroacetyloxy group, a sulfonyl group such as a methyl sulfonyl group, a dodecyl sulfonyl group, a benzene sulfonyl group or a p-chlorobenzene sulfonyl group, a sulfinyl group such as a methylsulfinyl group, a 3-phenoxybutyl sulfinyl group, a phenyl sulfinyl group or a m-pentadecylphenyl sulfinyl group, a sulfamoyl group such as a N-propyl sulfamoyl group, a N-phenyl sulfamoyl group, a N,N-diethyl sulfamoyl group or a N-ethyl-N-dodecylsulfamoyl group, a phosphonyl group such as an ethoxyphosphonyl group or a phenoxyphosphonyl group and a heterocyclic group such as a pyrrolyl group or a 5-chlorotetrazolyl group. These groups may be substituted.

According to an additional embodiment of the invention, the cyan dye-forming coupler coupler is of Formula [1]-6: 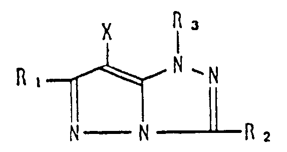
wherein R₂ is a halogen atom or a sulfonyl, sulfonyloxy, sulfinyl, sulfamoyl, phosphoryl, tetrazolyl, pyrrolyl, halogenated alkoxy, halogenated aryloxy, cyano, nitro, acyl or carbamoyl group; R₁ and R₃ are each a hydrogen atom or a substituent; and X is a hydrogen atom or a group capable of splitting off upon reaction with an oxidized product of a color developing agent.

The couplers of Formula [1]-6 have an electron withdrawing group in the pyrazoloazole nucleus.

The substituents represented by at least R₂ include sulfonyl, sulfinyl, sulfonyloxy, sulfamoyl, cyano, acyl, phosphoryl, tetrazolyl, pyrrolyl, carbamoyl, halogenated alkoxy, halogenated aryloxy and nitro groups and a halogen atom.

The sulfonyl groups include alkylsulfonyl, arylsulfonyl, halogenated alkylsulfonyl and halogenated arylsulfonyl groups.

The sulfinyl groups include alkylsulfinyl, arylsulfinyl, halogenated alkylsulfinyl and halogenated arylsulfinyl groups.

The sulfonyloxy groups include alkylsulfonyloxy and arylsulfonyloxy groups.

The sulfamoyl groups may be substituted with, for example, alkyl or aryl groups.

The acyl groups include alkylcarbonyl and arylcarbonyl groups.

The phosphoryl groups include alkoxyphosphoryl and alkylphosphoryl groups.

The tetrazolyl groups include a 1-tetrazolyl group.

The pyrrolyl groups include a 1-pyrrolyl group.

The carbamoyl groups may be substituted by, for example, an alkyl or aryl group.

The halogenated alkoxy groups include a 1- halogenated alkoxy group such as a trifluoromethoxy group.

The halogenated aryloxy groups include, preferably, a tetrafluoroaryloxy group and a pentafluoroaryloxy group.

The halogen atoms include a bromine atom and a chlorine atom.

The particularly preferred groups include sulfonyl, sulfinyl, sulfonyloxy, sulfamoyl, acyl, cyano, phosphoryl, tetrazolyl and pyrrolyl groups . In Formula [1]-6, at least R₁ being an above-mentioned group, is capable of providing particularly desirable spectral absorption characteristics.

Among the above-mentioned groups the preferred groups include sulfonyl, sulfinyl, sulfonyloxy, sulfamoyl acyl and cyano groups. These groups provide particularly desirable spectral absorption characteristics.

The above-mentioned groups may have a substituent, including a ballast group such as a long-chained hydrocarbon group and a polymer residue, or an elecron withdrawing group.

In Formula [I]-6, R₁ may be any of the above-mentioned groups, any other group or a hydrogen atom.

The groups represented by R₁ include, typically, alkyl, aryl, anilino, acylamino, sulfonamido, alkylthio, arylthio, alkenyl, cycloalkyl, cycloalkenyl, alkynyl, heterocyclic, alkoxy, aryloxy, heterocyclicoxy, siloxy, carbamoyloxy, amino, alkylamino, imido, ureido, sulfamoylamino, carbonylamino and heterocyclicthio groups, a spiro-compound residue and a cross-linked hydrocarbon compound residue.

The alkyl groups represented by R₁ preferably have 1 to 32 carbon atoms. They may be straight-chained or branched.

The aryl groups represented by R₁ include, preferably, a phenyl group.

The acylamino groups represented by R₁ include alkylcarbonylamino and arylcarbonylamino groups.

The sulfonamido groups represented by R₁ include alkylsulfonylamino and arylsulfonylamino groups.

The alkyl components and aryl components of the alkylthio groups and arylthio groups represented by R₁₁ include the alkyl groups and aryl groups represented by R_{.1} respectively.

The alkenyl groups represented by R₁ preferably have 2 to 32 carbon atoms. The cycloalkyl groups represented thereby preferably have 3 to 12 carbon atoms, more preferably 5 to 7 carbon atoms. The alkenyl groups may be straight-chained or branched.

The cycloalkenyl groups represented by R₁ preferably have 3 to 12 carbon atoms, more preferably 5 to 7 carbon atoms.

The carbamoyloxy groups include, for example, an alkylcarbamoyloxy group and an arylcarbamoyloxy group.

The ureido groups include, for example, an alkylureido group and an arylureido group.

The sulfamoylamino groups include, for example, an alkylsulfamoylamino group and an arylsulfamoylamino group.

The heterocyclic groups are preferably 5- to 7-membered. They include typically a 2-furyl group, a 2-thienyl group, a 2-pyrimidinyl group and a 2-benzothiazolyl group.

The heterocyclicoxy groups are preferably 5- to 7-membered heterocyclic rings They include, for example, a 3,4,5,6-tetrahydropyranyl-2-oxy group and a 1-phenyltetrazole-5-oxy group.

The heterocyclicthio groups are preferably a 5- to 7-membered heterocyclicthio group and they include, for example, a 2-pyridylthio group, a 2-benzothiazolylthio group and a 2,4-diphenoxy-1,3,5-triazole-6-thio group.

The siloxy groups include, for example, a trimethylsiloxy group, a triethylsiloxy group and a dimethylbu- tylsiloxy group.

The imido groups include succinimido, 3-heptadecyl succinimido, phthalimido and glutarimido groups.

The spiro-compound residues include a spiro [3.3] heptane-1-yl residue.

The cross-linked hydrocarbon compound residues include bicyclo [2.2.1] heptane-1-yl, tricyclo [3.3.1.1³⁷] decane-1-yl and 7,7-dimethyl-bicyclo [2.2.1] heptane-1-yl residues.

The carbonylamino groups include alkoxycarbonylamino and aryloxycarbonylamino groups.

The above-mentioned groups may have a substituent, including a ballast group such as a long-chained hydrocarbon group or a polymer residue or an electron withdrawing group.

As for X, the same definition applies as in formula [)]-1.

Furthermore, for R₃ those substituents as mentioned in formula [1]-4 can be mentioned. Among them a hydrogen atom is most preferred.

According to another embodiment of the Invention, the cyan dye-forming coupler is of Formula [1]-7: 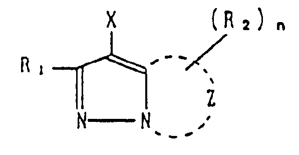
wherein at least two groups selected from R₁ and R₂, are independently electron withdrawing groups; Z is a non-metallic group which completes, together with the atoms to which it is attached, nitrogen-containing heterocyclic ring; the or each R₂ is bonded to a carbon atom of the heterocyclic ring; X is a hydrogen atom or a substituent capable of splitting off upon reaction with an oxidized product of a color developing agent; and n is 1 or 2. When n is 1, R₂ is an electron withdrawing group, and when n is 2, each or both R₂ groups are electron withdrawing groups. They may be the same or different front each other.

The couplers of Formula [1]-7 have an electron withdrawing group in the pyrazoloazole nucleus. At least two of the electron withdrawing groups selected from the R₁ and R₂ preferably have a substituent constant σρ (defined by Hammett) of not less than +0.20. They include, typically, sulfonyl, sulfonyloxy, sulfinyl, sulfamoyl, phosphoryl, halogen, halogenated alkoxy, halogenated aryloxy, nitro, pyrrolyl, tetrazolyl, cyano, carbamoyl, acyl, acyloxy, carboxyl and oxycarbonyl groups.

The sulfonyl groups include alkylsulfonyl, arylsulfonyl, halogenated alkylsulfonyl and halogenated arylsulfonyl groups.

The sulfonyloxy groups include alkylsulfonyloxy and arylsulfonyloxy groups.

The sulfinyl groups include alkylsulfinyl, arylsulfinyl, halogenated alkylsulfinyl and halogenated arylsulfinyl groups.

The sulfamoyl groups may be substituted with, for example, alkyl or aryl groups.

The phosphoryl groups include alkoxyphosphoryl, alkylphosphoryl and arylphosphoryl groups.

The halogen atoms include bromine and chlorine groups.

The halogenated alkoxy groups include, preferably, a 1-halogenated alkoxy group.

The halogenated aryloxy groups include, preferably, a tetra- or pentafluoroaryloxy group.

The pyrrolyl groups include a 1-pyrrolyl group.

The tetrazolyl groups include a 1-tetrazolyl group.

The acyl groups include alkylcarbonyl and arylcarbonyl groups.

The acyloxy groups include alkylcarbonyloxy groups.

The oxycarbonyl groups include alkoxycarbonyl and aryloxycarbonyl groups.

For halogenated alkyl groups, a 1-halogenated alkyl group such as a trifluoromethyl group is preferred.

For the halogenated aryl groups, a tetra- or penta-fluoroaryl group is preferred.

An arylsulfonylmethyl group or an alkylsulfonylmethyl group may also be used.

The above-mentioned groups may have a substituent, including a ballast group such as a long-chained hydrocarbon group and a polymer residue, or an elecron withdrawing group.

In Formula [1]-7, when n is 2 and R₁₁ or R₂ is a substituent other than an electron withdrawing group, the substituent is preferably a hydrogen atom, or it may be any other substituent.

The substituents include, typically, alkyl, aryl, anilino, acylamino, sulfonamido, alkylthio, arylthio, alkenyl, cycloalkyl, cycloalkenyl, alkynyl, heterocyclic, alkoxy, aryloxy, heterocyclicoxy, siloxy, carbamoyloxy, amino, alkylamino, imido, ureido, sulfamoylamino, alkoxycarbonylamino, alkoxycarbonyl, aryloxycarbonyl, and heterocyclicthio groups, a spiro-compound residue group and a cross-linked hydrocarbon compound residue group.

The alkyl groups represented by R₁ or R₂ preferably have 1 to 32 carbon atoms and may be straight-chained or branched.

The aryl groups include, preferably, a phenyl group.

The sulfonamido groups include alkylsulfonylamino and arylsulfonylamino groups.

The alkyl components and aryl components of the alkylthio groups and arylthio groups include the alkyl groups and aryl groups mentioned above respectively.

The alkenyl groups preferably have 2 to 32 carbon atoms. The cycloalkyl groups preferably have 3 to 12 carbon atoms, more preferably 5 to 7 carbon atoms. The above-mentioned alkenyl groups may be straight-chained or branched.

The cycloalkenyl groups preferably have 3 to 12 carbon atoms, more preferably 5 to 7 carbon atoms.

The carbamoyloxy groups include an alkyl carbamoyloxy group and an arylcarbamoyloxy group.

The ureido groups include an alkylureido group and an arylureido group.

The sulfamoylamino groups include an alkylsulfamoylamino group and an arylsulfamoylamino group.

The heterocyclic groups are preferably be the 5- to 7-membered and include typically a 2-furyl group, a 2-thienyl group, a 2-pyrimidinyl group and a 2-benzothiazolyl group.

The heterocyclicoxy groups preferably have a 5- to 7-membered heterocyclic ring and include, for example, a 3,4,5,6-tetrahydropyranyl-2-oxy group and a 1-phenyltetrazole-5-oxy group.

The heterocyclicthio groups are preferably a 5- to 7-membered heterocyclicthio group and include, for example, a 2-pyridylthio group, a 2-benzothiazolylthio group and a 2,4-diphenoxy-1,3,5-triazole-6-thio group.

The siloxy groups include, for example, a trimethylsiloxy group, a triethylsiloxy group and a dimethylbu- tylsiloxy group.

As for X and Z, those atoms and groups as in formula [I]-1 are mentioned.

The cyan dye-forming couplers which are advantageously used in the present invention are represented by the following Formulae [II] through [VI]:

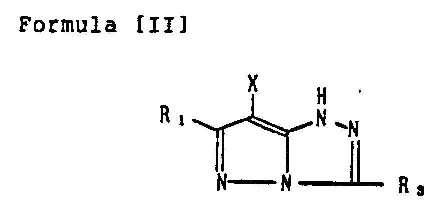

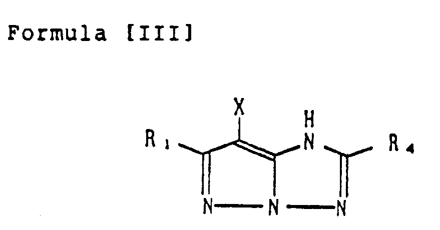

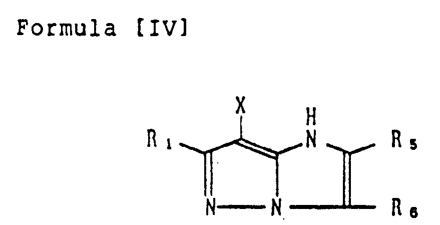

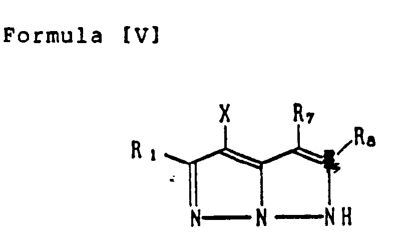

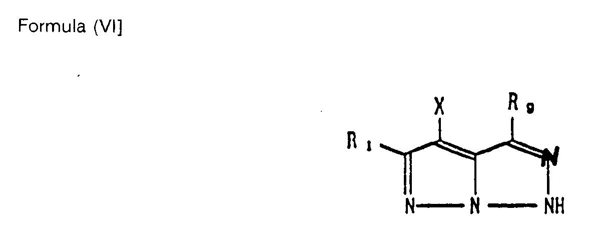

In Formulae (II] through [VI], R₁ is synonymous with R₁ in Formula [I]-I; and R₃ through R₉ and X are synonymous with R₂ and X, respectively.

Typical examples of the cyan couplers are:

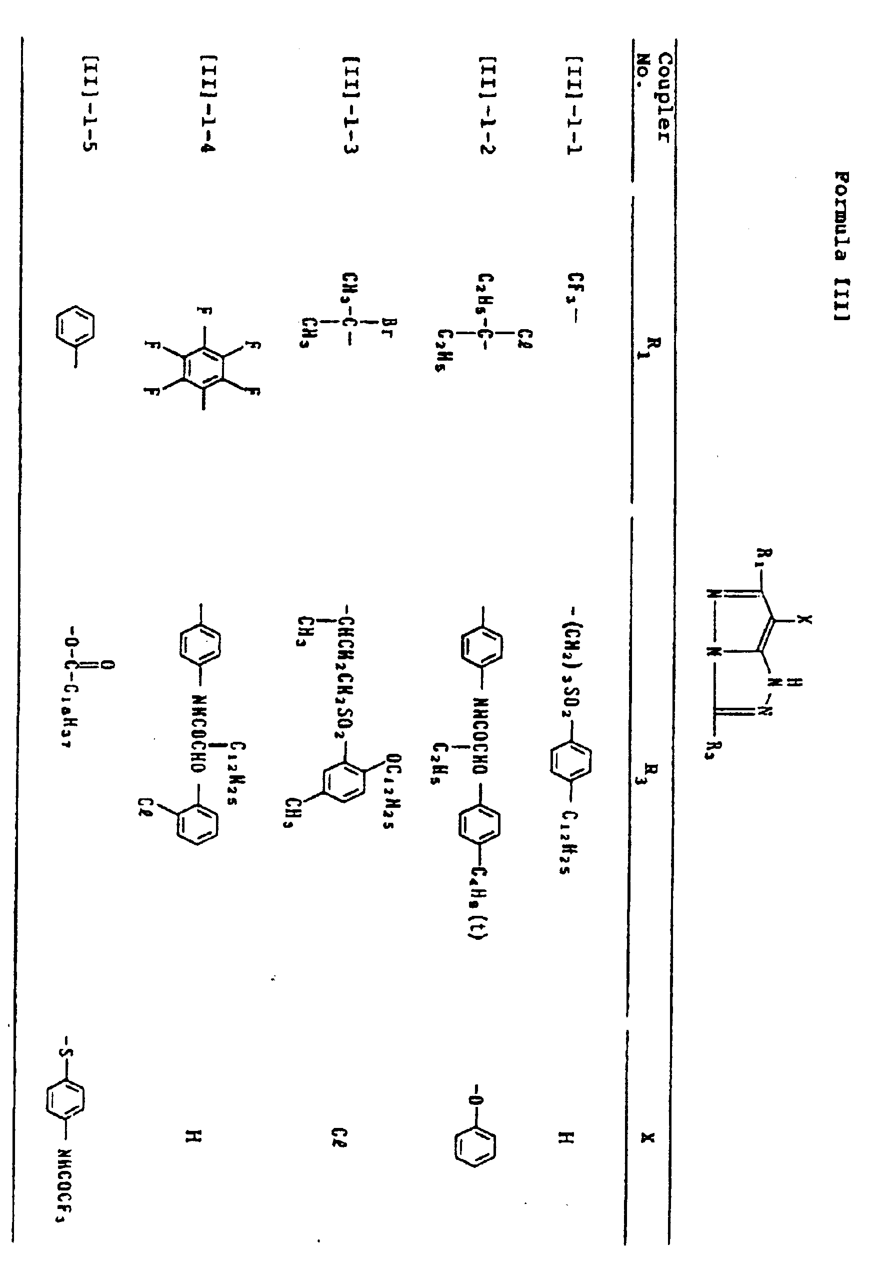

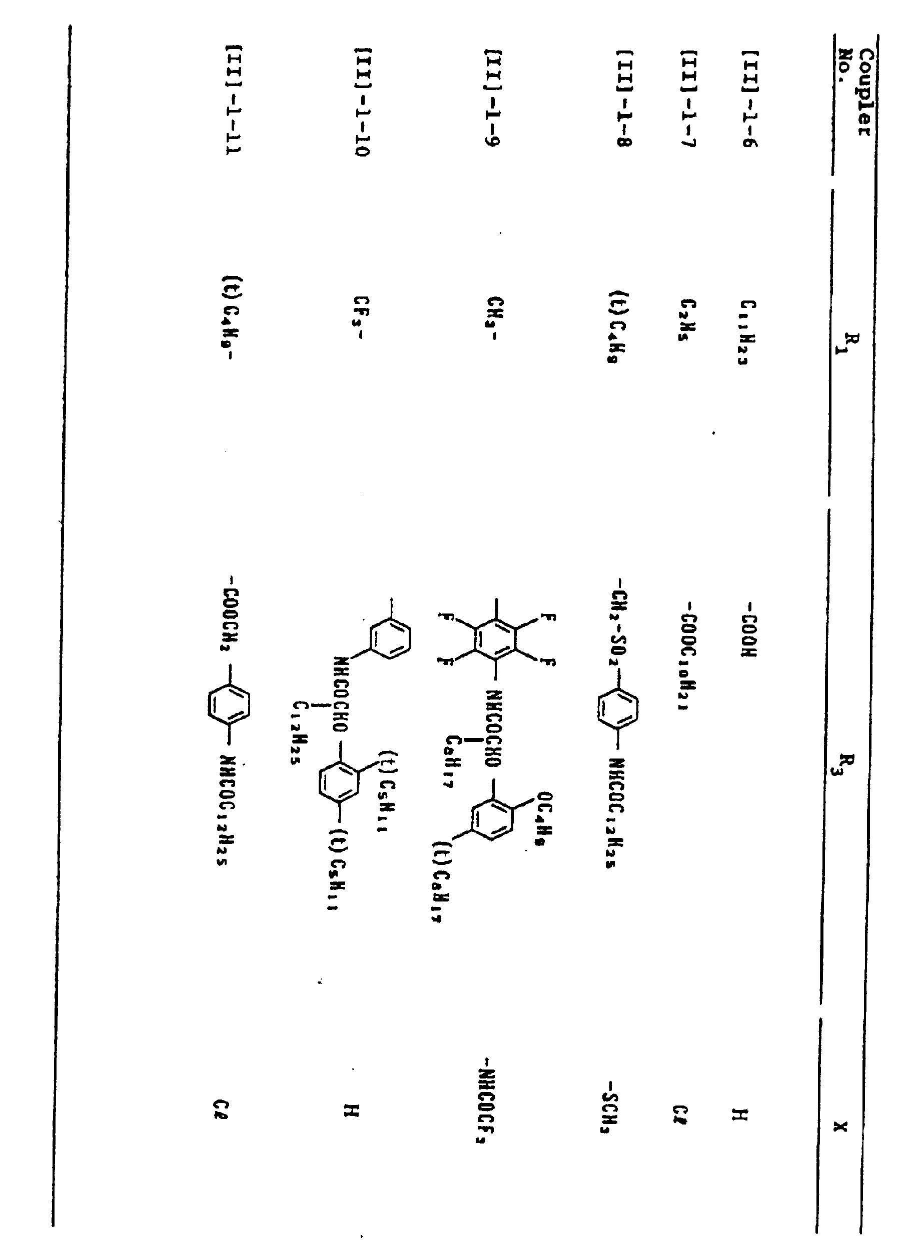

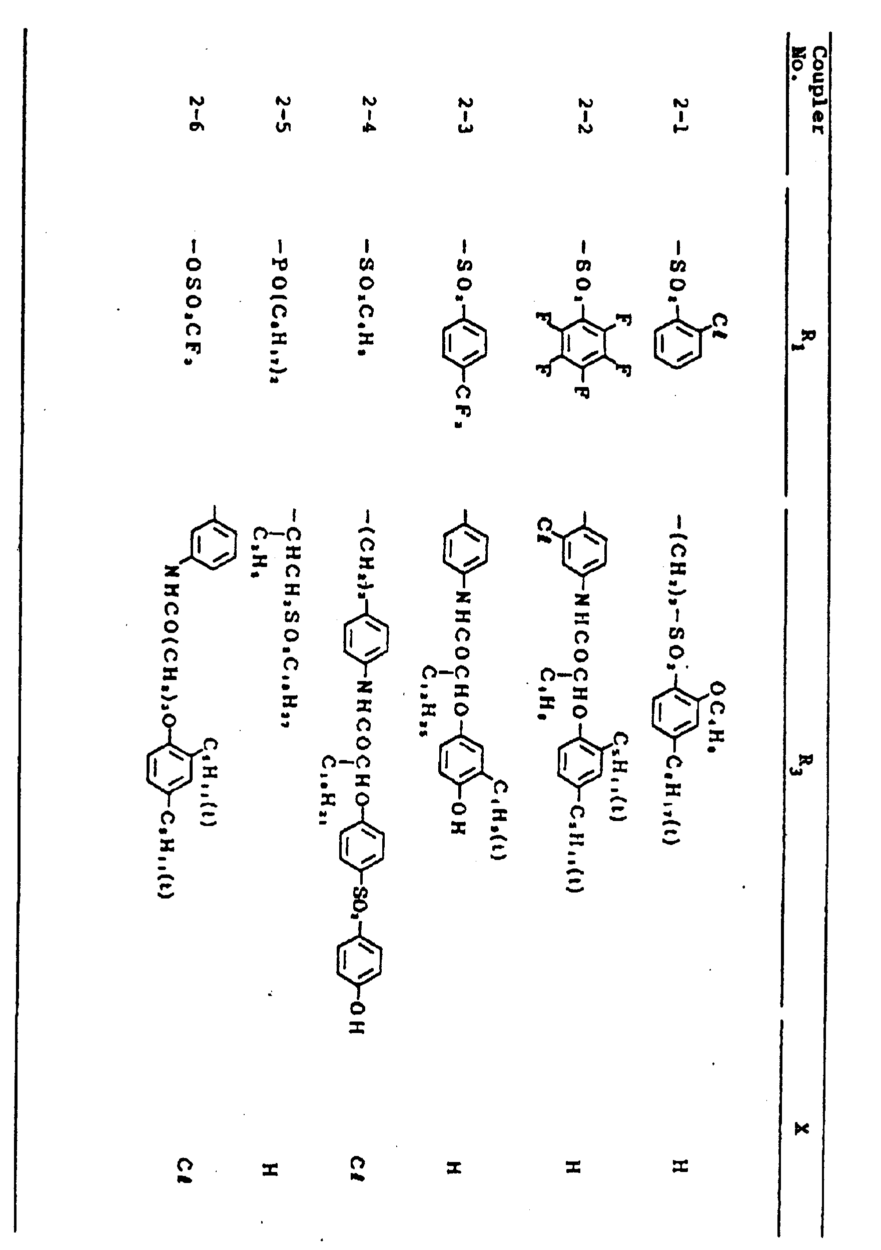

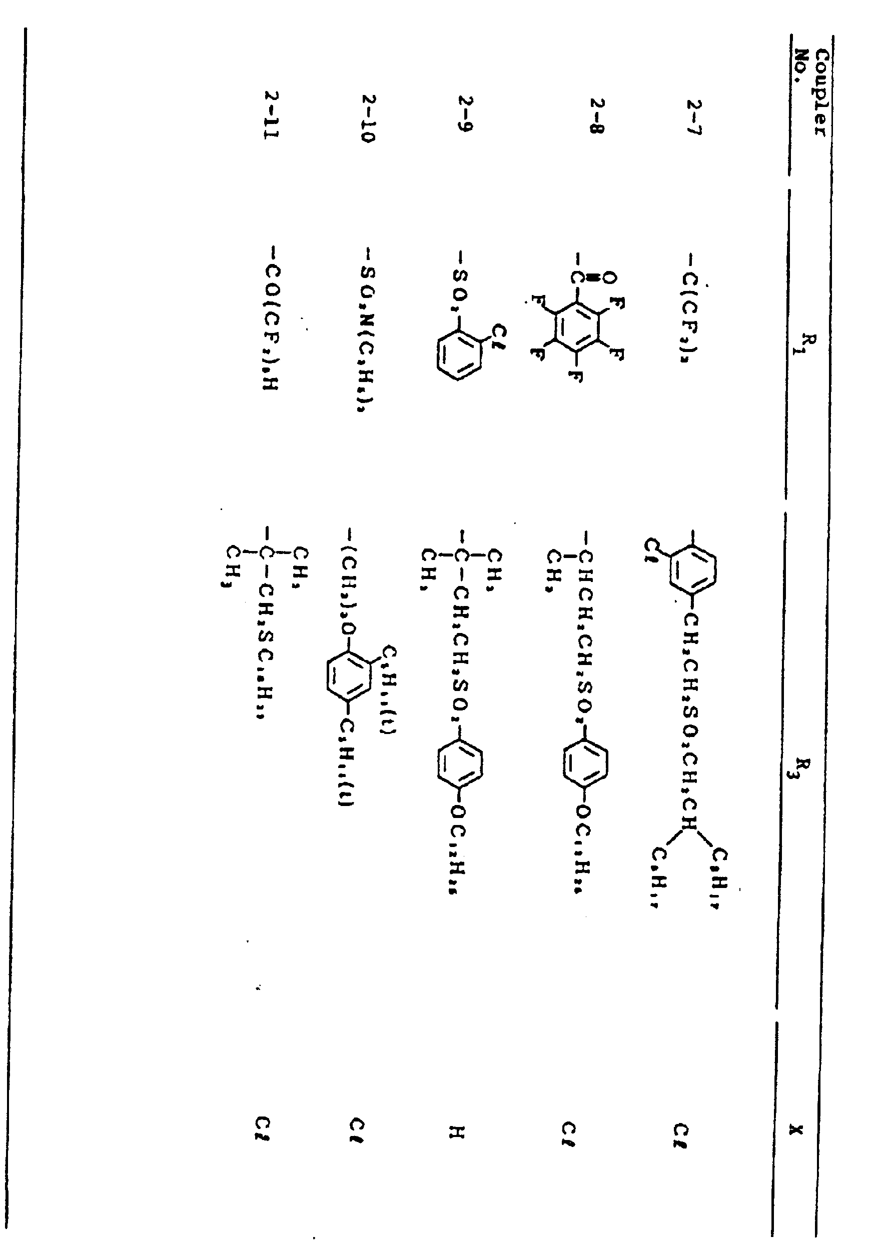

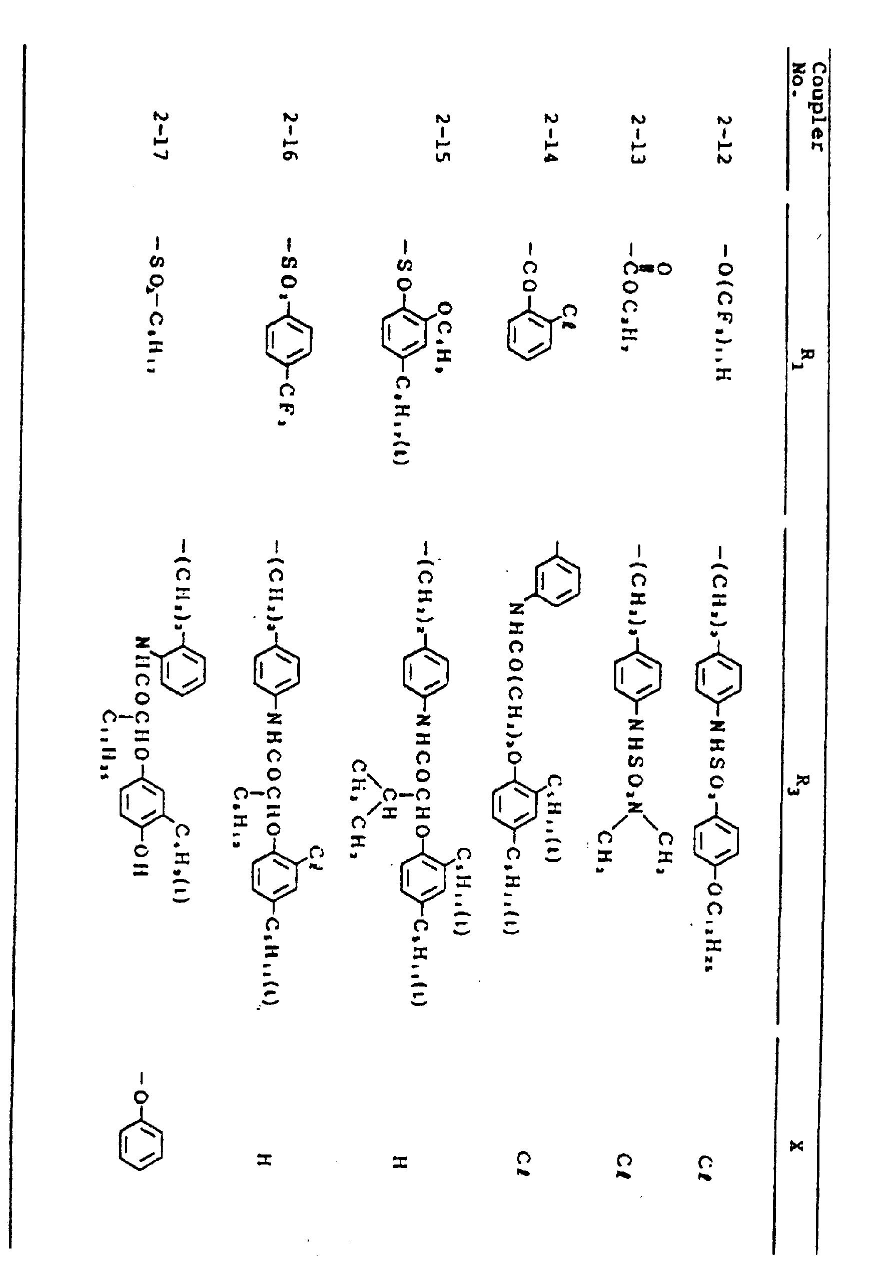

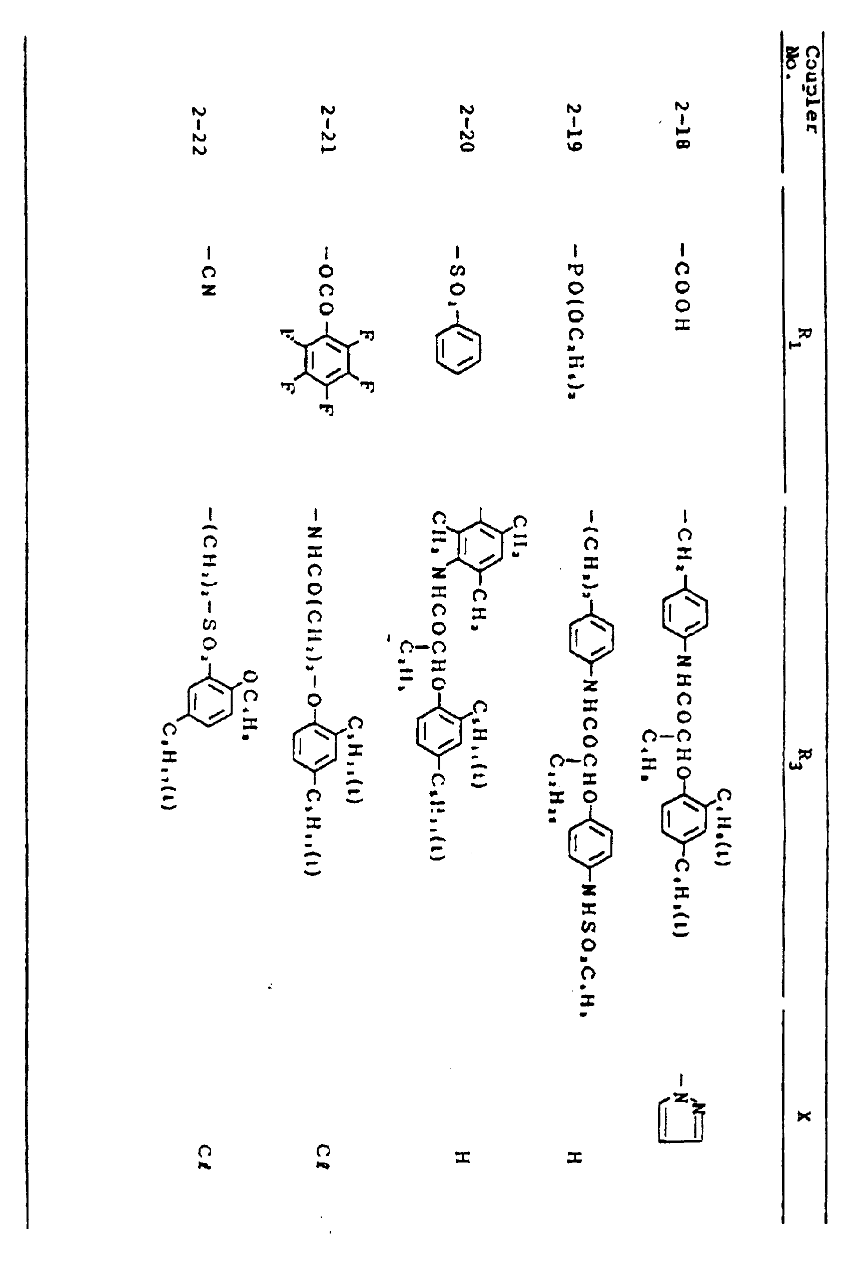

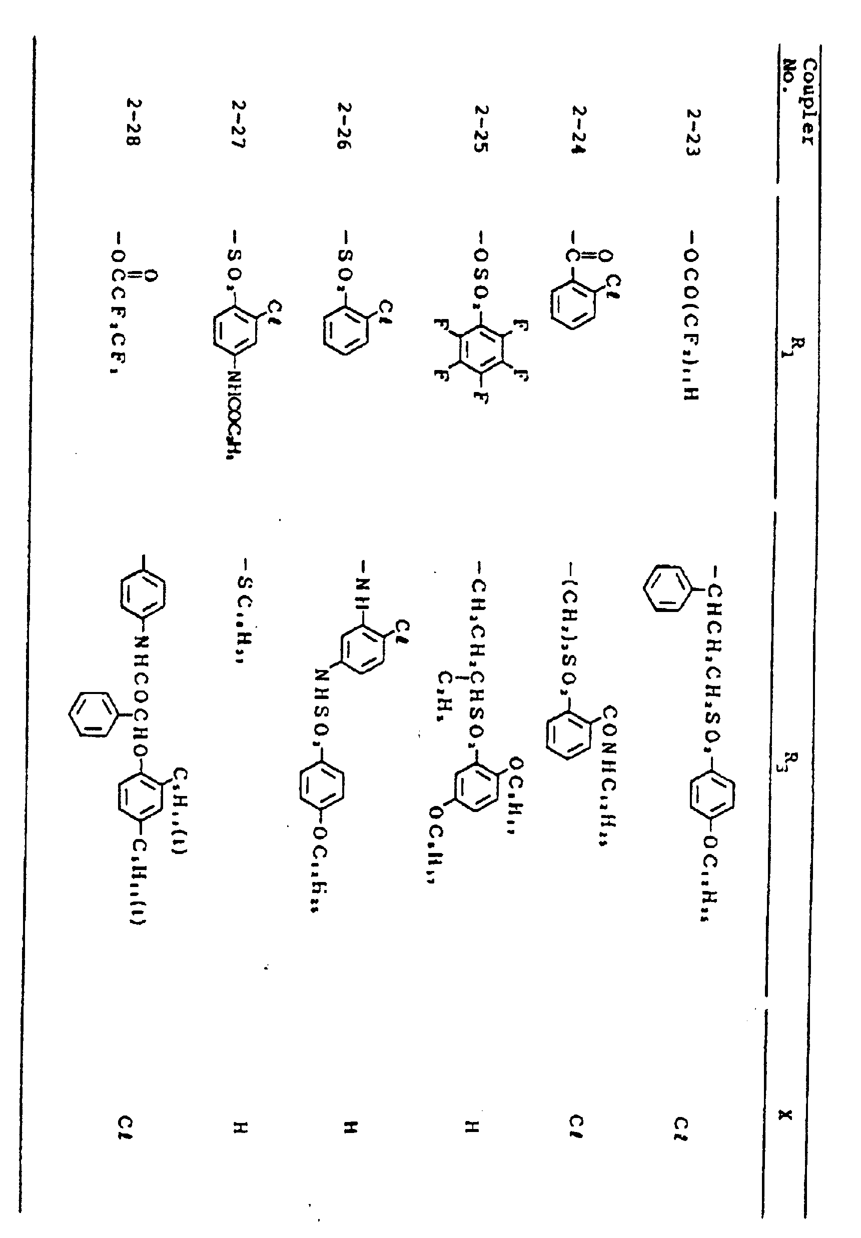

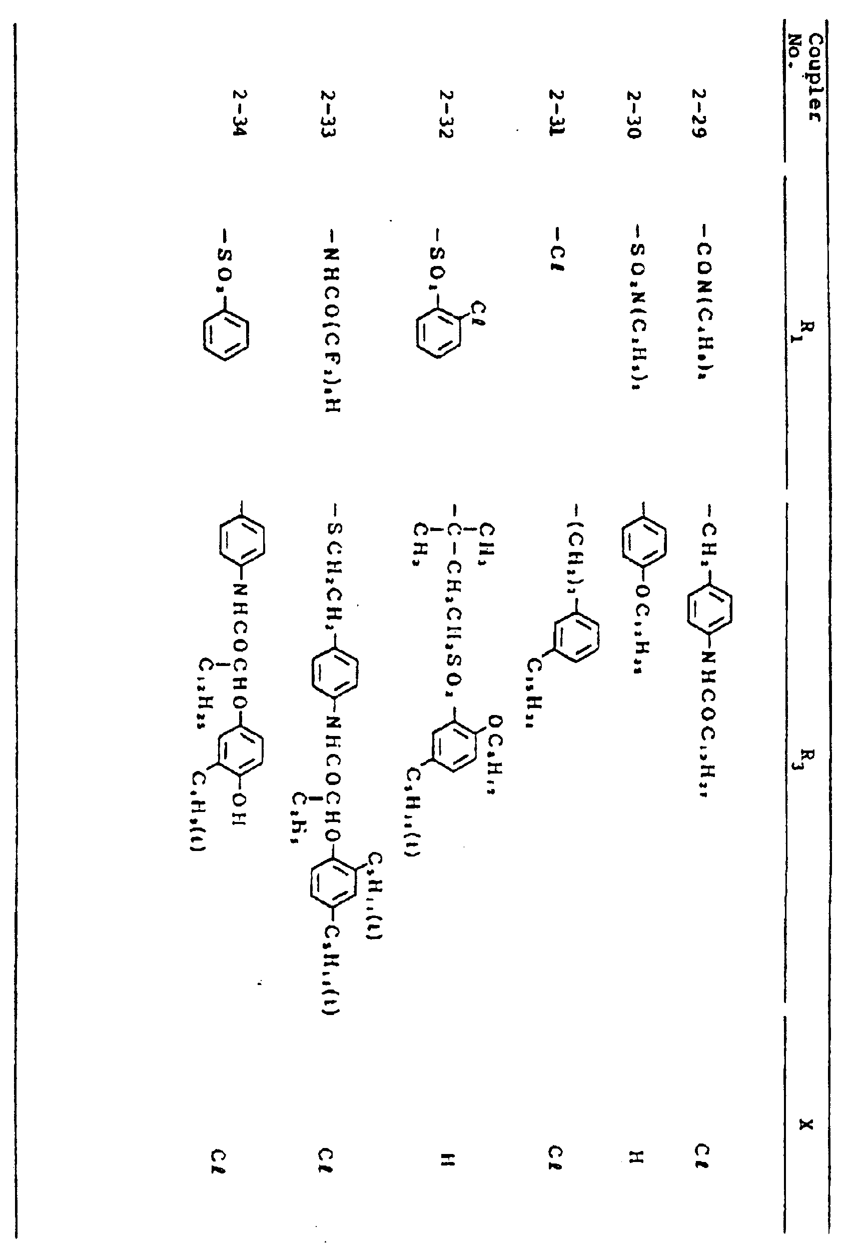

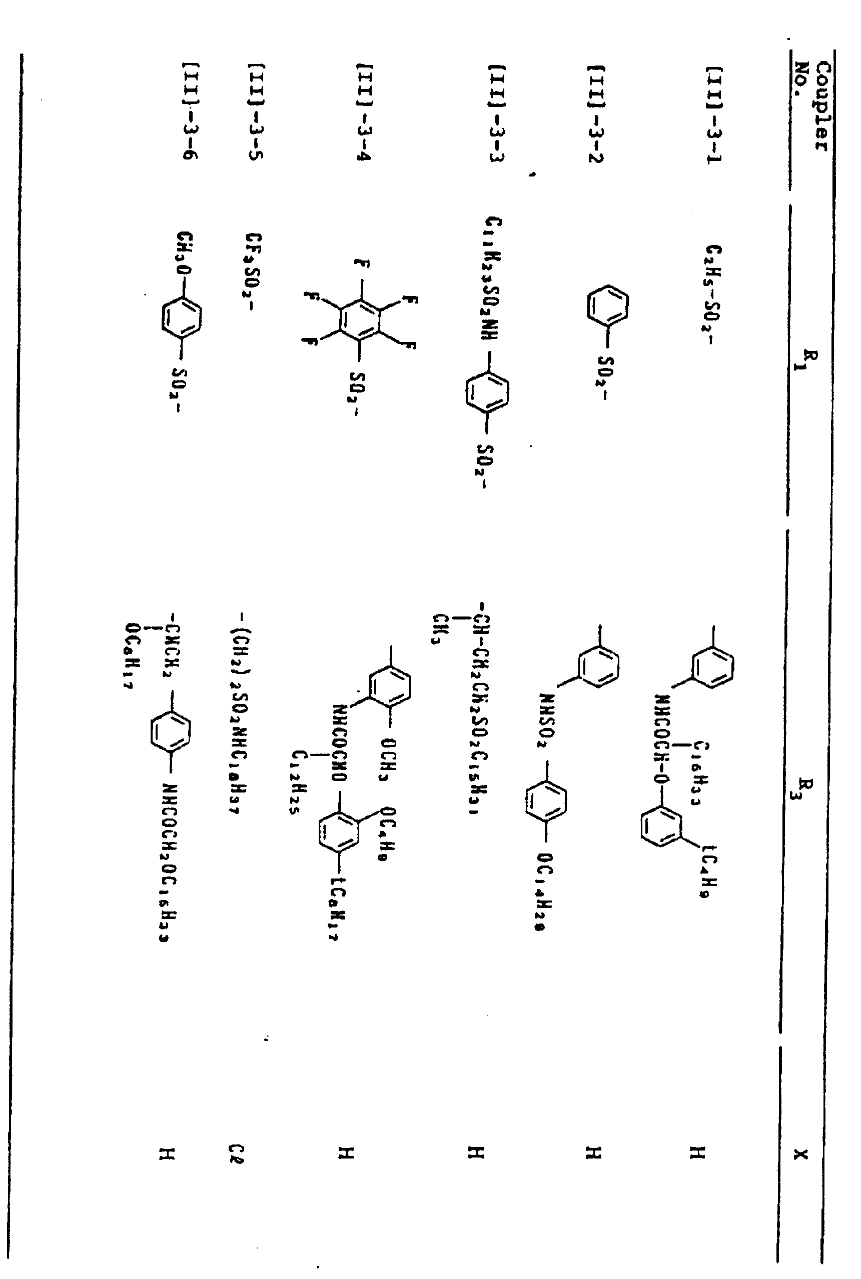

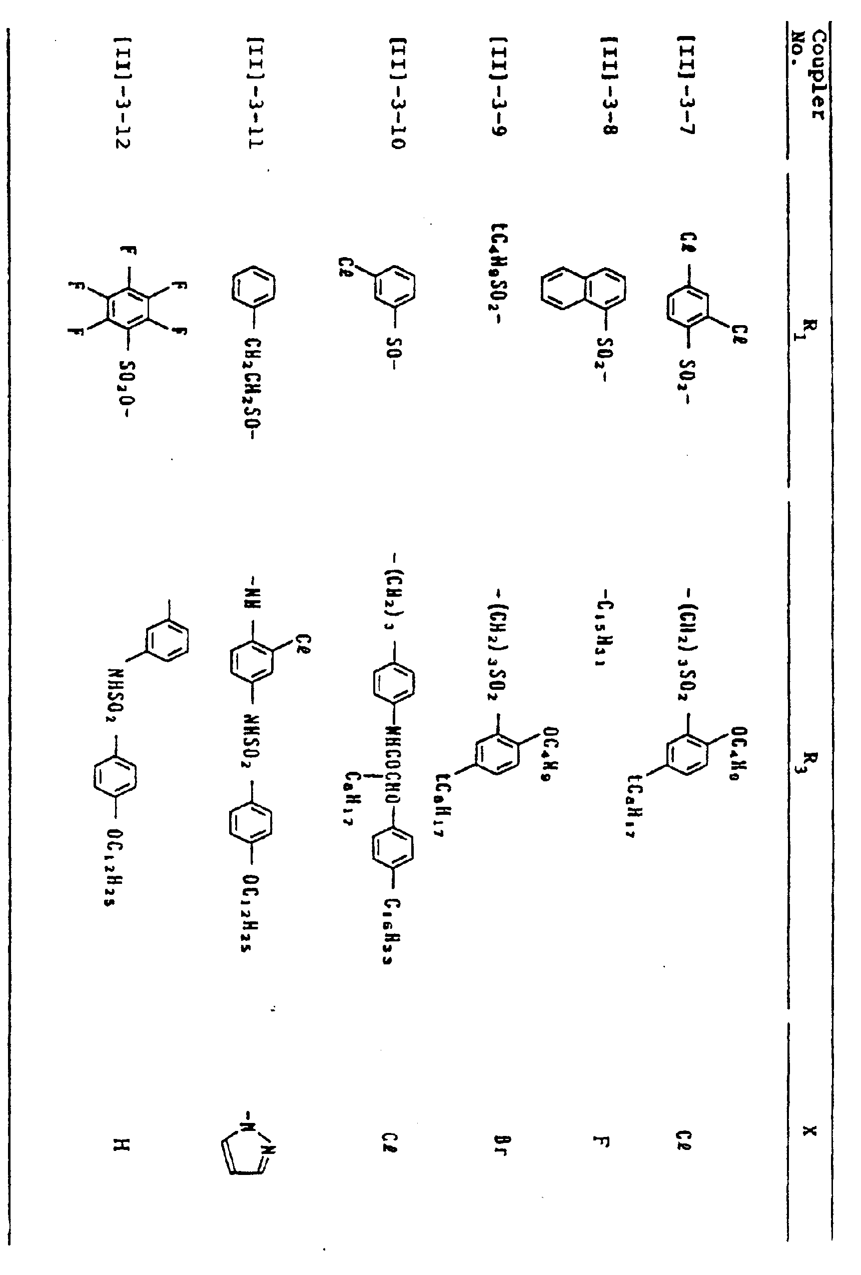

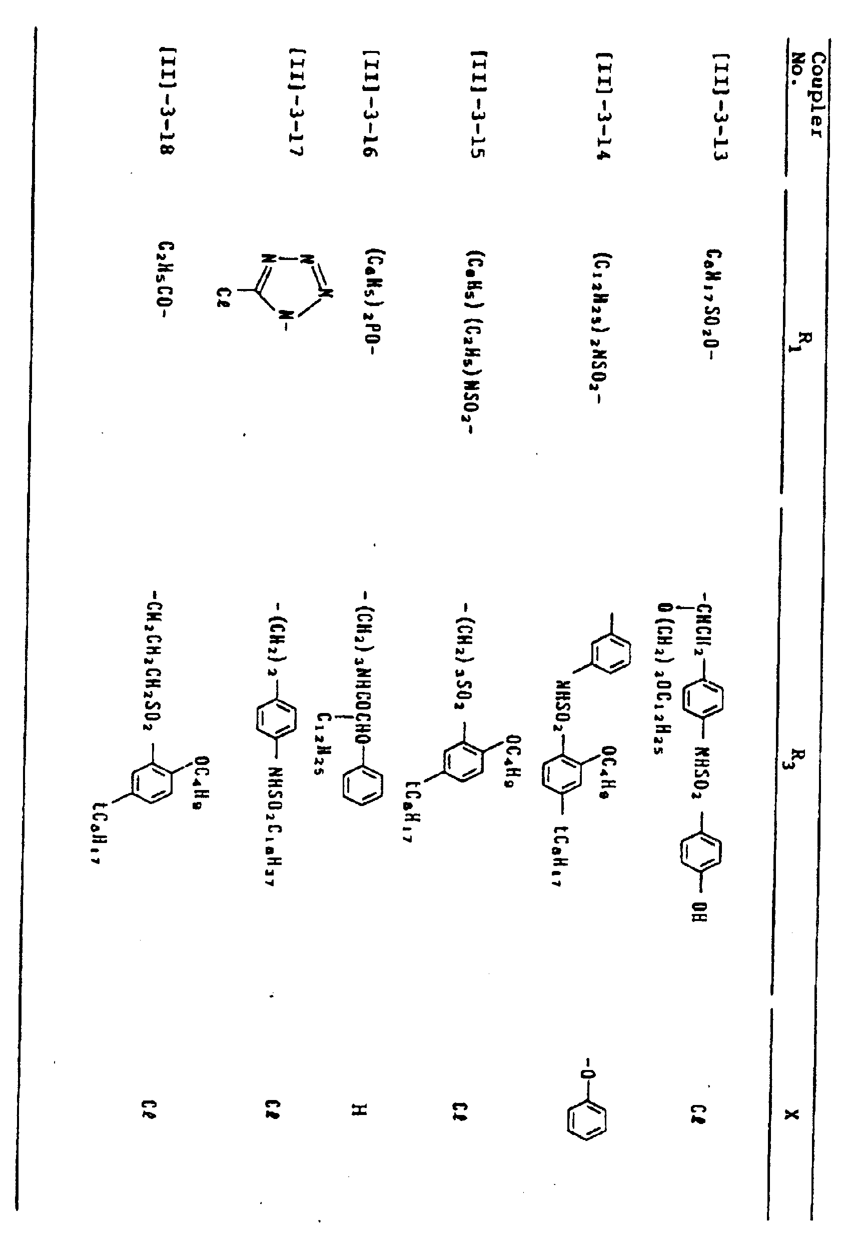

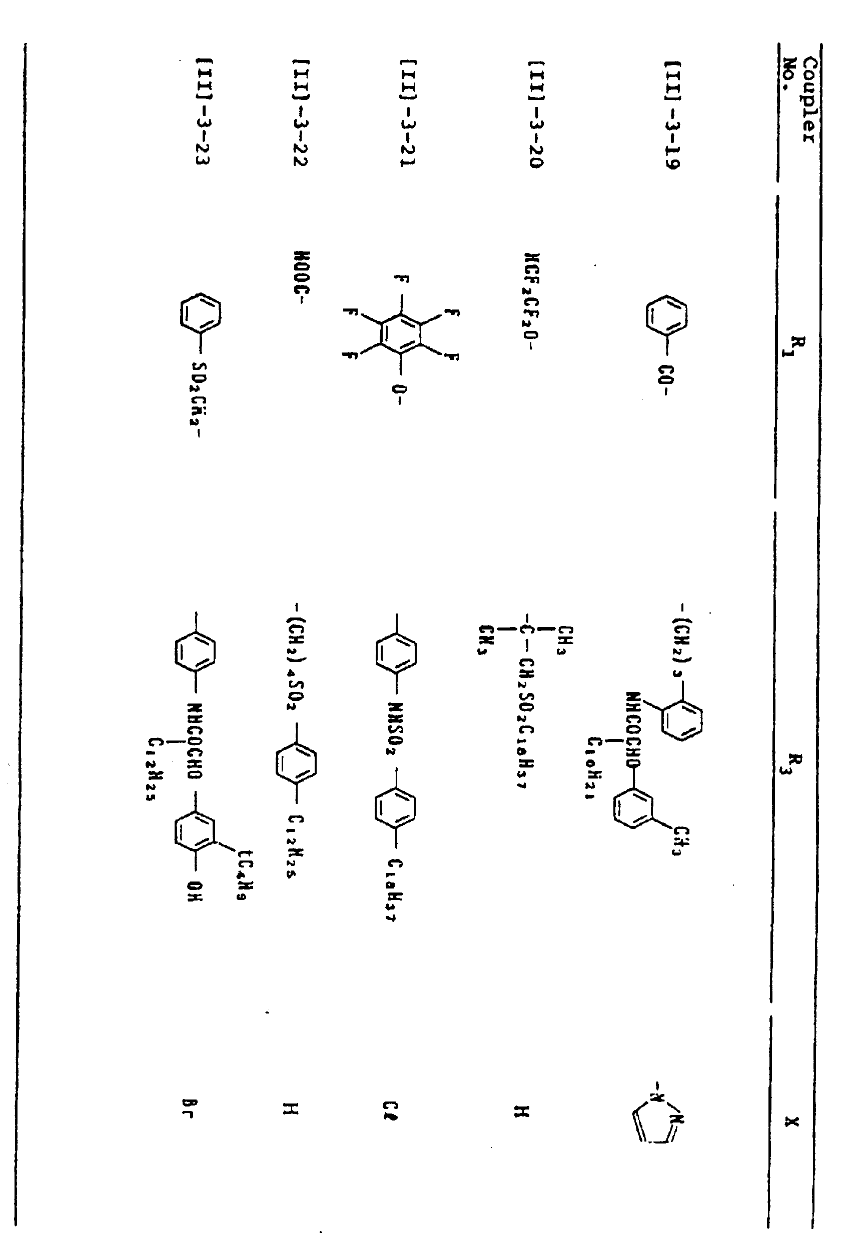

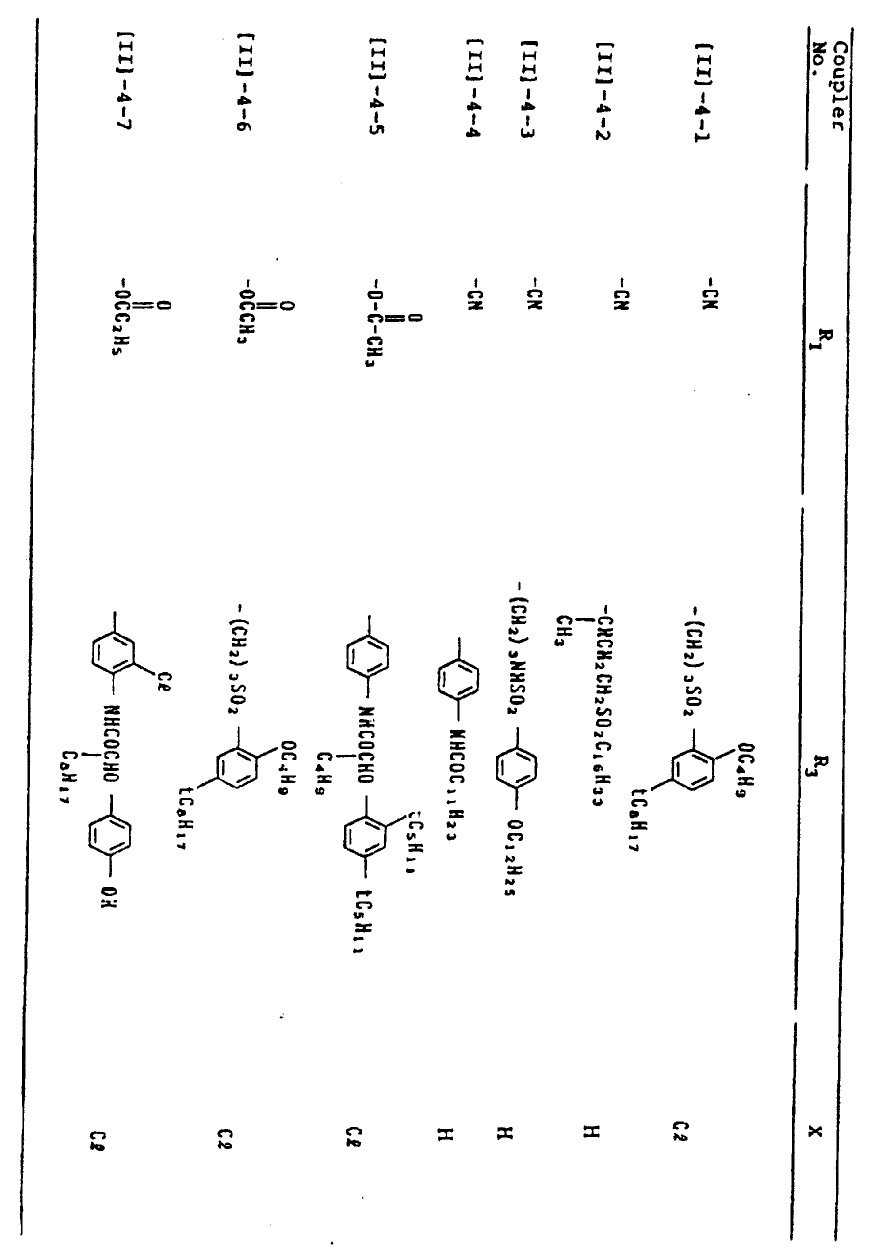

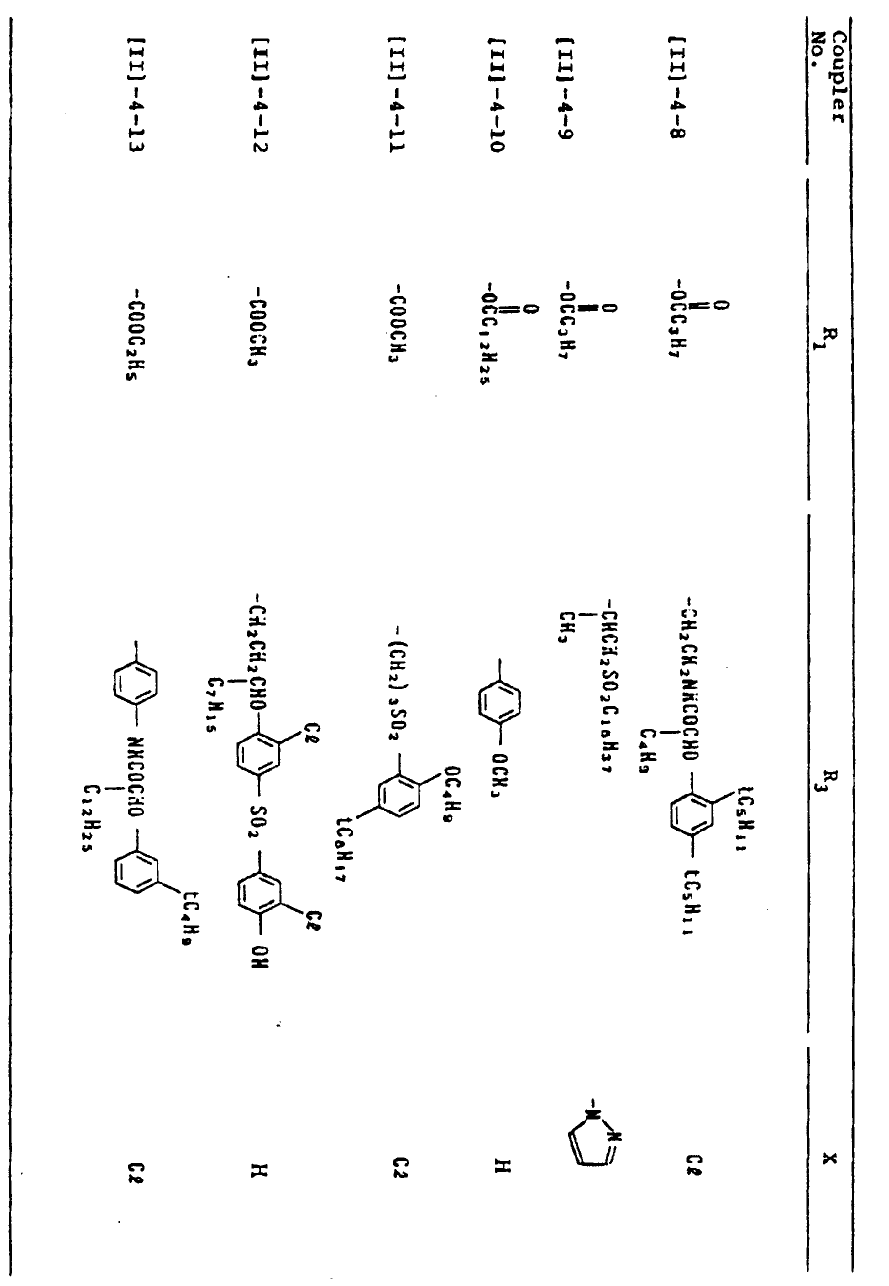

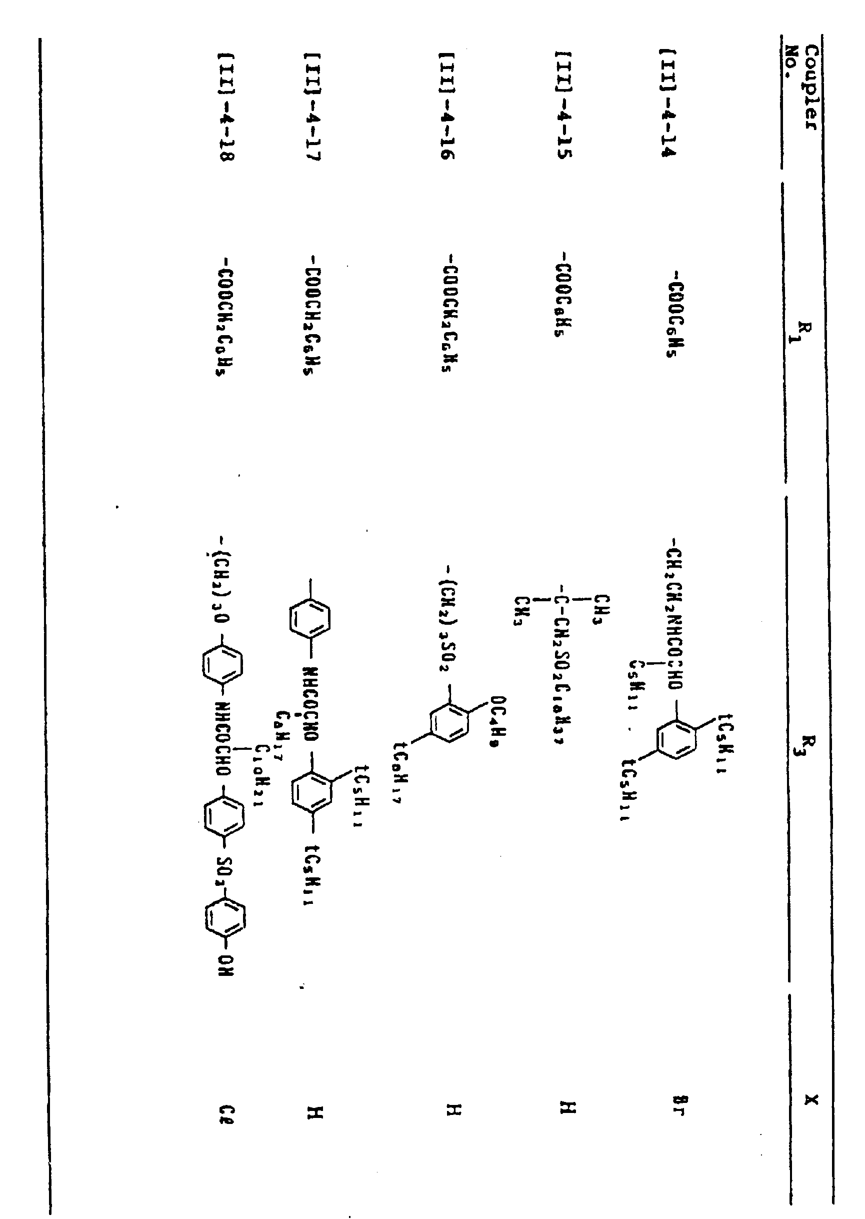

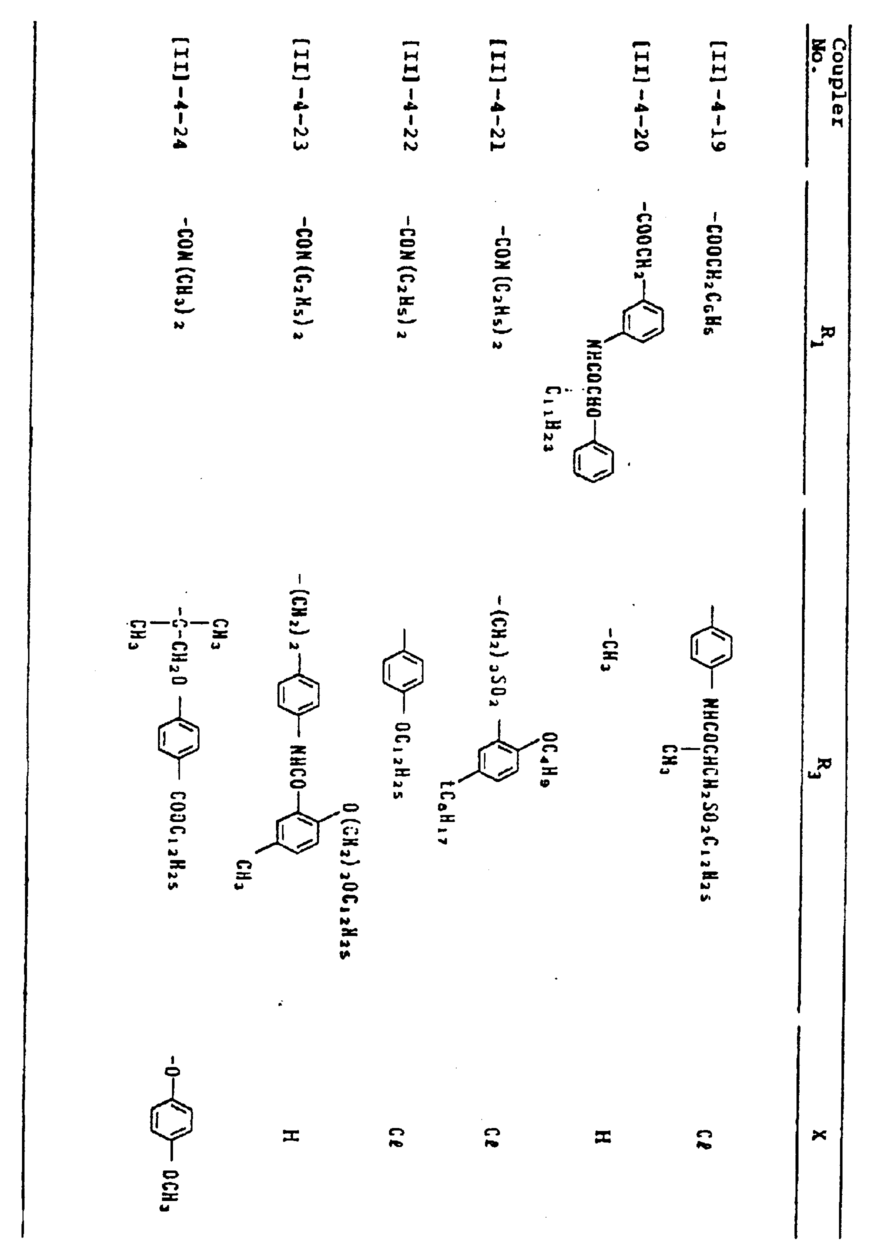

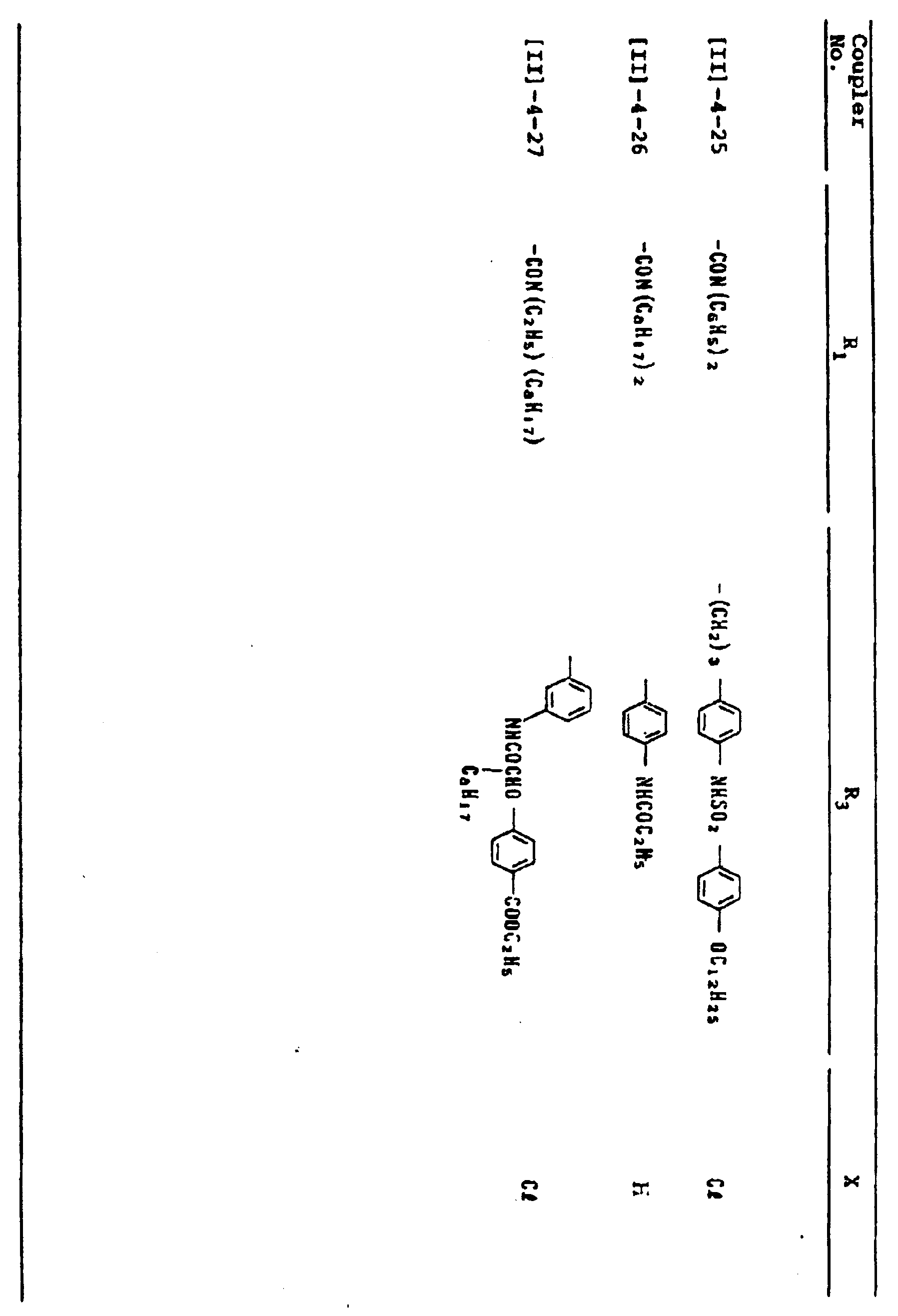

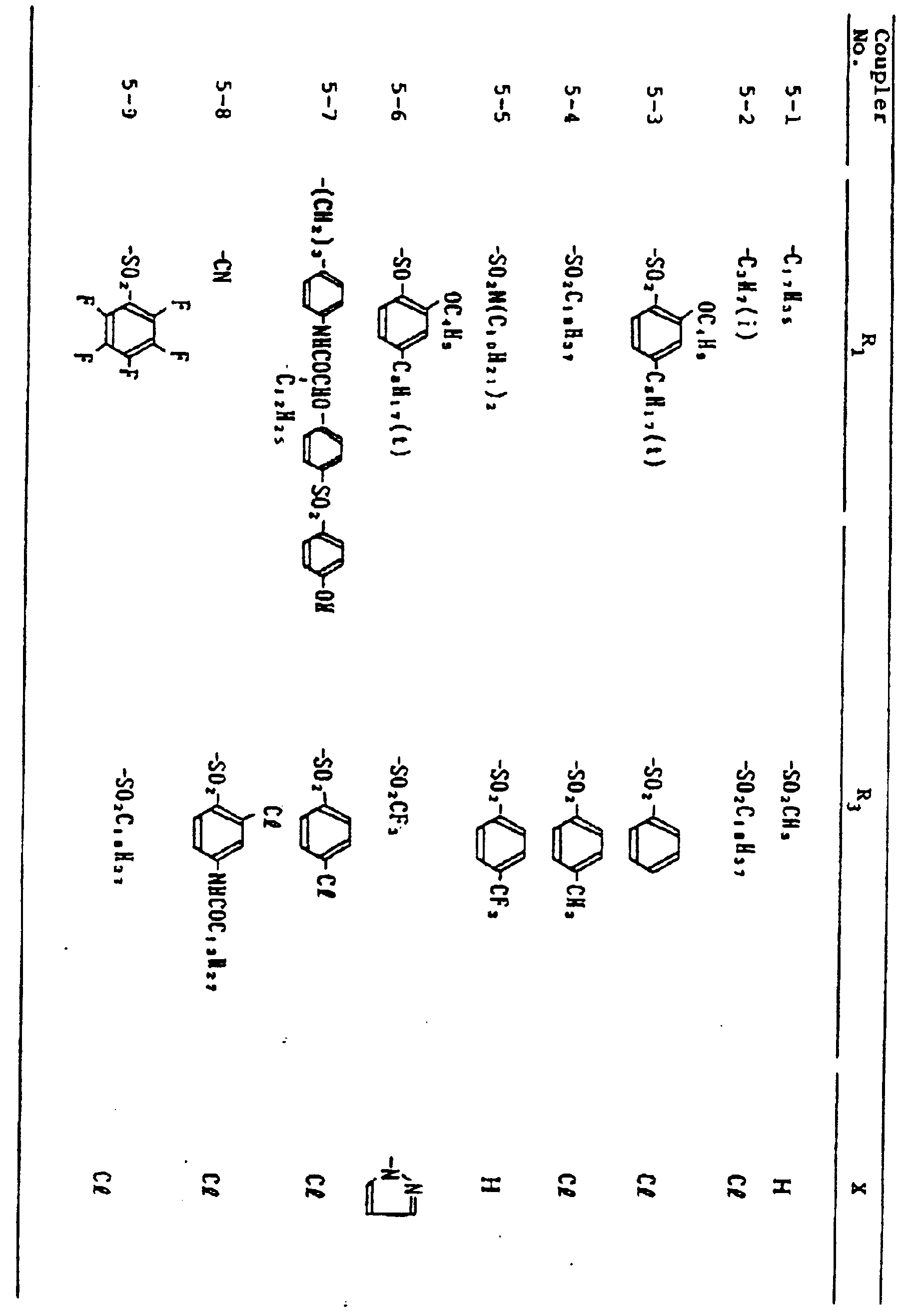

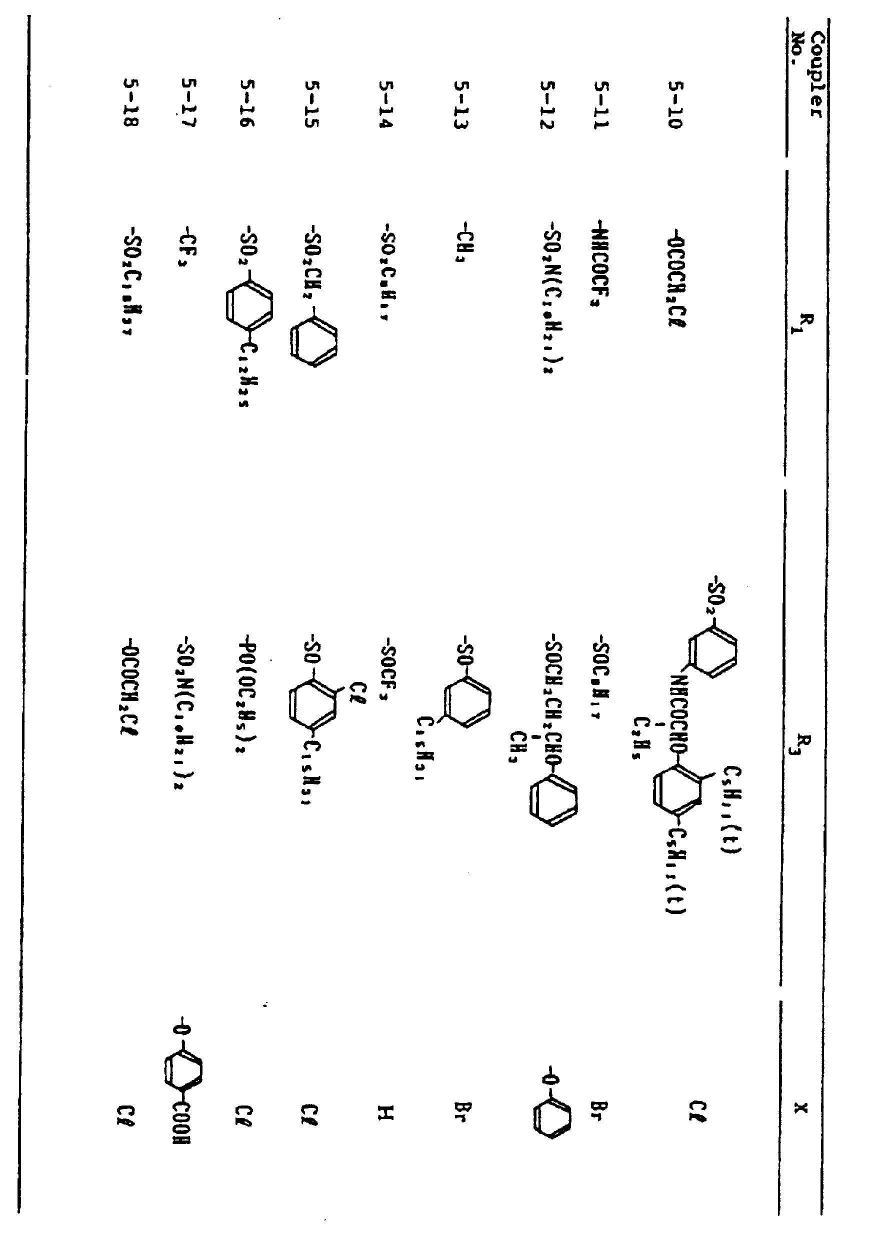

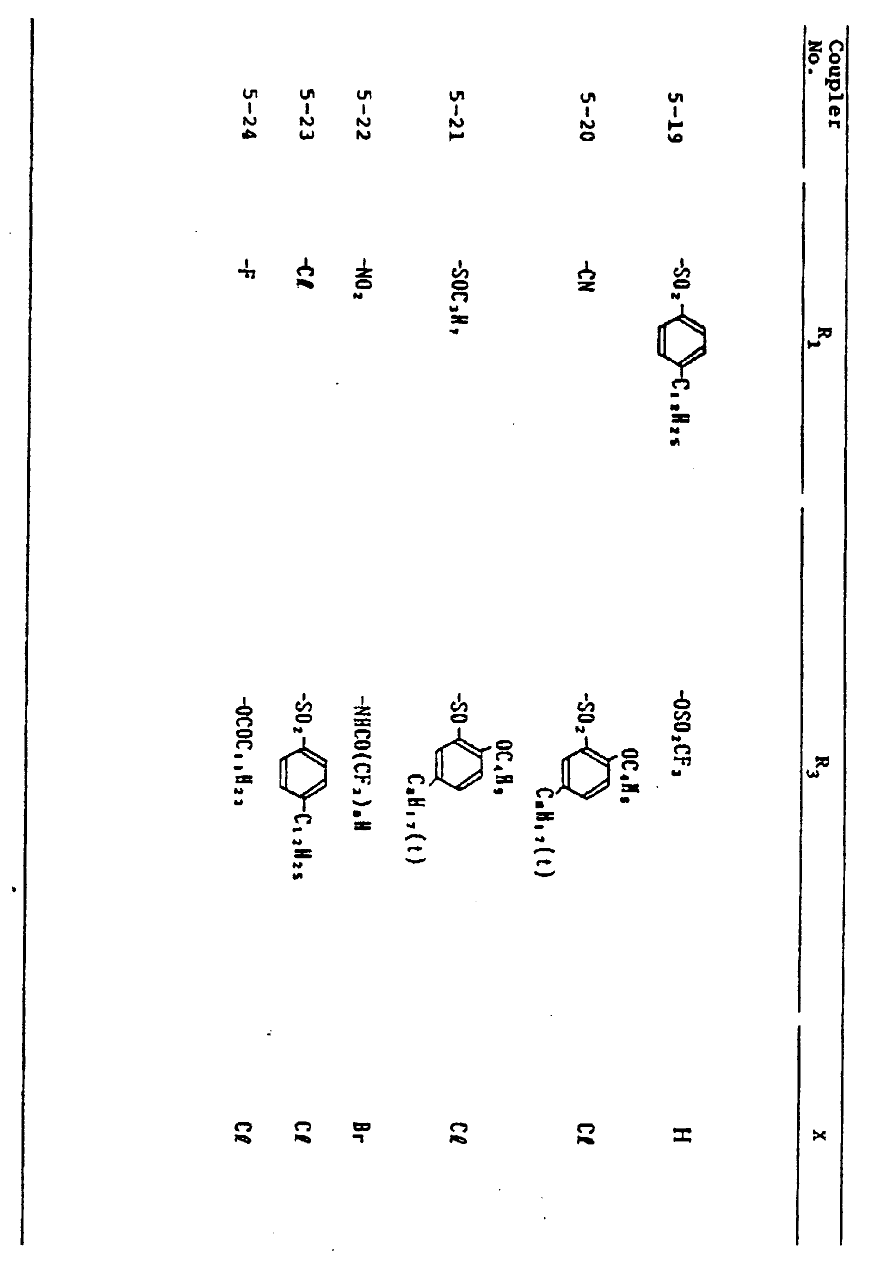

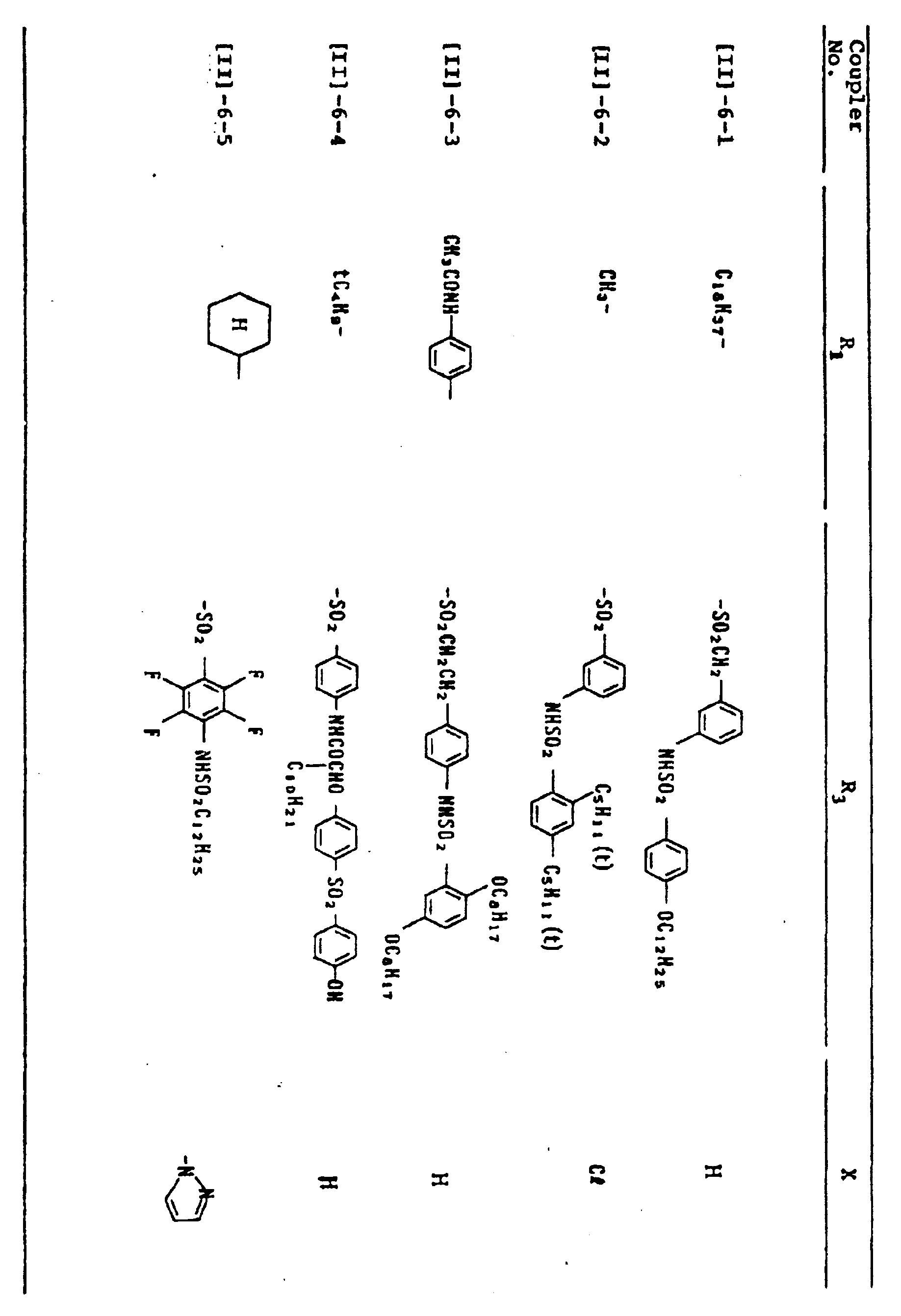

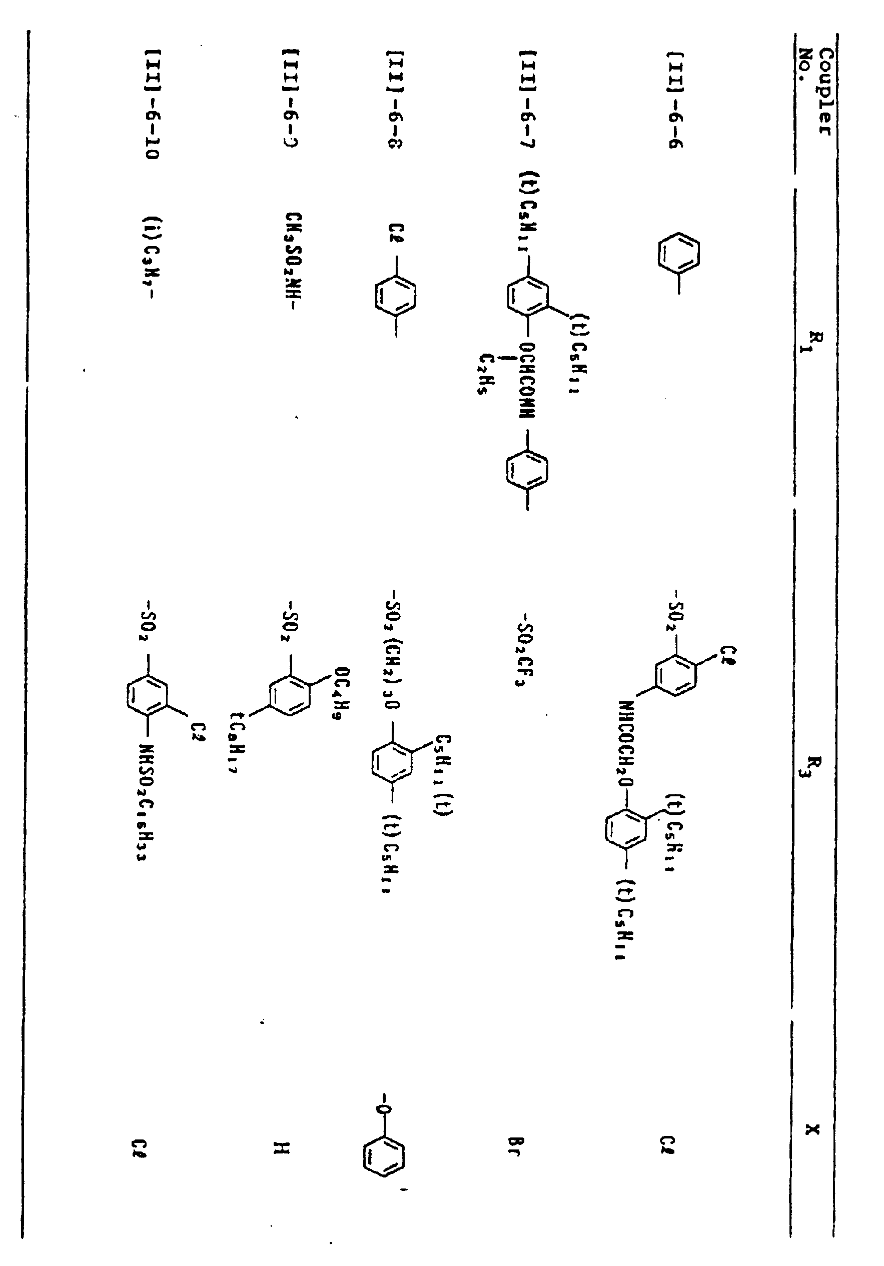

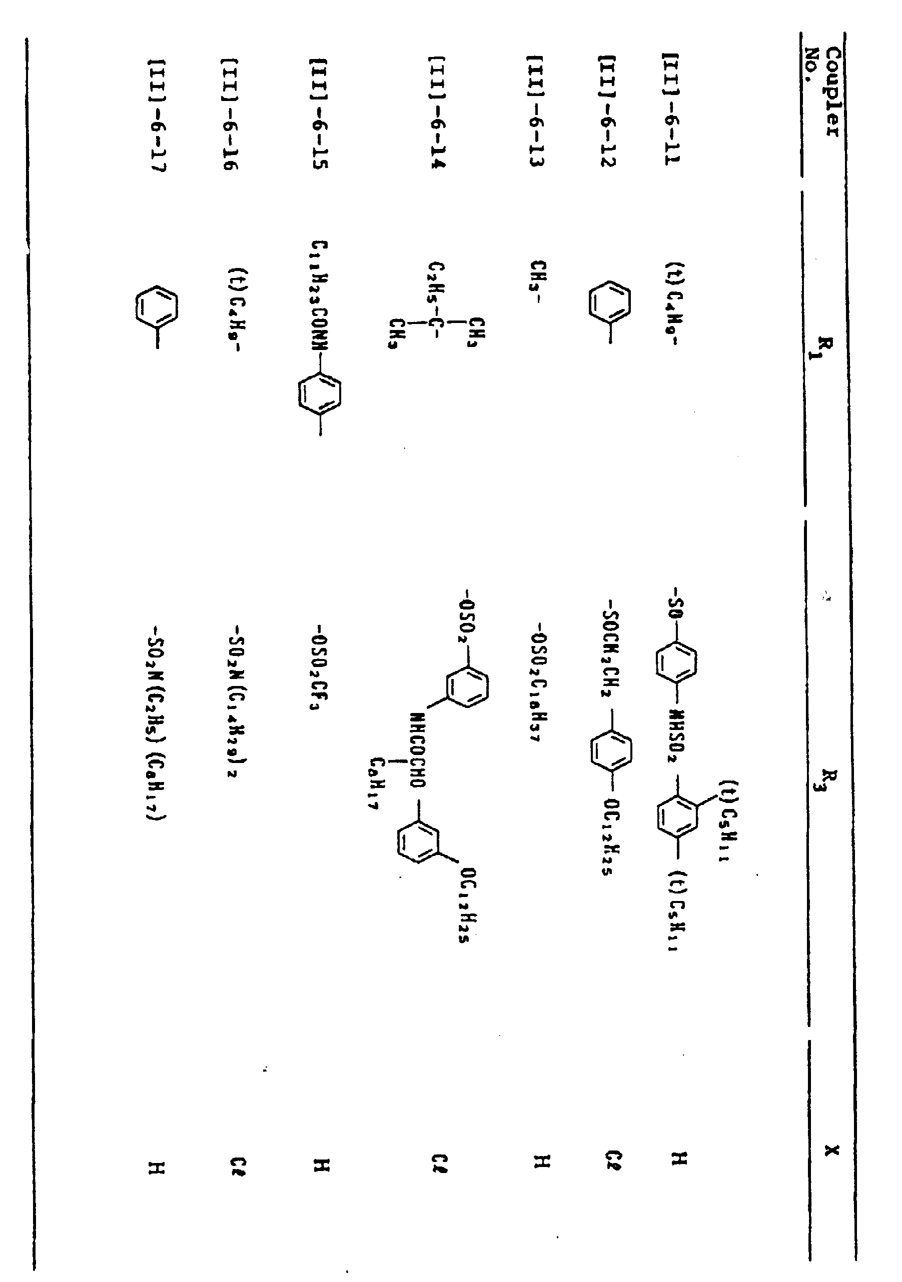

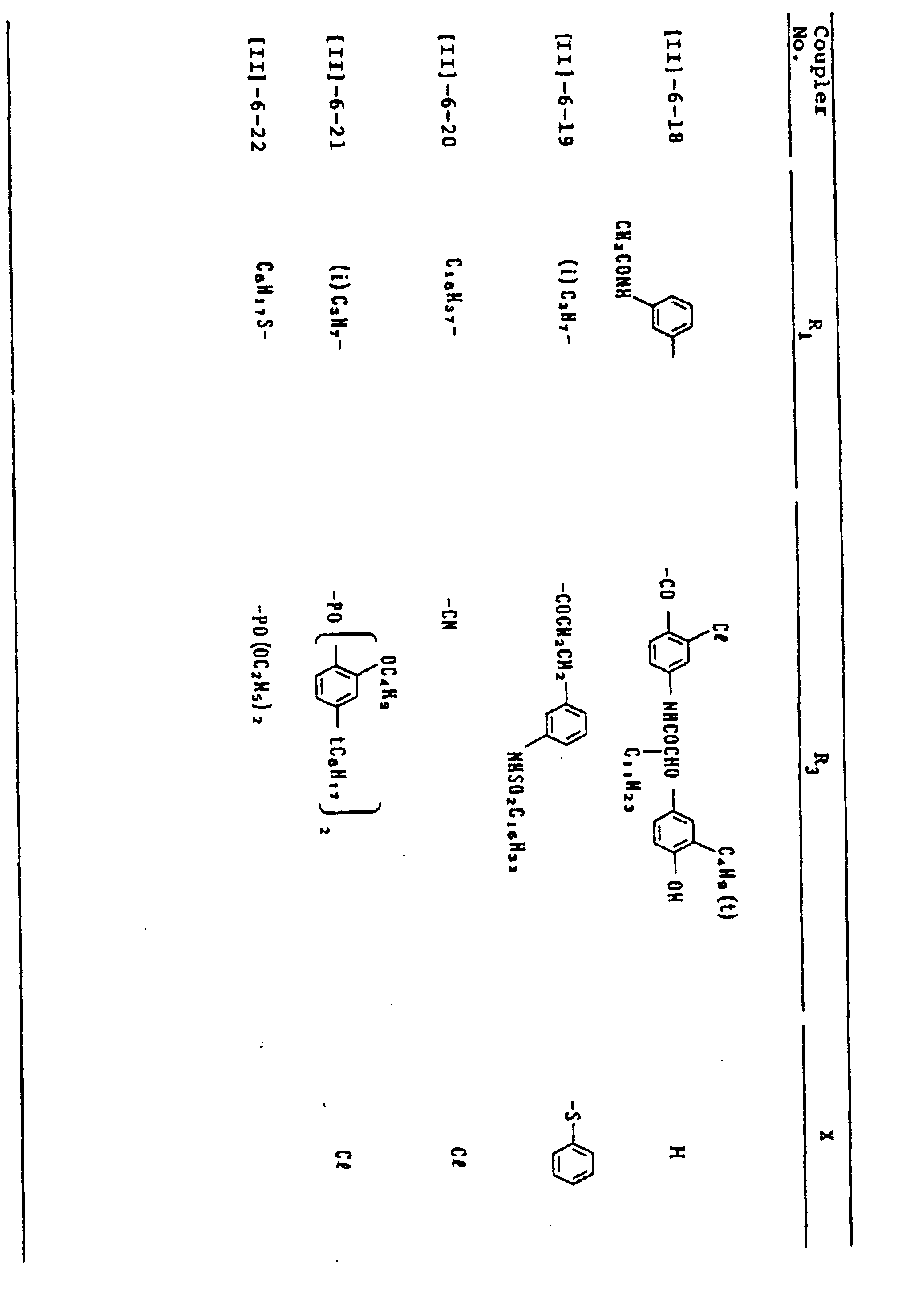

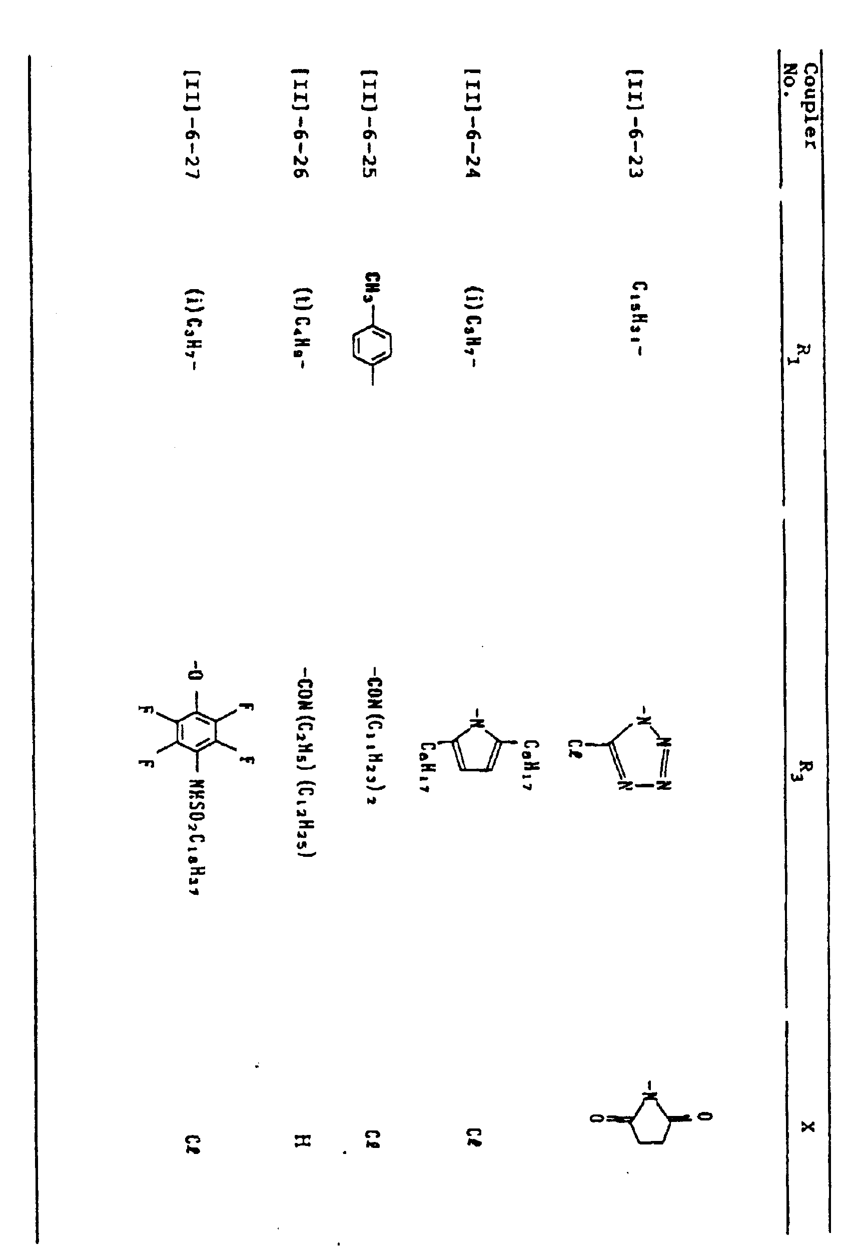

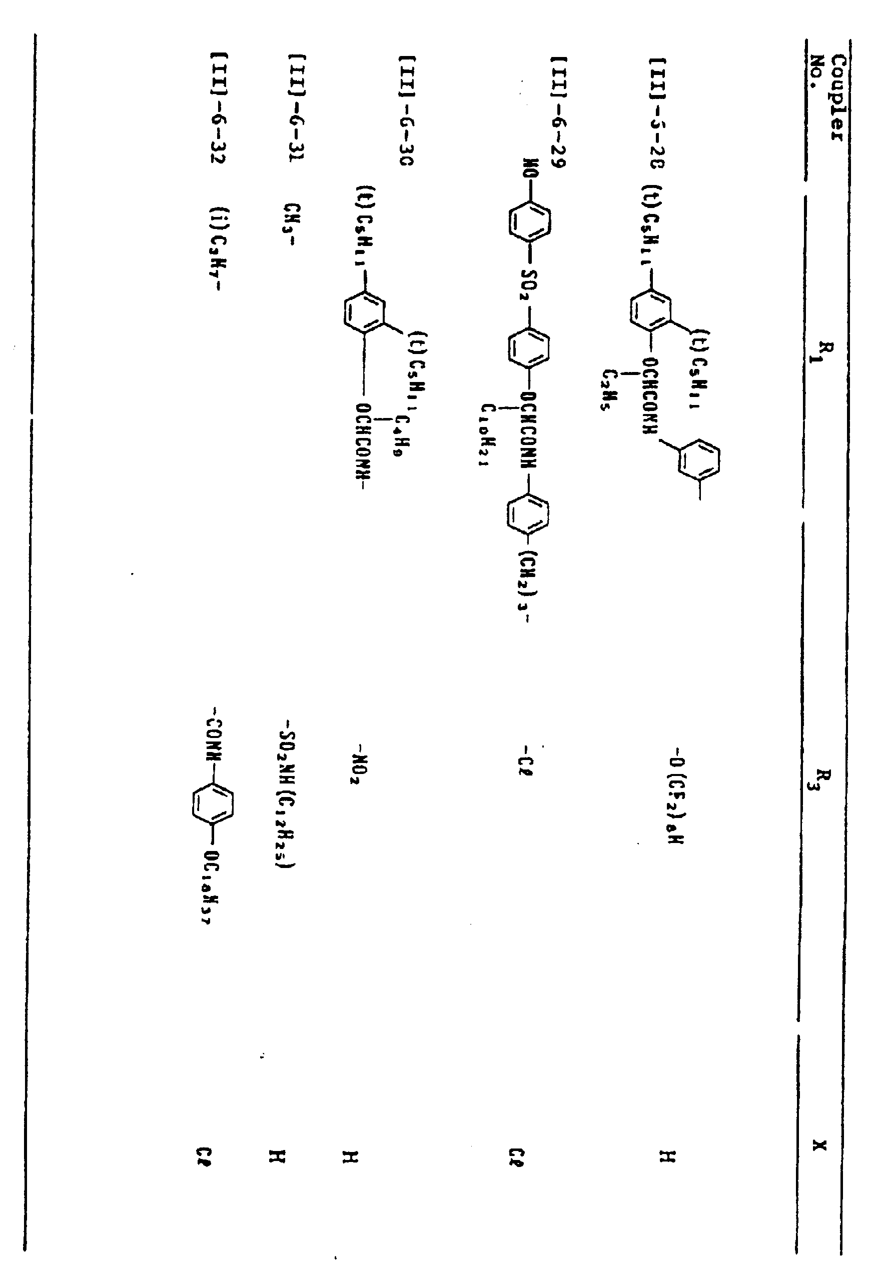

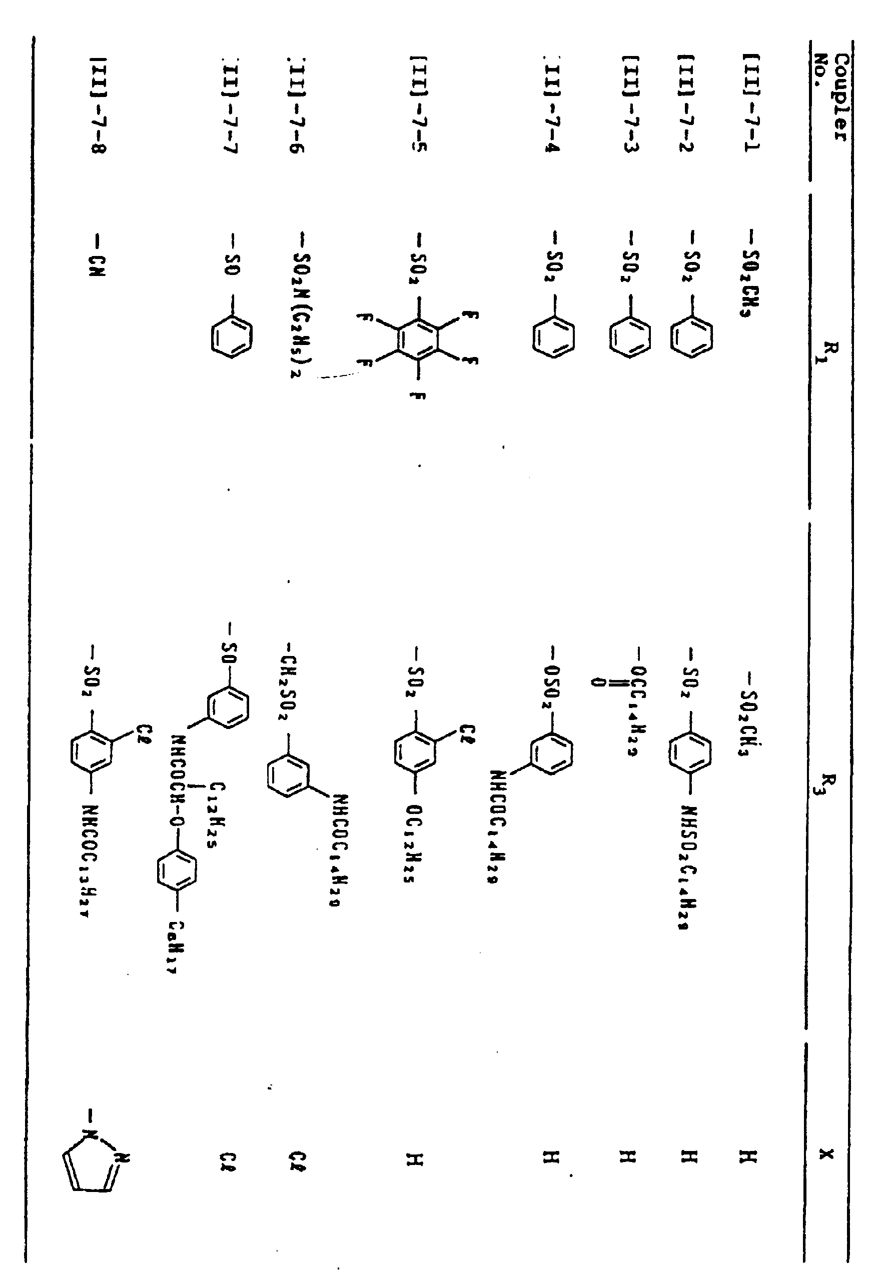

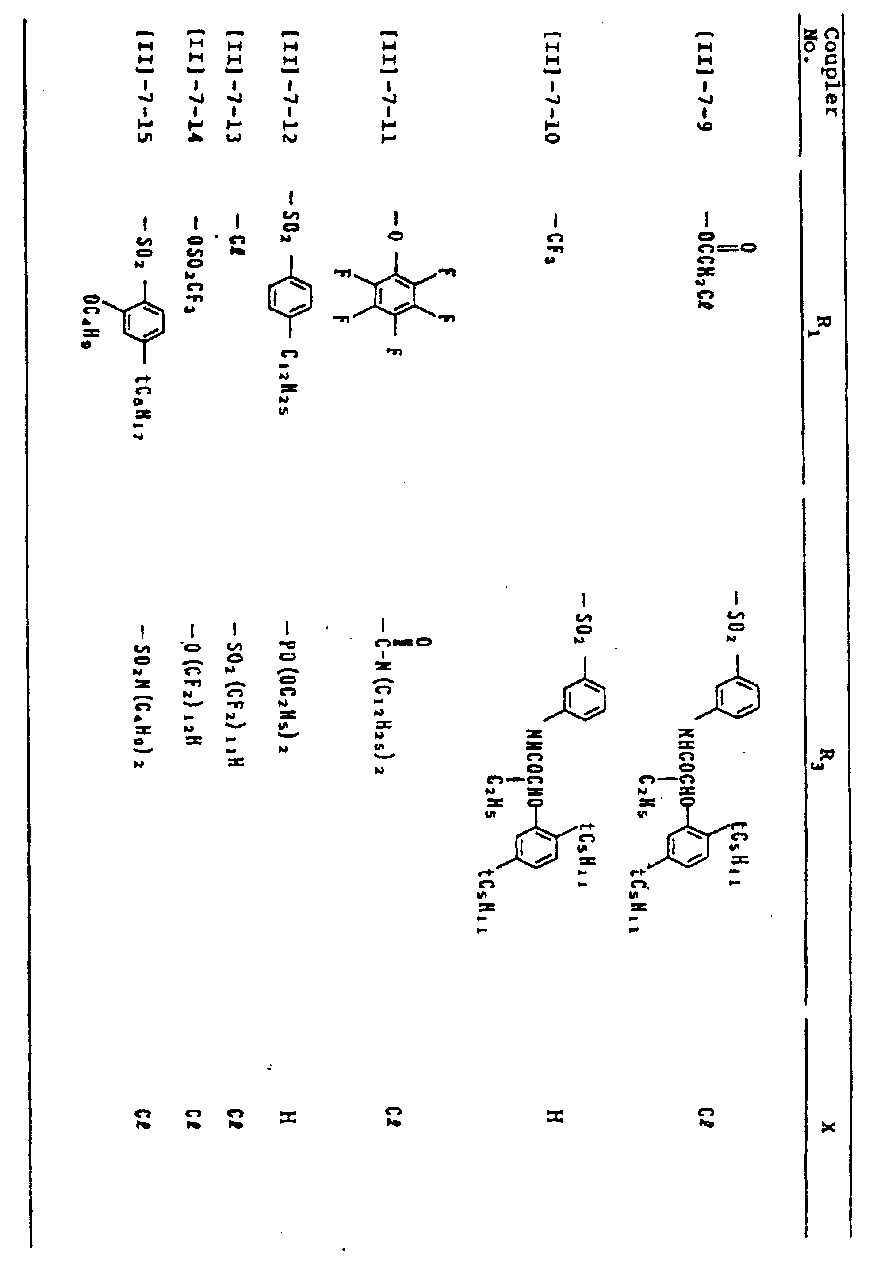

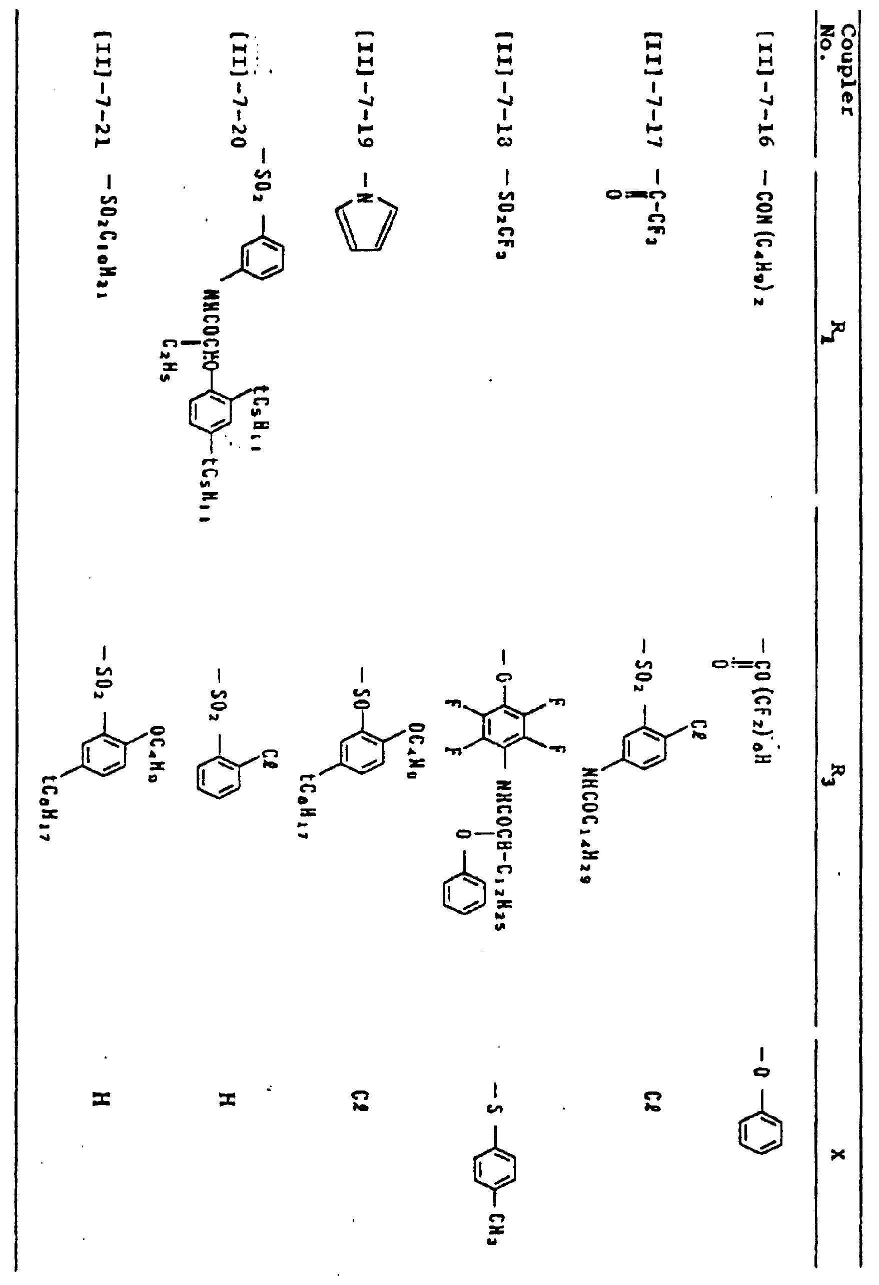

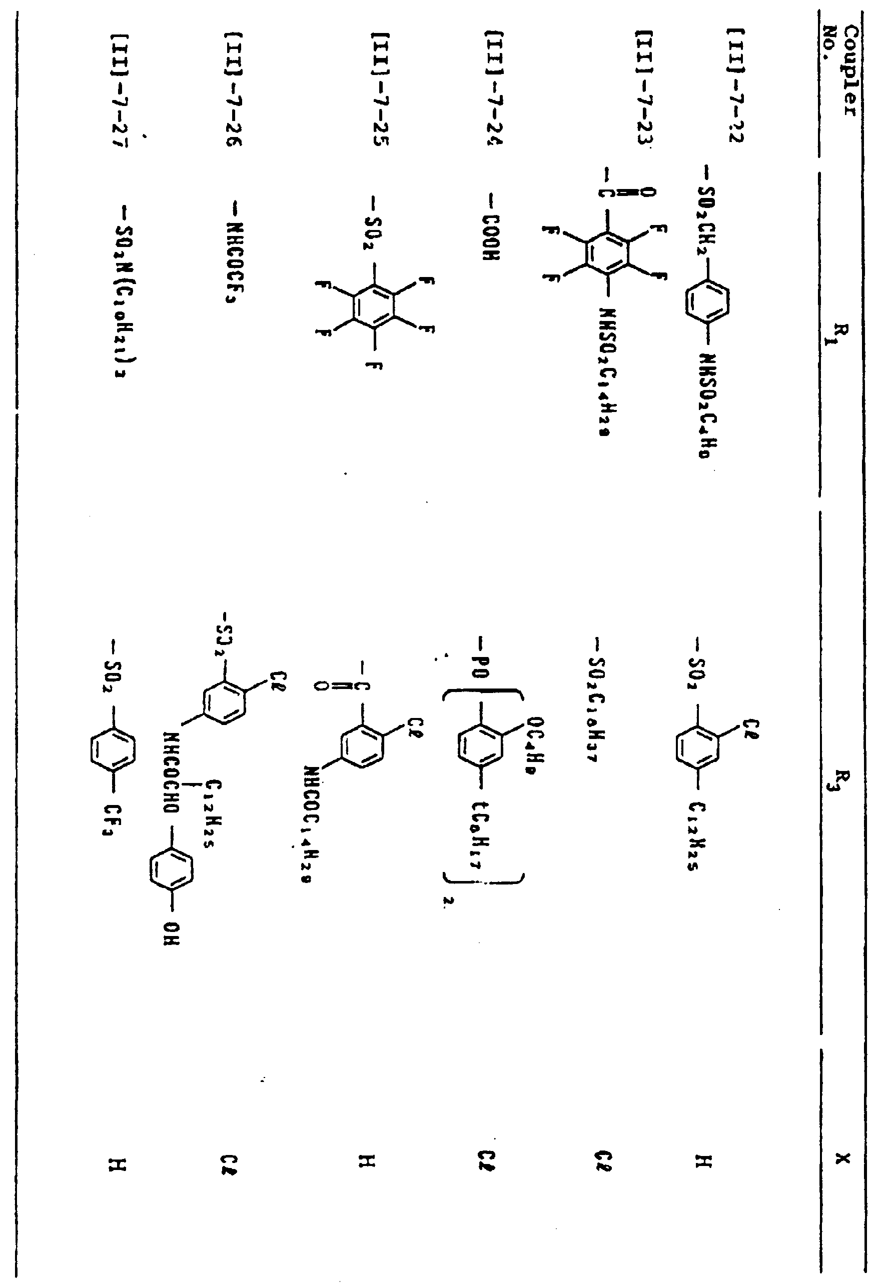

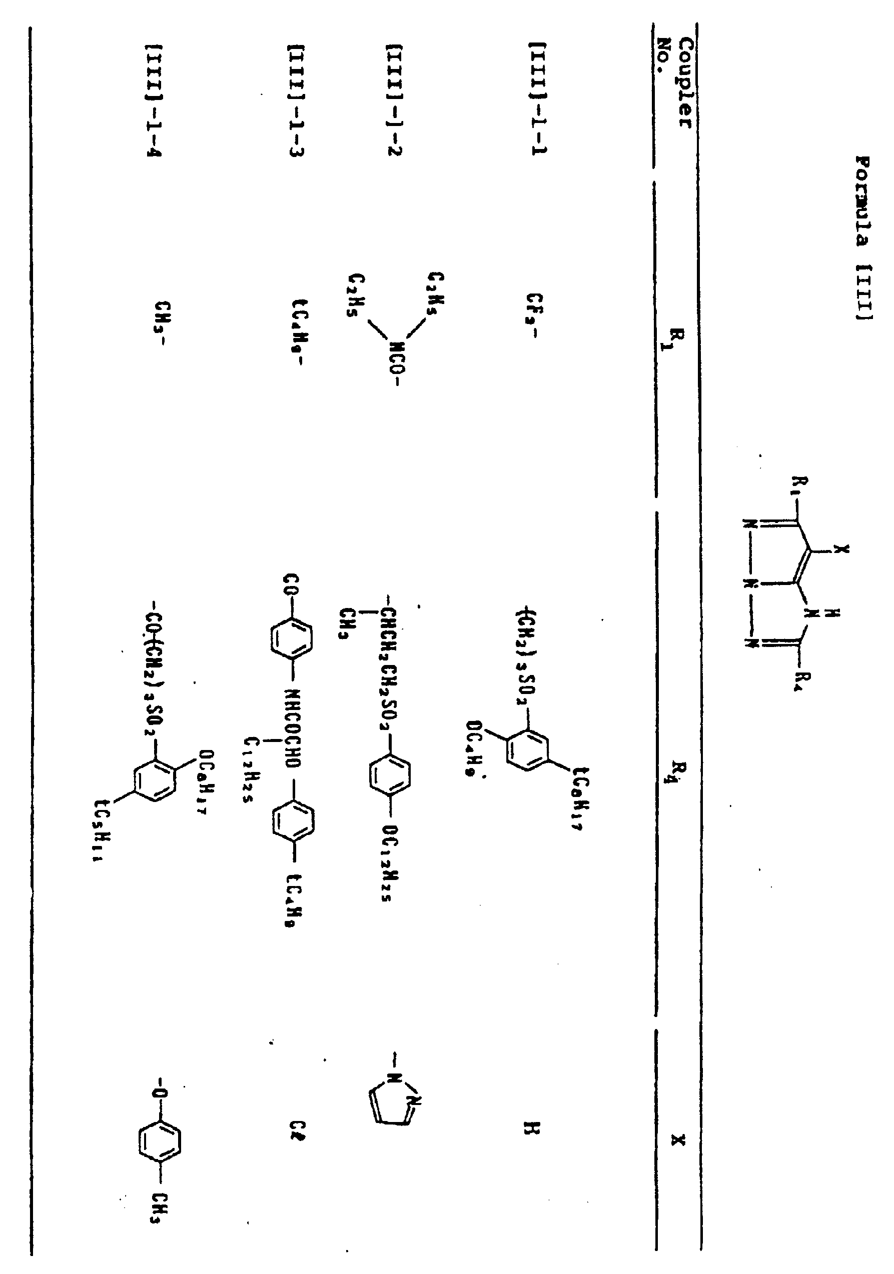

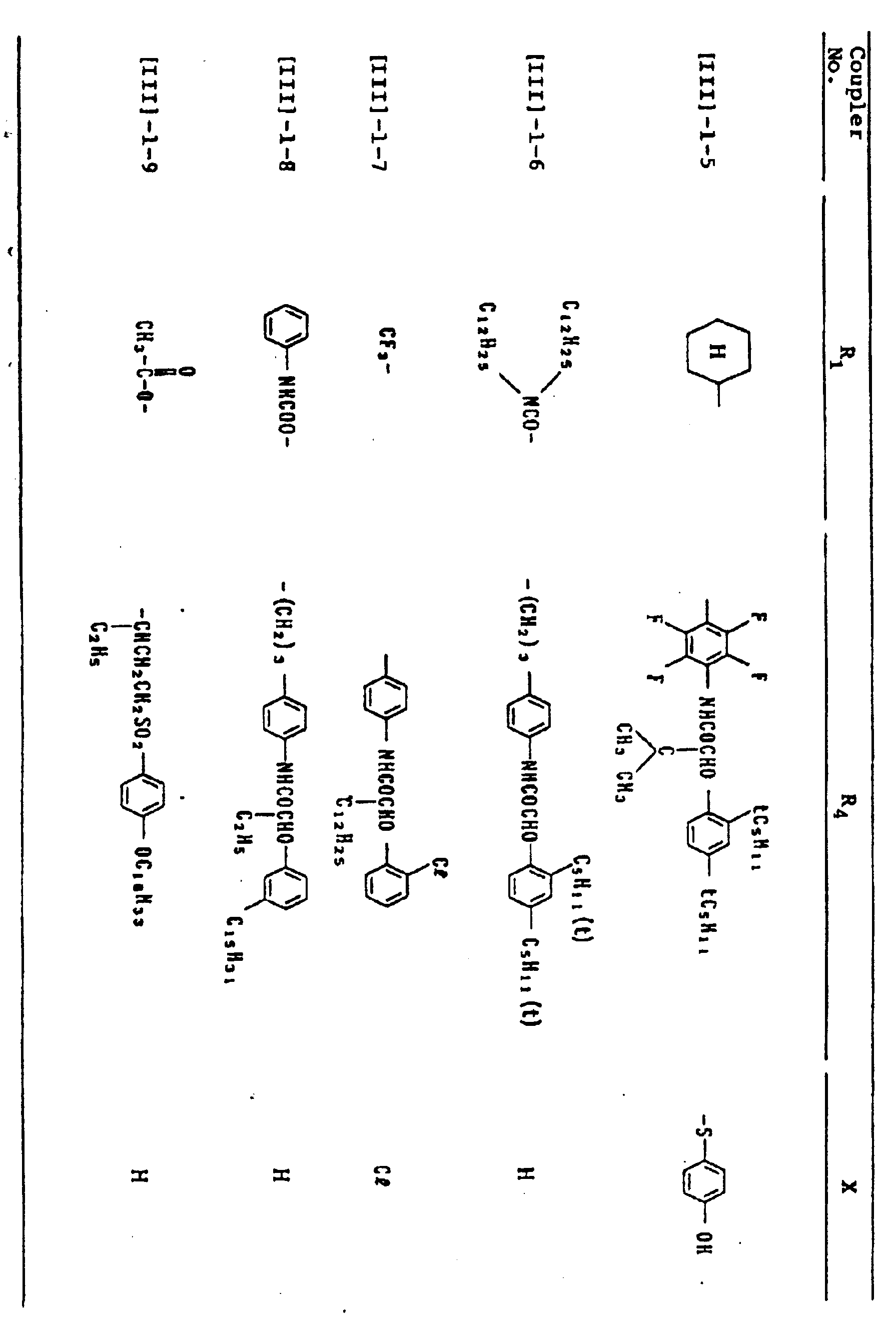

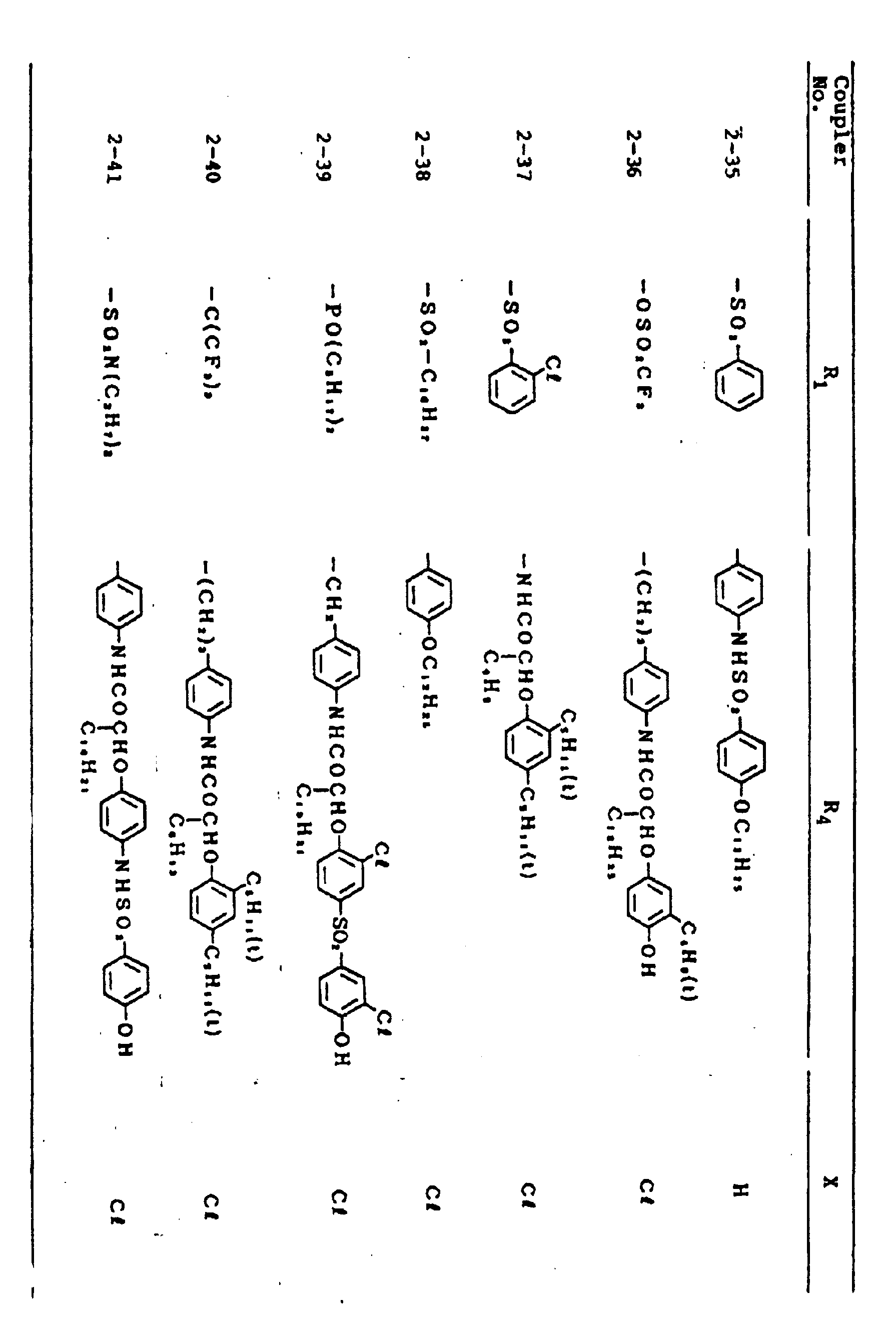

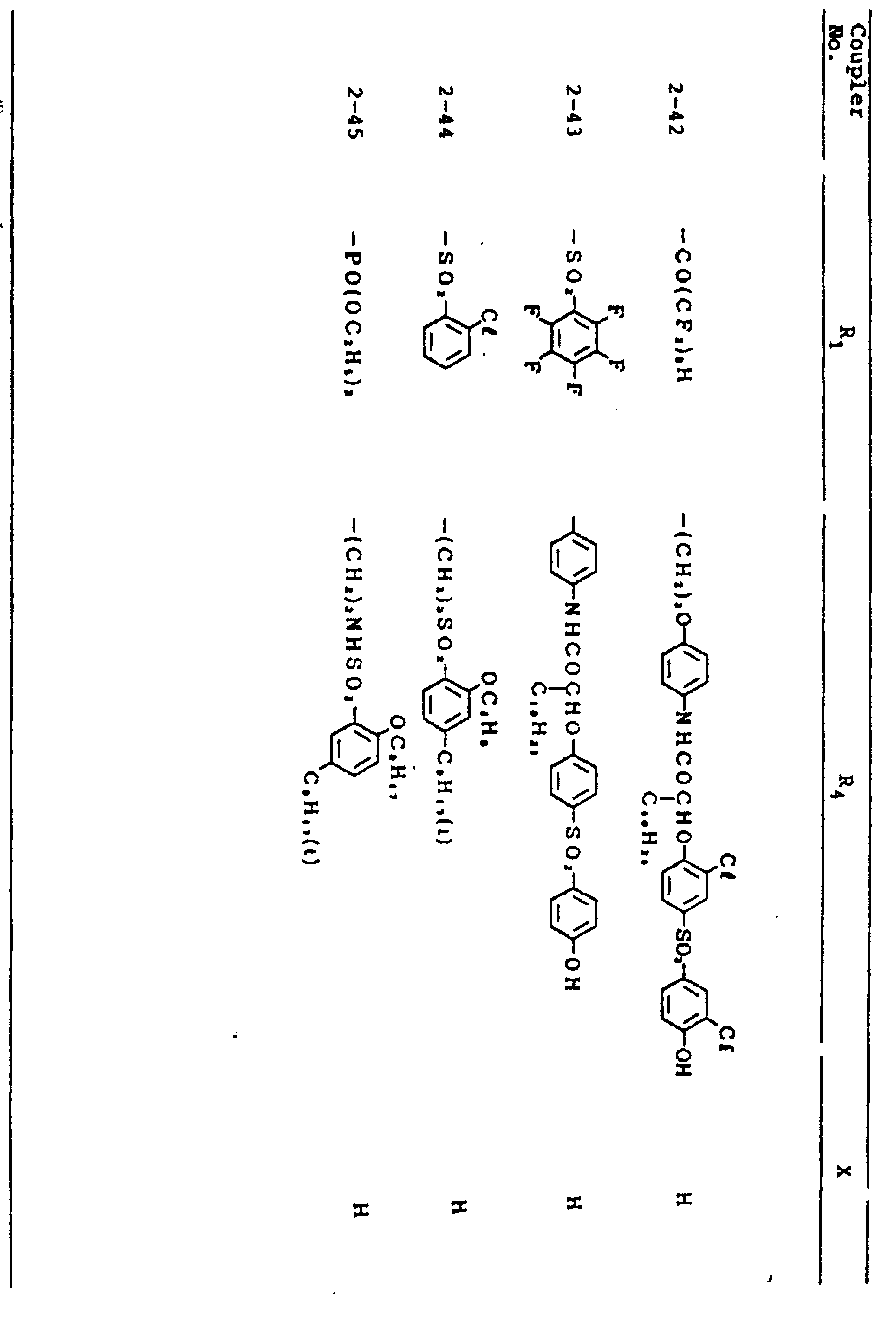

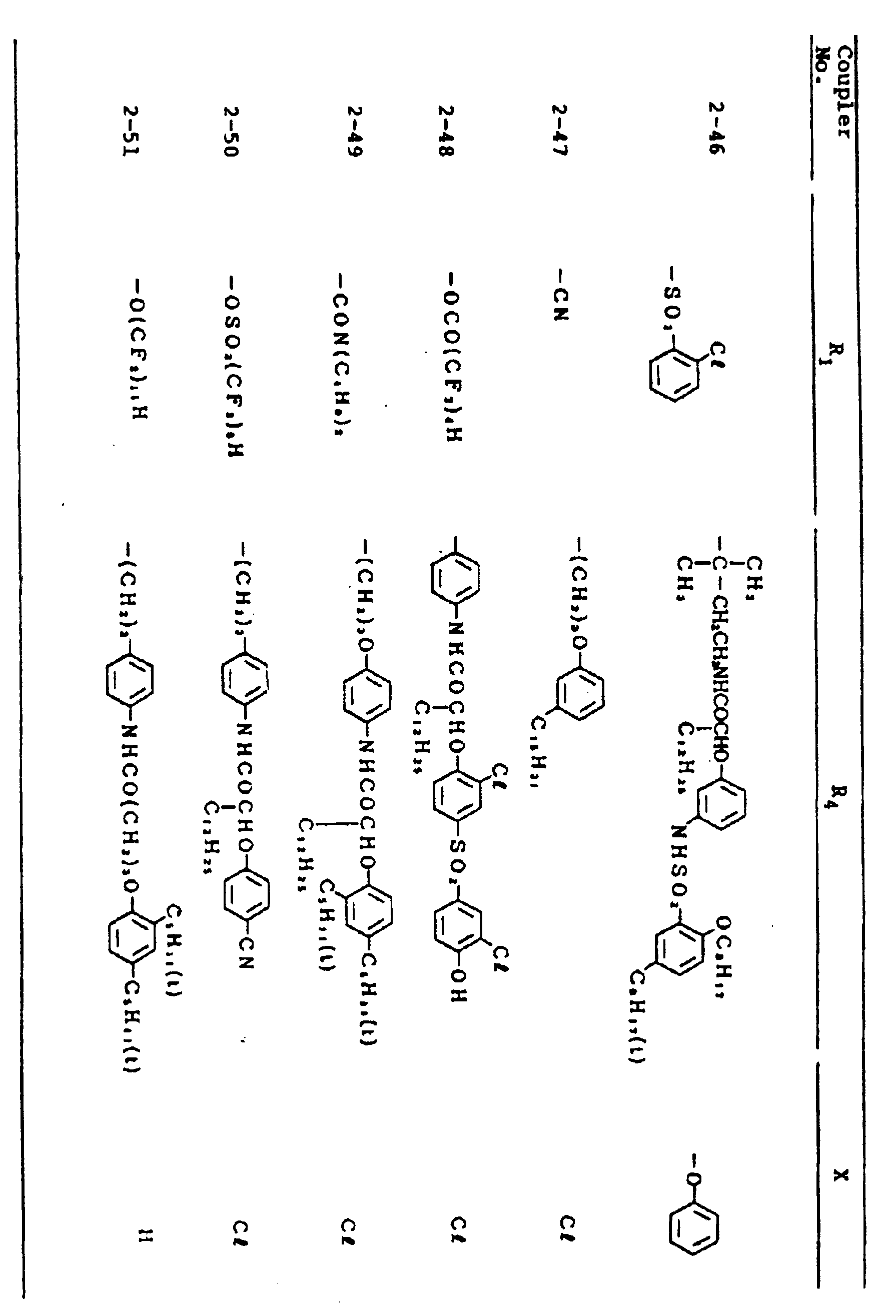

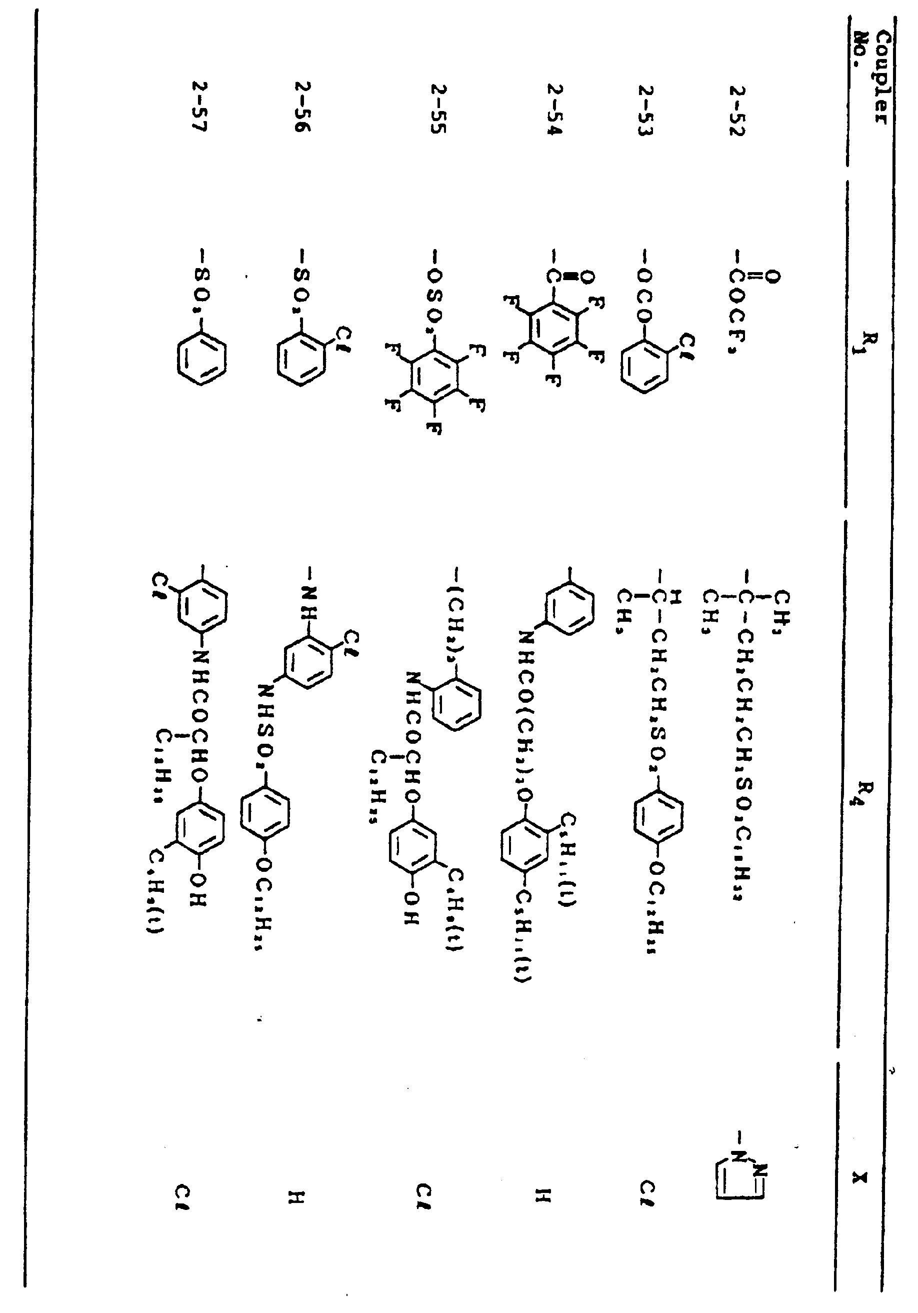

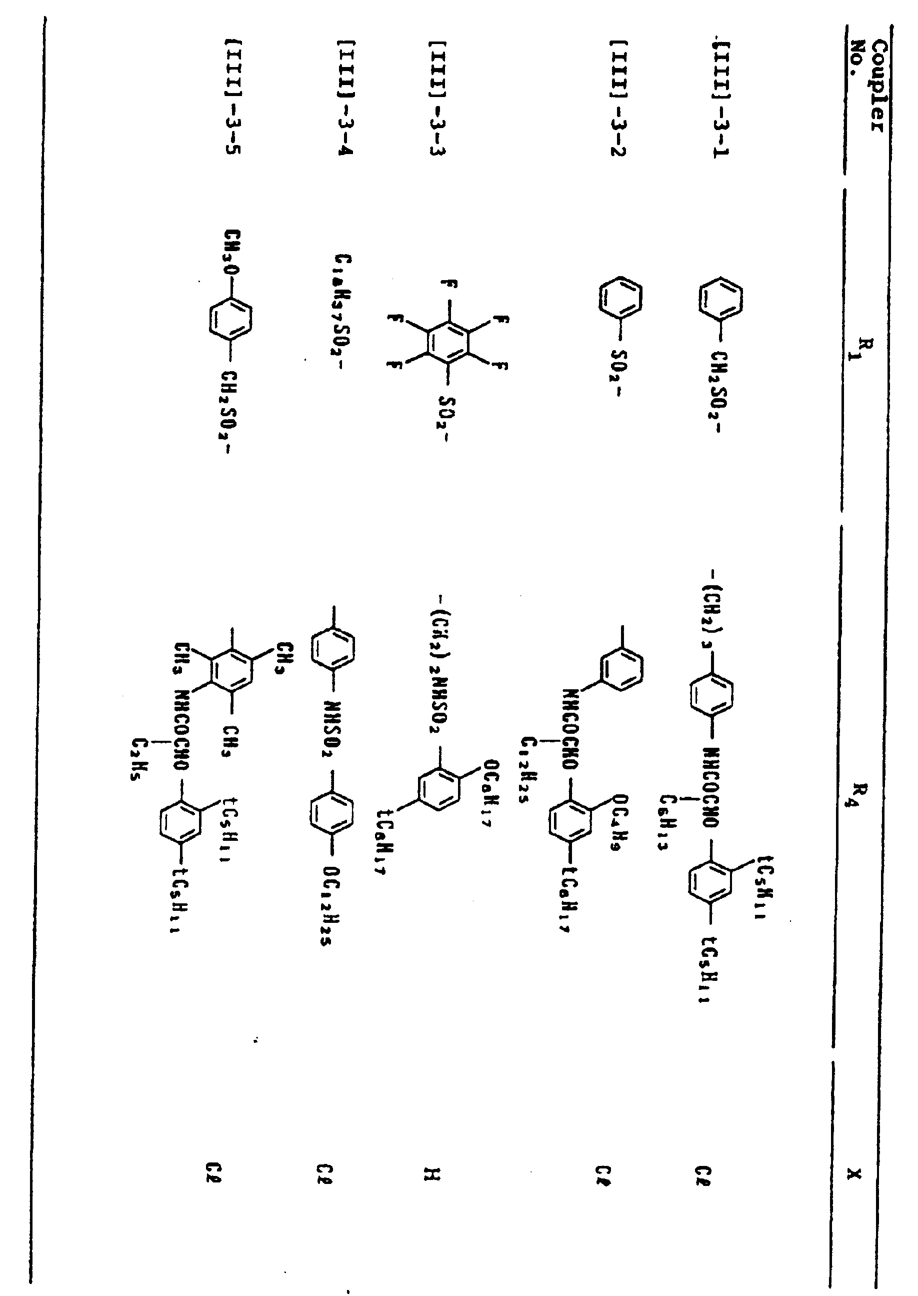

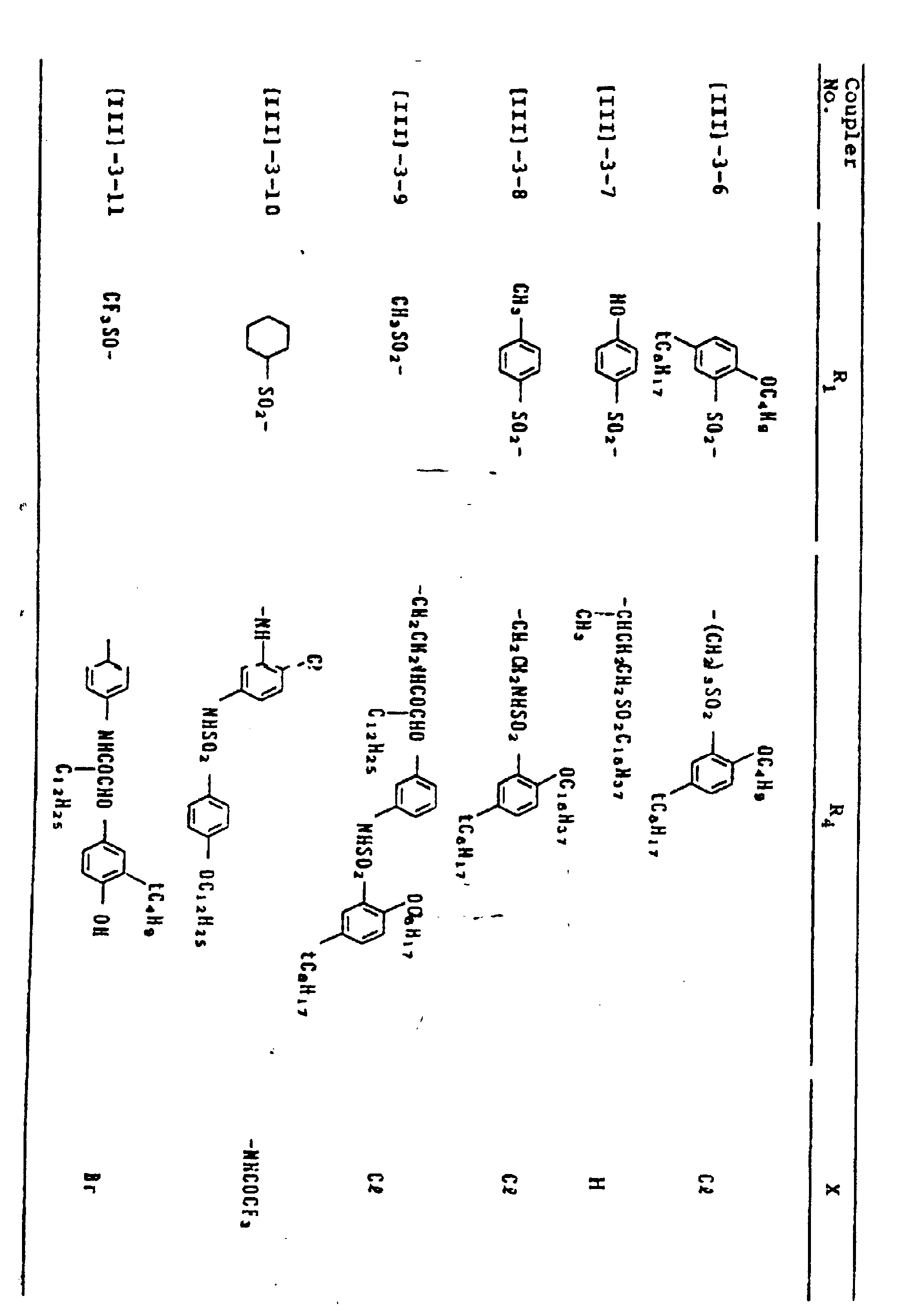

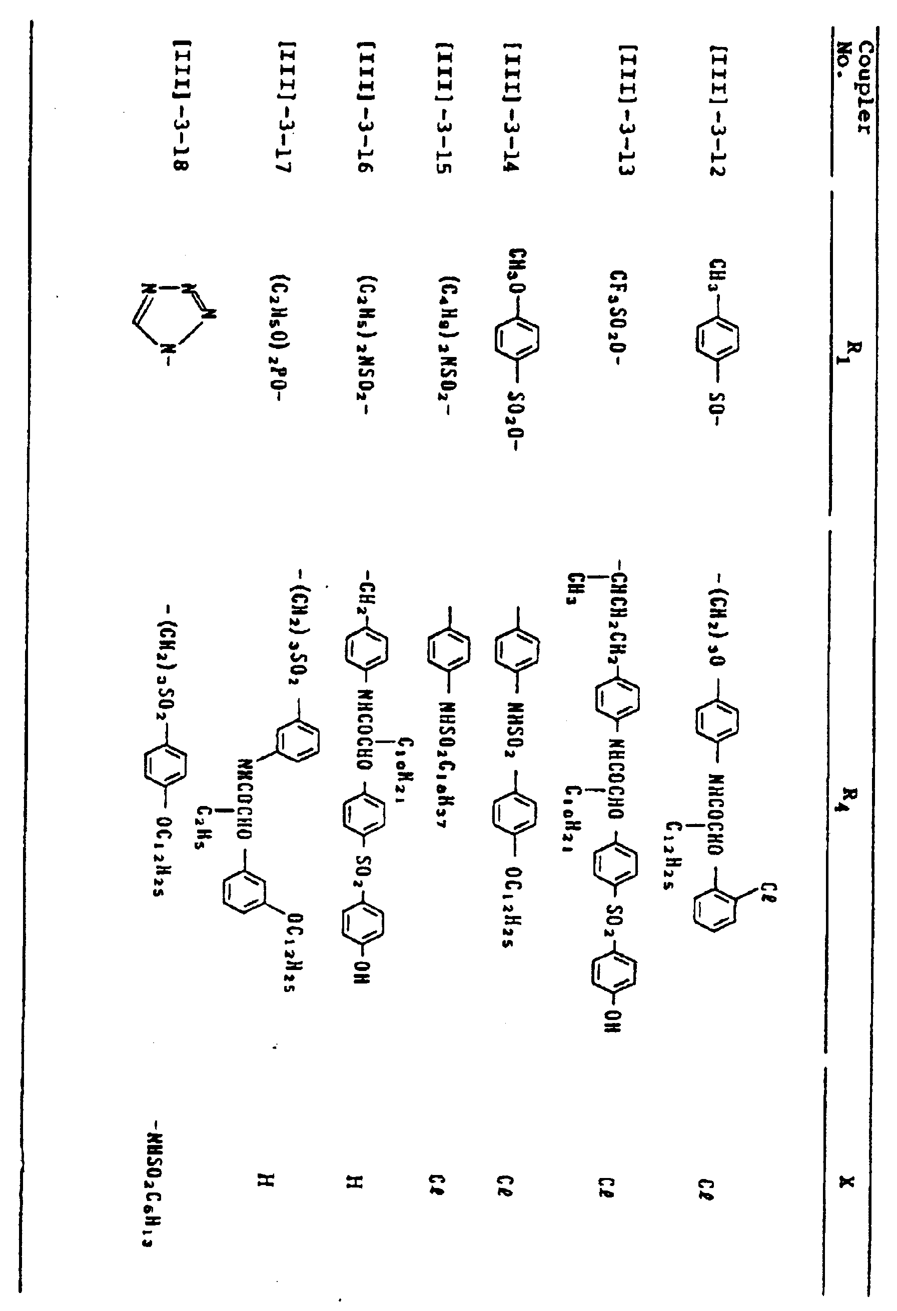

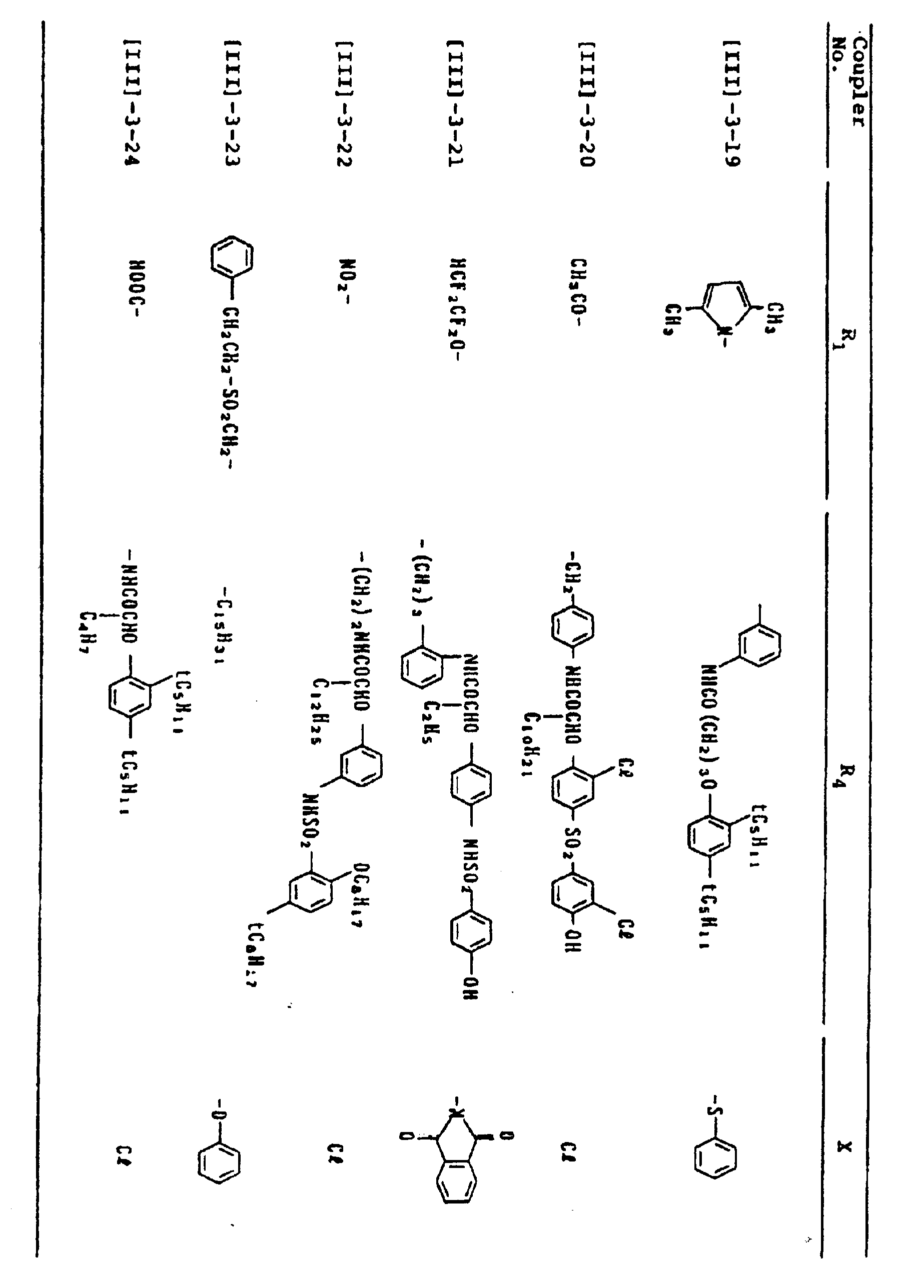

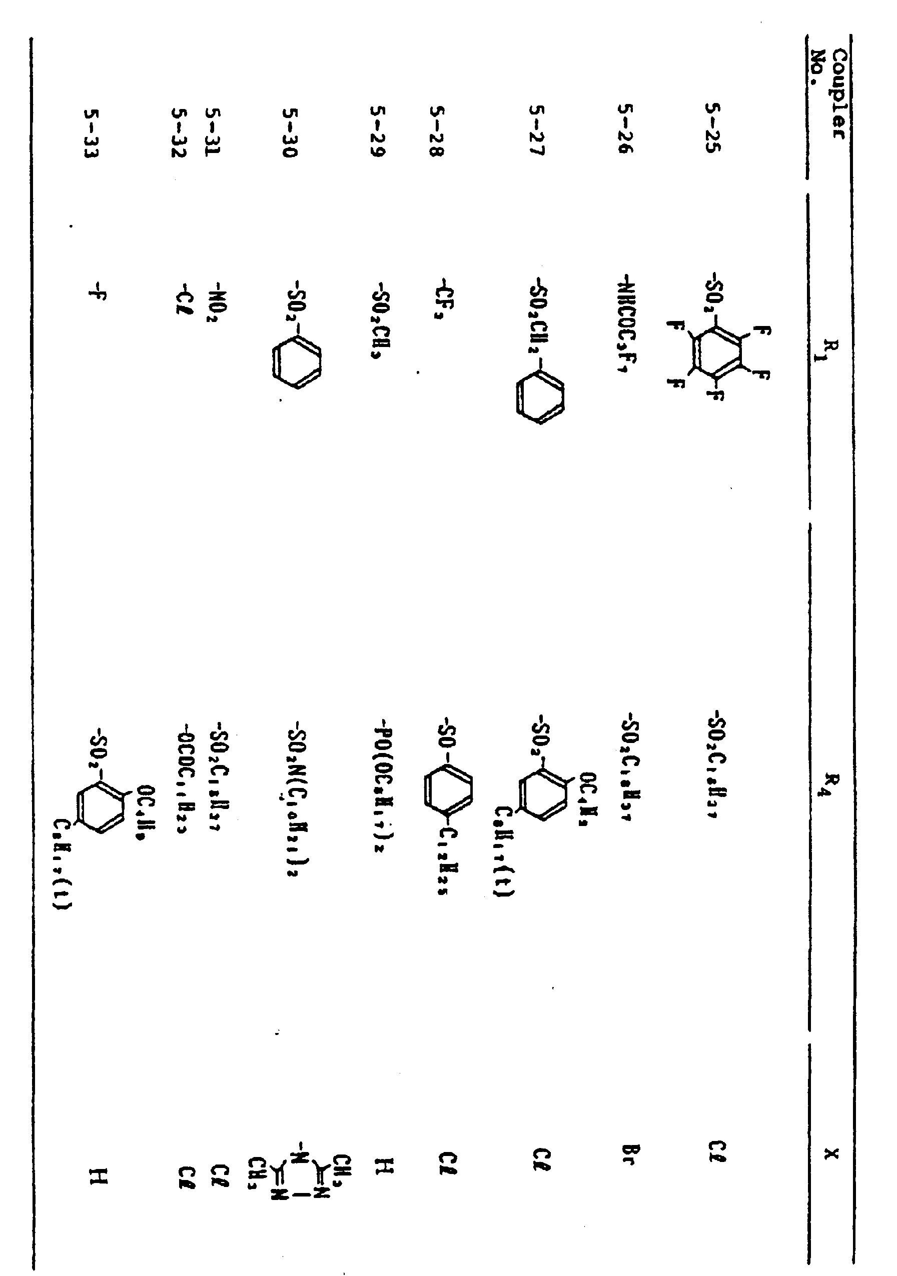

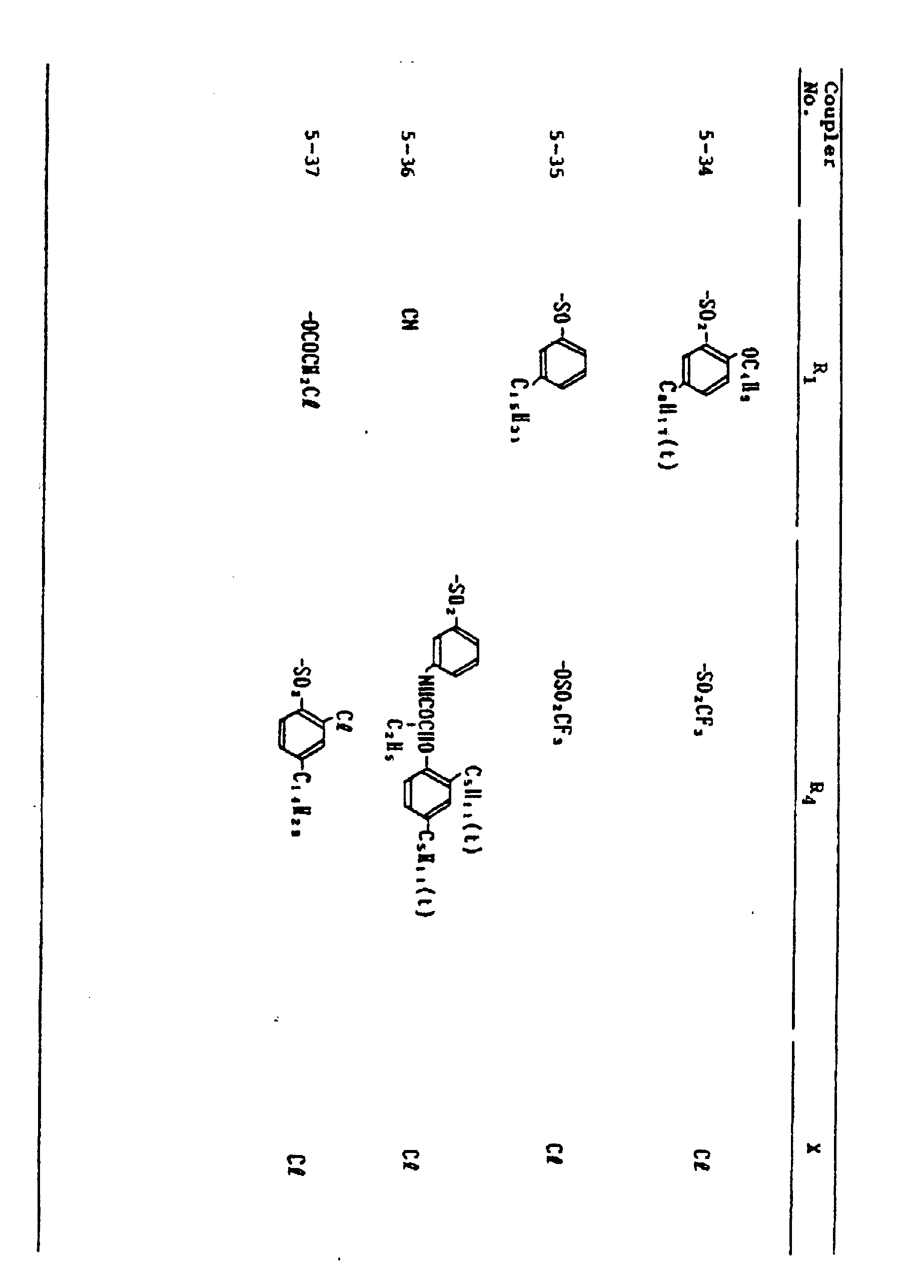

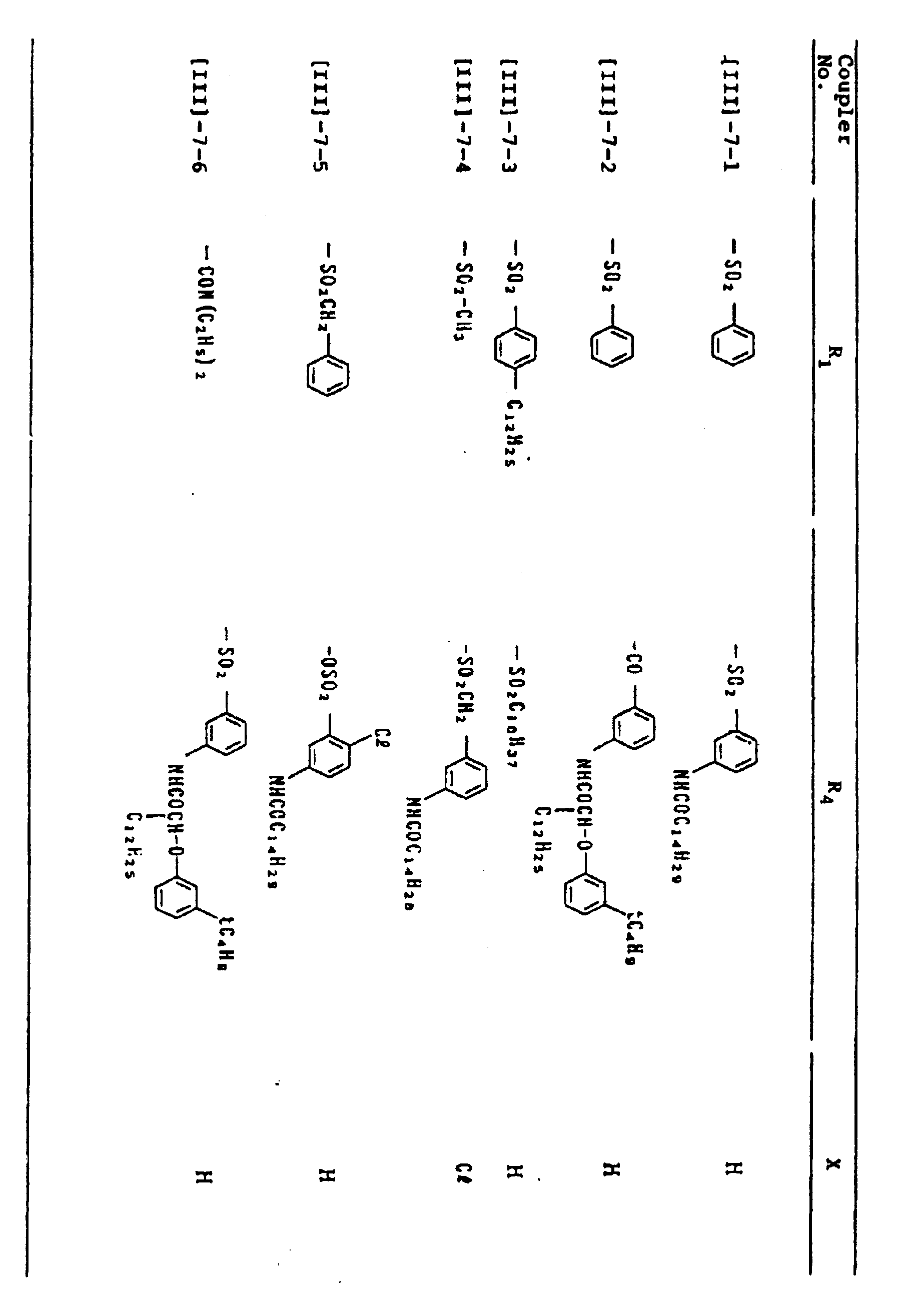

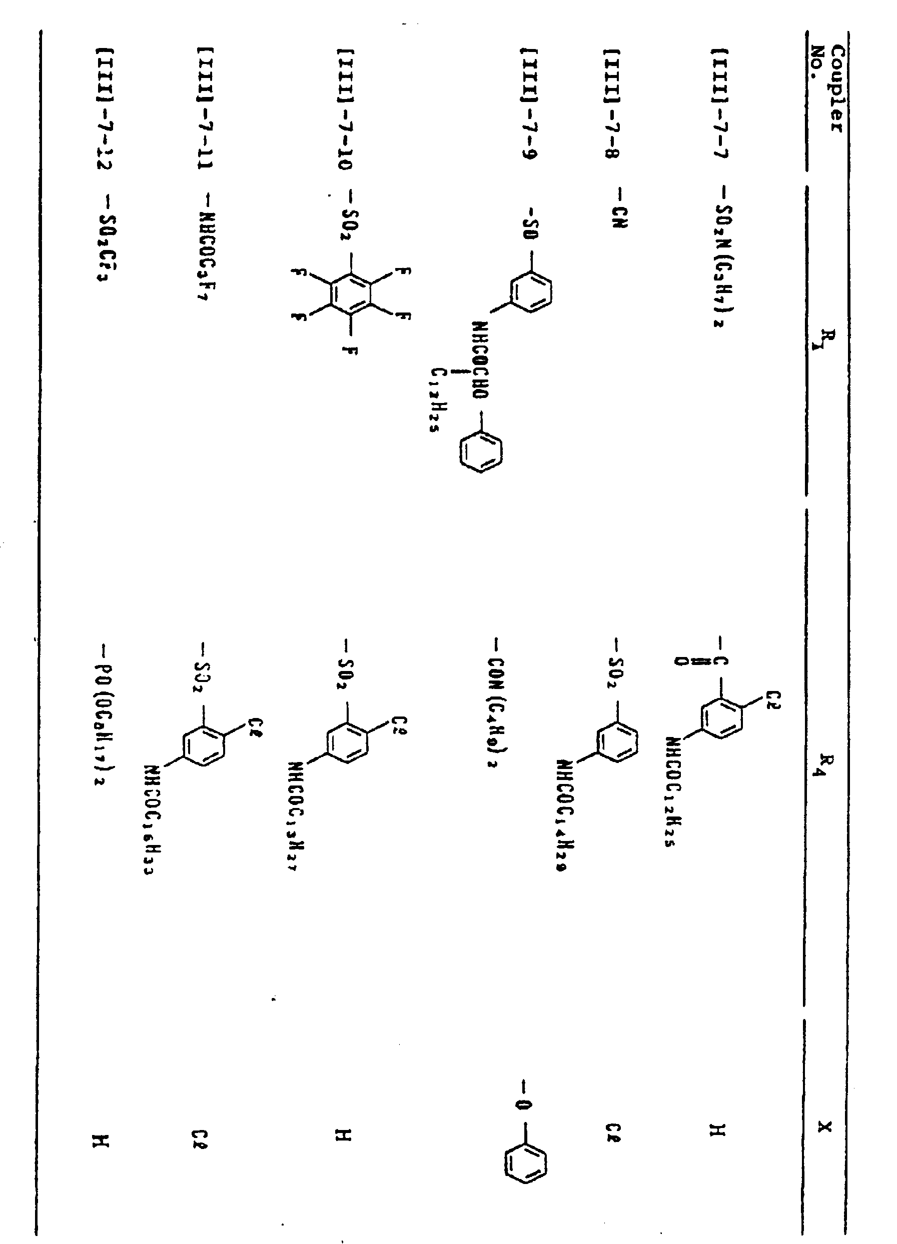

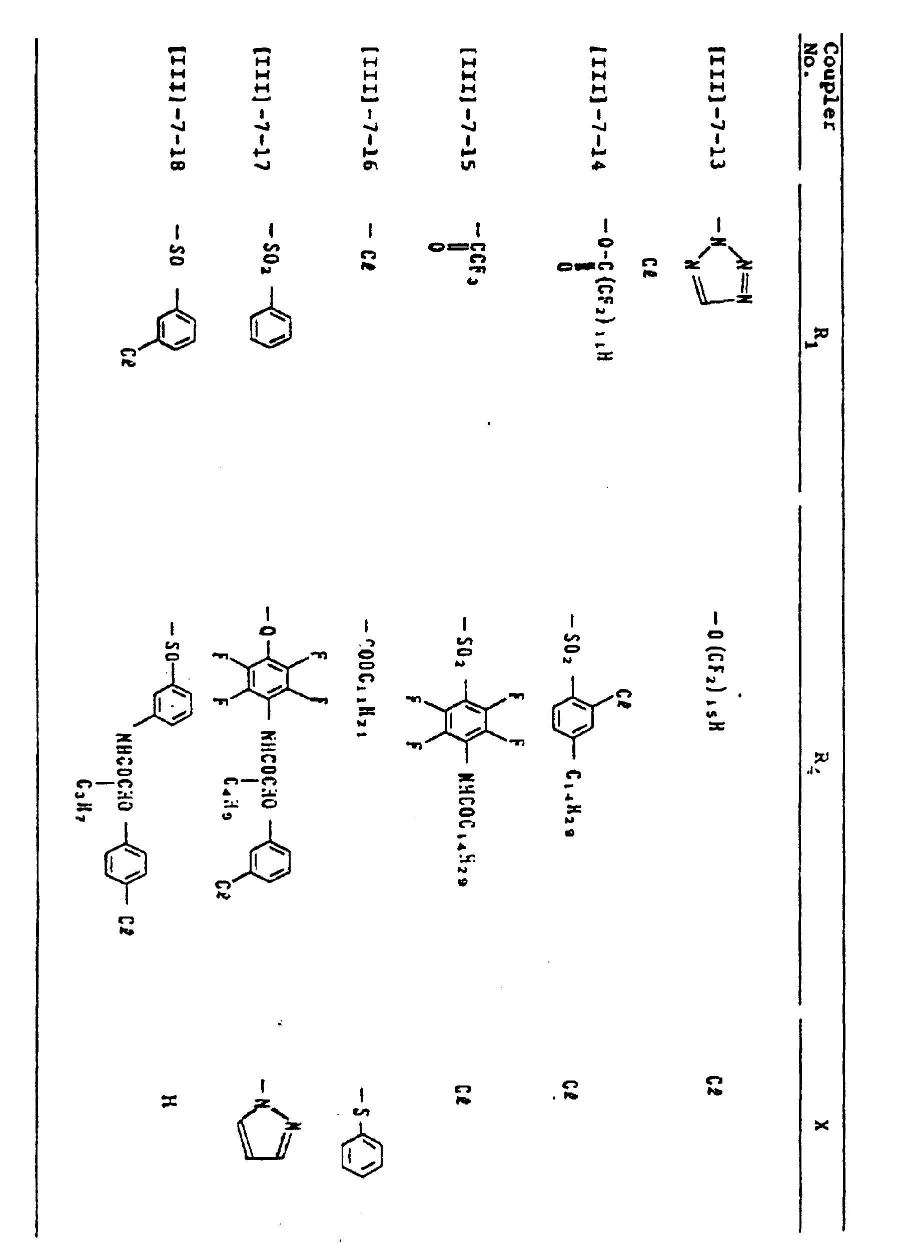

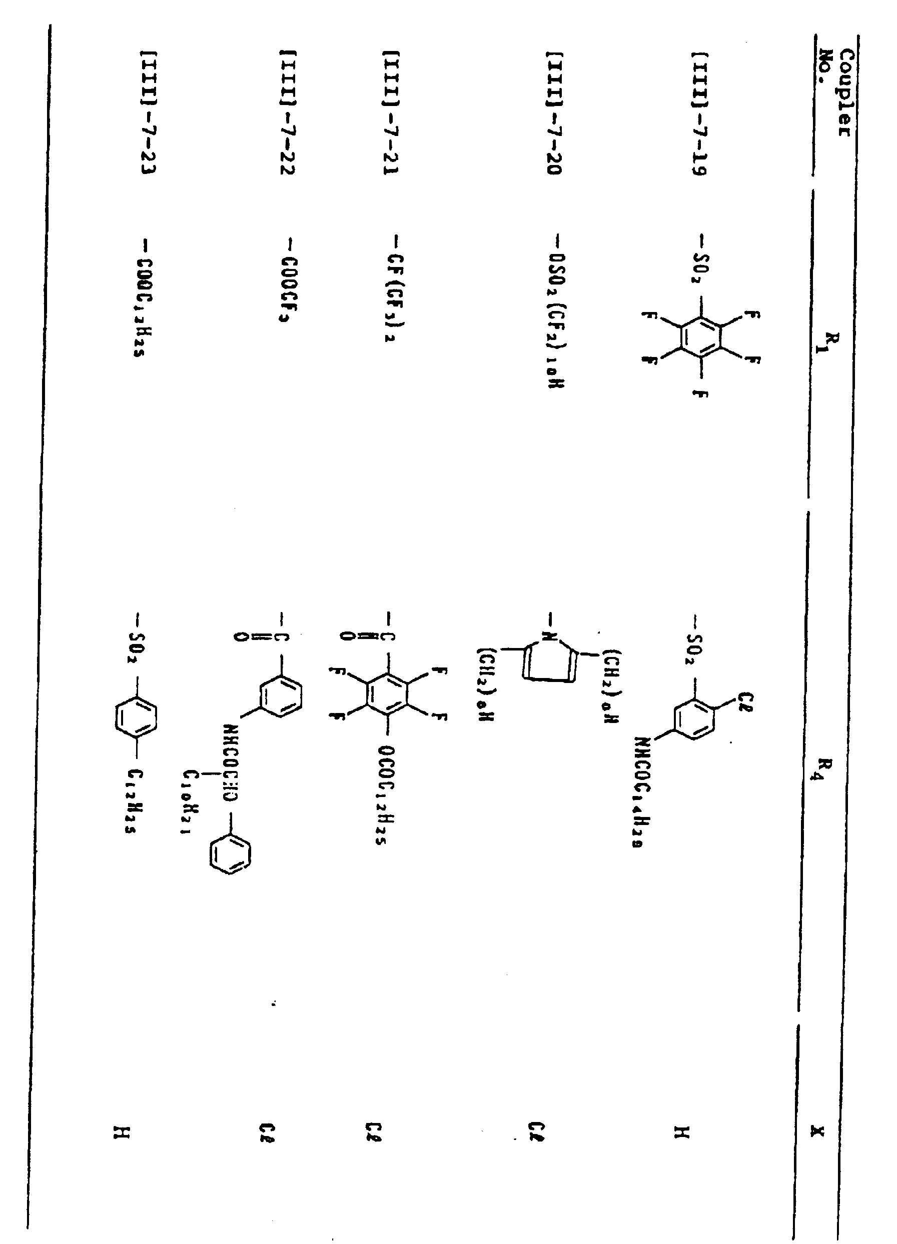

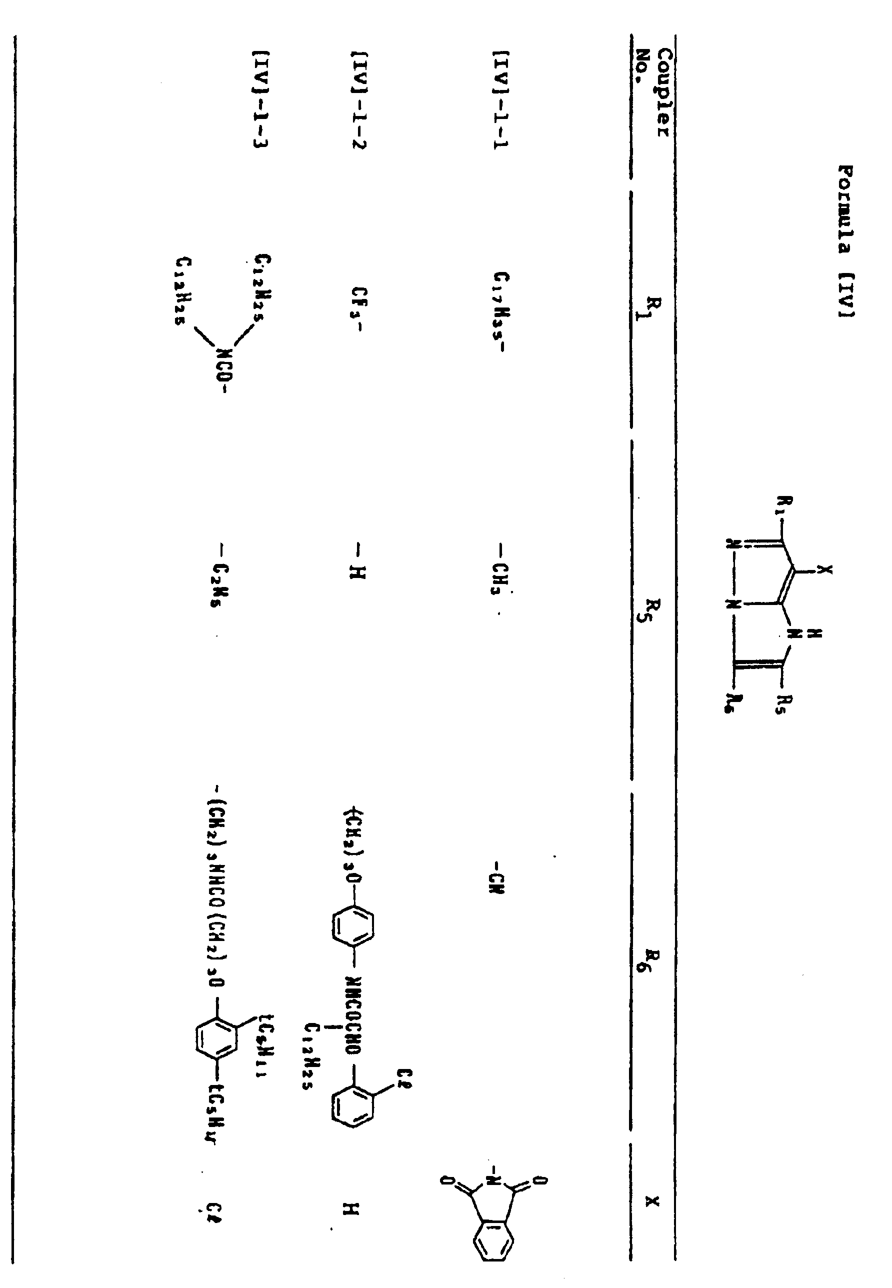

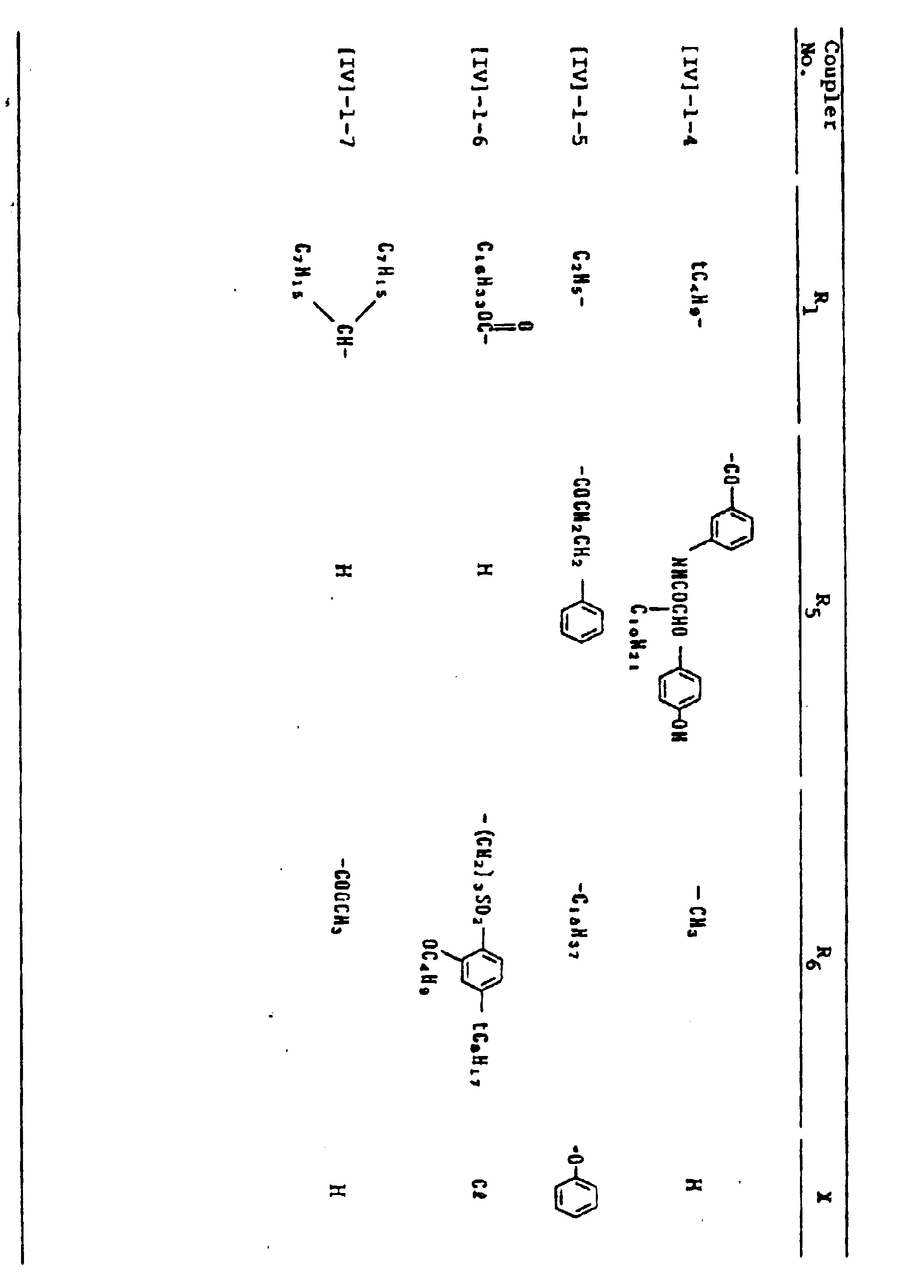

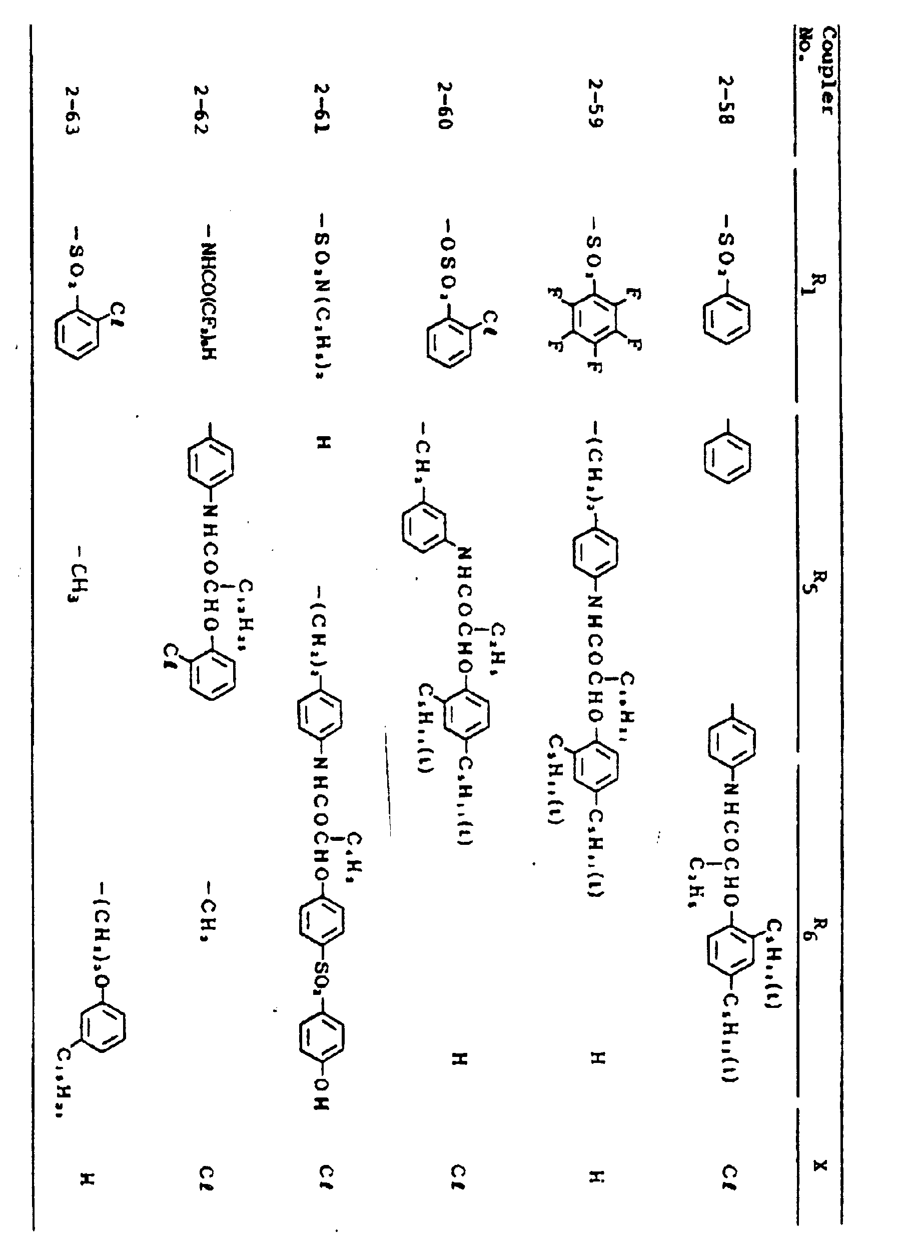

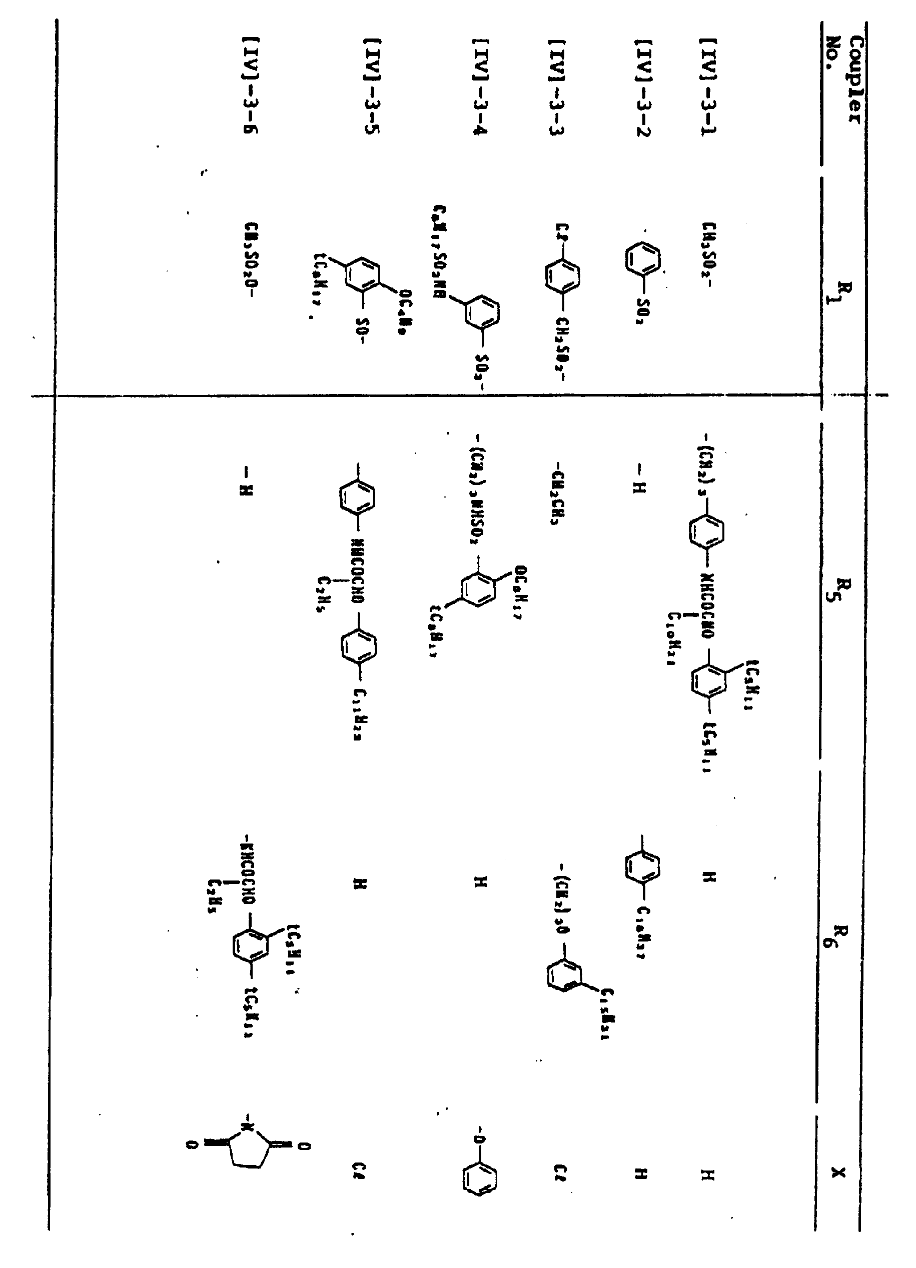

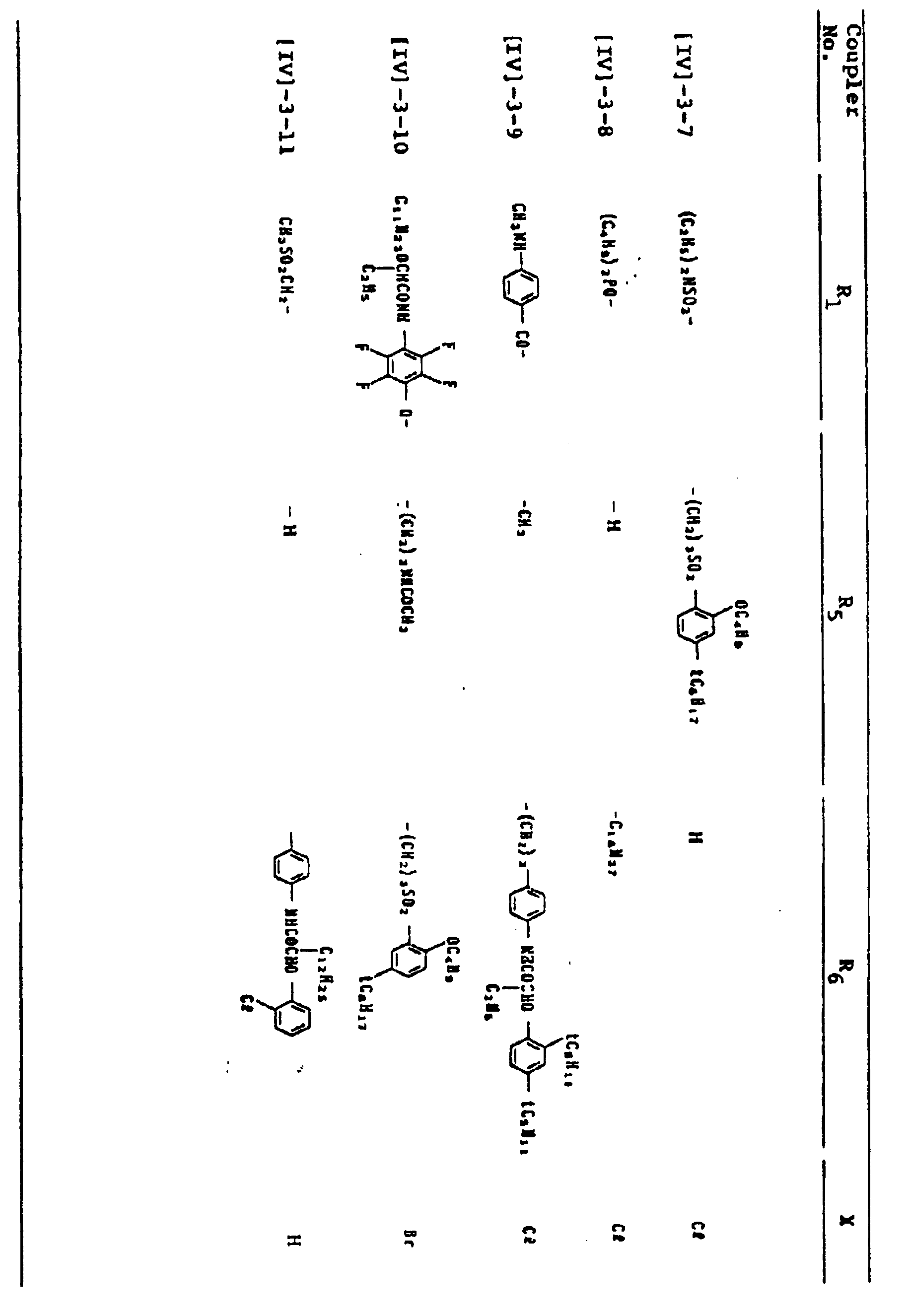

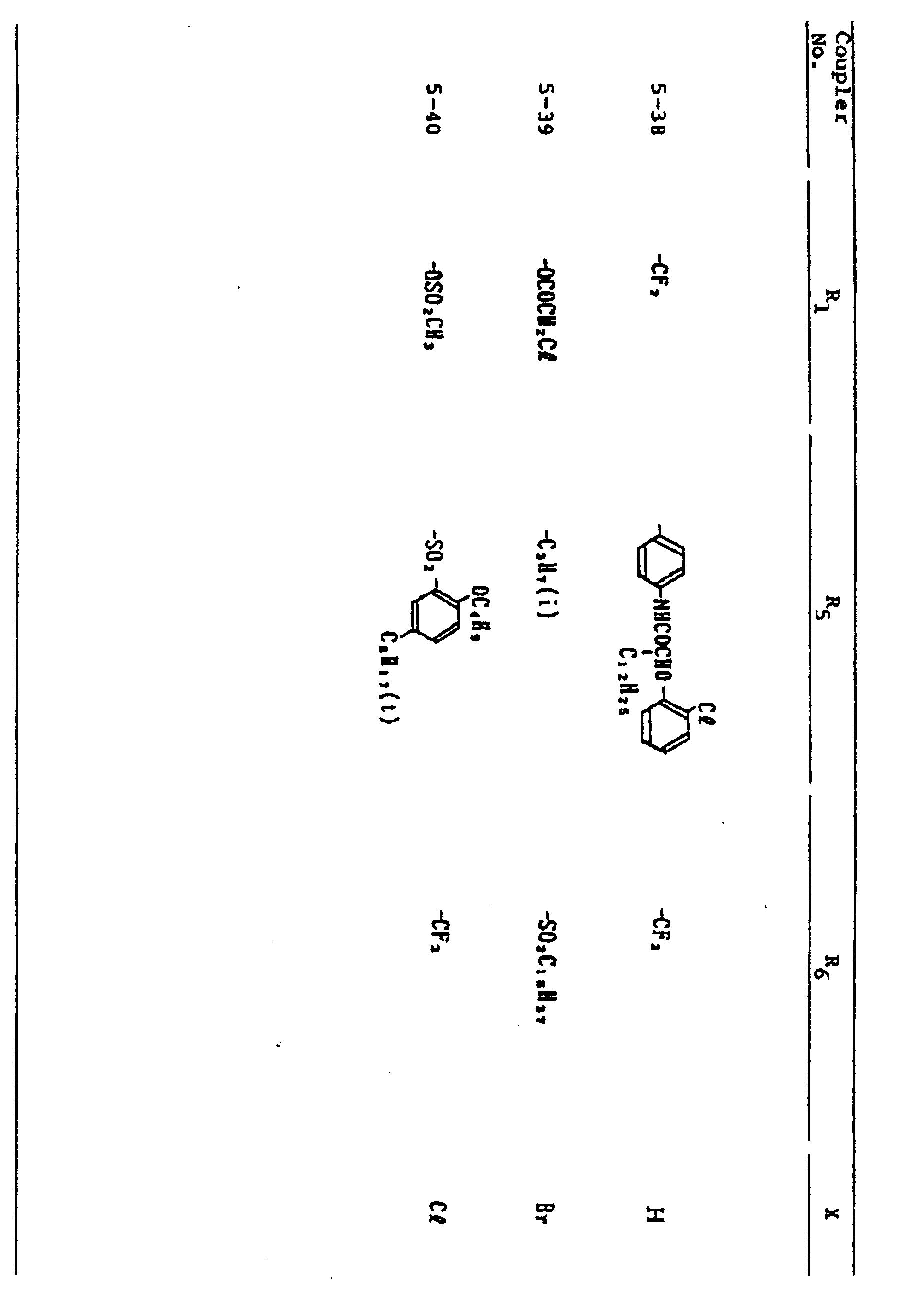

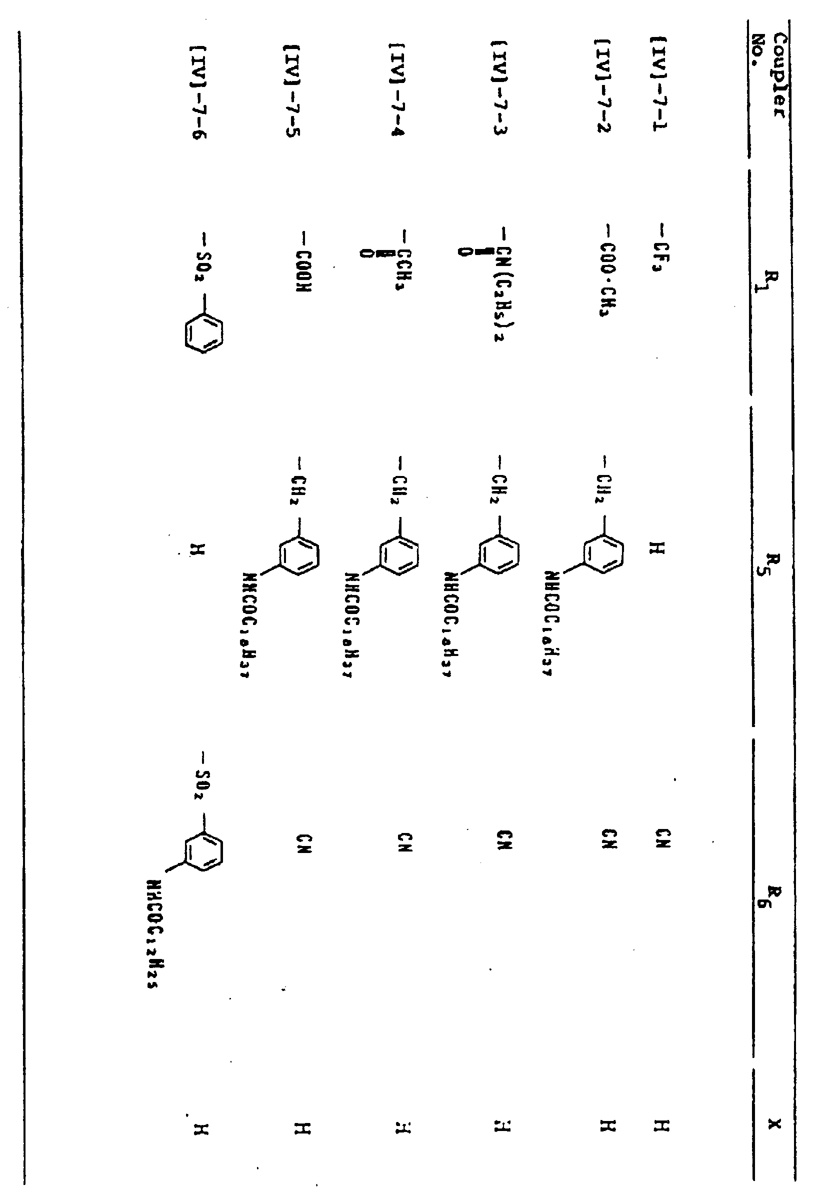

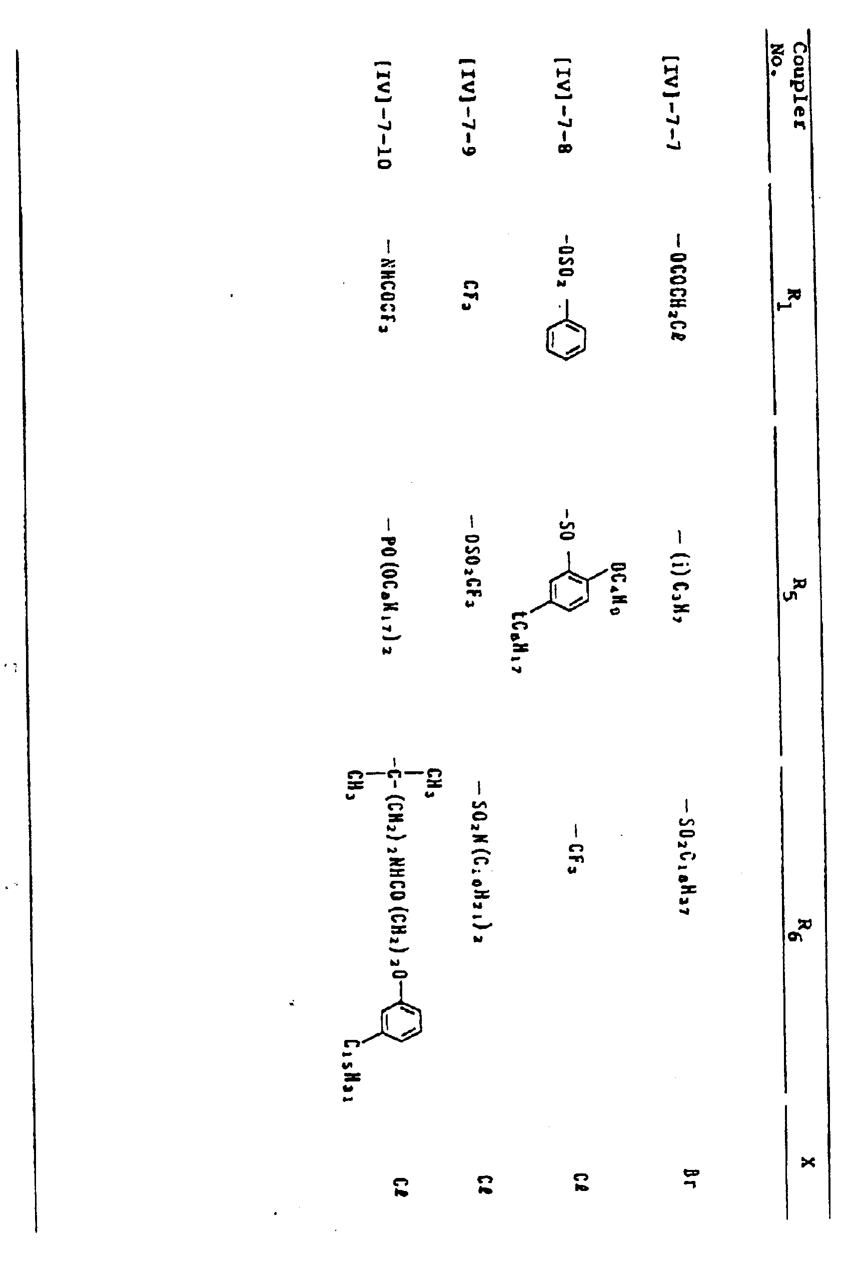

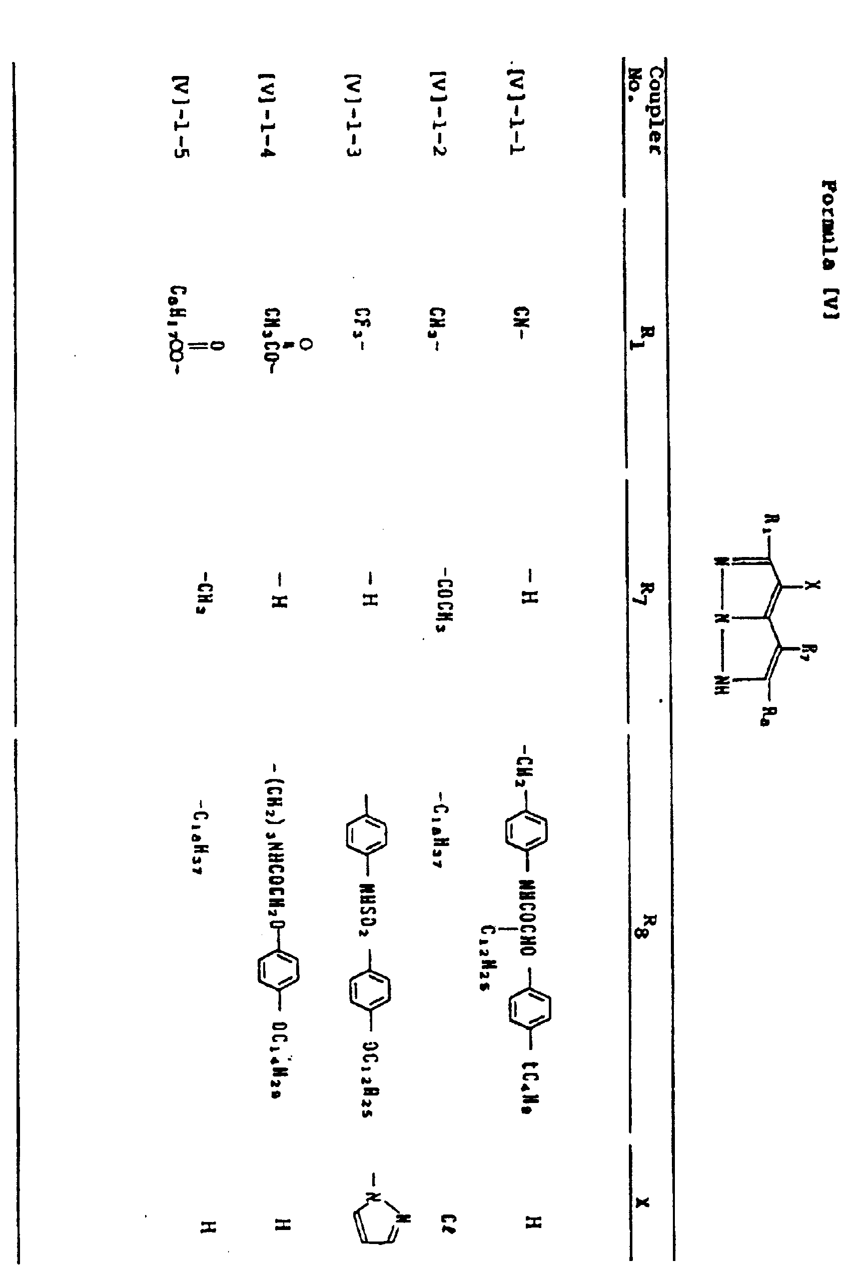

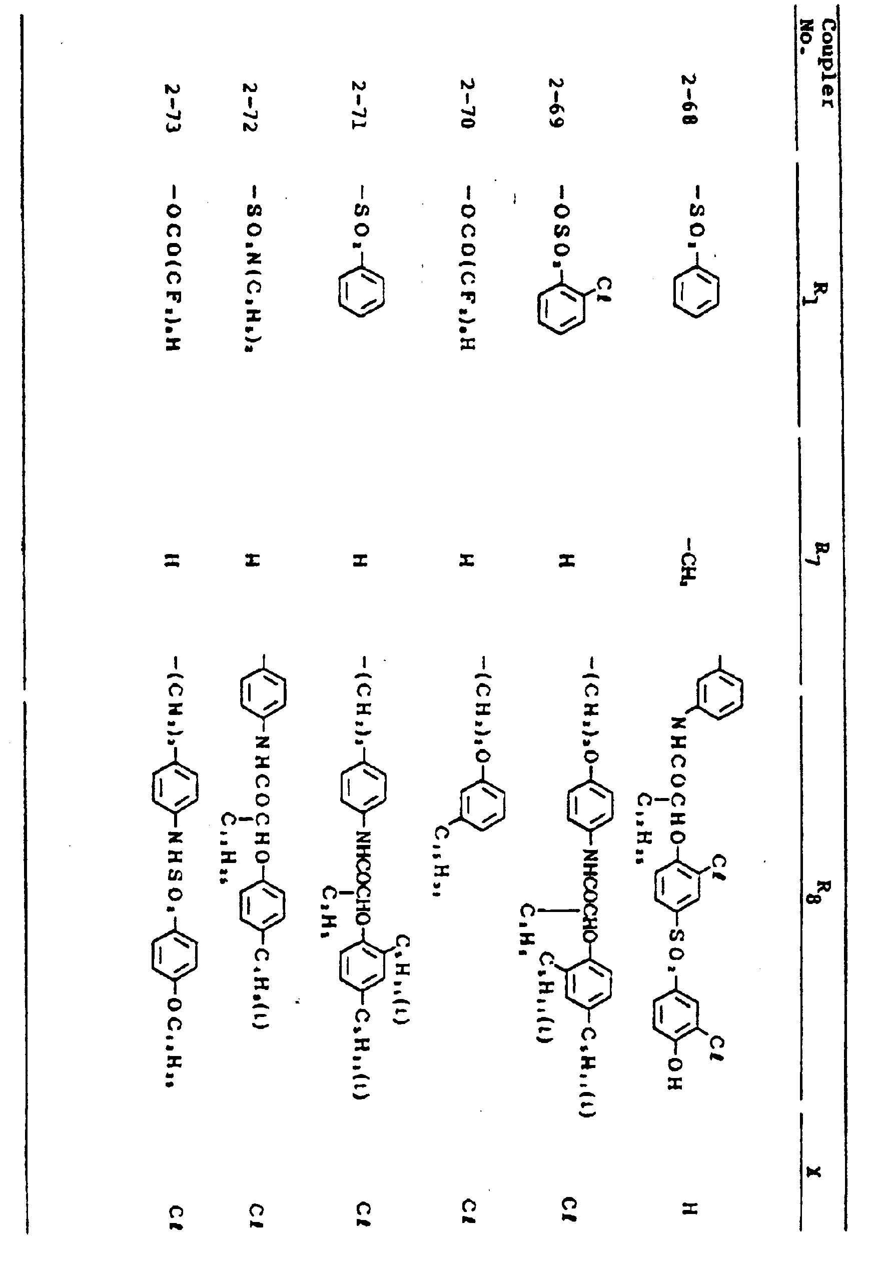

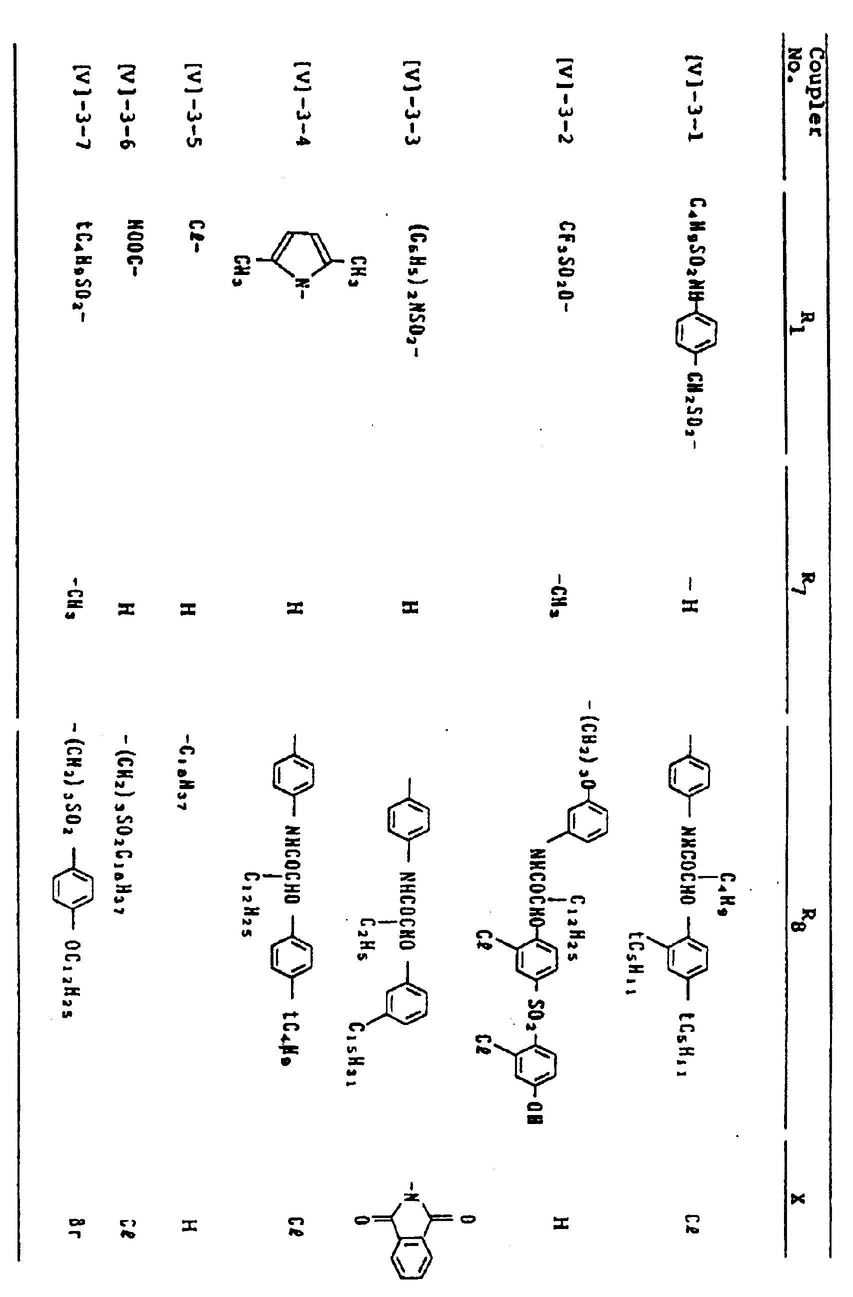

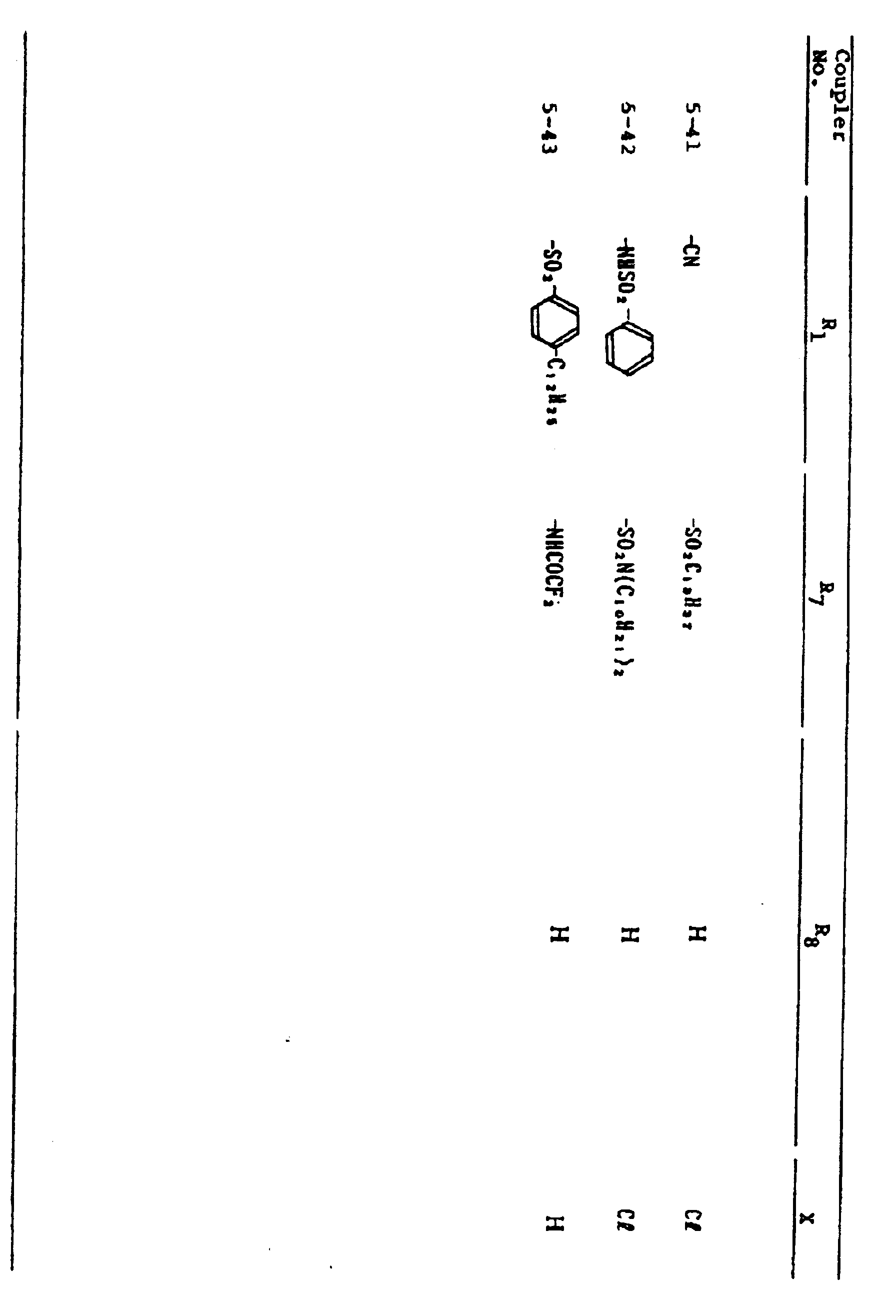

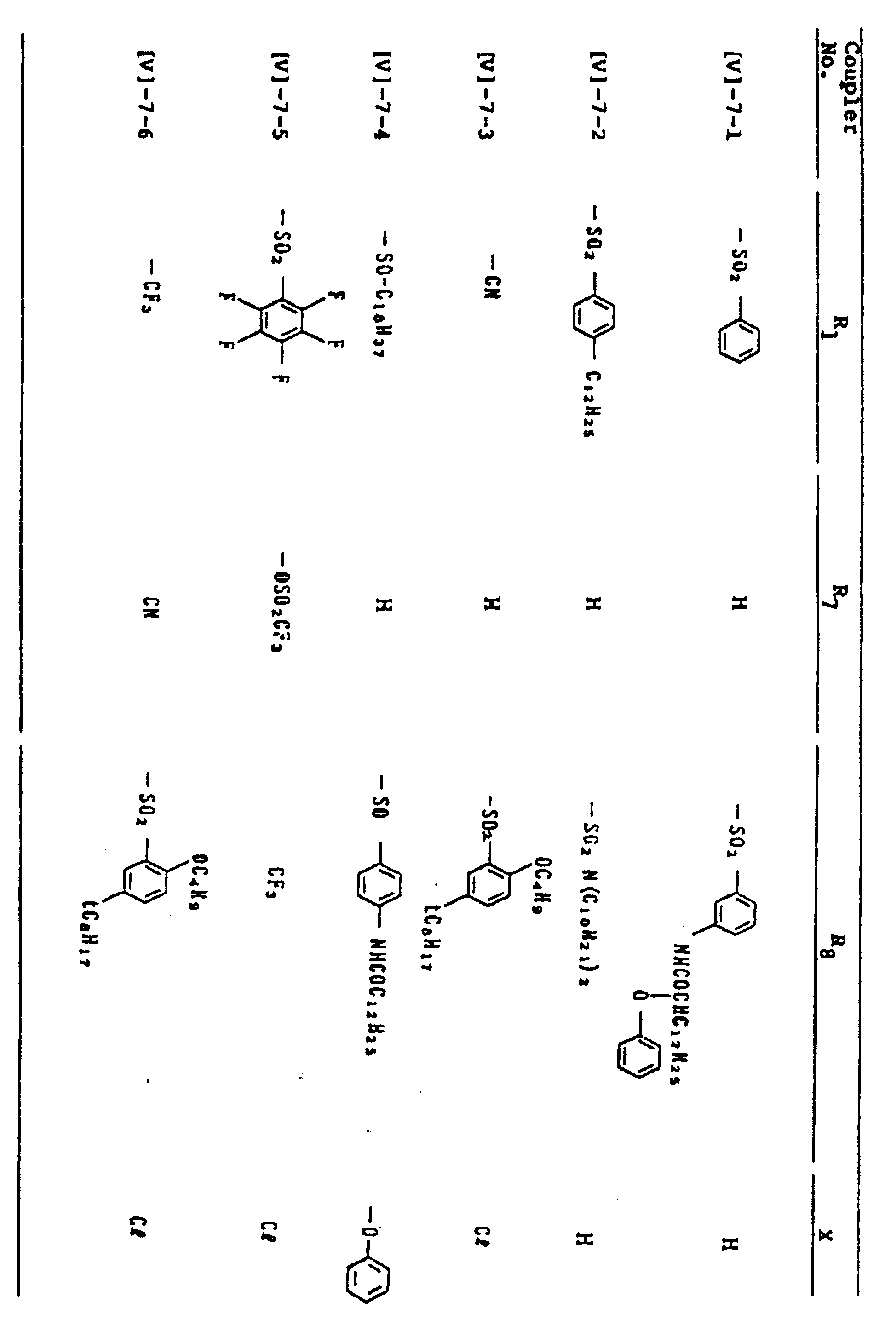

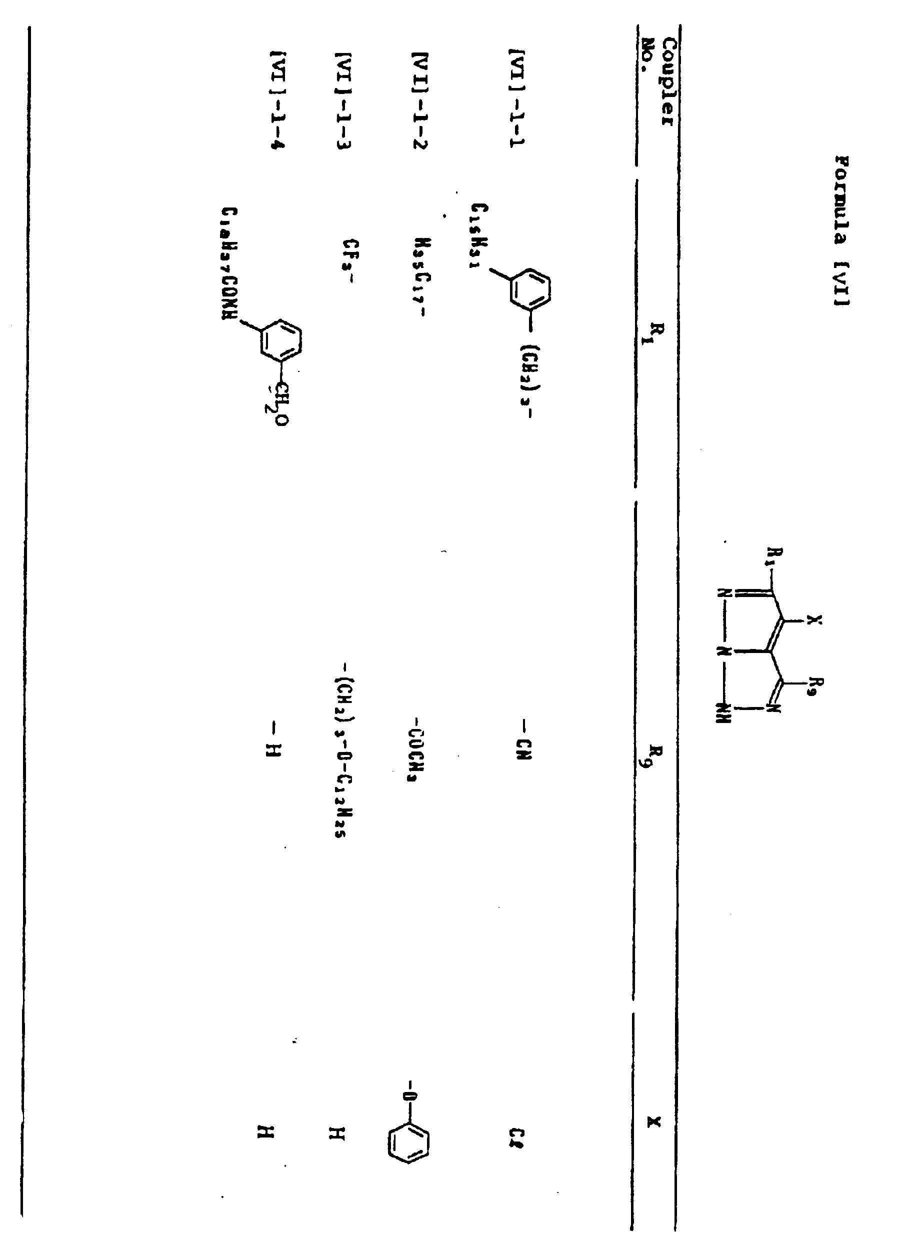

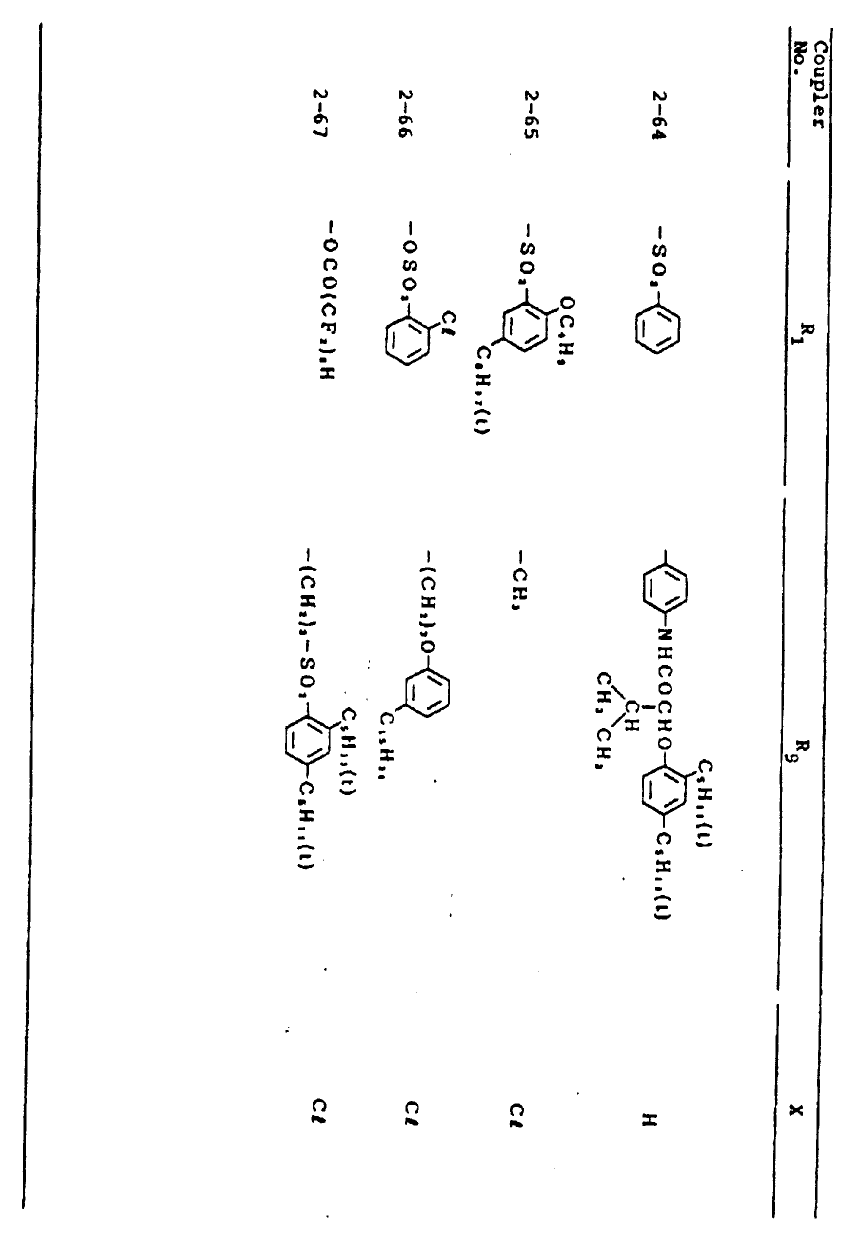

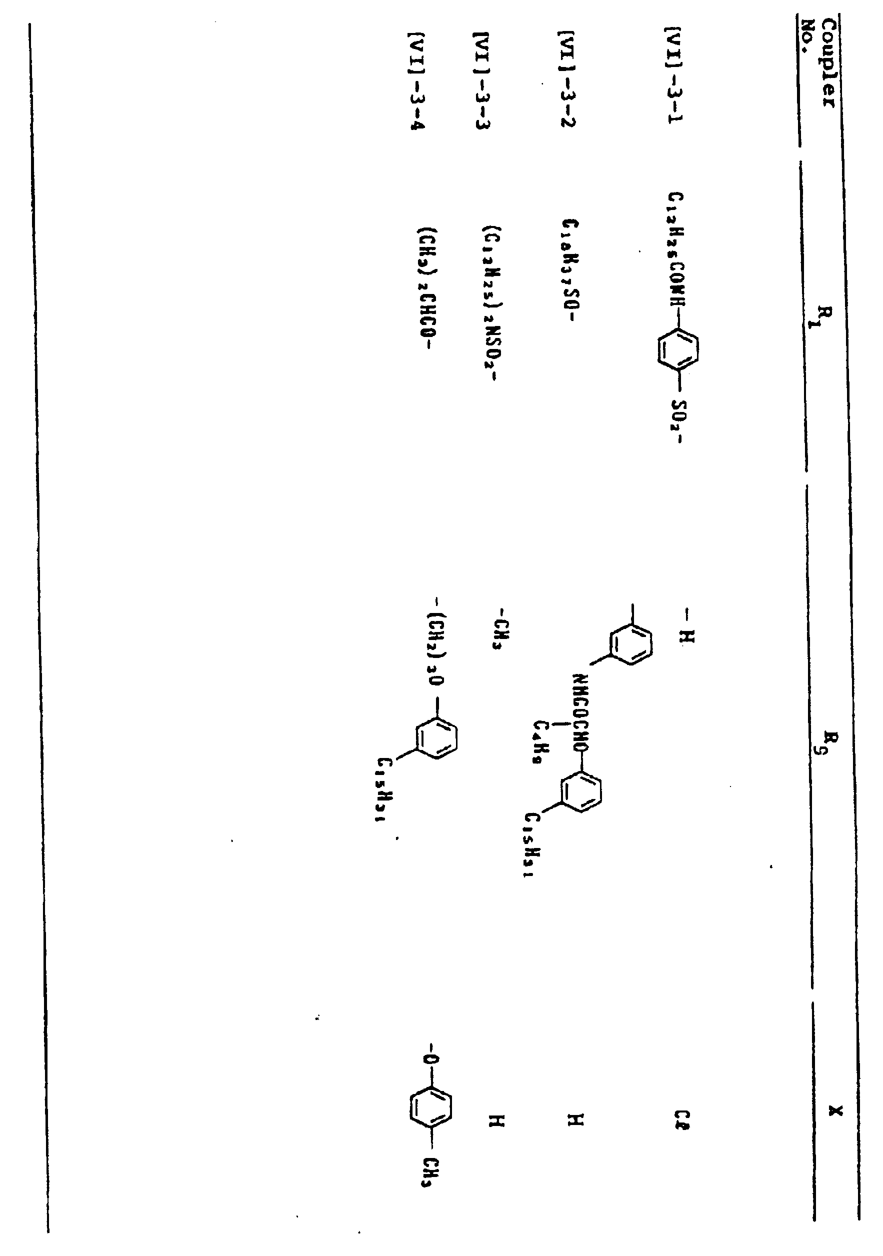

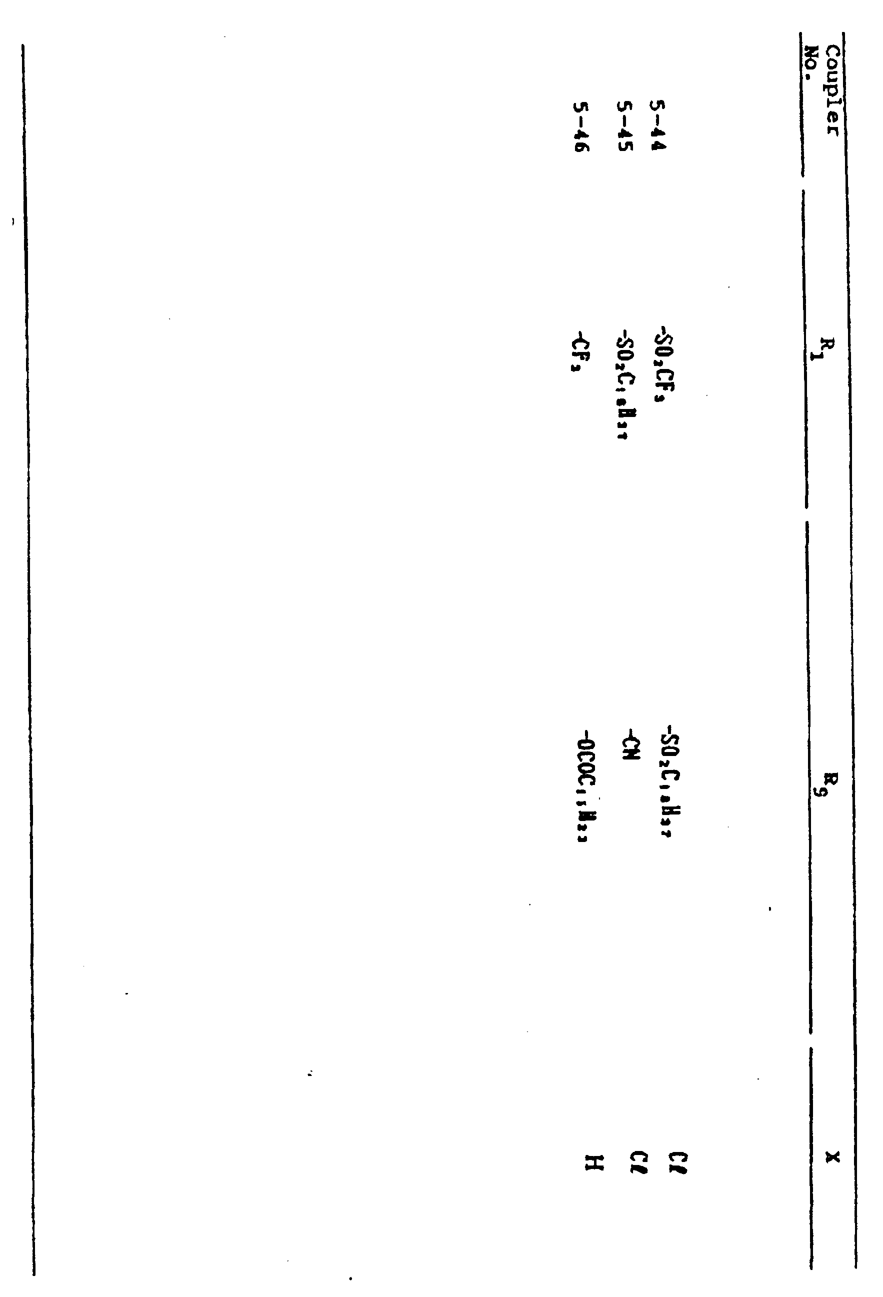

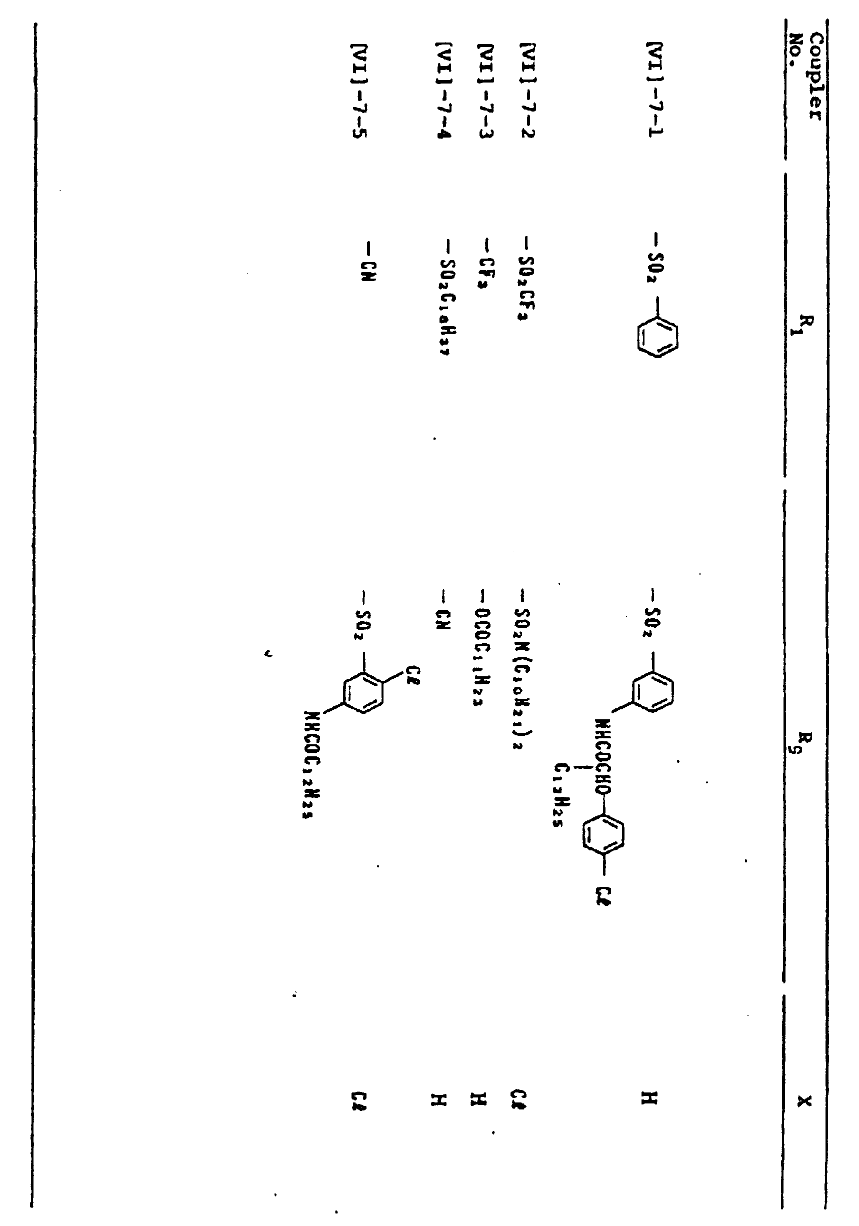

The above-mentioned cyan couplers may readily be synthesized by anybody who is skilled in the art, with reference to, for example, Journal of the Chemical Society, Perkin I, 1977, pp. 2047-2052; J. Heterocycl. Chem., 11 , p.423, 1974; Chem. Ber., 32 p.797, 1899 and 95 , p.2861 and p.2881, 1962; U.S. Patent Nos. 3,705,896 and 3,725,067; Japanese Patent O.P.I. Publication Nos. 99437-1984, 42045-1983, 162548-1984, 171956-1984, 33552-1985, 43659-1985, 172982-1985, 190779-1985, 197688-1985 and 65247-1986; Japanese Patent Publication Nos. 43947-1971 and 43977-1971; and Japanese Patent Publication Nos. 120054-1986 and 122450-1986.

Typical examples of synthesizing cyan couplers are given below.

### Synthesis Example 2-1

The reaction scheme is:

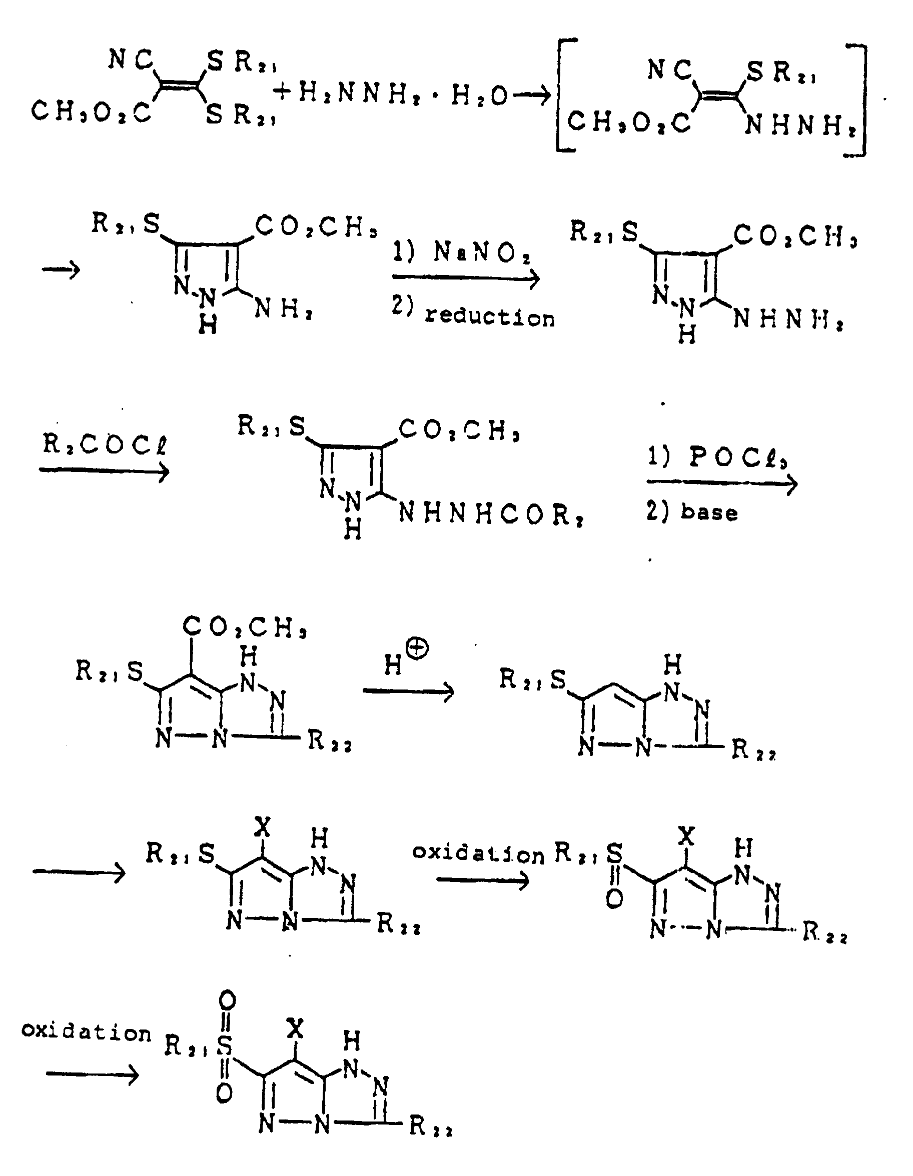

In the above scheme, R₂₁, R₂₂ and X are synonymous with those given in Formula [I].

X can be introduced by the method described in Japanese Patent Publication No. 43947-1971.

Synthesis Example 3-1 (Synthesis of Cyan coupler II-3-1

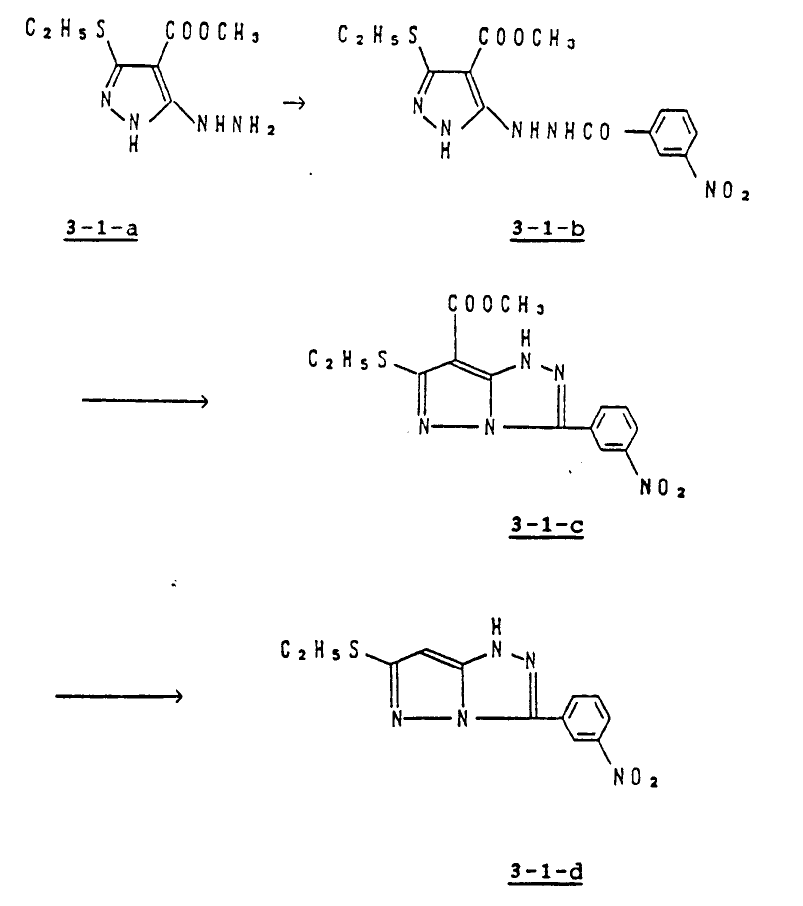

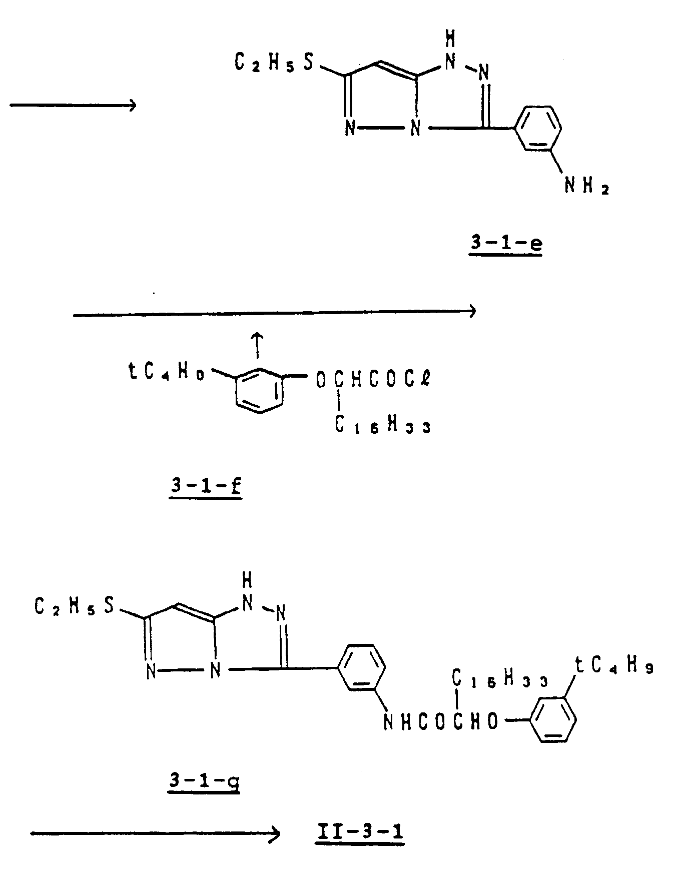

3-1-a was synthesized in the method described in Japanese Patent Application No. 120054-1986.

### [ 3-1-a → 3-1-b ]

0.01 mole of 3-1-a was dissolved in 50 ml of acetonitrile and 0.012 moles of m-nitrobenzoyl chloride were then added thereto. 0.1 mole of triethylamine was dropped into the resulted solution. After stirring for 3 hours at room temperature, the resultant crystals were filtered out. Thus, 0.0072 moles of 3-1-b were obtained.

### [ 3-1-b → 3-1-c ]

0.0072 moles of 3-1-b and 0.0095 moles of phosphorus oxychloride were added to 70 ml of toluene and the resultant solution was refluxed for 3.5 hours. After the toluene was distilled off, 0.1 mole of pyridine and 50 ml of acetonitrile were added and the resultant solution was refluxed for 2.5 hours. Afterfiltering while still warm, 0.0041 moles of 3-1-c were obtained.

### [ 3-1-c → 3-1-d ]

0.0041 moles of 3-1-c was added to a solvent comprising 25 ml of acetic acid, 7 ml of sulfuric acid and 0.8 ml of water. The resultant solution was refluxed for 1.5 hours.

The resultant matter was neutralized with an aqueous sodium hydroxide solution and, after extraction and condensation, water was added. Thus, 0.0030 moles of 3-1-d were filtered out.

### [ 3-1-d → 3-1-e → 3-1-g ]

0.0030 moles of 3-1-d were dissolved in 50 ml of THF and the resultant solution was hydrogenated using Pd/C. After the Pd/C was filtered out the solvent was distilled off. The resulting deposited matter 3-1-d was dissolved in 100 ml of acetonitrile and 0.0052 moles of 3-1-f were added 0.004 moles of triethyleamine were dropped into the resulting solution. After stirring for 2 hours at room temperature, the deposited crystals were filtrated and recrystallized with ethyl acetate, so that 0.0024 moles of 3-1-g were obtained.

### [ 3-1-g → 11-3-1

0.0024 moles of 3-1-g were dissolved in 15 ml of acetic acid and 5 ml of an aqueous 35% hydrogen peroxide solution was then dropped in gradually. After stirring for 2.5 hours at 55°C, 50 ml of water were added and the resulting solution was neutralized gradually with an aqueous sodium hydroxide solution. It was then extracted and distilled with ethyl acetate. The deposited matter thus obtained thereby was recrystallized with acetonitrile. Thus, 0.0019 moles of the white needle crystals 11-3-1 were obtained.

Synthesis Example 3-2 (Synthesis of II-3-18 )

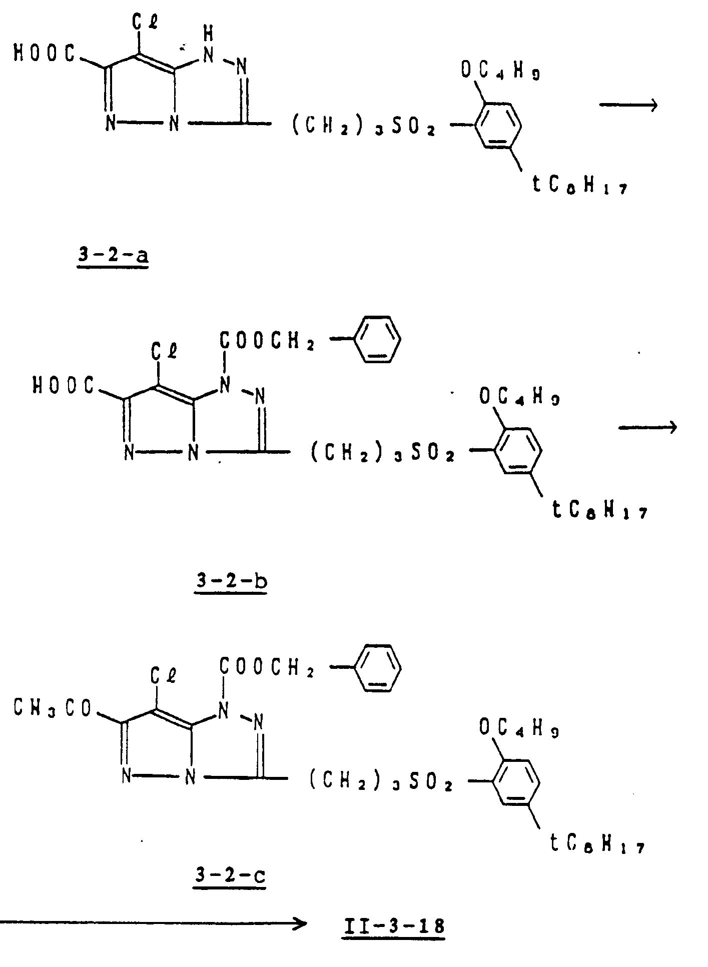

3-carboxy-4-chloro-5-aminopyrazole was used as the raw material and 3-2-a was synthesized in an ordinary method via a hydrorazine substance through a reaction with an acid chloride.

### [ 3-2-a → 3-2-b ]

While 3-2-a of 0.01 mole and 5 ml of an aqueous 2N-sodium hydroxide solution were kept cool with ice and stirred in 20ml of acetonitrile, 0.01 mole of benzyloxy carbonyl chloride and 3 ml of an aqueous 4N-sodium hydroxide solution were gradually dropped thereinto and the resulted solution was stirred for 30 minutes. After neutralizing with chloric acid, the neutralized matter was cooled and the precipitates were filtered out and washed with cooled water. Thus, 0.008 moles of 3-2-b were obtained.

### [ 3-2-b → 3-2-c ]

Twenty (20) ml of a benzene solution containing 0.008 moles of 3-2-b were added with stirring into 22 ml of an diethyl ether solution containing 1 N-methyl lithium and the resulting solution was stirred for 20 hours. After adding 100 ml of water with cooling, extraction was carried out using ethyl acetate, and the solvents were then distilled off. The deposited matter was recrystallized with acetonitrile, so that 0.0040 moles of 3-2-c were obtained.

### [3-2-c → 11-3-18 ]

0.0040 moles of 3-2-c were dissolved in 60 ml of THF and a reduction was made with Pd/C.

After the Pd/C was removed and the solvents distilled off, the resultant matter was recrystallized with ethyl acetate, so that 0.0030 moles of light-yellowish needle crystals of 11-3-18 were obtained.

Synthesis Example 3-3 (Synthesis of III-3-1)

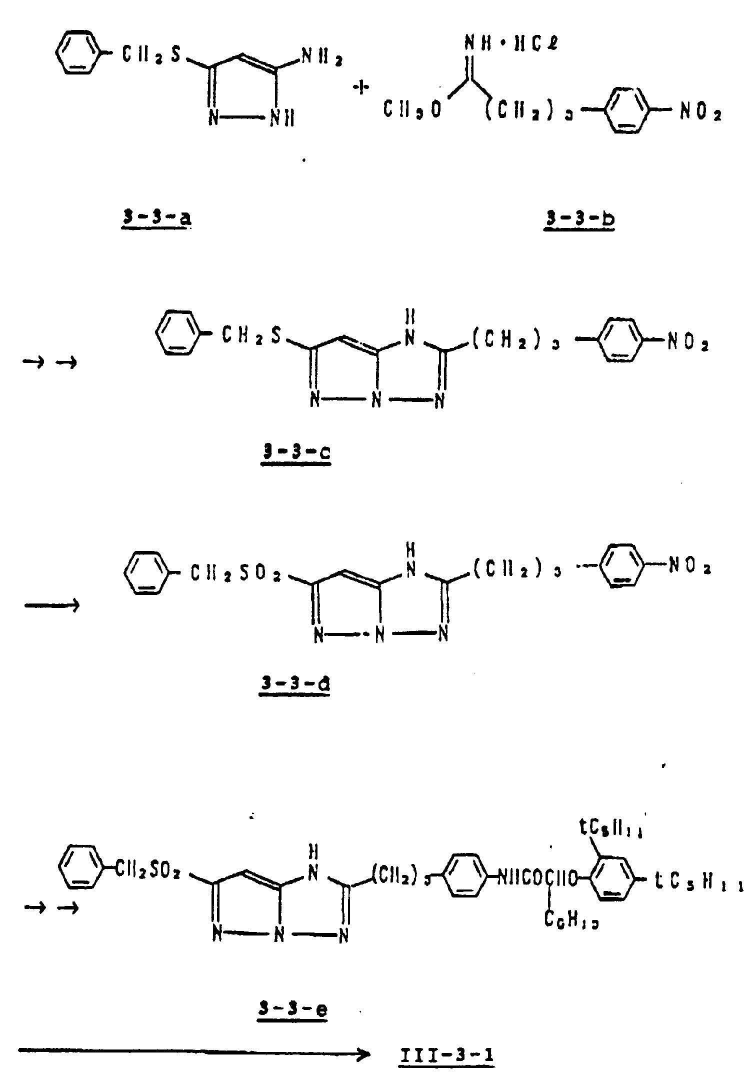

3-3-a was synthesized in an ordinary method with reference to Japanese Patent Application No. 120054-1986.

### [Synthesis of 3-3-c ]

In 20 ml of toluene, 0.01 mole of 3-3-a and 0.011 moles of 3-3-b were refluxed for 20 hours and the toluene was then distilled off. The residues were dissolved in 20 ml of methanol and a methanol solution containing hydroxylamine was added at 0 °C. The resultant solution was stirred for 1.5 hours at room temperature and was then poured into 200 ml of water. The deposited matter was then filtered out and dissolved in 60 ml of THF. The resultant solution was added to 0.004 moles of triethylamine and stirred.

While stirring, a THF solution containing 0.7 g of p-toluene-sulfonic acid chloride was added and further stirred. The insoluble matter was then filtered out and the filtrate was refluxed for 7 hours in a nitrogen atmosphere so that the THF was distilled off. The residues were put into a small amount of 50 ml of methanol, so that 0.0045 moles of 3-3-c were obtained.

### [ 3-3-c → 3-3-d ]

of 0.0045 of 3-3-c moles were oxidized in the same manner as in [ 3-1-g] → II-3-1 ] of Synthesis Example 3-1, so that 0.0039 moles of 3-3-d were obtained.

### [ 3-3-d → 3-3-e ]

0.0039 moles of 3-3-d were hydrogenated and reduced in the same manner as in [ 3-1-d → 3-1-e → 3-1-g ] of Synthesis Example 3-1 and were further reacted with an appropriate acid chloride, so that 0.0031 moles of coarse crystals of 3-3-e were obtained.

### [ 3-3-e → III-3-1 ]

0.0031 moles of 3-3-e were dissolved in 50 ml of chloroform and 0.0050 moles of N-chlorosuccinimide were added thereto.

### The resultant solution was stirred for 30 minutes at room temperature and the solvents were distilled off. Recrystallization was carried out made with a mixed solvent of ethyl acetate and n-hexane, so that 0.0022 moles of white powdered crystals of III-3-1 were obtained.

Synthesis Example 3-4 (Synthesis of III-3-16 )

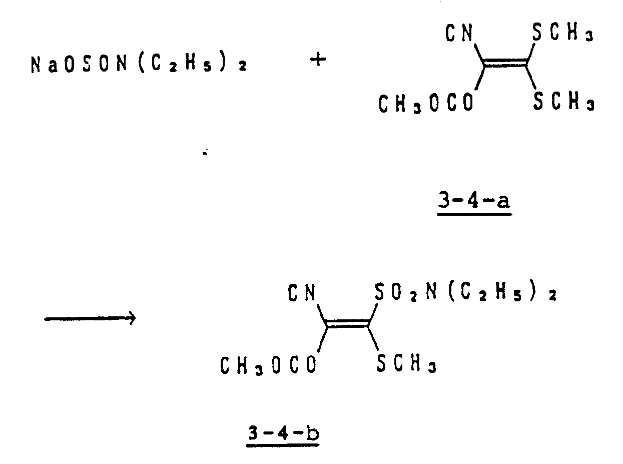

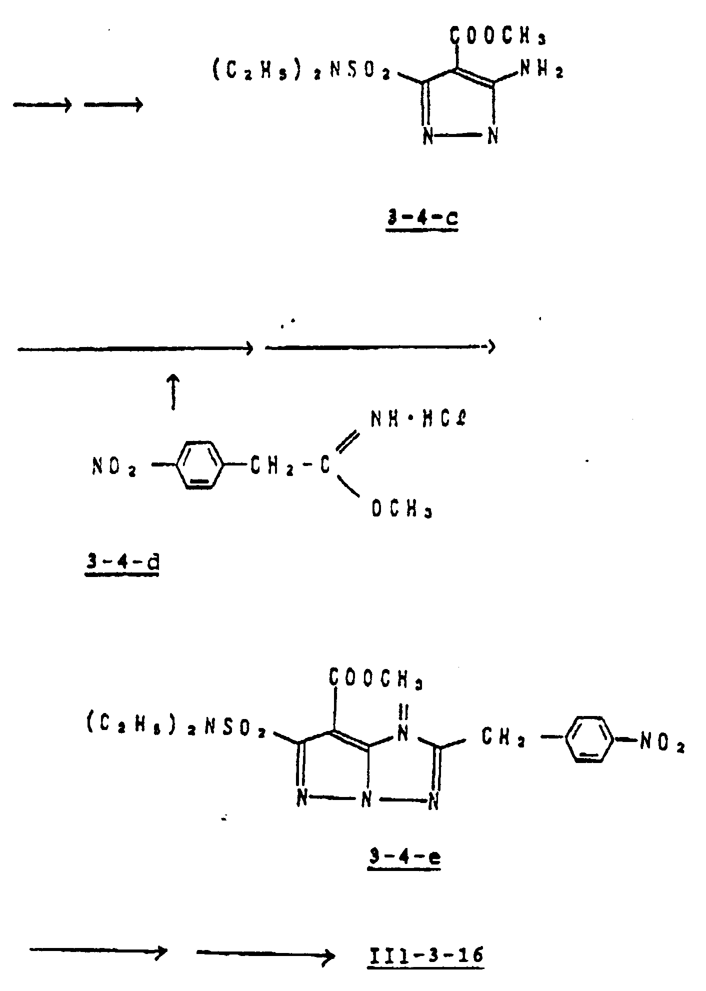

### [ 3-4-a → 3-4-b ]

0.01 mole of 3-4-a and 0.022 moles of sodium diethylamino sulfinate were dissolved in 50 ml of methanol and the resulting solution was refluxed for 3 hours. The solvent was distilled off and the insoluble matter was filtered out after adding acetic acid to the residue and the filtrates were condensed, so that 0.0061 moles of 3-4-b were obtained.

### [ 3-4-b → 3-4-c ]

0.0061 moles of 3-4-b were added to 100 ml of ethanol, and 0.0080 moles of 100% hydrazine hydrate were dropped thereinto. After refluxing for 7 hours, the solvent was distilled off and the residue was recrystallized out of the ethanol, so that 0.0031 moles of 3-4-c were obtained.

### [ 3-4-c → 3-4-e ]

0.0031 moles of 3-4-c were reacted with 3-4-d and, the ring was then closed in the same manner as in [Synthesis of 3-3-c ] of Synthesis Example 3-3, so that 0.0014 moles of 3-4-e were obtained.

### [ 3-4-e → III-3-16 ]

0.0041 moles of 3-4-e were hydrogenation-reduced and then reacted with an acid chloride in the same manner as in [ 3-1-d → 3-1-e → 3-1-g ] of Synthesis Example 3-1. The resulting coarse crystals of III-3-16 were recrystallized from acetonitrile, so that 0.0010 mole of III-3-16 was obtained.

Synthesis Example 3-5 (Synthesis of IV-3-1 )

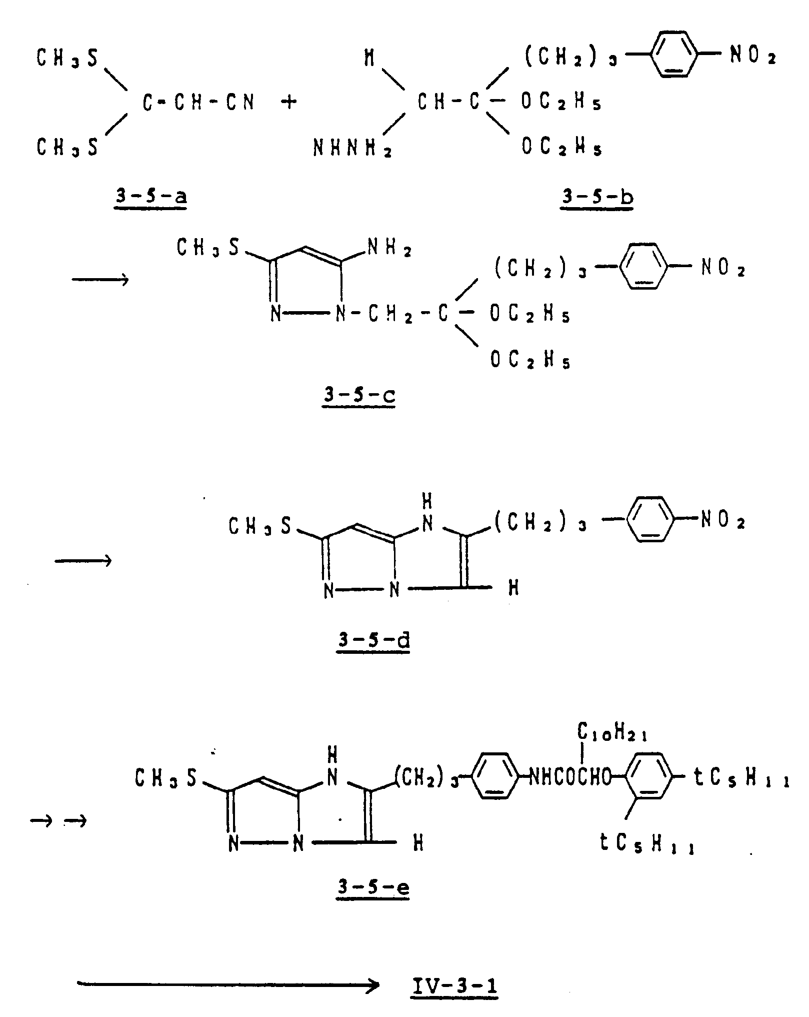

### [Synthesis of 3-5-c ]

0.010 mole of 3-5-a and 0.010 mole of 3-5-b were refluxed for 17 hours in 50 ml of anhydrous ethanol and the solvent was distilled off. The resulting matter was then distilled under reduced pressure, so that 0.0060 moles of 3-5-c were obtained.

### [Synthesis of 3-5-d ]

0.0060 moles of 3-5-c were refluxed for 6.5 hours in 70 ml of ethanol and 30 ml of a 25% sulfuric acid solution. After cooling, 0.05 moles of sodium carbonate was added and stirred. The resulting insoluble matter was filtered out and the solvent was distilled off. The resulting residue was recrystallized from acetonitrile, so that 0.0040 moles of 3-5-d were obtained.

### [ 3-5-d → 3-5-e ]

0.0040 moles of 3-5-d was hydrogenation-reduced in the same manner as in [ 3-1-d → 3-1-e 3-1-g ] of Synthesis Example 3-1 and were further reacted with an acid chloride, so that 0.0032 moles of 3-5-e were synthesized. A mixed solvent of ethyl acetate and hexane was used as the recrystallizing solvent.

### [ 3-5-e → III-3-1 ]

A peracetic acid oxidation was carried out on 0.0032 moles of 3-5-e in the same manner as in [ 3-1-g → II-3-1 ] of Synthesis Example 3-1. 0.0020 moles of white needle crystals of IV-3-1 were obtained recrystallization from acetonitrile.

Synthesis Example 4-I

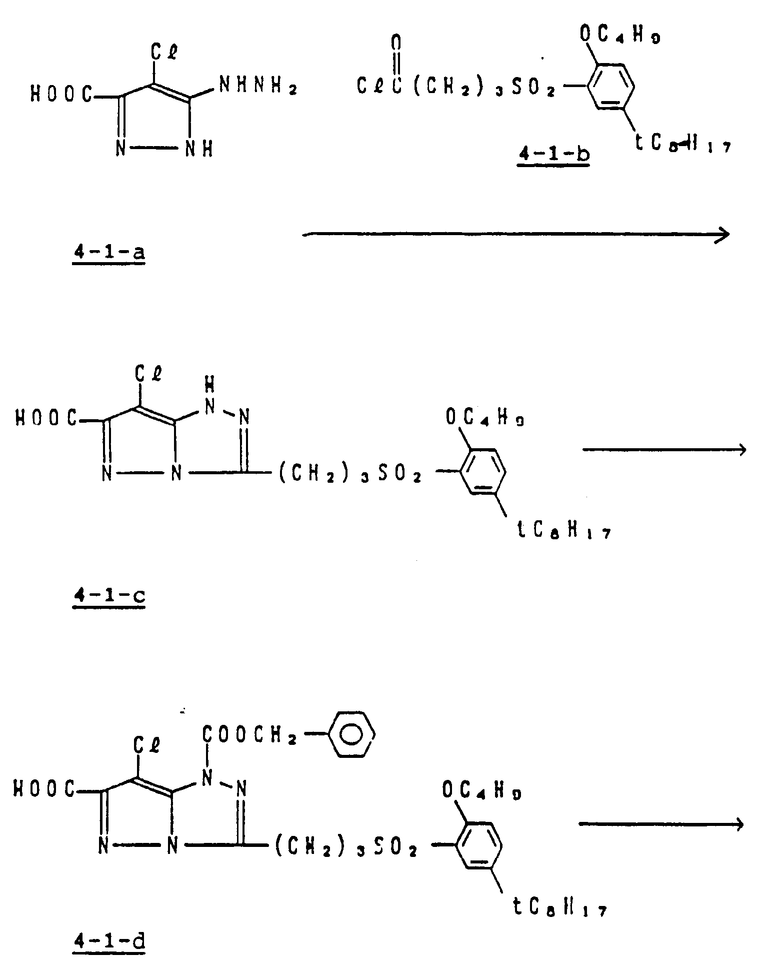

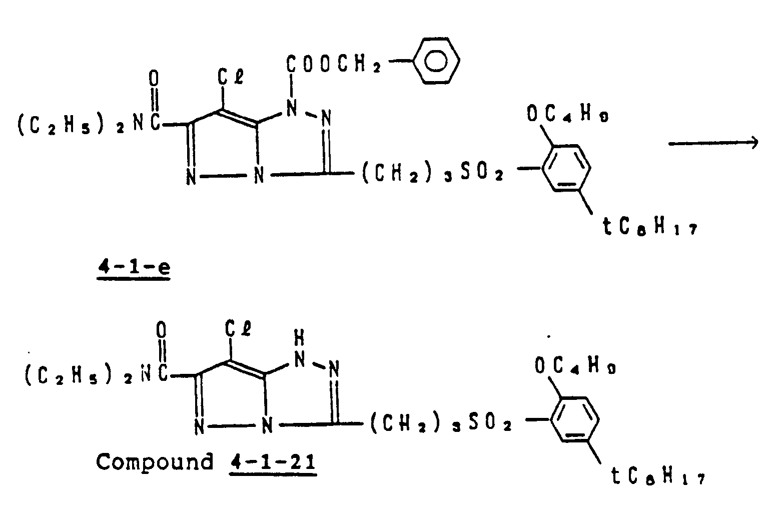

### [Synthesis of 4-1-a ]

0.25 moles of 3-carboxy-4-chloro-5-aminopyrazole were added to 1.5 liters of 6N-chloric acid. After the resulting solution was completely dissolved once, it was cooled to 5° C or lower and 60 ml of an aqueous solution containing 19 g of sodium nitrite were added thereto at a temperature of from 5 to 10° C. The resulting solution was stirred for 30 minutes at 0° C.

Next, 120 g of stannous chloride dihydrate were dissolved in 500 ml of a conc. chloric acid solution. The resulting solution was gradually dropped into the aforegoing solution at a temperature of 0° C or lower. The resulting mixture was stirred for one hour as it was and further stirred for another 2.5 hours at room temperature.

The solution was made to 4 liters by adding water and hydrogen-sulfide gas was then introduced for one hour. The deposited solids were filtered out and the filtrates were concentrated. Then, 400 ml of ethanol were added to the concentration and the deposited white crystals were filtered out The filtrates were dissolved in water and neutralized in an aqueous sodium hydrogencarbonate solution, so that 0.15 moles of 4-1-a were obtained.

### [4-1-a → 4-1-c ]

0.15 moles of 4-1-a were dissolved in 750 ml of acetonitrile and 0.18 moles of 4-1-b were added. 210 ml of triethylamine were then dropped thereinto. The resulting solution was stirred for 3 hours at room temperature, and the deposited crystals were filtered out. The crystals and 0.15 moles of phosphorus oxychloride were added to one liter of toluene and the resulting solution was refluxed for 4 hours. The toluene was then distilled off and 750 ml of acetonitrile and 120 ml of pyridine were added. The resulting mixture was further refluxed for 3.5 hours and was then filtered when still warm so that 0.062 moles of 4-1-c were obtained.

### [ 4-1-c → 4-1-d ]

While cooling with ice and stirring in 120 ml of acetonitrile, 0.062 moles of 4-1-c and 31 ml of an aqueous 2N-sodium hydroxide solution were dropped gradually into 20 ml of an aqueous 4N-sodium hydroxide solution containing 0.062 moles of benzyloxycarbonyl chloride. The resulting solution was stirred for 30 minutes and neutralized with chloric acid. It was then cooled and the precipitates were filtered out and washed with cooled water, so that 0.050 moles of 4-1-d were obtained.

### [ 4-1-d → 4-1-e ]

0.050 moles of 4-1-d and 7 ml of triethylamine were dissolved in 100 ml of dichlormethane, and 24 ml of a dichlormethane solution containing 6mi of thionyl chloride were dropped thereinto. The resulted solution was stirred for one hour at room temperature and was further stirred while adding iced water. An organic laye separated therefrom and the solvents were distilled off. After this, the residue obtained was dissolved in 100 ml of acetonitrile. To the resulting solution was added 7 ml of pyridine and 0.06 moles of diethylamine , and it was then stirred for one hour. After further stirring for 2 hours at 55° C, the solution was poured into 300 ml of water and the deposit was filtered out and recrystallized from ethyl acetate, so that 0.022 moles of 4-1-e were obtained.

### [ 4-1-e → Compound 4-1-21 ]

4-1-e of 0.022 moles were dissolved in 300 ml of THF and the resulting solution was reduced with Pd/c. The Pd/c was removed therefrom and the solvent was distilled off. After this, the resulting matter was recrystallized from ethyl acetate, so that 0.014 moles of Compound 4-1-21 in the form of white needle crystals were obtained.

Synthesis Example 4-2

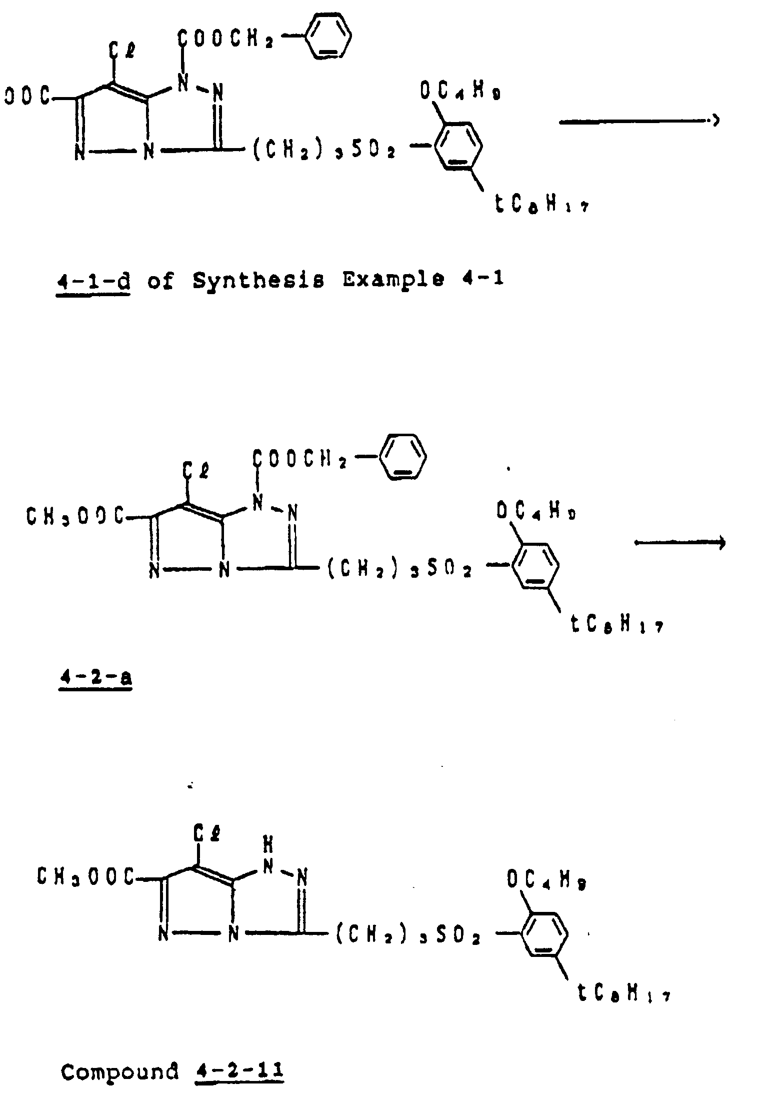

### [Synthesis of 4-2-a]

4-1-d of Synthesis Example 4-1 was used as the raw material. A mixture of 0.025 moles of 4-1-d 3.05 ml of methanol and 50 ml of dichloroethane were added to 0.125 ml of sulfuric acid and the solution was refluxed for 7 hours. After cooling, 100 ml of water was added and an extraction was carried out with ethyl acetate. The extracted matter was washed with an aqueous sodium hydrogencarbonate solution and the solvent was distilled off. After then, the residue was recrystallized from acetonitrile, so that 0.015 moles of 4-2-a were obtained.

### [ 4-2-a → Compound 4-2-11 ]

0.015 moles of 4-2-a were dissolved in 200 ml of methanol and the solution was reduced with Pd/C. After removing the Pd/C, the solvents was distilled off. The resulting matter was recrystallized from ethyl acetate, so that 0.008 moles of Compound 4-2-11 in the form of a white powder were obtained.

Synthesis Example 6-1 (Synthesis of Compound 6-1-1 )

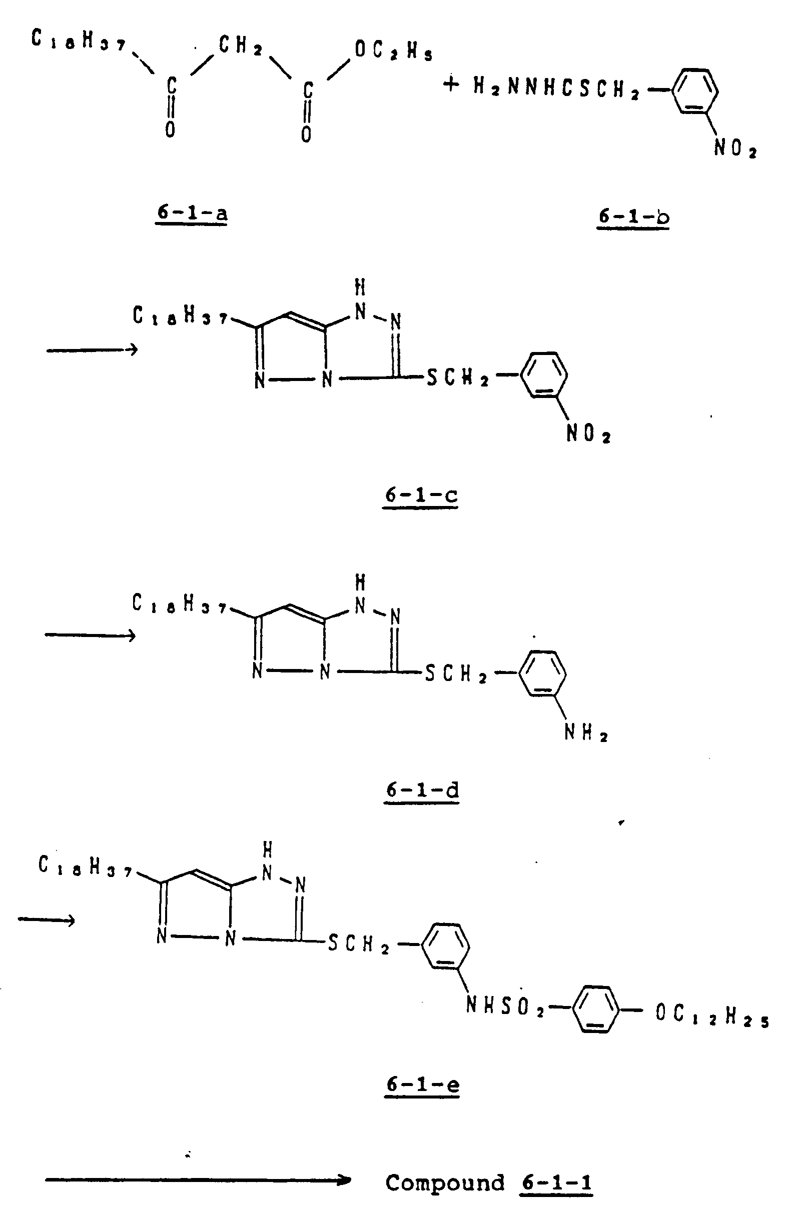

### [Synthesis of 6-1-c ]

0.012 moles of 6-1-a and 0.01 mole of 6-1-b, hydroiodide were heated in 30 ml of amyl alcohol for 30 minutes. After cooling, the deposited matter was filtered off and then washed with ether, so that 0.0061 moles of 6-1-c were obtained.

### [6-1-c→ 6-1-e] ]

0.0061 moles of 6-1-c was dissolved in 100 ml of THF and the resulting solution was hydrogenated using Pd/C. After the Pd/C was filtered off, the solvent was distilled off. Thus, coarse crystals, 6-1-d, were obtained. The 6-1-d was dissolved in 200 ml of acetonitrile and 0.0092 moles of p-dodecaoxyphenylsulfonyl chloride were added. 0.0081 moles of trithylamine were dropped into the resulting solution and stirred for 2 hours at room temperature. After this, the deposited crystals were filtered off and recrystallized from ethyl acetate, so that 0.0042 moles of 6-1-e were obtained.

### [ 6-1-e → Compound 6-1-1 ]

0.0042 moles of 6-1-e were dissolved in 30 ml of acetic acid and, 10 ml of a 35% hydrogen peroxide solution were gradually dropped thereinto and stirred for 3 hours at 50° C. After then, 100 ml of water were added and gradually neutralized with an aqueous sodium hydroxide solution and an extraction was carried out with ethyl acetate. The deposited matter obtained by distillating the solvent off was recrystallized from acetonitrile, so that 0.0030 moles of Compound 6-1-1 in the form of white needle crystals were obtained.

Synthesis Example 6-2 (Synthesis of Compound 6-2-13 )

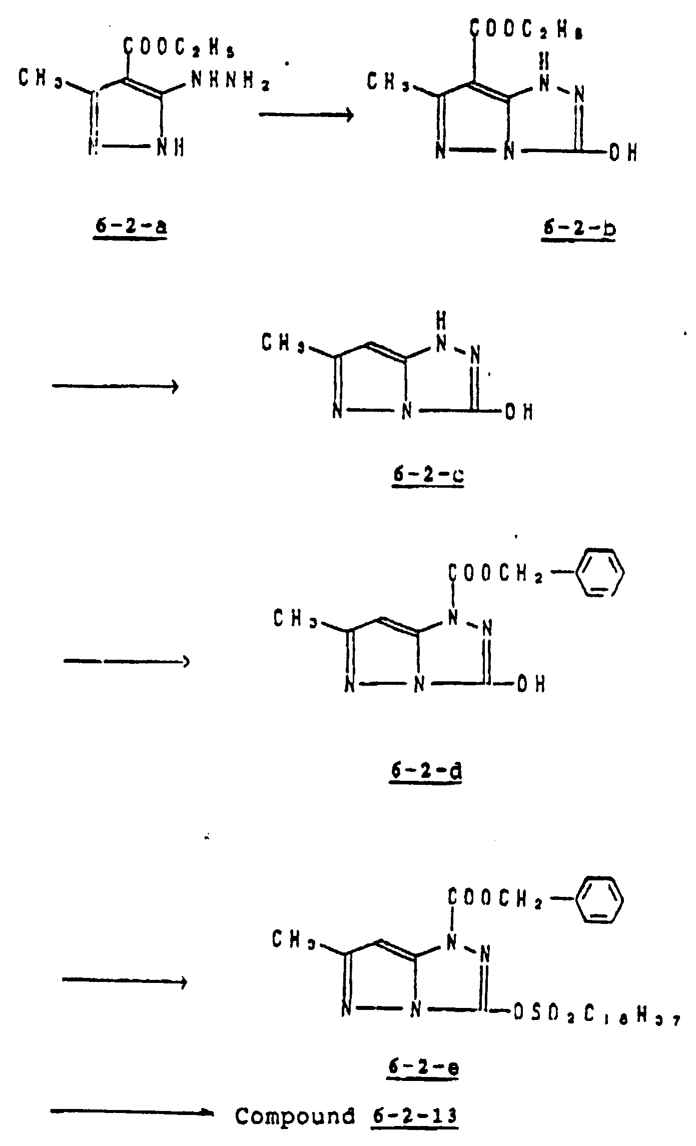

### 6-2-a was synthesized by an ordinary method.

### [ 6-2-a → 6-2-b → 6-2-c ]

0.010 moles of 6-2-a was dissolved in 50 ml of acetonitrile and with 0.015 moles of urea was added. The resulting solution was stirred for 2 hours and the deposited matter, 6-2-b , was filered out. The filtered deposited matters was refluxed for one hour in a mixed solvent of 30 ml of 6-2-b, 18 ml of acetic acid, 0,7 ml of sulfuric acid and water and was then neutralized with an aqueous sodium hydroxide solution. An extraction was made ethyl acetate and the solvent was distilled off, so that 0.0049 moles of 6-2-c were obtained.

### [ 6-2-c → 6-2-d ]

0.0049 moles of 6-2-c and 3 ml of an aqueous 2N-sodium hydroxide solution were added to 15 ml of acetonitrile. While cooling with ice and stirring the resulting solution, 0.0049 moles of benzyloxycarbonyl chloride and 1.5 ml of an aqueous 4N-sodium hydroxide solution were gradually dropped thereinto and then stirred for 30 minutes. The solution was neutralized with chloric acid and then cooled. The precipitate was filtered off and washed with cool water, so that 0.0034 moles of 6-2-d were obtained.

### [ 6-2-d → 6-2-e ]

0.0034 moles of 6-2-d were dissolved in 30 ml of acetonitrile and thereinto 0.0040 moles of 6-2-d were dropped and 3 ml of pyridine were added. The resulting solution was stirred at room temperature. The resulting reaction solution was poured into water, the deposited matter was filtered off and the filtrates were recrystallized with a mixed solvent of ethyl acetate and hexane, so that 0.0020 moles of 6-2-e were obtained.

### [ 6-2-e → Compound 6-2-13 ]

0.0020 moles of 6-2-e were dissolved in 30 ml of THF and the solution was reduced with Pd/C. The Pd/C was removed and the solvent was distilled off. The resulting matter was recrystallized from ethyl acetate, so that 0.0015 moles of Compound 6-2-13 in the form of light-yellow powdered crystals were obtained.

Synthesis Example 7-1 (Synthesis of 11-7-1 )

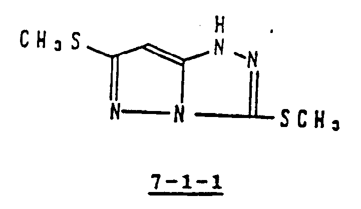

### [Synthesizing Process of 7-1-1 ]

0.012 moles of o-ethyl-S-methyldiethyl malonate and 0.01 moles of S-methylisothiocarbohydrazide hydroiodide were heated in 25 ml of amyl alcohol for 30 minutes.

After cooling, the deposited matter was filtered out and the filtrates were washed with ether, so that 0.006 moles of coarse crystals 7-1-1 were obtained.

### [Synthesizing Process 7-1-2 ]

0.006 moles of 7-1-1 were dissolved in 15 ml of acetic acid. Then, 5 ml of an aqueous 35% hydrogen peroxide solution were dropped gradually into the resulting solution and stirred for 2 hours. After this, 50 ml of water were added and the mixture was neutralized with an aqueous sodium hydroxide solution. The resulting reaction solution, having a PH of 6.5, was extracted with ethyl acetate and the solvent was distilled off. The resulting deposited matter was washed with acetonitrile, so that 0.005 moles of white needle crystals 11-7-1 were obtained.

Synthesis Example 7-2 (Synthesis of 11-7-2)

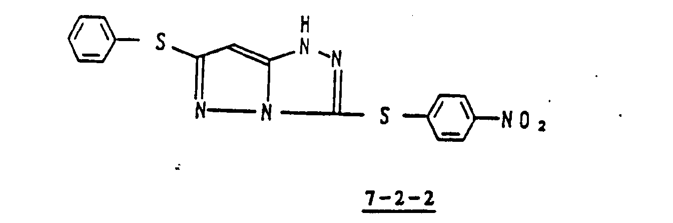

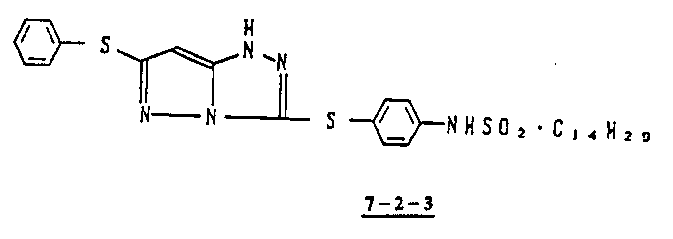

### (Synthesizing Process 7-2-1 )

0.012 moles of o-ethyl-S -methyldiethyl malonate 0.01 moles of S-(p-nitrophenyl)isothiocarbohydrazide hydroiodide were heated in 25 ml of amyl alcohol for one hour.

After cooling, the deposited matter was filtered out and the filtrates were washed with ether and dried , so that 0.0059 moles of 7-2-2 were obtained.

### (Synthesizing Process 7-2-2 )

0.0059 moles of 7-2-2 were dissolved in 50 ml of THF and hydrogenated using Pd/C. After the Pd/C was filterd out, the reaction solution was distilled off and the deposited matter was dissolved in 100 ml of acetonitrile. Then, 0.01 mole of tetradecanedulfonyl chloride was added and 0.012 moles of triethylamine were dropped thereinto. After stirring for hours at room temperature, the deposited crystals were filtered out and further recrystallized from ethyl acetate, so that 0.0055 moles of Compound 7-2-3 were obtained.

### Synthesizing Process 7-2-3

The 7-2-3 was oxidized in the same manner as in Synthesizing Process 7-1-2 of Synthesis Example 7-1, so that 0.0039 moles of white needle crystals 11-7-2 were obtained.

### Synthesis Example 7-3 (Synthesis of II-7-3)

### Synthesizing Process 7-3-1

According to the method described in Japanese PantentApplication No. 120054-1986, 7-3-4 was obtained.

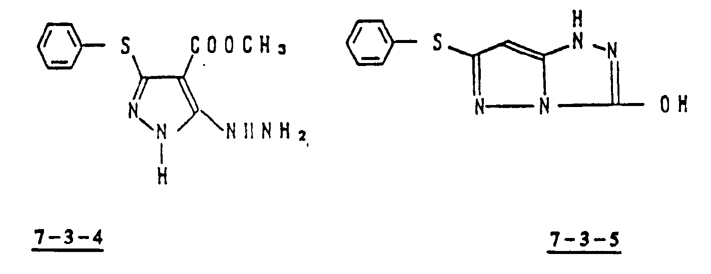

0.02 moles of 7-3-4 were dissolved in 100 ml of acetonitrile and 0.030 moles of urea were added. The resulting solution was then stirred for 2 hours and the deposited matter was filtered out. The filtrates were heated and refluxed for one hour in a mixed solvent of 50 ml of acetic acid, 20 ml of sulfuric acid and 1.0 ml of water. The resulting matter was then neutralized with an aqueous sodium hydroxide solution and extracted with ethyl acetate, and the solvent was distilled off, so that 0.0128 moles of 7-3-5 were obtained.

### Synthesizing Process 7-3-2

7-3-5 0.0062 moles of 7-3-5 were dissolved in 60 ml of acetonitrile and 0.0070 moles of tetradecanecarbonyl chloride were dropped. thereinto. After a small amount of pyridine was added, the resulting solution was stirred at room temperature. The resulting reaction solution was poured into water and the deposited matter was filtered out, so that 7-3-6 was obtained. Furthermore, 0.0035 moles of 11-7-3 were obtained in the same manner as in Synthesizing Process 7-1-2 of Synthesis Example 7-1.

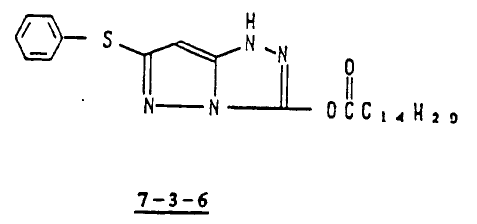

### Synthesis Example 7-4 (Synthesis of 11-7-4)

Compound 7-3-5 of Synthesis Example 7-3 was used.

0.006 moles of 7-3-5 were dissolved in 60 ml of acetonitrile and 0-0065 moles of m-nitrophenylsulfonyl chloride were dropped thereinto. After a small amount of pyridine was added, the resulting solution was stirred at room temperature. The resulting reaction solution was poured into water and the deposited matter was filtered out. The filtrates were further hydrogenated and reduced in the same manner as in Synthesizing Process 7-2-2 of Synthesis Example 7-2 and reacted with tetradecanecarbonyl chloride. The reacted matter was oxidized in the same manner as in Synthesizing Process 7-1-2 of Synthesis Example 7-1, so that 0.0028 moles of 11-7-4 were obtained.

### Synthesis Example 7-5 (Synthesis of 11-7-6)

According to the methods described in, for example J. Heterocycl. Chem., Voi.11, p.423, 1974 and Japanese Patent Application No. 120054-1986, 7-5-7 was synthesized.

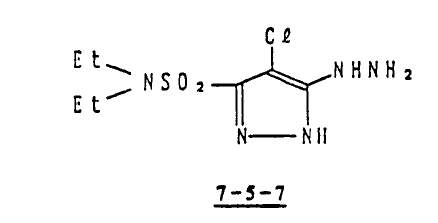

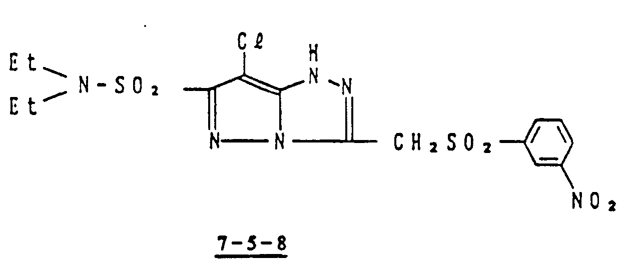

0.01 moles of 7-5-7 and 0.015 moles of m-nitrophenylsulfonylbenzoyl chloride were added to 50 ml of ethyl acetate and 1.0 g of triethylamine was dropped thereinto. After stirring for 2 hours, the deposited crystals were filtered out and washed. The resulting crystals and 0.01 mole of phosphorus oxychloride were refluxed for 2 hours in 30 ml of toluene and the solvent was distilled off. After 10 g of pyridine and 30 ml of acetonitrile were added and further refluxed for hours, the deposited matterwas recrystallized from acetonitrile, so that 0.0050 moles of 7-5-8 were obtained. Successively, a reaction was carried out in. the same manner as in Synthesizing Process 7-2-2 of Synthesis Example 7-2, so that 0.0031 moles of the objective compound were obtained.

### Synthesis Example 7-6 (Synthesis of III-7-1)

Synthesizing Process 7-6-1

According to ordinary methods, the following aminopyrazoles 7-6-9, 7-6-10 and 7-6-11 were obtained.

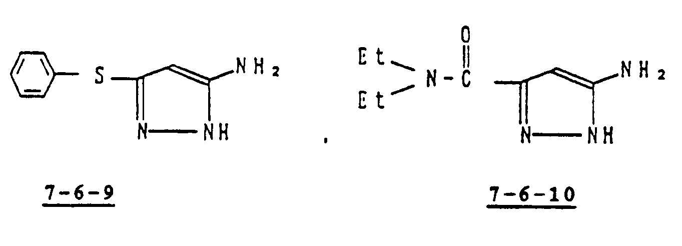

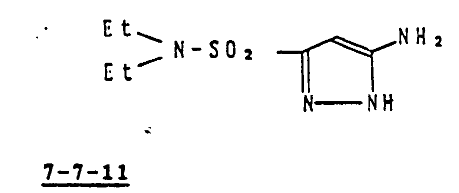

According to the method described in Japanese Patent O.P.I. Publication No. 65247-1986, 7-6-12 and 7-

6-13 were synthesized with the corresponding nitrile compounds.

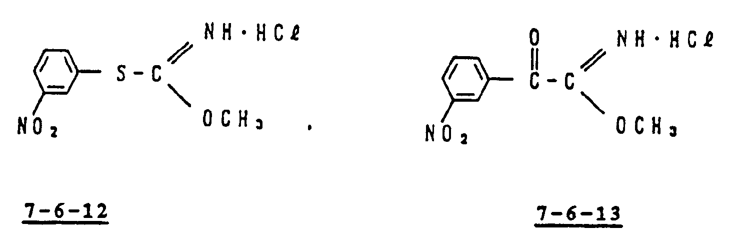

### Synthesizing Process 7-6-2

0.01 moles of 7-6-9 and 0.011 moles of 7-6-12 were refluxed for 20 hours in 20 ml of toluene and the solvents were then distilled off. The resulting residue was dissolved in 20 ml of methanol and a methanol solution containing hydroxylamine was added at 0° C. The resulting solution was stirred for one hour at room temperature. The solution was the poured into 200 ml of water, and the deposited matters was filtered out.

The fi It rates were dissolved in 50 cc of THF and 0.004 moles of triethylamine were added and stirred. While stirring, a THF solution containing 0.7 g of p-toluene-sulfonic chloride was added. After further stirring, the resulting insoluble matterwas were filtered out and the filtrate was refluxed for 7 hours in a nitrogen atmosphere. After then, THF was distilled off and the residue was dissolved in a small amount of methanol. When the resulting solution was poured into 50 ml of water, 0.0014 moles of 7-6-14 were obtained.

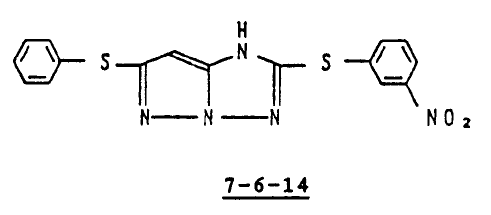

0.0014 moles of 7-6-14 were hydrogenated and reduced in the same manner as in Synthesizing Process 7-2-2 of Synthesis Example 7-2 and were then reacted with an acid chloride. The resulting matter was further oxidized in the same manner as in Synthesizing Process 7-2-3 of Synthesis Example 7-2, so that 0.0026 moles of 111-7-1 were obtained.

### Synthesis Example 7-7 (Syntheses of III-7-2 , -6 and -7)

According to the method of Synthesizing Process 7-6-2 of Synthesis Example 7-6, III-7-2 was synthesized by making use of 7-6-9 and 7-6-13 , each synthesized in Synthesizing Process 7-6-1 of Synthesis Example 7-6, III-7-6 by 7-6-10 and 7-6-12 and III-7-7 by 7-6-11 and 7-6-13 ; their yields were 29%, 47% and 30%, respectively.

### Synthesis Example 7-8 (Synthesis of IV-7-1 )

According to the method of J. Heterocycl. Chem. 10 , p.411, 1973 and Japanese Patent O.P.I. Publication No. 162548-1984, 7-8-15 and 7-8-16 were synthesized.

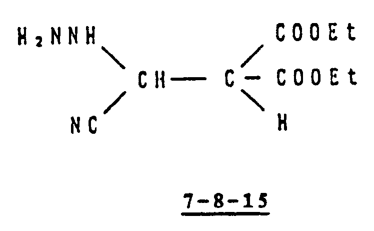

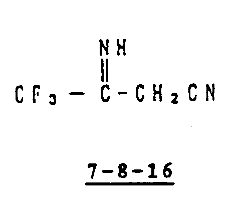

0.01 moles of 7-8-15 and 0.012 moles of 7-8-16 were refluxed for 10 hours in anhydrous ethanol and the solvent was removed. After this a distillation was applied under reduced pressure. so that 0.0072 moles of 7-8-17 were obtained.

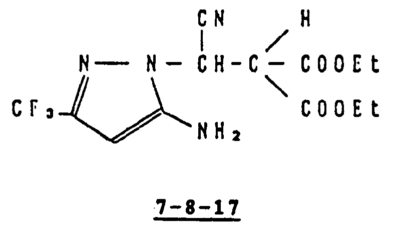

0.0072 moles of 7-8-17 were refluxed for 3 hours in 300 ml of ethanol and 80 ml of a 20% sulfuric acid solution. After cooling, excessively solidified sodium carbonate was added and filtered out, and the solvent was removed. The residue was recrystallized from acetonitrile, so that 0.0040 moles of IV-7-1 were obtained.

### Synthesis Example 7-9 (Synthesis of IV-7-5 )

7-9-18 and 7-9-19 were synthesized in the same manner as in Synthesis Example 7-8.

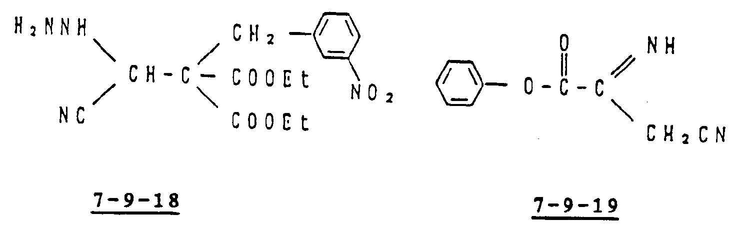

In the same manner as in Synthesis Example 7-8, 0.031 moles of 7-9-20 were obtained by reaction with 0.06 moles of7-9-19.

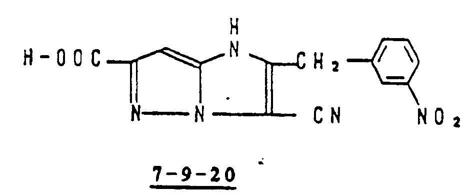

In the same manner as in Synthesizing Process 7-2-2 of Synthesis Example 7-2, 7-9-20 was hydrogenated and reduced, and was then reacted in ethyl acetate in the presence of pyridine with an acid chloride in an amount of 1.2 times than the moles of 7-9-20 to produce coarse crystals. The coarse crystals were recrystallized from acetonitrile, so that 0.025 moles of IV-7-5 were obtained.

### Synthesis Example 7-10 (Synthesis of IV-7-2)

A mixture of 0.01 moles of 7-9-20 , 0.03 moles of methanol and 20 ml of dichloroethane were added to 0.05 moles of sulfuric acid and refluxed for 8 hours. After cooling, 50 ml of water were added and the resulting solution was extracted with ethyl acetate. The ethyl acetate solution was washed with aqueous sodium hydro- carbonate and the solvent was removed. After this, the residue was recrystallized from acetonitrile. Furthermore, a hydrogenation and reduction were carried out in the same manner as in Synthesizing Process 7-2-2 of Synthesis Example 2-2, and a reaction was carried out with an acid chloride, so that 0.0065 moles of IV-7-2 were obtained.

The couplers of the present invention are ordinarily used in an amount of from 1x10-a moles to 1 mole, and more preferably from 1x10-² moles to 8x10-' moles, per mole of silver halide used.

The couplers of the invention may be used in similar methods and techniques as with ordinary cyan dye forming couplers. It is typical to prepare a color light-sensitive material of the invention in such a manner that the cyan couplers of the invention are compounded into a silver halide emulsion and the resulting emulsion is coated on a support.

When the red light-sensitive silver halide emulsion containing a coupler of Formulae [1-1] through [1-7] is provided with at least one spectral sensitizing dye selected from the following Formulae [A], [B], [C], [D], [E] and [F], excellent effects can be displayed for both photosensitivity of the emulsion layer and aging stability of the coating liquid.

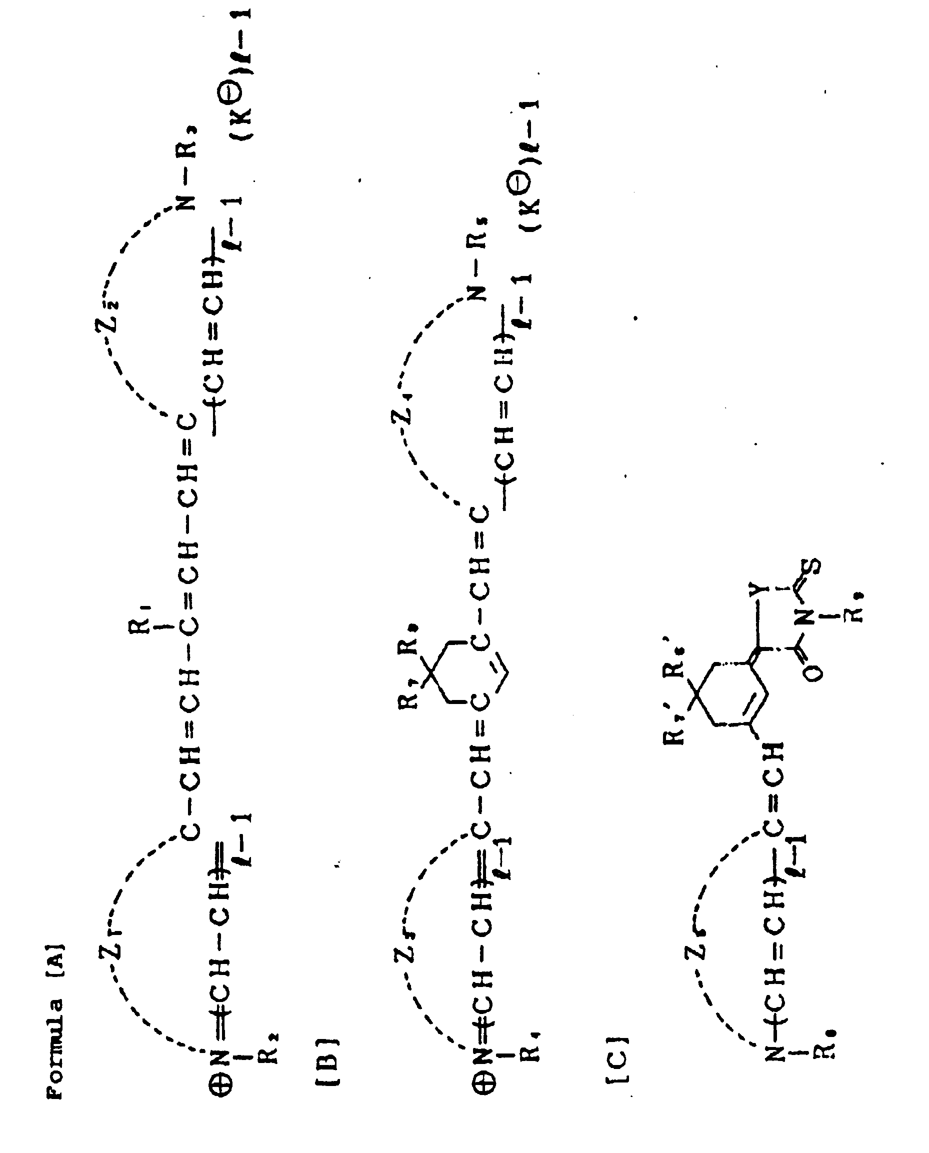

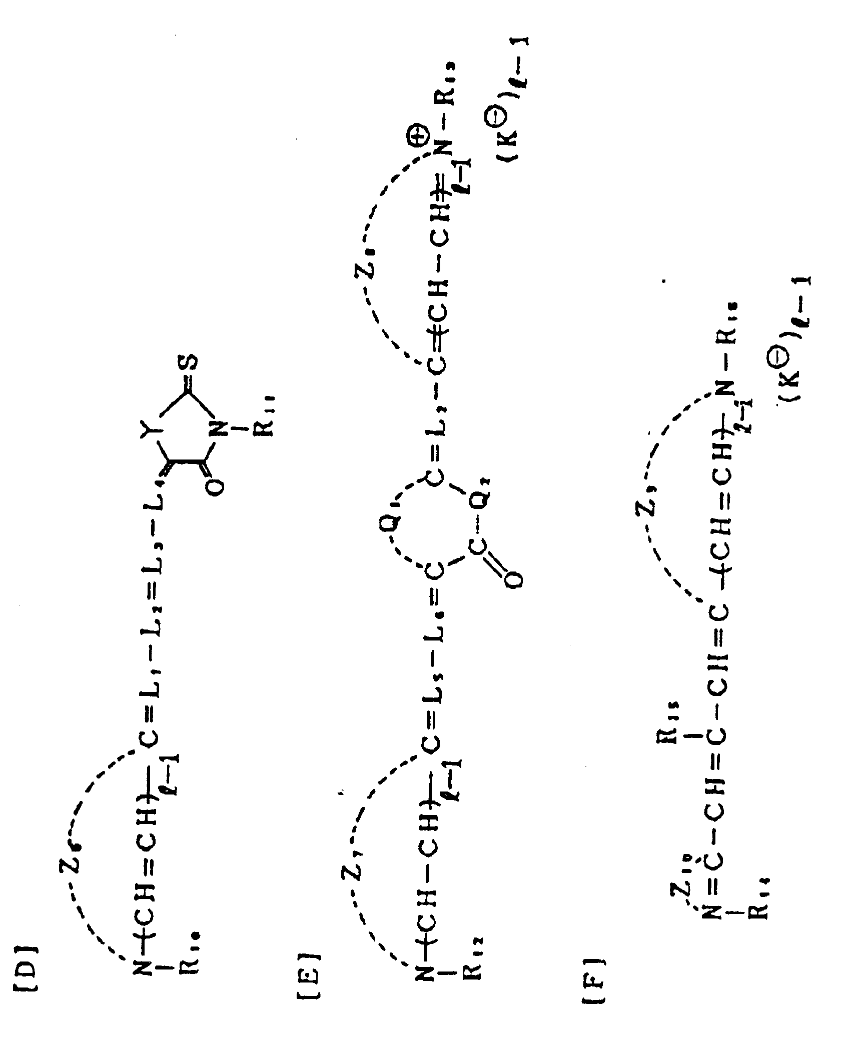

Wherein Z₁ through Z₉ each is a group which completes a benzene ring or a naphthalene ring condensed with a pyridine ring, an imidazole ring, a thiazole ring, a selenazole ring, an oxazole ring or a tetrazole ring; Z₁₀ is a group which completes a benzothiazole ring, a benzoselenazole ring, a (β-naphthothiazole ring, a (β-naphthoselenazole ring, a benzimidazole ring ora 2-quinoline ring; Q₁₁ and Q₂ each is a group which completes a 4-thiazolidinone, 5-thiazolidinone or 4-imidazolidinone; R₁ and R₁₅ each is a hydrogen atom, an alkyl group or an aryl group; R₇, R₈, R₇, and R₈, each is an alkyl group; R₉ and R₁₁ each is an alkyl group, an aryl group or a heterocyclic group; R₂, R₃, R₄, R₅, Rₛ, R₁₀, R₁₂, R₁₃, R₁₄ and R₁₆ each is an alkyl group or an aryl group;

f is 1 or 2; Y is a sulfur atom or a selenium atom; L₁ through L₆ each is a substituted or unsubstituted methyne group; and K is an acid anion.

The spectral sensitizing dyes of Formulae [A], [B], [C], [D], [E] and [F] are well-known. They may readily be synthesized by a method described in, for example, F.M. Hamer, 'The Chemistry of Heterocyclic Compounds', Vol. 18; and A. Weissberger, 'The Cyanine Dyes and Related Compounds', edited by Interscience, New York, 1964.

Examples of the spectral sensitizing dyes which are preferably be used in the invention are:

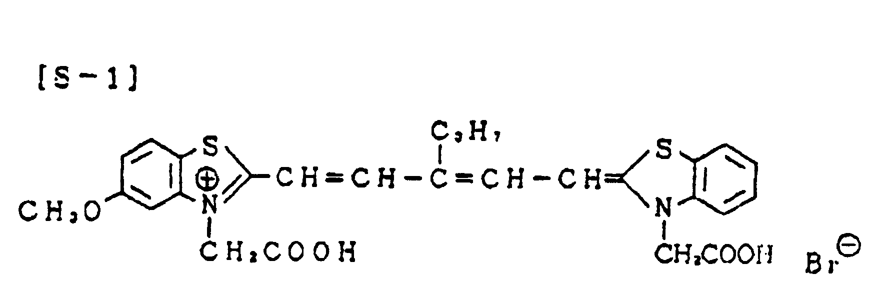

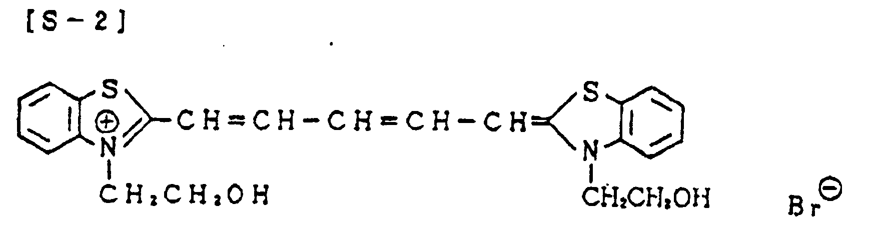

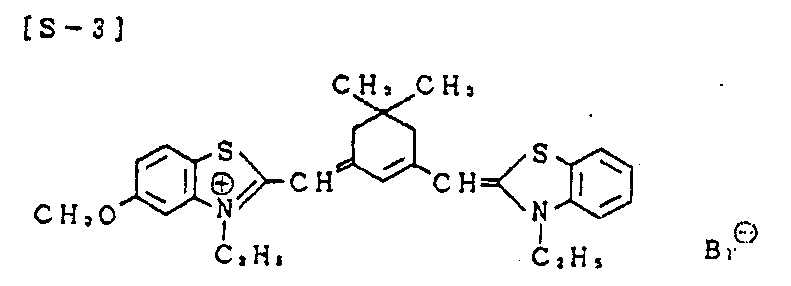

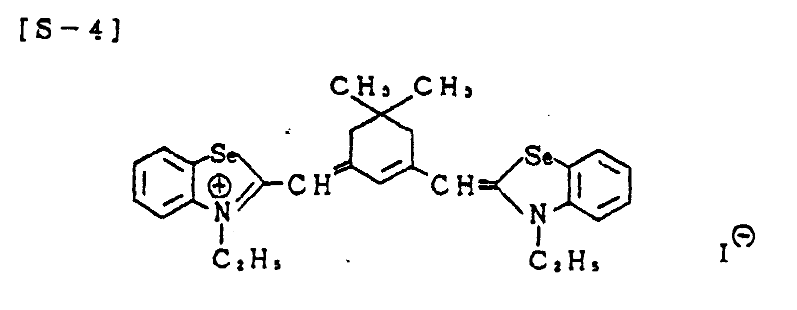

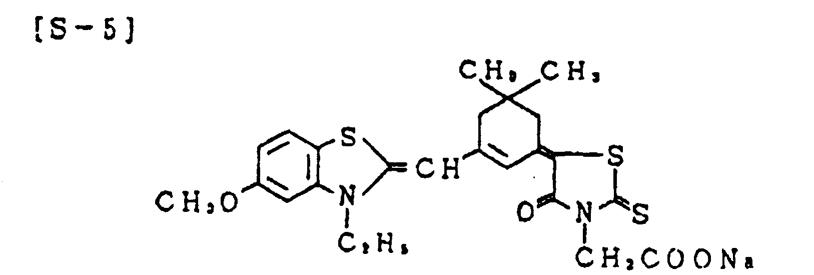

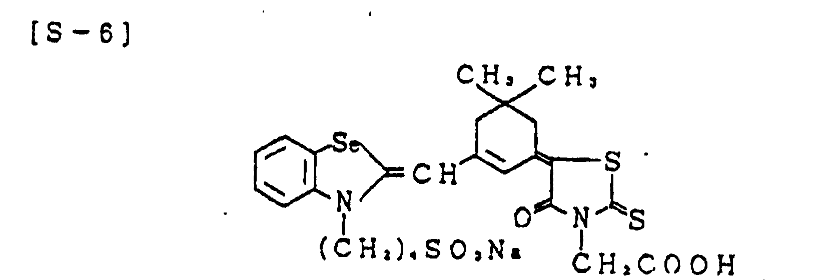

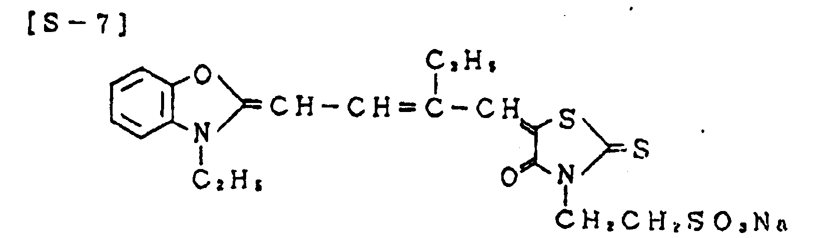

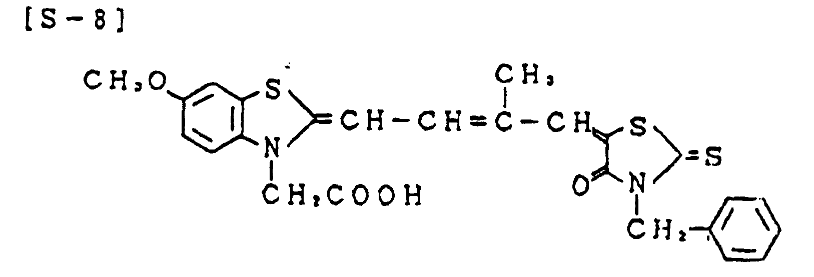

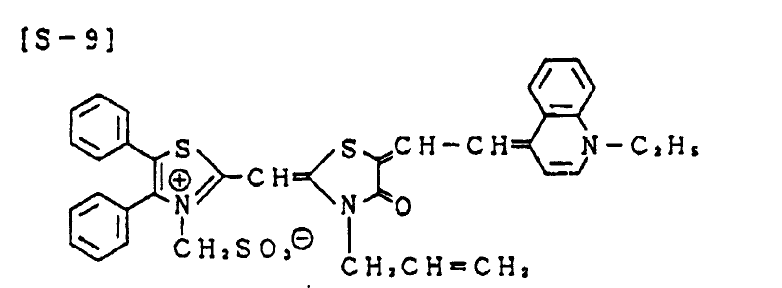

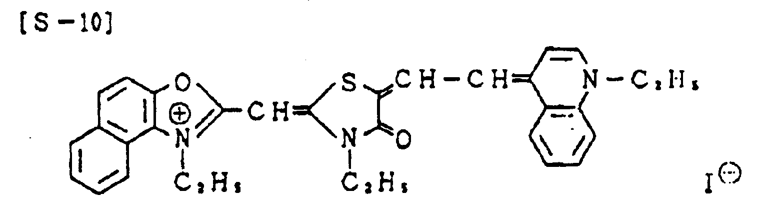

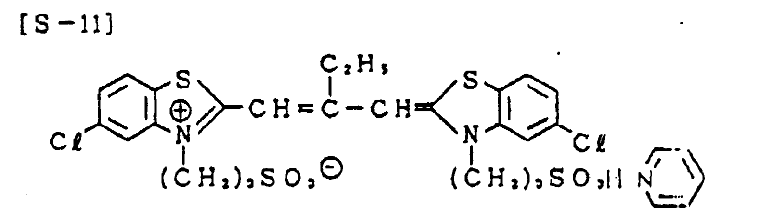

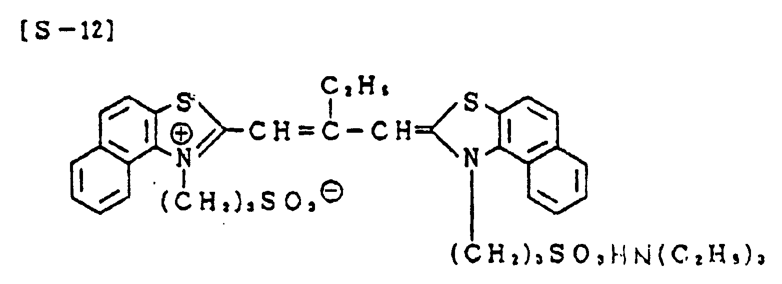

The above-mentioned spectral sensitizing dyes may be added to an emulsion by any method well-known in the art. For example, the spectral sensitizing dyes may be dispersed directly into an emulsion; or they are dissolved in water-soluble solvent such as pyridine, methyl alcohol, ethyl alcohol, methyl cellosolve, acetone or a mixture thereof, they are diluted with water, or they are dissolved in water, so that they may be added in the form of a solution to the emulsion. Supersonic wave oscillation may be used in the course of the dissolution.

It is also possible to use a method in which the spectral sensitizing dye is dissolved in a volatile organic solvent, the resulting solution is dispersed in a hydrophilic colloid and the resulting dispersion is added to an emulsion, as described in U.S. Patent No. 3,469,987; and a method in which a water-insoluble dye is dispersed in a water-soluble solvent without dissolving the dye and the resulting dispersion is added to an emulsion, as described in Japanese Patent Publication No. 24185-1971. In addition to the above, the spectral sensitizing dye may be added to an emusion in the form of a dispersion prepared by an acid dissolution-dispersion method. Besides the above, the addition of the spectral sensitizing dye to an emulsion may be by the methods described in for example U.S. Patent Nos. 2,912,345, 3,342,605, 2,996,287 and 3,425,835.

The spectral sensitizing dyes may be added to the emulsion at any time during the course of preparing the emulsion. It is, however, preferred to add the dye during or after chemical sensitization. It is also possible to add the dyes in parts during or after the chemical sensitization.

The spectral sensitizing dyes of Formulae [A] through [F] may be used together with other spectral sensitizing dyes, in the form of a so-called supersensitizing combination. In this case, the spectral sensitizing dyes are dissolved in solvents which are the same or different from each other. The resulting solutions are mixed together before adding them to an emulsion, or each of the solutions may be added separately to the emulsion. When adding the solutions separately, the order and intervals of the addition may be determined according to the purpose.

The amount of the spectral sensitizing dyes used is generally from 1.20x10-⁴ to 0.15x10⁻⁴ moles, preferably from 1.0x1 0-4 to 0.3xl 0-⁴ moles more preferably from 0.8x10-⁴ to 0.4xl 0-⁴ moles, per mole of silver halide used.

The color light-sensitive materials of the invention may be in the form of, for example, a color negative or positive film or a color print paper.

The light-sensitive materials, including the above-mentioned color print paper, may be for either monochromatic or multicolor use. In multicolor light-sensitive materials, the cyan couplers of the invention are contained in a red light-sensitive silver halide emulsion layer thereof. The multicolor light-sensitive materials each have dye image forming constitution units which are sensitive to the three primary color spectral regions. Each con- sitution unit may be a single-layered or multi-layered emulsion layer sensitive to a particula spectral region. The component layers of a light-sensitive material, including an image forming constitution unit layer, may be arranged in various orders as known in the art.

Typical multicolor light-sensitive materials comprise a support carrying thereon a cyan dye image forming constitution unit, (in which at least one cyan coupler is a cyan coupler of the present invention), comprising at least one red light-sensitive silver halide emulsion layer containing at least one cyan coupler; a magenta dye image forming constitution unit comprising at least one green light-sensitive silver halide emulsion layer containing at least one magenta coupler; and a yellow dye image forming constitution unit comprising at least one blue light-sensitive silver halide emulsion layer containing at least one yellow coupler. The light-sensitive materials may be provided with supplementary layers such as a filter layer, an interlayer, a protective layer and a subbing layer.

Conventional methods may be used to add the cyan couplers of the present invention to an emulsion. For example, a silver halide emulsion may be prepared in the following manner. The cyan couplers of the invention are dissolved independently or in combination in a single solution of either a high boiling solvent such as tricresyl phosphate or dibutyl phthalate, having a boiling point of not lower than 175° C, or a low boiling solvent- such as butyl acetate or butyl propionate, or a mixed solution thereof if required. The resulting solution is the mixed in an aqueous gelatin solution containing a surface active agent. Next, the mixed solution is homogenized by a high-speed rotary mixer or a colloid-mill and a silver halide is added to the resulting emulsion.

The silver halides preferably used in the invention include silver chloride, silver chlorobromide and silver chloroiodobromide. Furthermore, a mixture such as silverchlorobromide and silver bromide may also be used. When a silver halide emulsion is used in a color print paper, high-speed developability in particular is demanded. It is, therefore, preferred that chlorine is included in the silver halide, and it is particularly preferred that silver chloride, silver chlorobromide or silver chloroiodobromide has a silver chloride content of at least 1%.

The silver halide emulsions may be chemically sensitized by an ordinary method and may also be optically sensitized to a desired spectral wavelength region.

To prevent fog and/or to keep the photographic characteristics stable in the course of manufacturing, storing or processing the light-sensitive materials in the photographic industry, it is possible to add an antifogging agent or a stabilizer.

Color light-sensitive materials of the invention may contain for example, an anti-color-foggant, a dye-image stabilizer, a UV absorbent, an antistatic agent, a matting agent or a surface active agent, each of which are ordinarily used in light-sensitive materials.

The above-mentioned additives may be those referred to in Research Disclosure, Vol. 176, pp. 22-31, December, 1978, for example.

An image may be formed by processing a color light-sensitive material of the invention in a color development process which is well-known in the art.

Color light-sensitive materials may be treated in an alkaline active bath, if the hydrophilic colloidal layer of the sensitive material contains a color developing agent or a precursor thereof

After color development, color light-sensitive materials of the invention may be bleached and fixed. The bleaching and fixing steps may be carried out at the same time.

After the fixing step, a washing step is ordinarily carried out, but the washing step may be either substituted by a stabilizing step or carried out in combination with a stabilizing step.

### EXAMPLES

In the following examples, the invention is further described in detail.

### Example 1-1

Sample 1-1 of red-sensitive color light-sensitive material was prepared by coating the following layers over to a paper support laminated with polyethylene on the both sides thereof in order from the support side, respectively. The amounts of the compounds added are expressed in terms of those added persq. meter, unless otherwise particularly stated. (Amounts of silver halides are expressed in terms of the contents of silver.)

### Layer 1: An emulsion layer

A red-sensitive emulsion layer comprising 1.2 g of gelatin, 0.30 g of red-sensitive silver chlorobromide emulsion (containing silver chloride of 96 mol%) and 0.45 g of comparative cyan coupler a dissolved in 1.35 g of dioctyl phthalate.

### Layer 2: A protective layer

A protective layer containing 0.50 g of gelatin, whereto 2,4-dichloro-6-hydroxy-s-triazine sodium salt was added as a hardener in an amount of 0.017 g per g of the gelatin used.

Next, Samples No. 1-2 through No. 1-13 each of the invention were prepared in quite the same manner as in Example 1-1, except that the comparative coupler a was replaced by the couplers indicated in Table 1-1 (provided that the amounts added were the same molar amounts as in the comparative coupler a).

The resulted Samples No. 1-1 through No. 1-13 were exposed to light through a wedge in an ordinary method and were then processed in the following processing steps.

### (Processing steps)

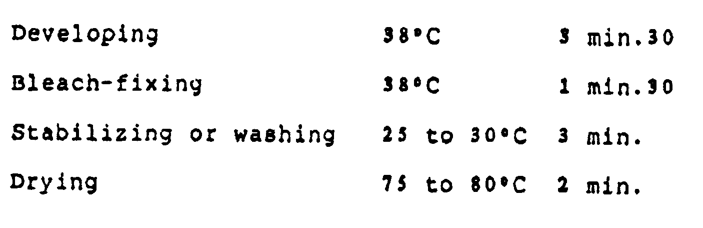

The processing liquids used in each of the steps were as follows.

(Color developer)

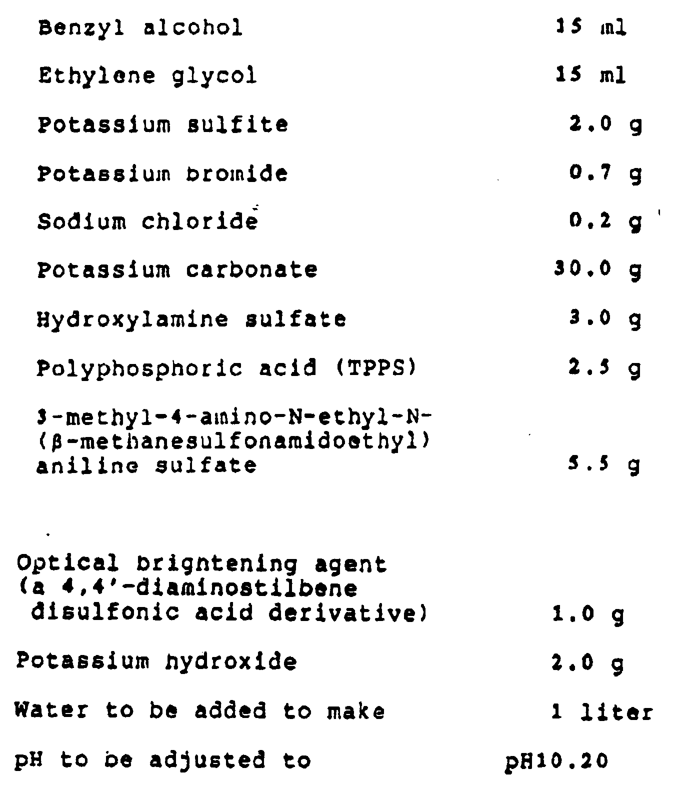

(Bleach-fixer)

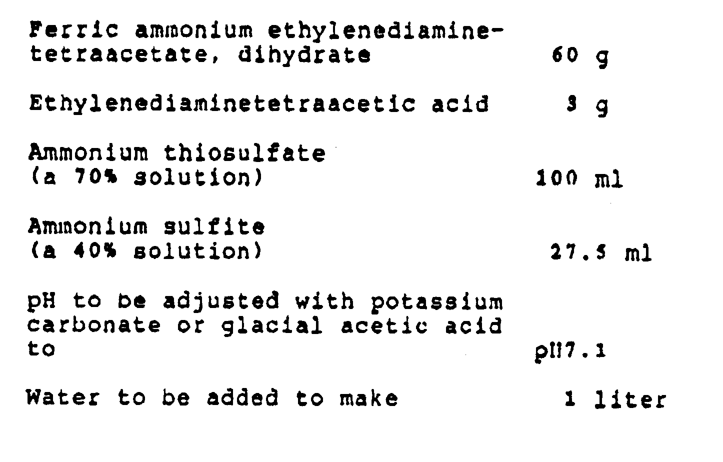

(Stabilizer)

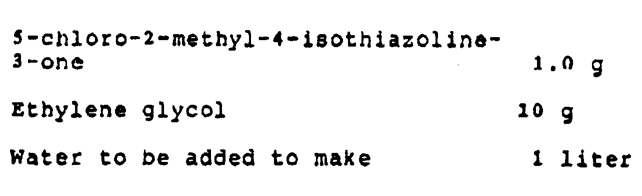

With respect to thus processed Samples No. 1-1 through No. 1-13, the measurements were made respectively each of the maximum spectral absorption wavelengths (λ,max), the reflection densities of 420 nm (DX420) when the reflection densities of λ,max were 1.0, and the half band widths (W1/2) which are the difference between one point on the longerwavelenth side than each λmax and the shorter wavelength side than each λmax when the reflection densities of λmax were 0.5. After then, the spectral absorption characteristics and the color reproducibility of each Sample were examined.

The smaller a value of DX420 is, the less an irregular absorption in green region is. The smaller a value of W1/2 is, the sharper an absorption is. The facts mean that a color reproducibility is excellent.

Fig. 1-1 shows the absorption spectra of Sample Nos. 1-1, 1-2 and 1-6. Wherein, represents Comparative Coupler a (of Sample 1-1), 2 and 6 represent Coupler 11-1-1 (of Sample 1-2) and Coupler III-1-1 (of Sample 1-6) each of the invention, respectively.

Further, each of the processed samples was allowed to stand for 14 days under the circumstances of a high temperature and humidity (at 60 °C and 80%RH), and the heat and moisture resisting properties of the resulted dye-images were checked up. The results thereof are also shown in Table 1-1, wherein the heat and moisture resisting properties of the dye-images are indicated by the percentage of the residual dyes which still remained after the heat and moisture resistance tests were tried, to the initial density of 1.0.

For those measurements, a densitometer, Model KD-7R (manufactured by Konishiroku Photo Ind. Co., Ltd.), was used.

Comparative Coupler a

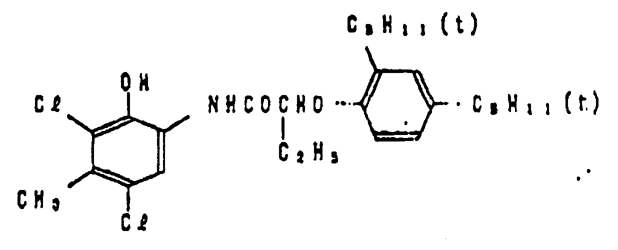

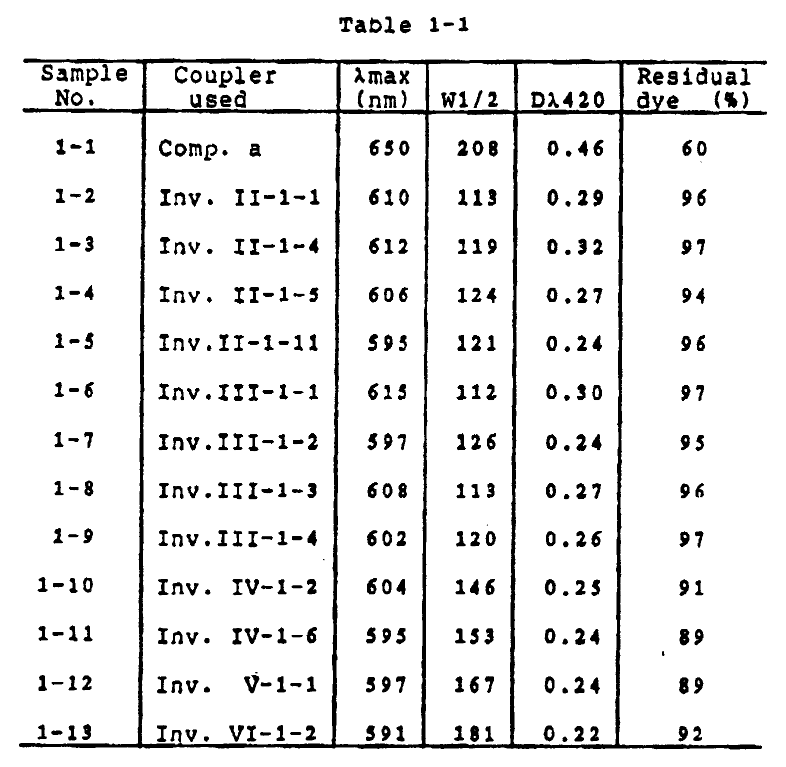

As is obvious from Table 1-1, it is found that every sample having the couplers of the invention has excellent spectral absorption characteristics, because their half band widths are extremely narrower than those of the sample having the comparative coupler and the irregular absorptions are relatively less, as compared with the sample used the comparative coupler. It is further found that every sample having the couplers of the invention is relatively solid, because of the high percentage of residual dyes and the relatively excellent moisture resisting property.

In addition, Fig. 1-1 shows the fact that the couplers of the invention are relatively less in undesirable irregular absorption caused in green region and relatively sharp in absorption in the regions around λmax, as compared with the conventional phenol type couplers.

### Example 1-2

Samples No. 1-14 through No. 1-19 of red-sensitive color reversal photographic light-sensitive materials were prepared by coating the following layers over to a triacetyl cellulose fi im support, in order from the support side.

The amounts of the compounds added are expressed by the amounts thereof per sq.meter unless otherwise specially stated. (Amounts of silver halides are expressed in terms of the contents of silver.)

### Layer 1: An emulsion layer

A red-sensitive emulsion layer comprising 1.4 g of gelatin, 0.5 g of red-sensitive si Iver chlorobromide emulsion (containing silver chloride of 96 mol%) and the couplers shown in Table 1-2 dissolved in 1.50 g of dibutyl phthalate.

### Layer 2: A protective layer

A protective layer containing 0.5 g of gelatin, whereto 2,4-dichloro-6-hydroxy-s-triazine sodium salt was added as a hardener in an amount of 0.017 g per g of the gelatin used.

The resulted Samples were exposed to light through a wedge in an ordinary method and were then processed in the following processing steps.

(Reversal processing steps)

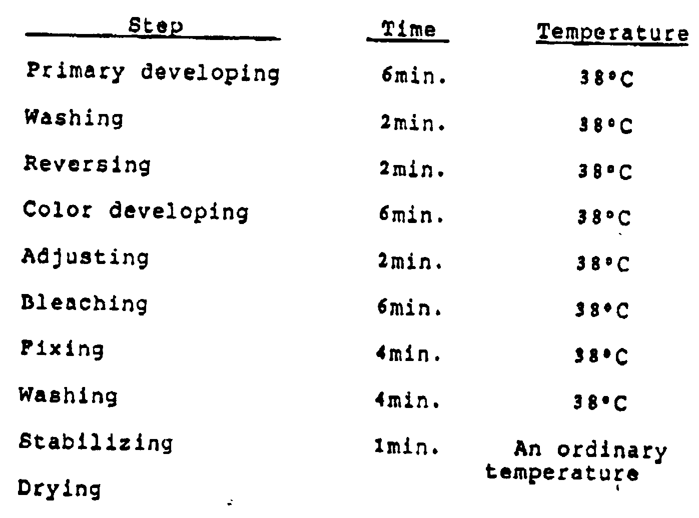

The compositions of the processing liquids were as follows.

(Primary developer)

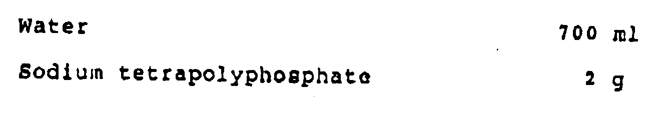

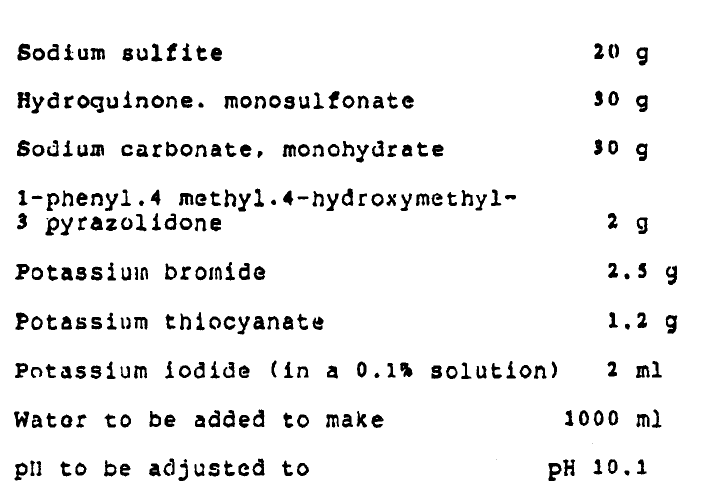

(Reversal bath)

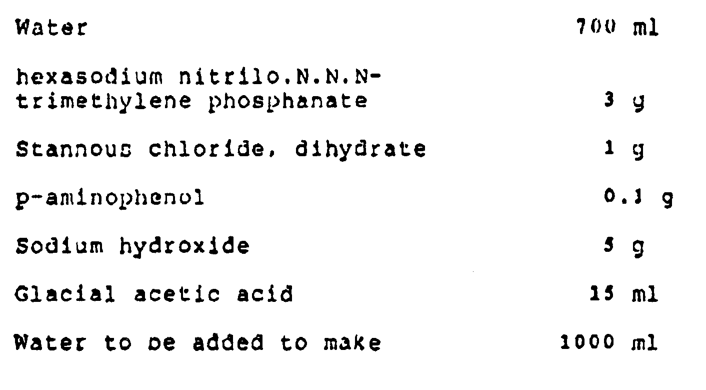

(Color developer C)

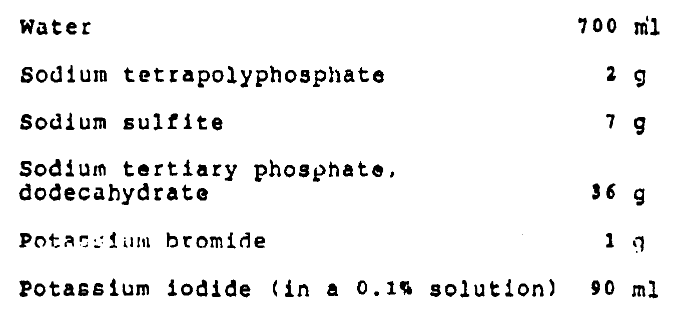

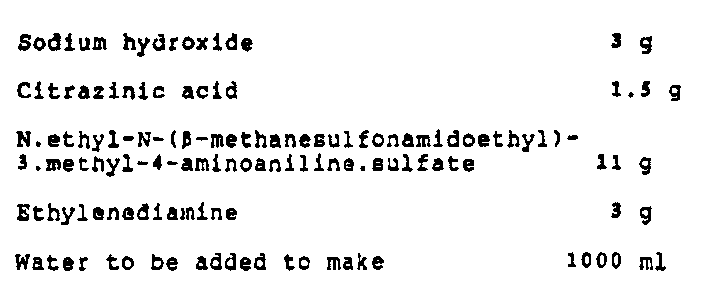

(Adjuster)

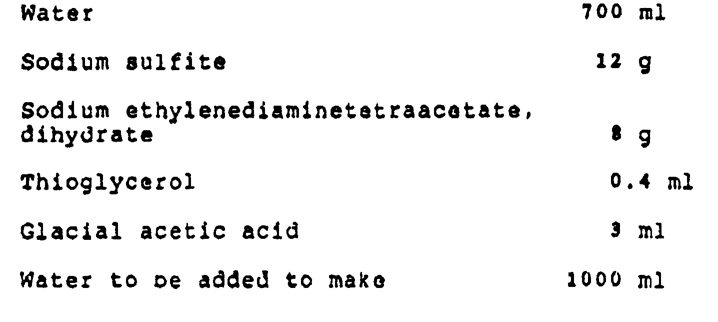

(Bleaching bath)

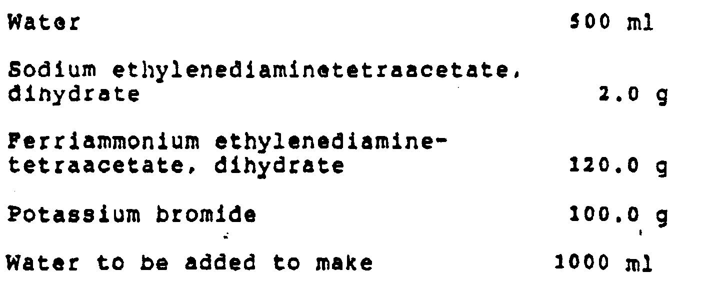

(Fixer)

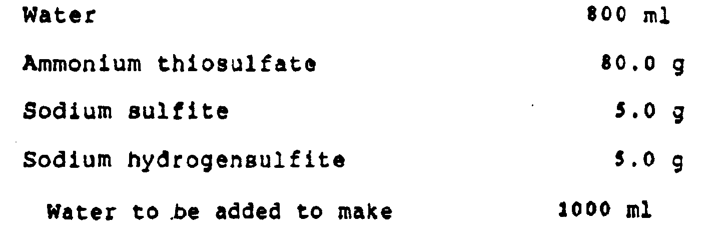

(Stabilizer)

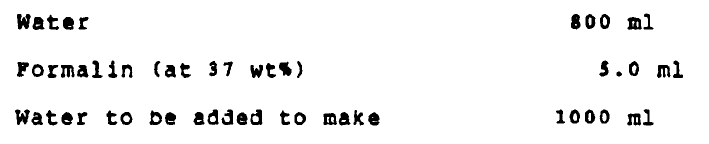

With each of the processed samples No. 1-14 through No. 1-19, the maximum spectral absorption wavelength (λmax), the half band width (W1/2) and DX420 were measured in the same manner as in Example 1-1. The results thereof are shown in Table 1-2.

The measurements of transmission densities relating to the Example 1-2 were made with a densitometer, Model KD-7R.

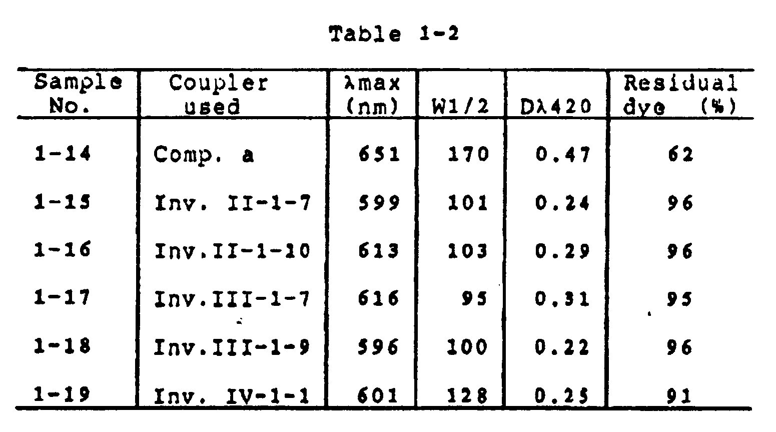

As is obvious from Table 1-2, it is found that every sample having the couplers of the invention has excellent spectral absorption characteristics and excellent color reproducibility, because their half band widths are extremely narrower and their DX420 are also less than those of the sample having the comparative coupler.

It is also found that the Samples used the couplers of the invention are relatively solid, because the percentage of the residual dye-images was remarkably improved.

### Example 2-1

### [Preparation of a red light-sensitive silver halide emulsion]

A silver chlorobromide emulsion containing silver chloride of 96 mol% was added with sodium thiosulfate in an amount of 1x1 0-5 moles per mole of the si Iver chlorobromide emulsion and was then chemically sensitized. Five minutes before completing the chemical sensitization, the resulted chemically sensitized emulsion was added with the following spectral sensitizing dye a, which is to be used in red light-sensitive emulsion layers and was in the form of a 0.1% solution, in an amount of 5.0x10⁻⁵ moles per mole of a silver halide used. Fiver minutes later, the resulted emulsion was added, at the point of time when the chemical sensitization was completed, with an aqueous 0.5% solution of 4-hydroxy-6-methyl-1,3,3a,7-tetrazaindene and, thereafter, with an aqueous 10% gelatin solution. The resulted mixture was stirred and cooled, so that a red light-sensitive silver halide emulsion was prepared.

### (Preparation of light-sensitive material)

Sample 2-1 of red-sensitive color light-sensitive material was prepared by coating the following layers over to a paper support laminated with polyethylene on the both sides thereof in order from the support side, respectively. The amounts of the compounds added are expressed in terms of those added per sq. meter, unless otherwise particularly stated. (Amounts of silver halides are expressed in terms of the contents of silver.)

### Layer 1: An emulsion layer

A red-sensitive emulsion layer comprising 1.2 g of gelatin, 0.30 g of red-sensitive silver chlorobromide emulsion and 0.45 g of comparative cyan coupler a dissolved in 0.24 g of dioctyl phthalate.

### Layer 2: A protective layer

A protective layer containing 1.2 g of gelatin, whereto 2,4-dichloro-6-hydroxy-s-triazine sodium salt was added as a hardener in an amount of 0.017 g per g of the gelatin used.

Next, Samples No.2-2 through No. 2-21 each of the invention were prepared in quite the same manner as in Example 2-1, except that the comparative coupler a and the spectral senstizing dye a were replaced by the couplers of the invention shown in Table 2-1 and the spectral sensitizing dyes shown in Table 2-1, respectively. (The amounts of the couplers and spectral sensitizing dyes were in the amounts of the same moles as in Example 2-1.)

With respect to the above-mentioned Samples No. 2-1 through No. 2-21, for the purpose of checking up the aging stability of the coating liquids used, there prepared two kinds of each sample, i.e., one kind thereof was coated with the first emulsion layer coating liquid aged for one hour after the coating liquid had been prepared and another kind thereof was coated with the same coating liquid but aged for five hours after it had been prepared.

The resulted Samples No. 2-1 through No. 2-21 were exposed to light through a wedge and were then processed in the following processing steps.

(Processing steps)

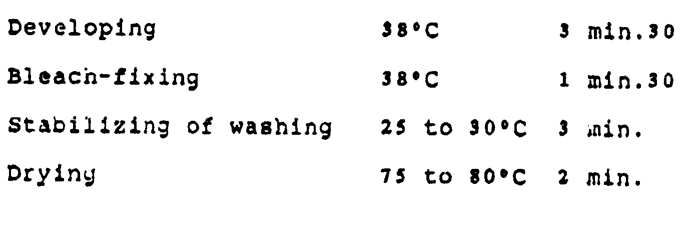

The processing liquids used in each of the steps were as follows.

(Color developer)

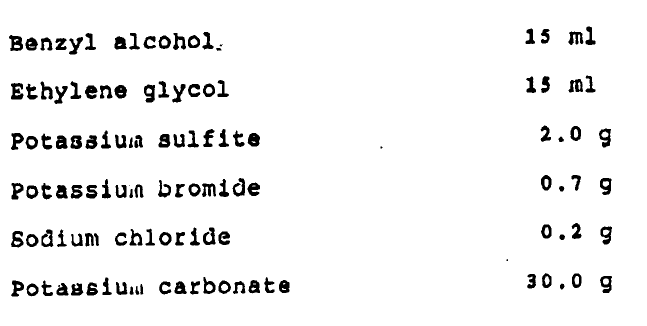

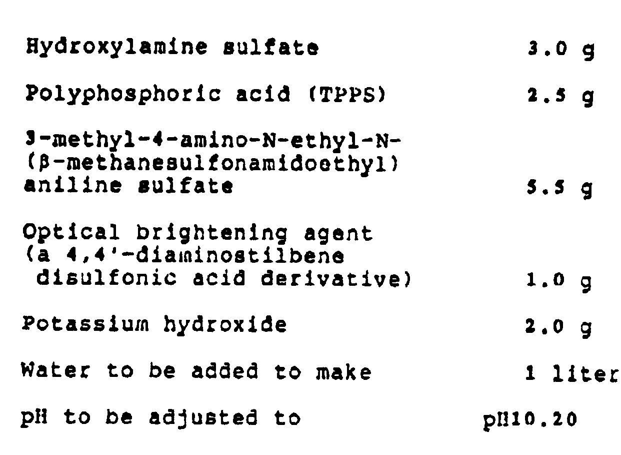

(Bleach-fixer)

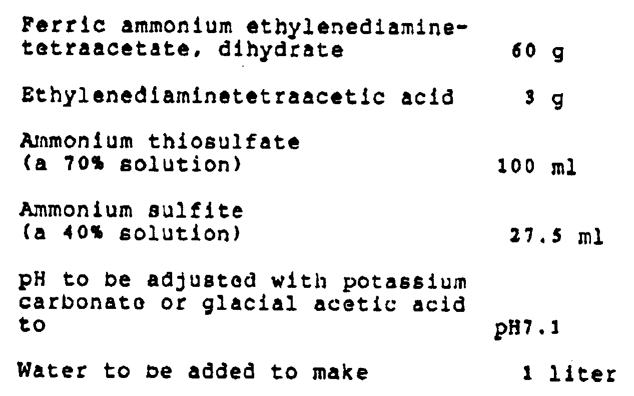

(Stabilizer)

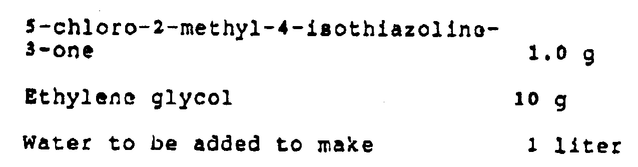

With respect to each sample thus processed and coated after aged for one hour, the measurements were made of the maximum spectral absorption wavelength (λmax), the reflection density of 420 nm when the reflection density of λmax was 1.0, and the half band width (i.e., the difference between the wavelength of the reflection density of 0.5 on the side of the wavelength longer than λmax and the wavelength of the reflection density of 0.5 on the side of the wavelength shorter than λmax). The smaller a value of DX420 is, the less an irregular absorption in red region is. The smaller a half band width is, the sharper an absorption is. The facts mean that a color reproducibility is excellent. Fig. 2-1 shows the absorption spectra of Sample Nos. 2-2, 2-5 and 2-14.

Further, each of the processed samples was allowed to stand for 14 days under the circumstances of a high temperature and humidity (at 60° C and 80%RH), and the heat and moisture resisting properties of the resulted dye-images were checked up. The results thereof are also shown in Table 2-1, wherein the heat and moisture resisting properties of the dye-images are indicated by the percentage of the residual dyes which still remained after the heat and moisture resistance tests were tried, to the initial density of 1.0.

With respect to the samples coated with the coating liquids respectively aged for one hour and for 5 hours, the densities thereof were measured with a densitometer to check up the sensitivities and fogs thereof. The standard optical density point for determining the sensitivities of the samples was set to be a point of fog plus 0.20.

For the relative sensitivity ratio, Sample No. 2-1 containing comparative coupler a and spectral sensitizing dye a, whose coating liquid was allowed to stand for one hour, was regarded as the criterion of 100%.

For those measurements, a densitometer, Model KD-7R (manufactured by Konishiroku Photo Ind. Co., Ltd.), was used.

The results thereof are correctively shown in Table 2-1.

Comparative coupler a

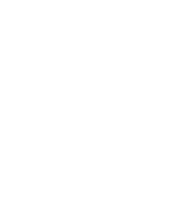

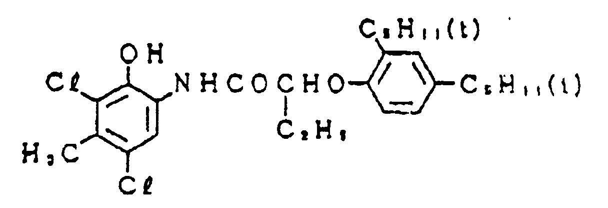

Spectral sensitizing dye a

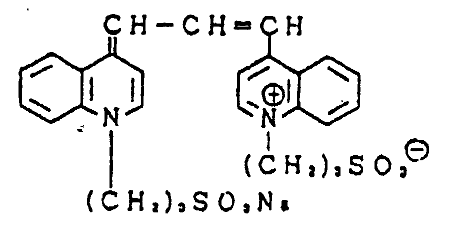

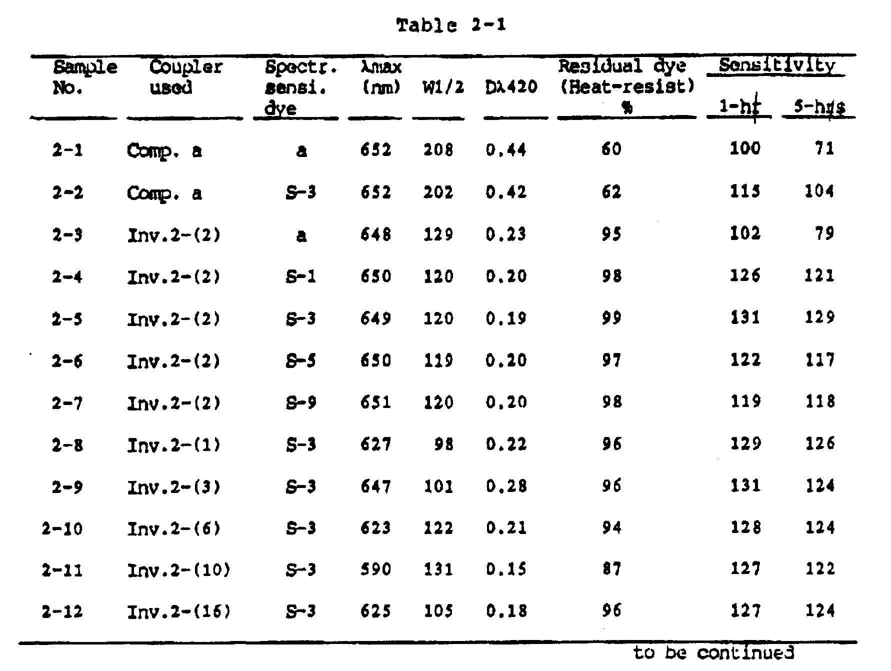

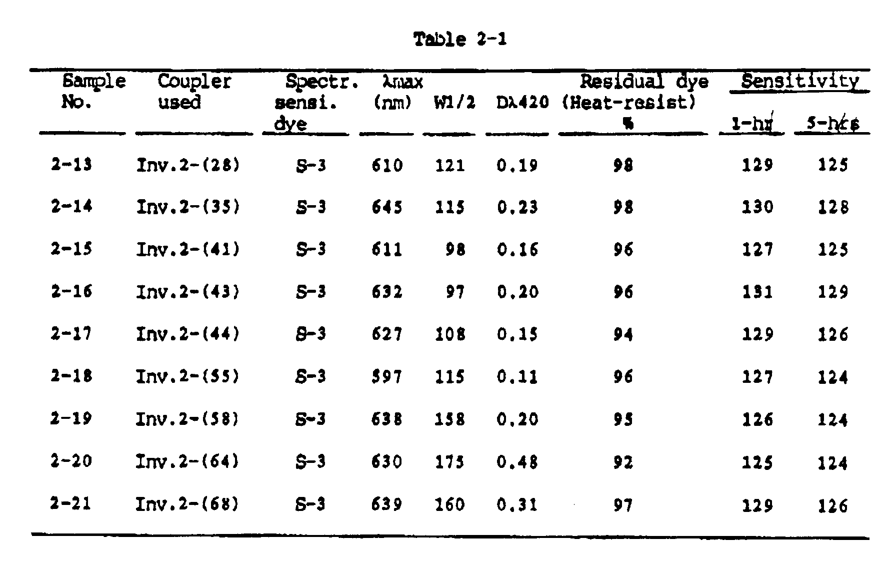

As is obvious from Table 2-1, it is found that every sample having the couplers of the invention has excellent color reproducibility, because their half band widths are narrower, irregular absorptions are less and the percentages of residual dyes are higher, as compared with those of the samples having the comparative couplers. It is further found that the examples of the invention are desirable embidiments, because a sensitivity lowering caused by aging coating liquids can be improved when using the spectral sensitizing dyes preferably applicable to the invention in combination.

In addition, Fig. 2-1 shows the fact that the couplers of the invention are relatively less in undesirable irregular absorption caused in green region (of 550 nm) and relatively sharp in absorption in the regions around λmax, as compared with the conventional phenol type couplers.

### Example 2-2

A red light-sensitive silver halide emulsion was prepared in the same amnner as in Example 2-1, except that the couplers and spectral sensitizing dyes used in Example 2-1 were replaced by those shown in Table 2-2 when preparing.

Samples No. 2-22 through No. 2-29 of red-sensitive color reversal photographic light-sensitive materials were prepared by coating the following layers over to a triacetyl cellulose fi 1m support, in orderfrom the support side.

The amounts of the compounds added are expressed by the amounts thereof per sq.meter unless otherwise specially stated. (Amounts of silver halides are expressed in terms of the contents of silver.)

### Layer 1: An emulsion layer

A red-sensitive emulsion layer comprising 1.4 g of gelatin, 0.5 g of red-sensitive silver chlorobromide emulsion and the couplers shown in Table 2-2 dissolved in 0.24 g of dibutyl phthalate.

### Layer 2: A protective layer

A protective layer containing 0.5 g of gelatin, whereto 2,4-dichloro-6-hydroxy-s-triazine sodium salt was added as a hardener in an amount of 0.017 g per g of the gelatin used.

The resulted Samples were exposed to light through a wedge in an ordinary method and were then processed in the following processing steps.

(Reversal processing steps)

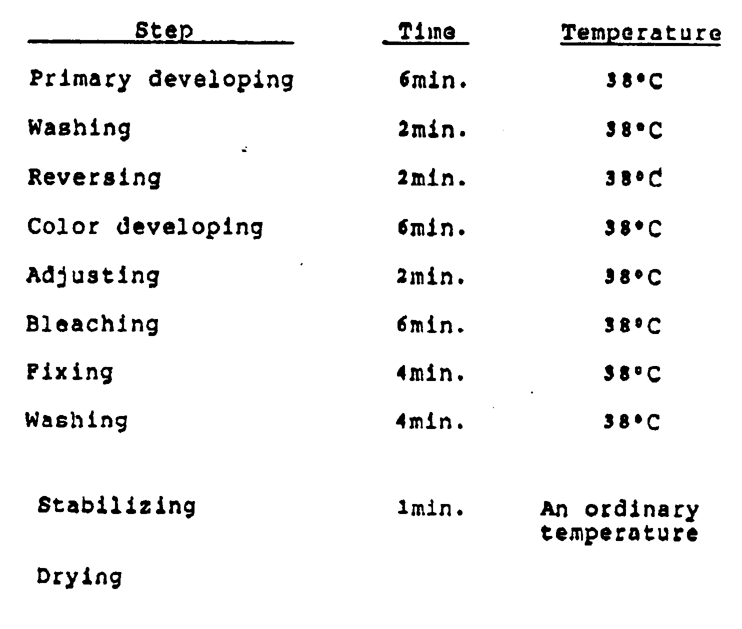

The compositions of the processing liquids were as follows.

(Primary developer)

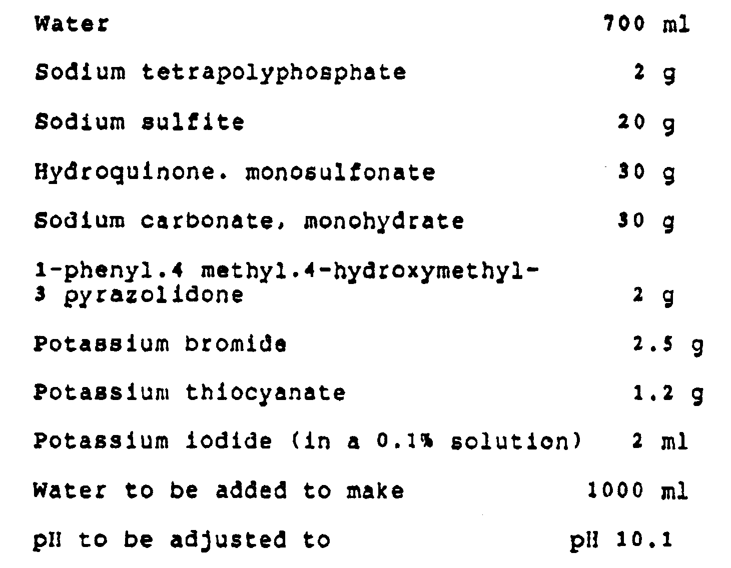

(Reversal bath)

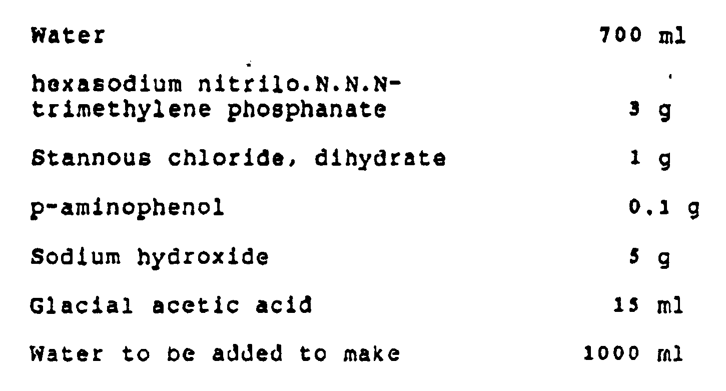

(Color developer C)

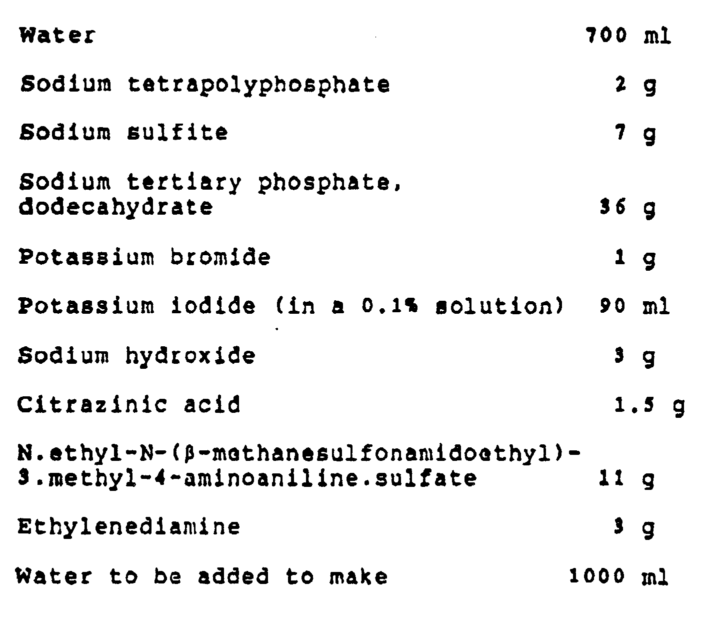

(Adjuster)

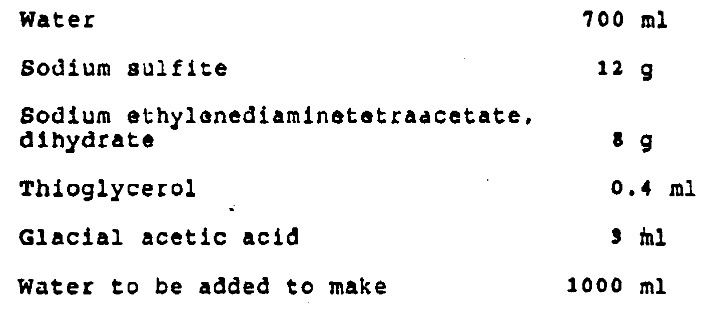

(Bleaching bath)

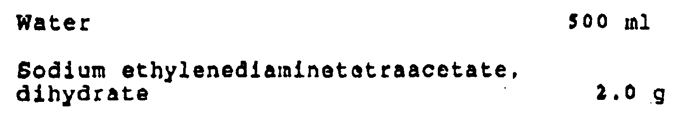

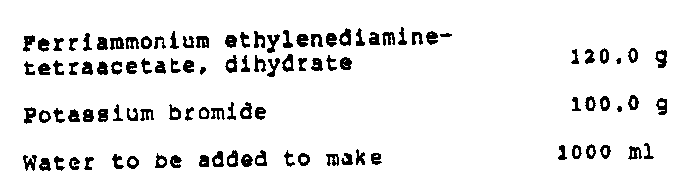

(Fixer)

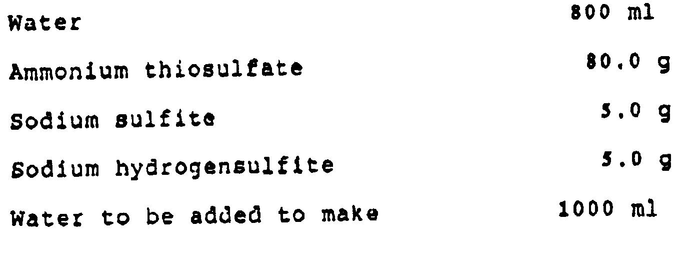

(Stabilizer)

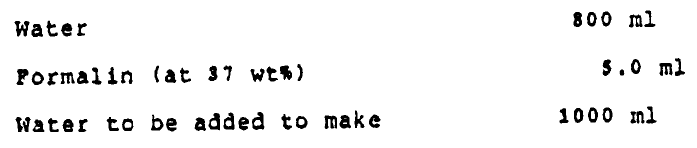

With respect to the samples coated with the primary emulsion layer coating liquid so prepared as to have the aforementioned composition, there obtained each sensitivitiy ratio of the samples coated with the coating liquids which were aged respectively for one hour and for 5 hours in the same manner as in Example 2-1 and, at the same time, there measured the maximum spectral absorption wavelength (Xmax) and half band width (W1/2) of each sample thus processed, in the same manner as in Example 2-1. The results thereof are shown in Table 2-2.

The measurements of transmission densities relating to the Example 2-2 were made with a densitometer, Model KD-7R.

Forthe relative sensitivity ratio, Sample No. 2-22 containing comparative coupler a and spectral sensitizing dye b, whose coating liquid was allowed to stand for one hour, was regarded as the criterion of 100%.

Spectral sensitizing dye b

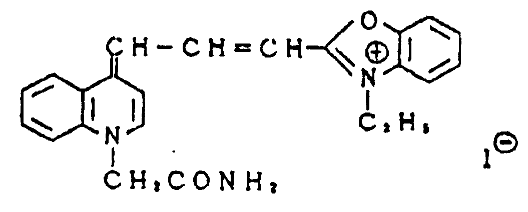

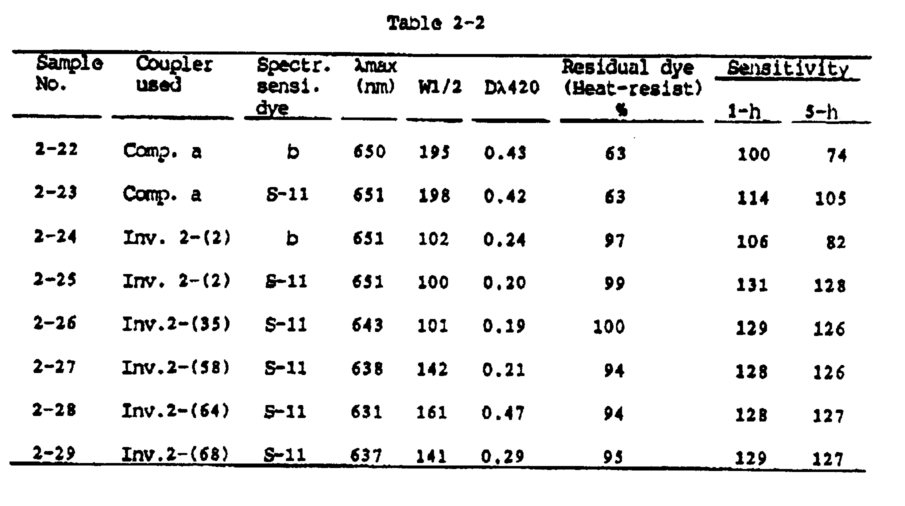

As is obvious from Table 2-2, it is found that the samples using the couplers of the invention are relatively narrow in half band width and excellent in color reproducibility and that the percentage of residual dye images are remarkably improved as compared to those of the comparative samples.

It is also found that the couplers of the invention are high in sensitivity and exellent in aging stability of coating liquids, provided that the couplers are used together with the spectral sensitizing dyes which may preferably be used in the invention.

### Example 3-1

Sample 3-1 of red-sensitive color light-sensitive material was prepared by coating the following layers over to a paper support laminated with polyethylene on the both sides thereof in order from the support side, respectively. The amounts of the compounds added are expressed in terms of those added persq. meter, unless otherwise particularly stated. (Amounts of silver halides are expressed in terms of the contents of silver.)

### Layer 1: An emulsion layer

A red-sensitive emulsion layer comprising 1.2 g of gelatin, 0.30 g of red-sensitive silver chlorobromide emulsion (containing silver chloride of 96 mol%) and 0.45 g of comparative cyan coupler a dissolved in 0.20 g of dioctyl phthalate.

### Layer 2: A protective layer

A protective layer containing 0.50 g of gelatin, whereto 2,4-dichloro-6-hydroxy-s-triazine sodium salt was added as a hardener in an amount of 0.017 g per g of the gelatin used.

Next, Samples No. 3-2 through No. 3-24 each of the invention were prepared in quite the same manner as in Example 3-1, except that the comparative coupler a was replaced by the couplers indicated in Table 3-1 (provided that the amounts added were the same molar amounts as in the comparative coupler a).

The resulted Samples No. 3-12 through No. 3-24 were exposed to light through a wedge in an ordinary method and were then processed in the following processing steps.

(Processing steps)

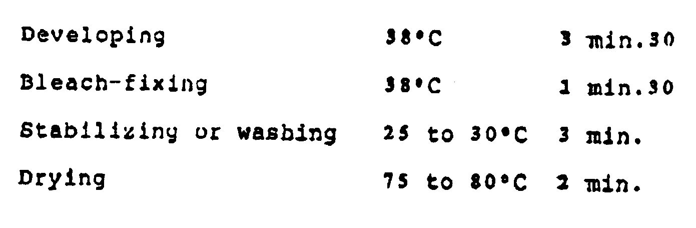

The processing liquids used in each of the steps were as follows.

(Color developer)

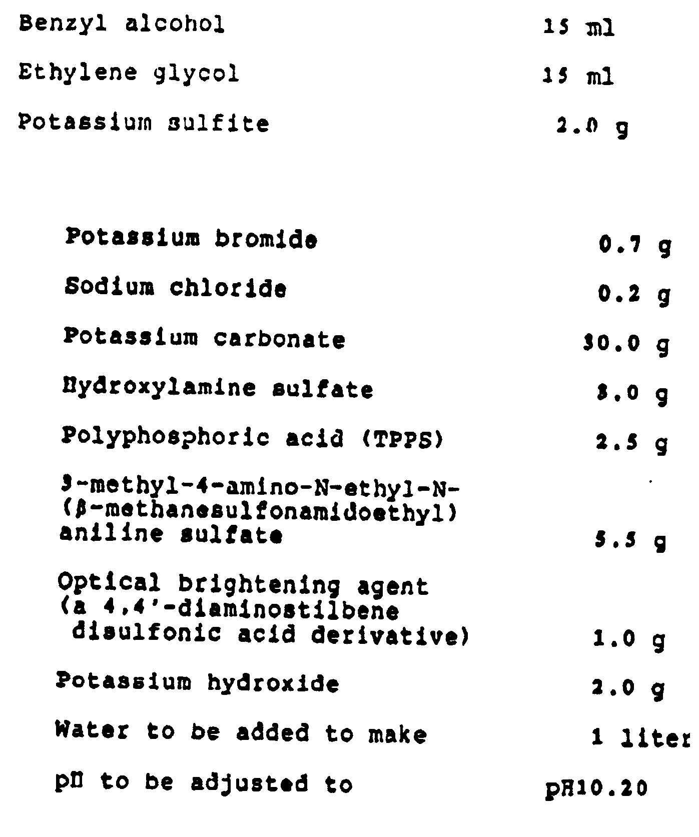

(Bleach-fixer)

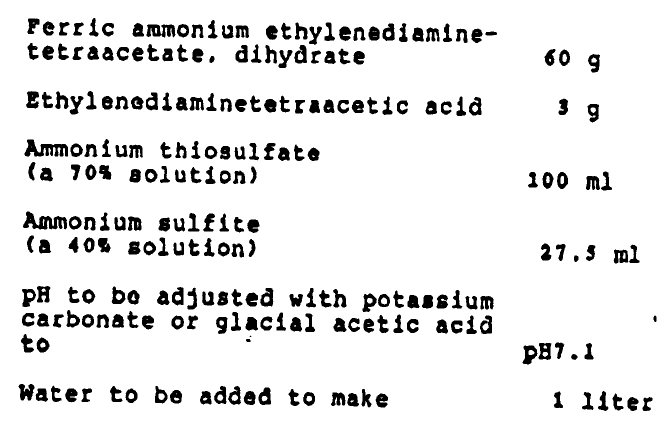

(Stabilizer)

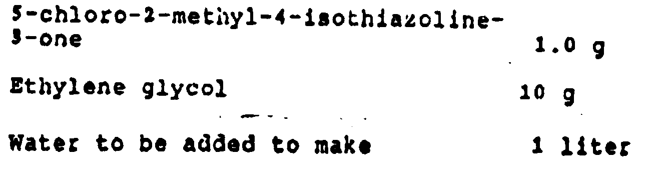

With respect to thus processed Samples No. 3-1 through No. 3-13, the measurements were made respectively each of the maximum spectral absorption wavelengths (λmax), the reflection densities of 420 nm (DX420) when the reflection densities of λmax were 1.0, and the half band widths (W1/2) which are the difference between one point on the longer wavelength side than each λmax and the shorter wavelength side than each λmax when the reflection densities of λmax were 0.5. After then, the spectral absorption characteristics and the color reproducibility of each Sample were examined.

The smaller a value of DX420 is, the less an irregular absorption in green region is. The smaller a value of W1/2 is, the sharper an absorption is. The facts mean that a color reproducibility is excellent.

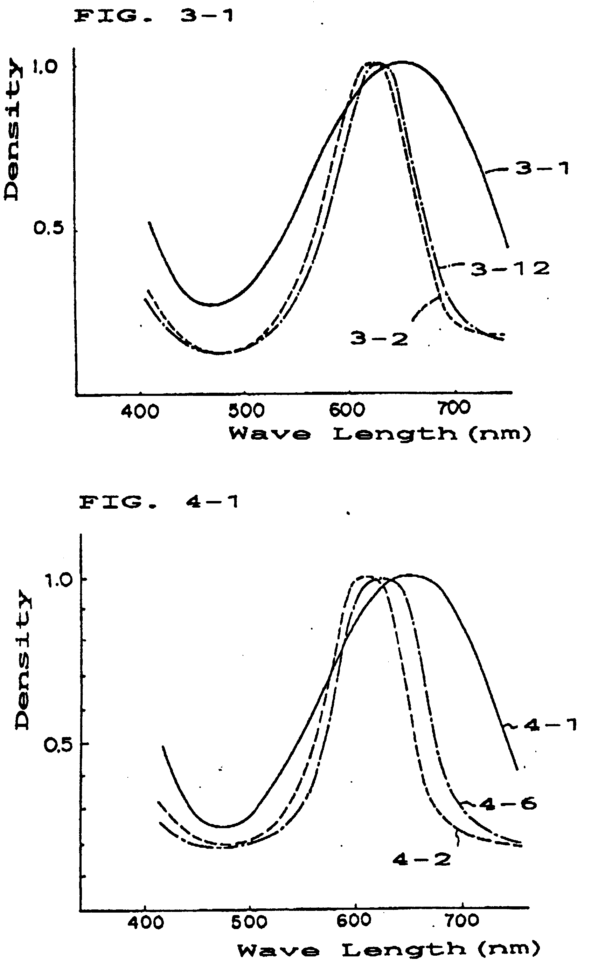

Fig. 3-1 shows the absorption spectra of Sample Nos. 3-1, 3-2 and 3-12.

Further, each of the processed samples was allowed to stand for 14 days under the circumstances of a high temperature and humidity (at 60° C and 80%RH), and the heat and moisture resisting properties of the resulted dye-images were checked up. The results thereof are also shown in Table 3-1, wherein the heat and moisture resisting properties of the dye-images are indicated by the percentage of the residual dyes which still remained after the heat and moisture resistance tests were tried, to the initial density of 1.0.

For those measurements, a densitometer, Model KD-7R (manufactured by Konishiroku Photo Ind. Co., Ltd.), was used.

Comparative Coupler a

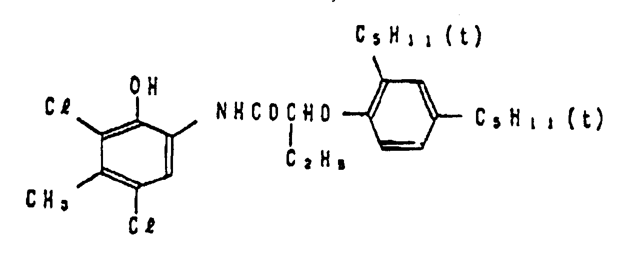

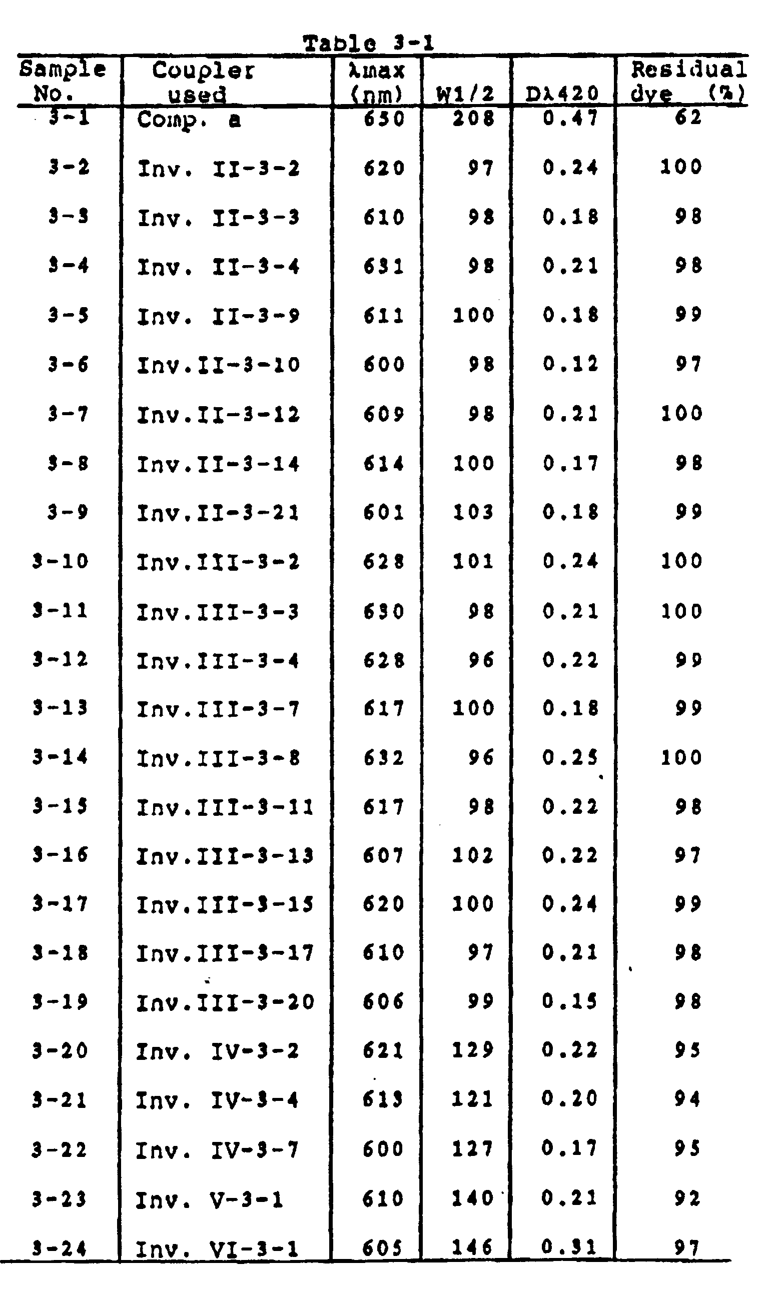

As is obvious from Table 3-1, it is found that every sample having the couplers of the invention has excellent spectral absorption characteristics, because their half band widths are extremely narrower and the irregular absorptions are relatively less, as compared with the sample used the comparative coupler. It is further found that every sample having the couplers of the invention is relatively solid, because of the high percentage of residual dyes and the relatively excellent moisture resisting property.

In addition, Fig. 3-1 shows the fact that the couplers of the invention are relatively less in undesirable irregular absorption caused in green region and relatively sharp in absorption in the regions around λmax, as compared with the conventional phenol type couplers.

### Example 3-2

Samples No. 3-25 through No. 3-32 of red-sensitive color reversal photographic light-sensitive materials were prepared by coating the following layers in order from a triacetyl cellulose film support side over to the support.

The amounds of the compounds added are expressed by the amounts thereof per sq.meter unless otherwise specially stated. (Amounts of silver halides are expressed in terms of the contents of silver.)

### Layer 1: An emulsion layer

A red-sensitive emulsion layer comprising 1.4 g of gelatin, 0.5 g of red-sensitive silver chlorobromide emulsion (containing silver chloride of 96 mol%) and the couplers shown in Table 3-2 dissolved in 0.24 g of dibutyl phthalate.

### Layer 2: A protective layer

A protective layer containing 0.5 g of gelatin, whereto 2,4-dichloro-6-hydroxy-s-triazine sodium salt was added as a hardener in an amount of 0.017 g per g of the gelatin used.

The resulted Samples were exposed to light through a wedge in an ordinary method and were then processed in the following processing steps.

(Reversal processing steps)

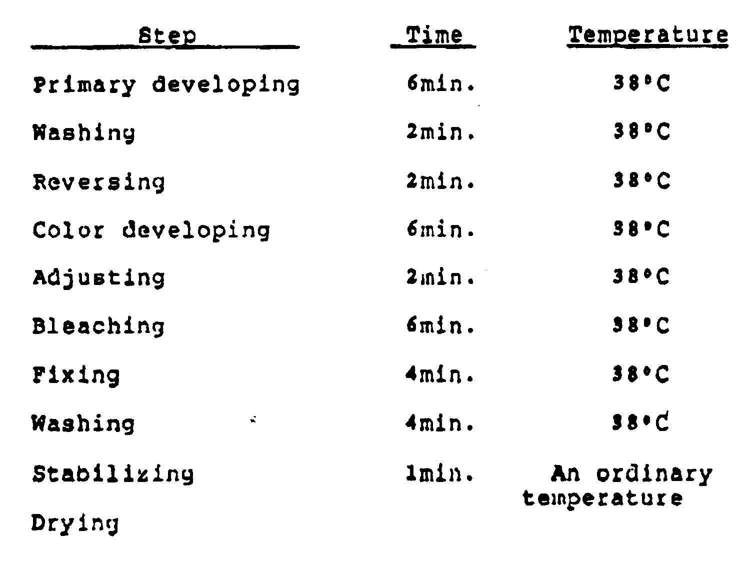

The compositions of the processing liquids were as follows.

(Primary developer)

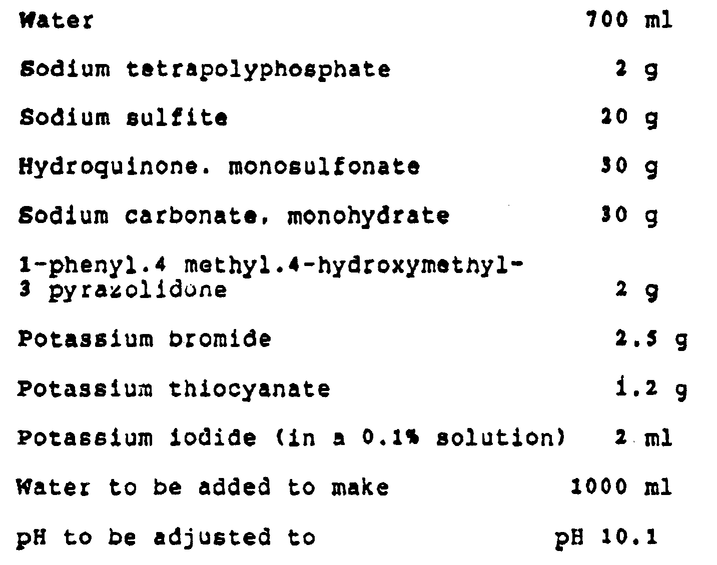

(Reversal path)

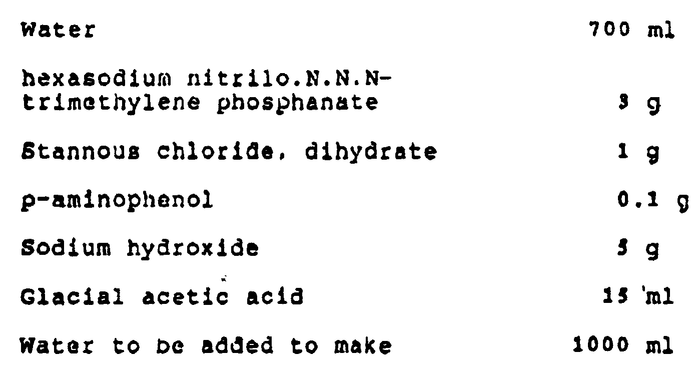

(Color developer C)

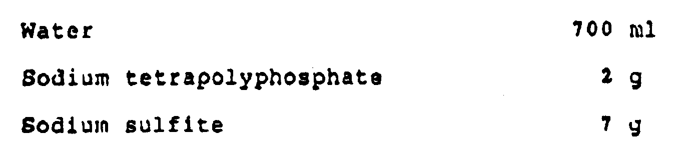

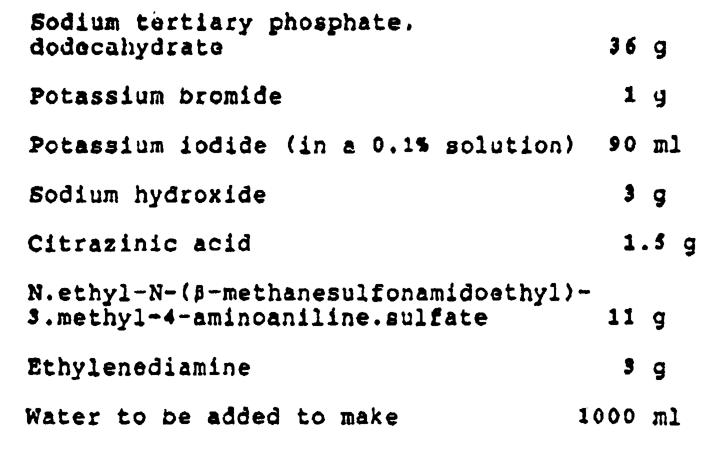

(Adjuster)

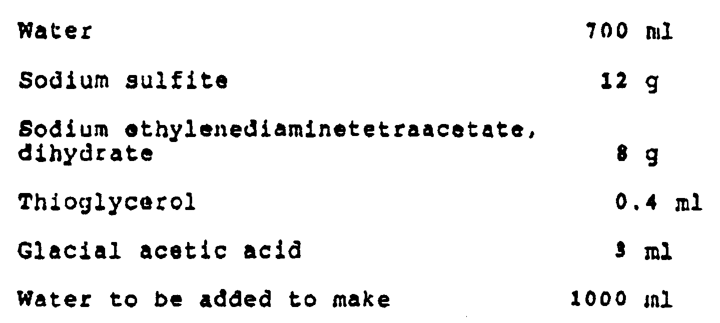

(Bleaching bath)

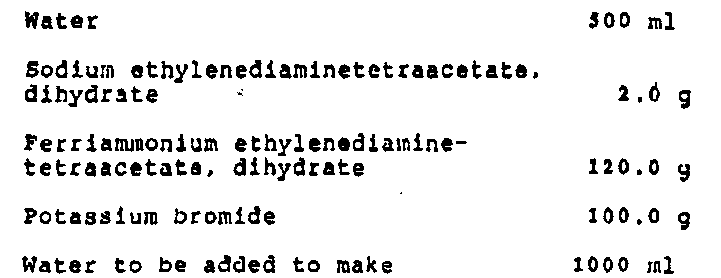

(Fixer)

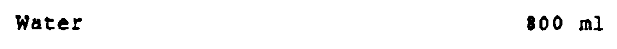

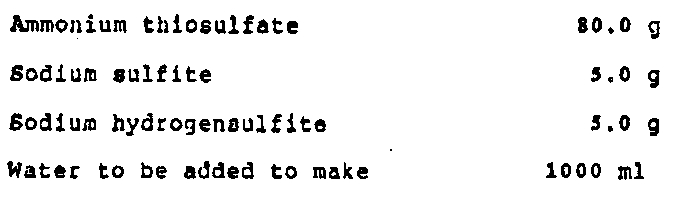

(Stabilizer)

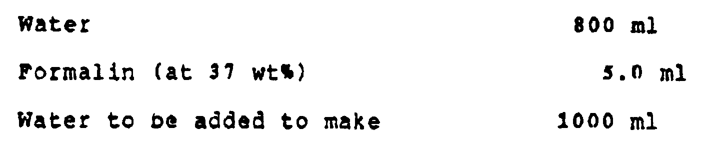

With each of the processed samples No. 3-25 through No. 3-32, the maximum spectral absorption wavelength (λmax) and the half band width (W1/2).Dλ420 were measured in the same manner as in Example 3-1. The results thereof are shown in Table 3-2.

The measurements of transmission densities relating to the Example 3-2 were made with a densitometer, Model KD-7R.

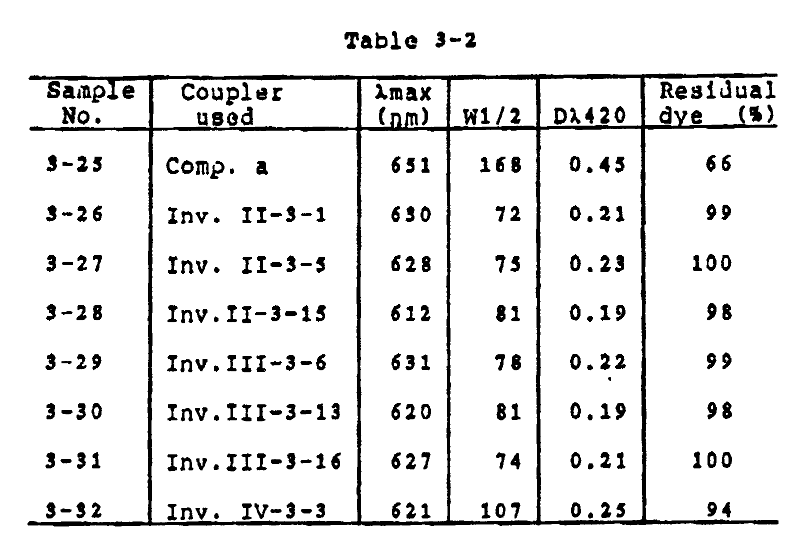

As is obvious from Table 3-2, it is found that every sample having the couplers of the invention has excellent spectral absorption characteristics and excellent color reproducibility, because their half band widths are extremely narrower and their DX420 are also less than those of the sample having the comparative coupler.

It is also found that the samples used the couplers of the invention were remarkably improved in the percentage of the residual dye-images and were also relatively solid.

### Example 4-1

Sample 4-1 of red-sensitive color light-sensitive material was prepared by coating the following layers over to a paper support laminated with polyethylene on the both sides thereof in order from the support side, respectively. The amounts of the compounds are expressed in terms of those added per sq. meter, unless otherwise particularly sated. (Amounts of silver halides are expressed in terms of the contents of silver.)

### Layer 1: An emulsion layer

A red-sensitive emulsion layer comprising 1.2 g of gelatin, 0.30 g of red-sensitive silver chlorobromide emulsion (containing silver chloride of 96 mol%) and 0.45 g of comparative cyan coupler a dissolved in 1.50 g of trioctyl phosphate.

### Layer 2: A protective layer

A protective layer containing 0.50 g of gelatin, whereto 2,4-dichloro-6-hydroxy-s-triazine sodium salt was added as a hardener in an amount of 0.017 g per g of the gelatin used.

Next, Samples No. 4-2 through No. 4-9 each of the invention were prepared in quite the same manner as in Example 4-1, except that the comparative coupler a was replaced by the couplers indicated in Table 4-1 (provided that the amounts added were the same molar amounts as in the comparative coupler a).

The resulted Samples No. 4-1 through No. 4-9 were exposed to light through a wedge in an ordinary method and were then processed in the following processing steps.

(Processing steps)

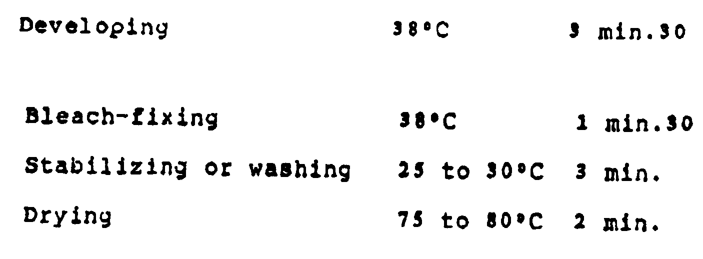

The processing liquids used in each of the steps were as follows.

(Color devoloper)

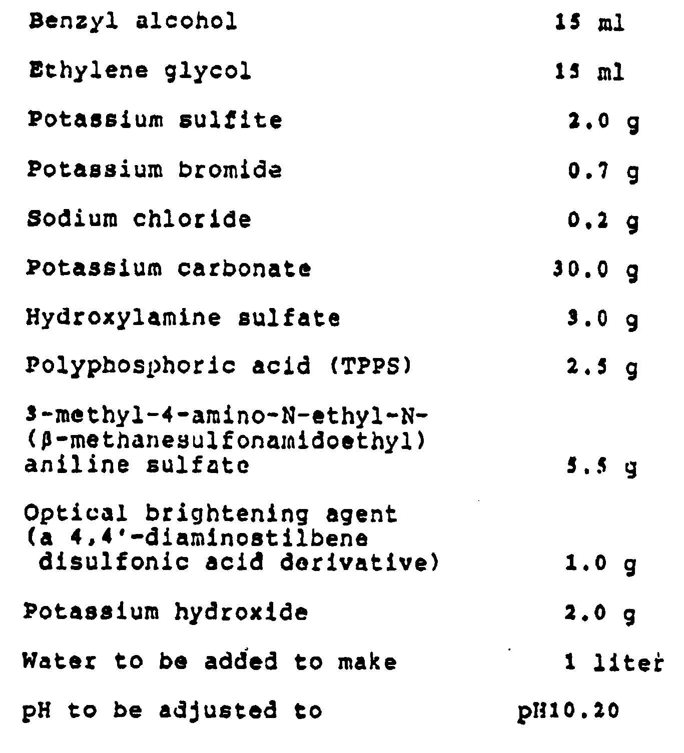

(Bleach-fixer)

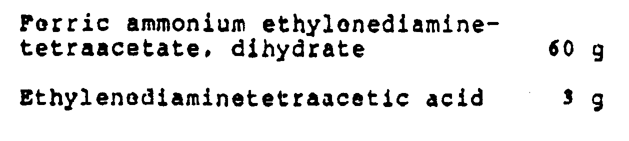

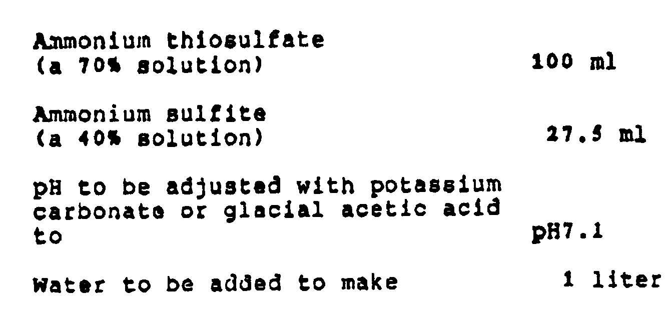

(Stabilizer)

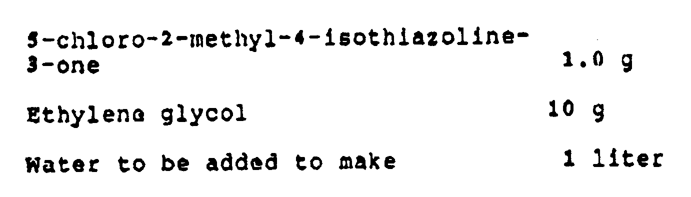

With respect to each processed Sample, the measurements were made respectively each of the maximum spectral absorption wavelengths (λmax), the reflexion densities of 420 nm (DX420) when the reflection densities of λmax were 1.0, and the half band widths (W1/2) which are the difference between the reflection density 0.5 on the side of the wavelength longer than λmax and the reflection density of 0.5 on the side of the wavelength shorter than λmax). After then, the spectral absorption characteristics and the color reproducibility of each sample were examined.

The smaller a value of DX420 is, the less an irregular absorption in green region is. The smaller a value of W1/2 is, the sharper an absorption is. The facts mean that a color reproducibility is excellent.

Fig. 4-1 shows the absorption spectra of Sample Nos. 4-1, 4-2 and 4-6.

Further, each of the processed samples was allowed to stand for 14 days under the circumstances of a high temperature and humidity (at 60° C and 80%RH), and the heat and moisture resisting properties of the resulted dye-images were checked up. The results thereof are also shown in Table 4-1, wherein the heat and moisture resisting properties of the dye-images are indicated by the percentage of the residual dyes which still remained after the heat and moisture resistance tests were tried, to the initial density of 1.0.

For those measurements, a densitometer, Model KD-7R (manufactured by Konishiroku Photo Ind. Co., Ltd.), was used.

The results thereof are correctively shown in Table 4-1.

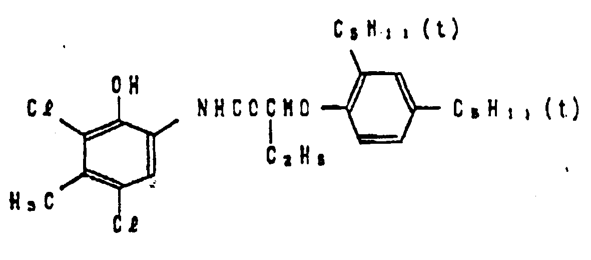

Comparative coupler a

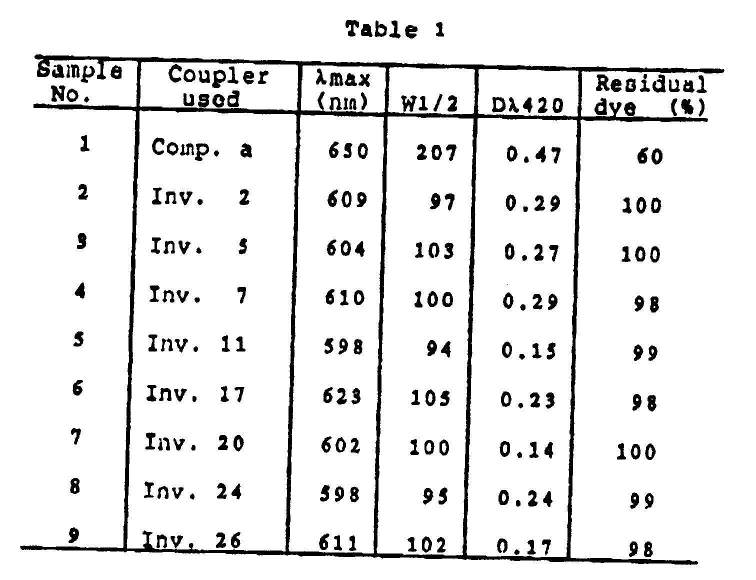

As is obvious from Table 4-1, it is found that every sample having the couplers of the invention has excellent spectral absorption characteristics, because their half band widths are extremely narrower than those of the sample having the comparative coupler and their irregular absorptions represented by DX420 are relatively less, as compared with the sample used the comparative coupler.

It is further found that every sample having the couplers of the invention can provide solid cyan images, because of their high percentage of residual dyes and excellent moisture resisting property.

In addition, Fig. 4-1 shows the fact that the couplers of the invention (e.g., Sample No. 4-2 and No. 4-6) are relatively less in undesirable irregular absorption caused in green region and relatively sharp in absorption in the regions around λmax, as compared with the conventional phenol type couplers.

### Example 4-2

Samples No. 4-10 through No. 4-16 of red-sensitive color reversal photographic light-sensitive materials were prepared by coating the following layers in order from the support side over to a triacetyl cellulose film support.

The amounts of the compounds added are expressed by the amounts thereof per sq.meter unless otherwise specially stated. (Amounts of silver halides are expressed in terms of the contents of silver.)

### Layer 1: An emulsion layer

A red-sensitive emulsion layer comprising 1.4 g of gelatin, 0.5 g of red-sensitive silver chlorobromide emulsion (containing silver chloride of 96 mol%) and the couplers (9.1x10-⁴ moles) shown in Table 4-2 dissolved in 1.65 g of dioctyl phosphate.

### Layer 2: A protective layer

A protective layer containing 0.5 g of gelatin, whereto 2,4-dichloro-6-hydroxy-s-triazine sodium salt was added as a hardener in an amount of 0.017 g per g of the gelatin used.

The resulted Samples were exposed to light through a wedge in an ordinary method and were then processed in the following processing steps.

(Reversal processing steps)

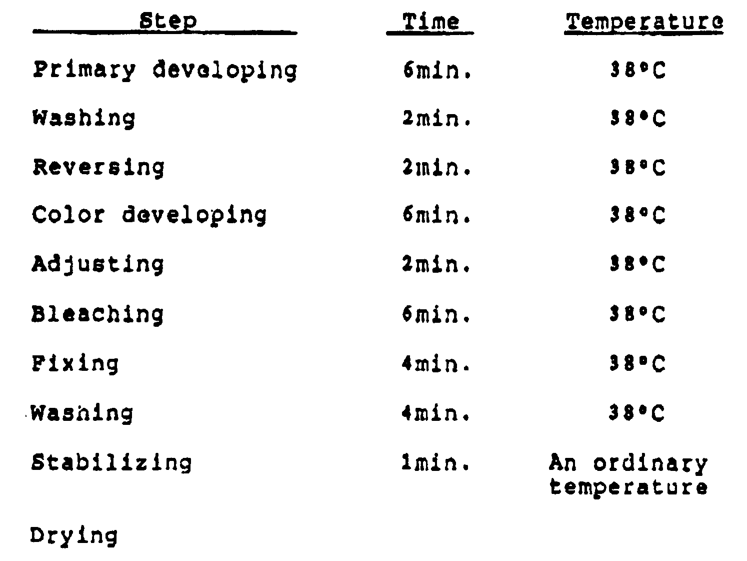

The compositions of the processing liquids were as follows.

(Primary developer)

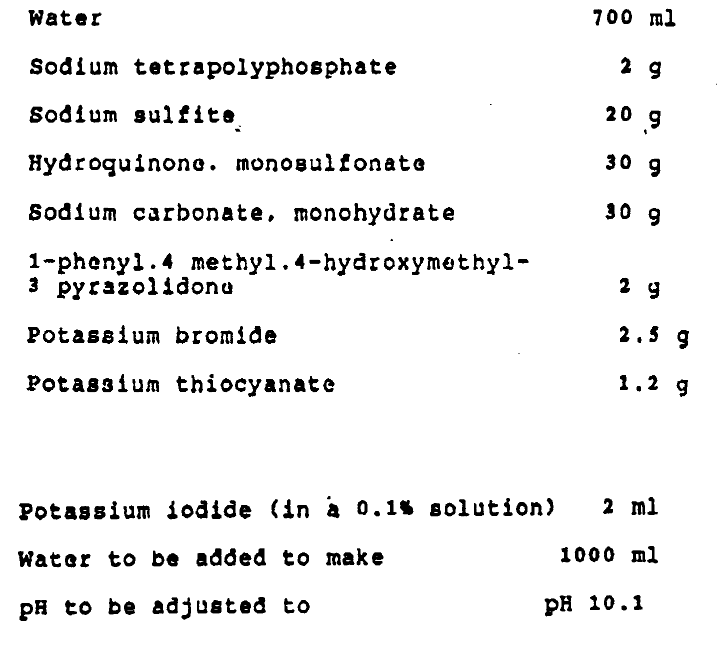

(Reversal bath)

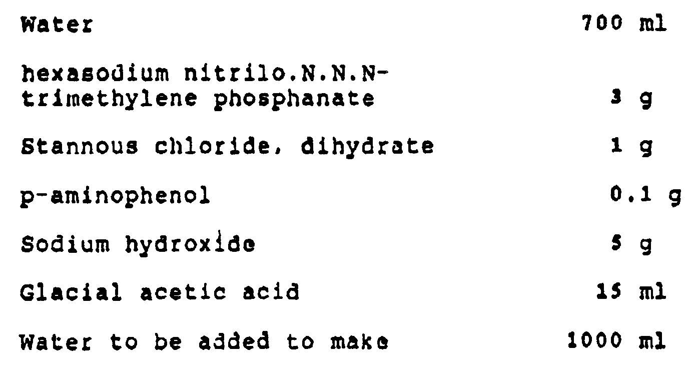

(Color developer)

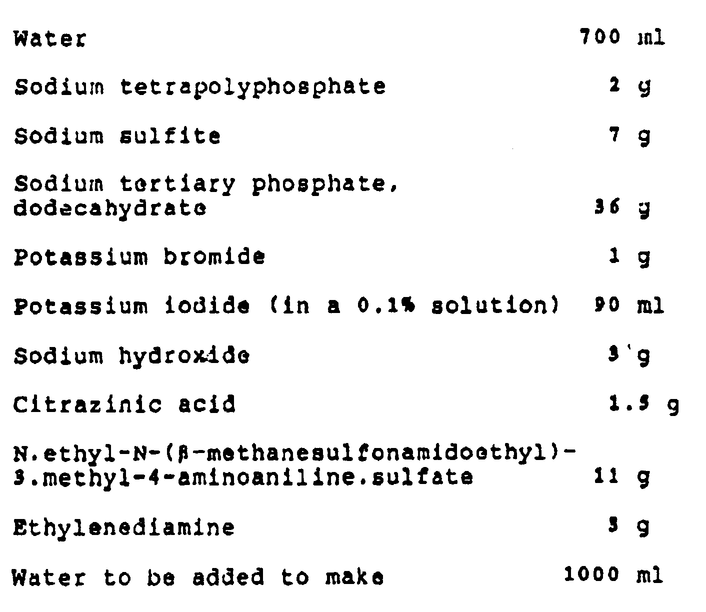

(Adjuster)

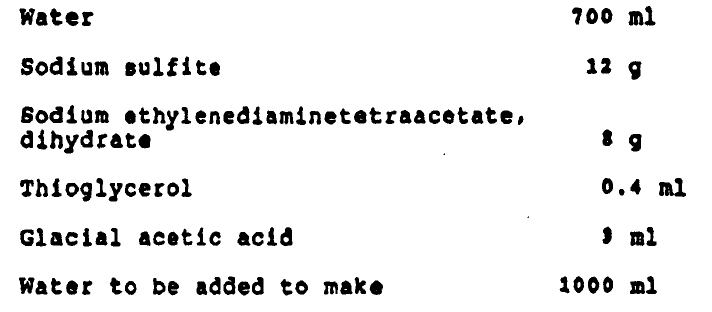

(Bleaching bath)

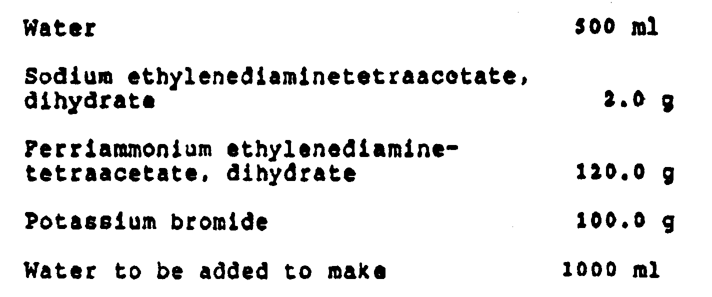

(Fixer)

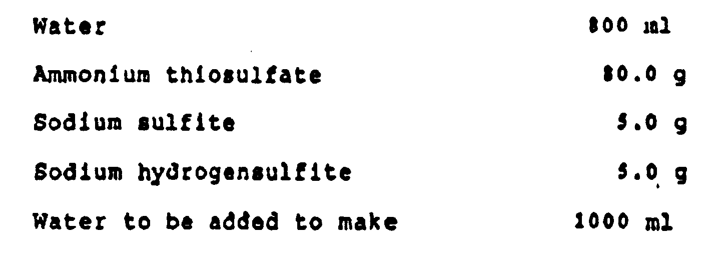

(Stabilizer)

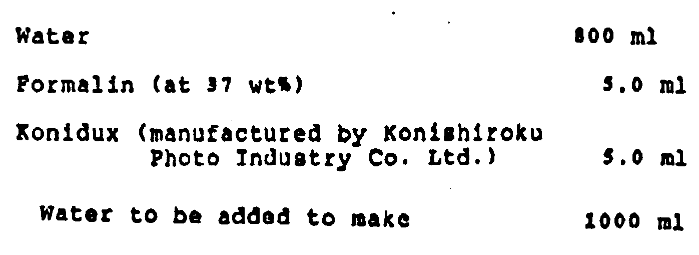

With each of the processed samples, the maximum spectral obsorption wavelength (λmax), the hale band width (W1/2) and DX420 were measured in the same manner as in Example 4-1. The results thereof are shown in Table 4-2.

The measurements of transmission densities relating to the Example 4-2 were made with a densitometer, Model KD-7R.

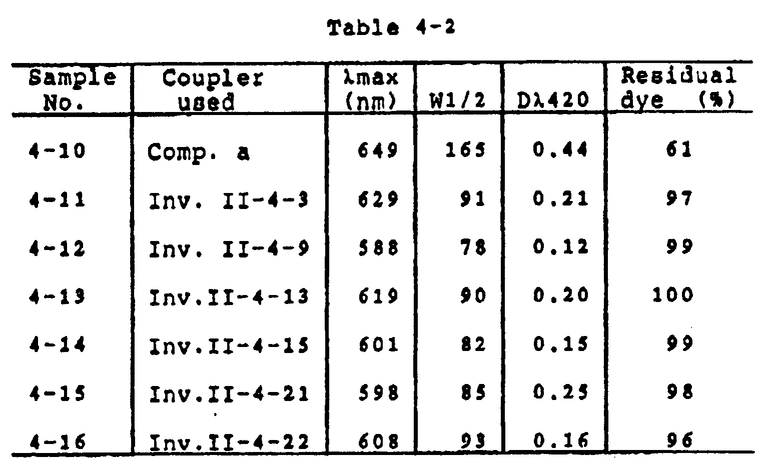

As is obvious from Table 4-2, i is found that every example having the couplers of the invention has excellent spectral absorption characteristics, because their half band widths are extremely narrower and their Dλ420 are also less than those of the sample having the comparative coupler. They can accordingly provide cyan images having excellent color reproducibility.

They can also provide solid cyan images, because their percentage of residual dyes is extremely high in heat resisting test.

### [Dispersion stability tests]

A dispersion was prepared in the following manner.

Comparative cyan coupler b of 2x10-³ moles, 3.0 g of dioctyl phthalate and 3 g of ethyle acetate were mixed and heated up to 60° C. The resulted solution was mixed with 17 ml of an aqueous 5% gelatin solution containing 1.7 ml of an aqueous solution of Alkanol B (i.e., alkylnaphthalene sulfonate, manufactured by DuPont). The resulted mixture solution was emulsified and dispersed by means of a supersonic homogenizer.

From the resulted dispersion, ethyl acetate was distilled off under reduced pressure, so that dispersion sample 4-1 was prepared. For the purpose of observing the dispersion stability of cyan couplers, the dispersion was stored in a refrigerator (at 5° C) for six days and was then put in a warm water bath at 40° C to keep temperature. The conditions of each crystal deposition were observed by an optical microscope, immediately after, 6 hours after and 12 hours after keeping temperature, respectively.

Further, dispersion samples No. 4-2 through No. 4-5 were prepared by making use of the couplers shown in Table 4-3 in the same manner as in Sample 4-1, and the dispersion stability of the couplers were also checked up. The results thereof are shown in Table 4-3.

Comparative coupler D

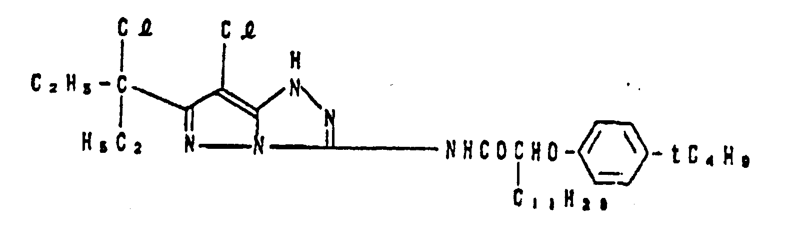

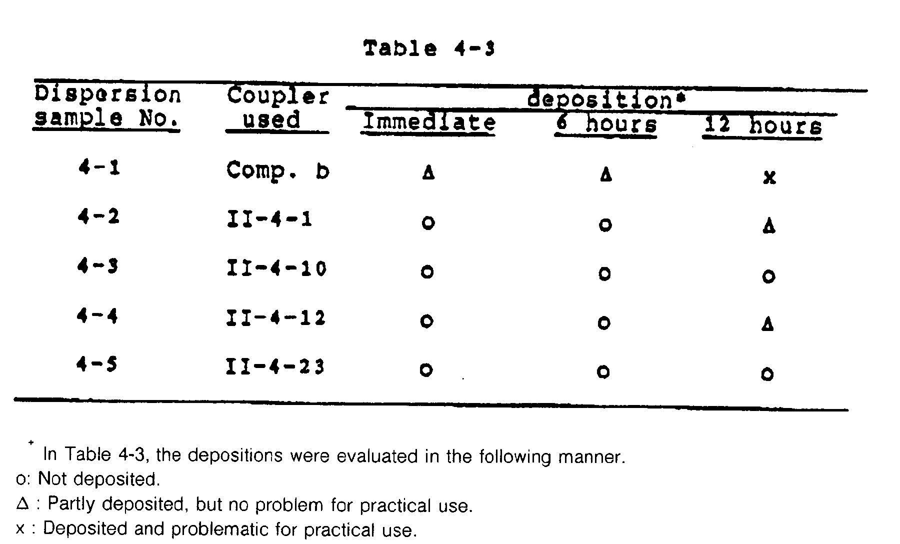

As is obvious from Table 4-3, it is found that, when using the cyan couplers of the invention are used, dispersions having excellent dispersion stability can be obtained, because deposition is hardly produced as compared to those produced with comparative coupler b.

### Example 5-1

Sample 5-1 of red-sensitive color light-sensitive material was prepared by coating the following layers over to a paper support laminated with polyethylene on the both sides thereof in order from the support side, respectively. The amounts of the compounds added are expressed in terms of those added persq. meter, unless otherwise particularly stated. (Amounts of silver halides are expressed in terms of the contents of silver.)

### Layer 1: An emulsion layer

A red-sensitive emulsion layer comprising 1.2 g of gelatin, 0.30 g of red-sensitive silver chlorobromide emulsion (containing silver chloride of 96 mol%) and 0.45 g of comparative cyan coupler a dissolved in 0.20 g of dioctyl phthalate.

### Layer 2: A protective layer

A protective layer containing 0.50 g of gelatin, whereto 2,4-dichloro-6-hydroxy-s-triazine sodium salt was added as a hardener in an amount of 0.017 g per g of the gelatin used.

Next, Samples No. 5-2 through No. 5-9 each of the invention were prepared in quite the same manner as in Example 5-1 except that comparative coupler a was replaced by the couplers of the invention (5-2), (5-9), 5-21), (5-25), (5-26), (5-39), (5-42) and (5-45), respectively, (provided that the amounts added are as shown in Table 5-1).

The resulted Samples No. 5-1 through No. 5-9 were exposed to light through a wedge in an ordinary method and were then processed in the following processing steps.

(Processing steps)

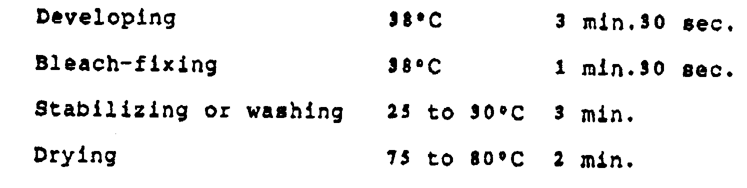

The processing liquids used in the steps are given below.

(Color developer)

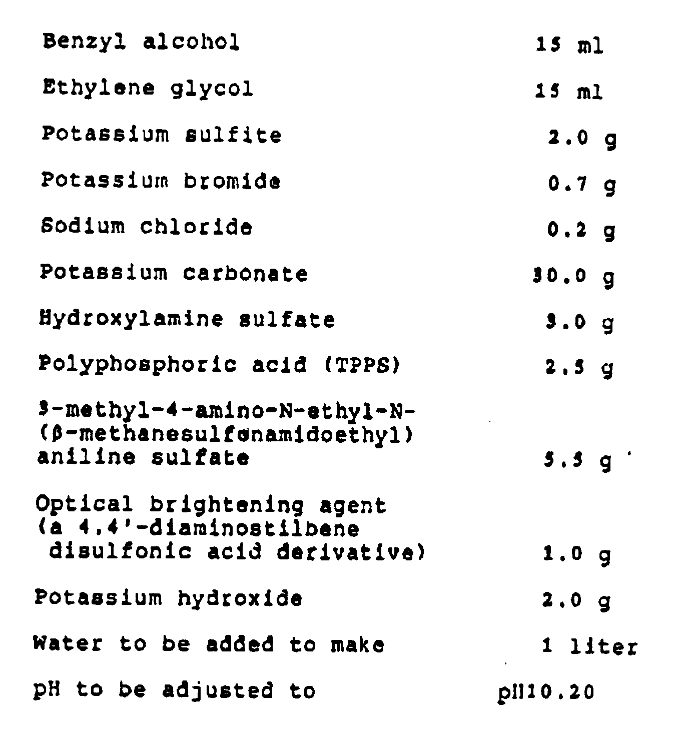

(Bleach-fixer)

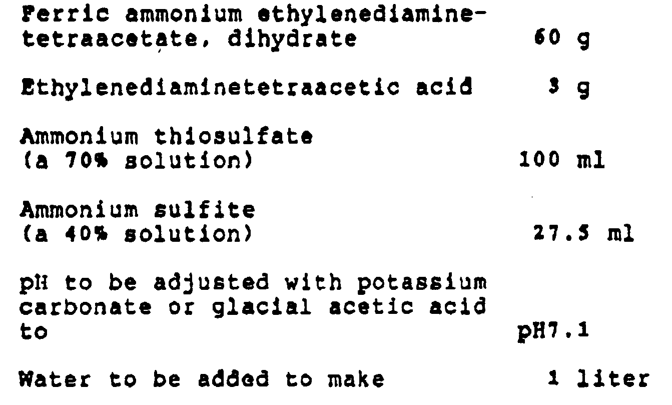

(Stabilizer)

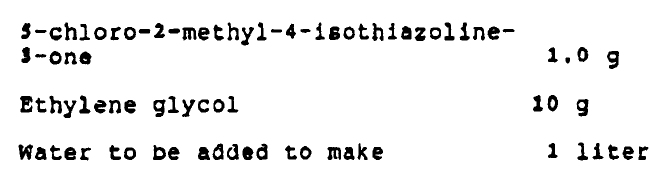

With respect to thus processed Samples No. 5-1 through No. 5-9, the measurements were made respectively each of the maximum spectral absorption wavelengths (λmax) and both of the reflection densities of 550 nm and 700 nm (Dλ550 and DX700)) at the time when the respective reflection densities of λmax were 1.0.

The smaller a value of Dλ550 is, the less an irregular absorption in green region is. The smaller a value of Dλ700 is, the sharper an absorption is. The facts mean that a color reproducibility is excellent.

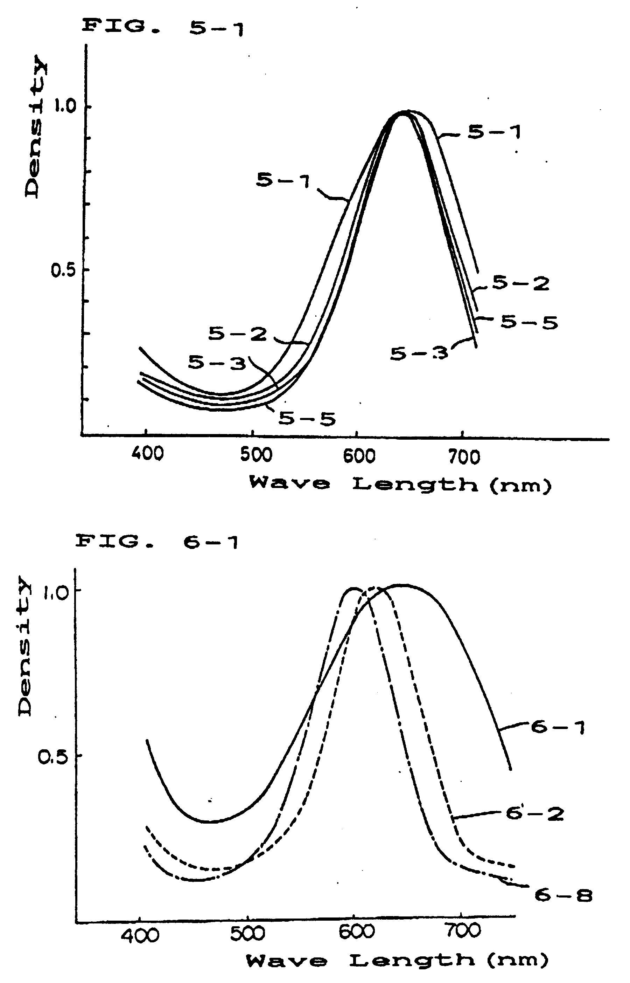

Fig. 5-1 shows the absorption spectra of Sample Nos. 5-1, 5-2, 5-3 and 5-5.

Further, each of the processed samples was allowed to stand for 14 days under the circumstances of a high temperature and humidity (at 60° C and 80%RH), and the heat and moisture resisting properties of the resulted dye-images were checked up. The results thereof are also shown in Table 5-1, wherein the heat and moisture resisting properties of the dye-images are indicated by the percentage of the residual dyes which still remained after the heat and moisture resistance tests were tried, to the initial density of 1.0.

For those measurements, a densitometer, Model KD-7R (manufactured by Konishiroku Photo Ind. Co., Ltd.), was used.

Comparative Coupler a

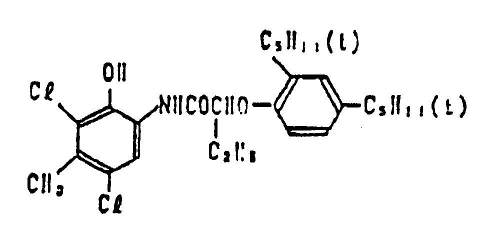

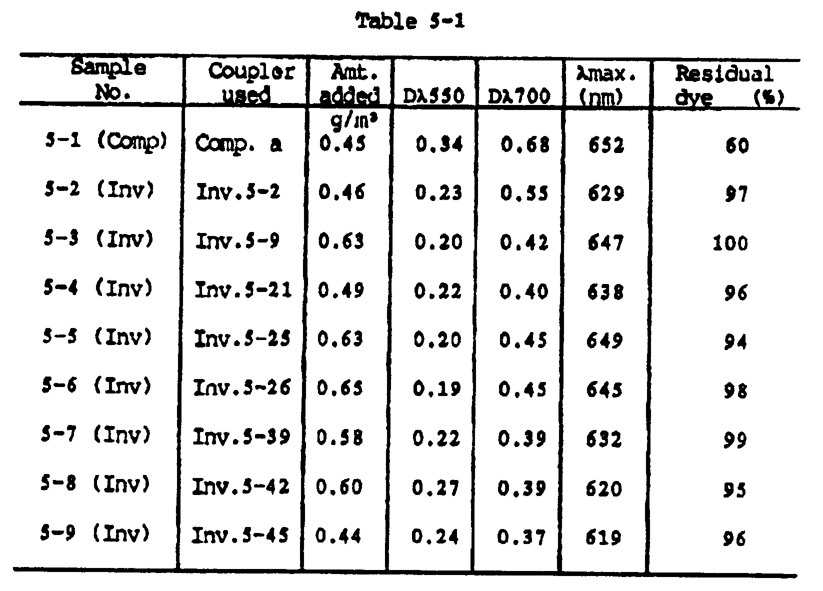

It is found from Table 5-1 that Samples No. 5-2 through No. 5-9 used the couplers of the invention are less in both Dλ550 and Dλ700 and excellent in color reproducibility. Among them, Couplers (5-9), (5-21), (5-25) and (5-26) are particularly excellent from the viewpoint of λmax. It is also found that the percentage of residual dye-images of the samples of the invention are remarkably improved as compared to those of the comparative samples and that color fading hardly occurs even when they are put in the circumstances of high temperature and humid.

Fig. 5-1 indicates that the couplers of the invention are relatively less in undesirable absorption in green spectral region and sharp in absorption caused around λmax.

### Example 6-1

Sample 6-1 of red-sensitive color light-sensitive material was prepared by coating the following layers over to a paper support laminated with polyethylene on the both sides thereof in order from the support side, respectively. The amounts of the compounds added are expressed in terms of those added persq. meter, unless otherwise particularly stated. (Amounts of silver halides are expressed in terms of the contents of silver.)

### Layer 1: An emulsion layer

A red-sensitive emulsion layer comprising 1.2 g of gelatin, 0.30 g of red-sensitive silver chlorobromide emulsion (containing silver chloride of 96 mol%) and 0.45 g of comparative cyan coupler a dissolved in 1.05 g of dioctyl phthalate.

### Layer 2: A protective layer

A protective layer containing 0.50 g of gelatin, whereto 2,4-dichloro-6-hydroxy-s-triazine sodium salt was added as a hardener in an amount of 0.017 g per g of the gelatin used.

Next, Samples No. 6-2 through No. 6-11 each of the invention were prepared in quite the same manner as in Example 6-1, except that comparative coupler a was replaced by the couplers shown in Table 6-1 (provided that the amounts added were the same molar amounts as in comparative coupler a).

The resulted Samples No. 6-1 through No. 6-11 were exposed to light through a wedge in an ordinary method and were then processed in the following processing steps.

(Processing steps)

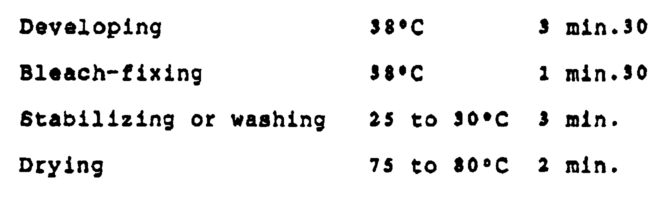

The processing liquids used in each of the steps were as follows.

(Color developer)

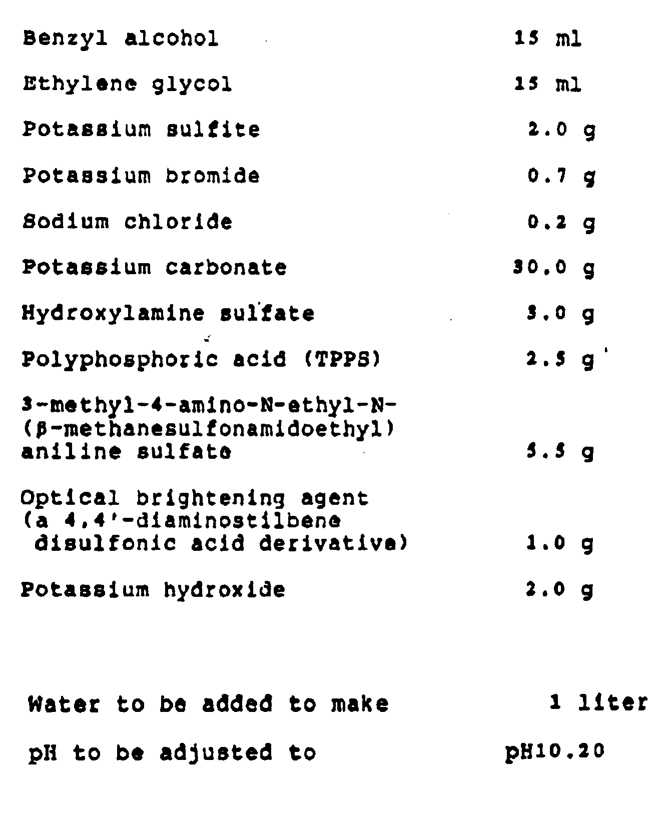

(Bleach-fixer)

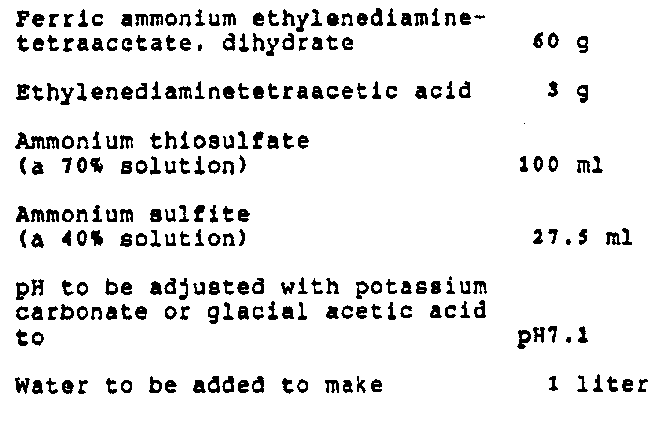

(Stabilizer)

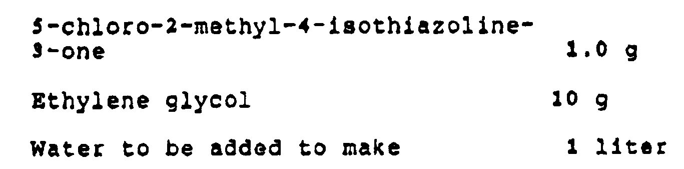

With respect to thus processed Samples No. 6-1 through No. 6-11, the measurements were made respectively each of the maximum spectral absorption wavelengths (λmax), the reflection densities of 420 nm (DX420) when the reflection densities of λmax were 1.0, and the half band widths (W1/2) which are the difference between one point on the longer wavelength side than each λmax and the shorter wavelength side than each λmax when the reflection densities of λmax were 0.5. After then, the spectral absorption characteristics and the color reproducibility of each Sample were examined.

The smaller a value of DX420 is, the less an irregular absorption in green region is. The smaller a value of W1/2 is, the sharper an absorption is. The facts mean that a color reproducibility is excellent.

Fig. 6-1 shows the absorption spectra of Sample Nos. 6-1, 6-2 and 6-8.

Further, each of the processed samples was allowed to stand for 14 days under the circumstances of a high temperature and humidity (at 60° C and 80%RH), and the heat and moisture resisting properties of the resulted dye-images were checked up. The results thereof are also shown in Table 6-1, wherein the heat and moisture resisting properties of the dye-images are indicated by the percentage of the residual dyes which still remained after the heat and moisture resistance tests were tried, to the initial density of 1.0.

For those measurements, a densitometer, Model KD-7R (manufactured by Konishiroku Photo Ind. Co., Ltd.), was used.

Comparative Coupler a

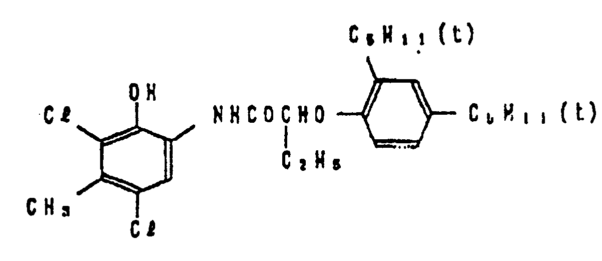

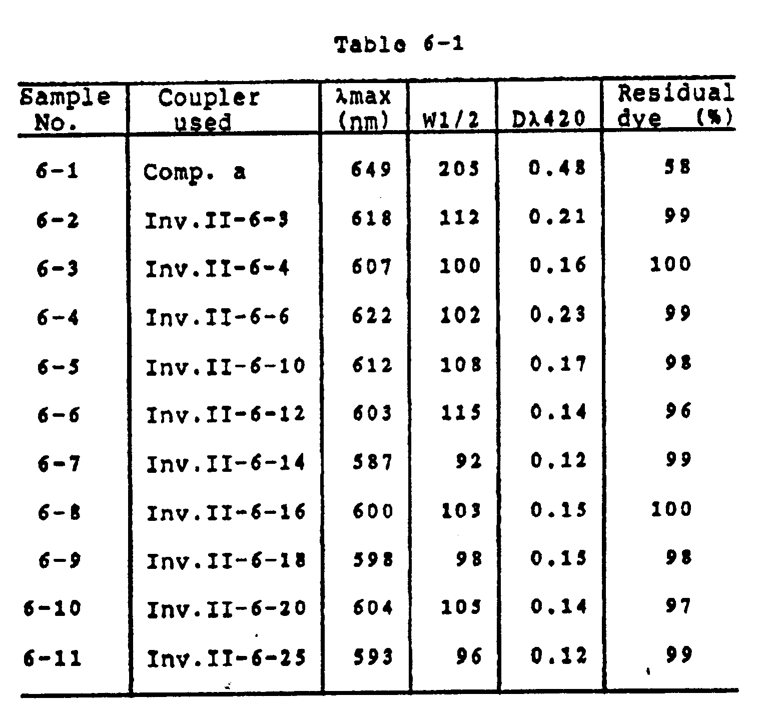

As is obvious from Table 6-1, it is found that every sample having the couplers of the invention has excellent spectral absorption characteristics, because their half band widths are extremely narrower and their irregular absorptions are also less, as compared with those of the sample having the comparative coupler.

It is also found that the Samples using the couplers of the invention are relatively solid, because the percentage of the residual dye-images was remarkably improved and the heat-and moisture-resisting properties are excellent.

In addition, Fig. 6-1 shows the fact that the couplers (11-6-2, 11-6-8) of the invention are relatively less in undesirable irregular absorption caused in green region and relatively sharp in absorption in the regions around λmax, as compared with the conventional phenol type coupler (1).

### Example 6-2

A red-sensitive color reversal potographic light-sensitive materials No. 6-12 through No. 6-16 were prepared by coating the following layers in order from a triacetyl cellulose film support over to the support.

The amounts of the compounds added are expressed by the amounts thereof per sq.meter unless otherwise specially stated. (Amounts of silver halides are expressed in terms of the contents of silver.) Layer 1: An emulsion layer

A red-sensitive emulsion layer comprising 1.4 g of gelatin, 0.5 g of red-sensitive si Iver chlorobromide emulsion and the couplers (9.1x10-⁴ moles) shown in Table 6-2 dissolved in 1.25 g of dibutyl phthalate.

### Layer 2: A protective layer

A protective layer containing 0.5 g of gelatin, whereto 2,4-dichloro-6-hydroxy-s-triazine sodium salt was added as a hardener in an amount of 0.017 g per g of the gelatin used.

The resulted Samples were exposed to light through a wedge in an ordinary method and were then processed in the following processing steps.

(Reversal processing steps)

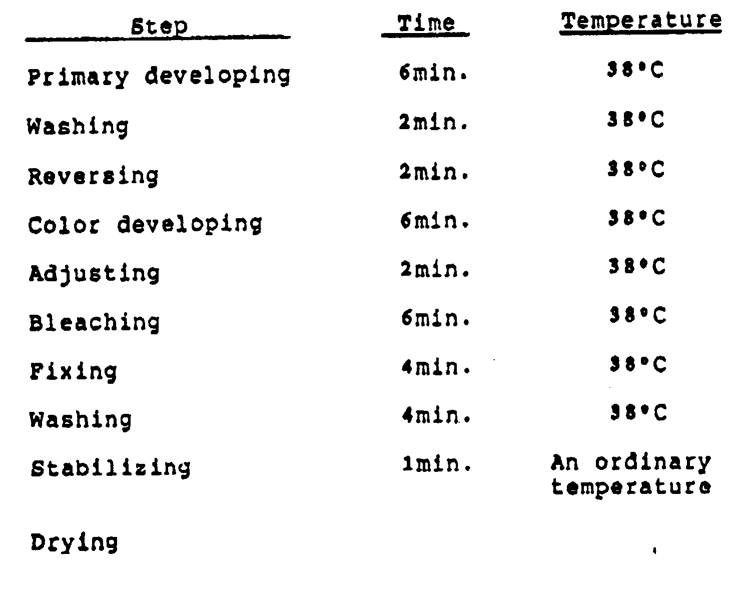

The compositions of the processing liquids were as follows.

(Primary developer)

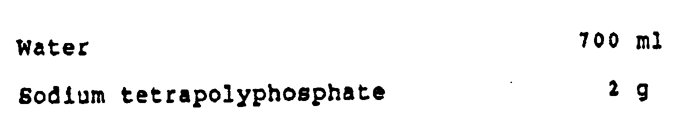

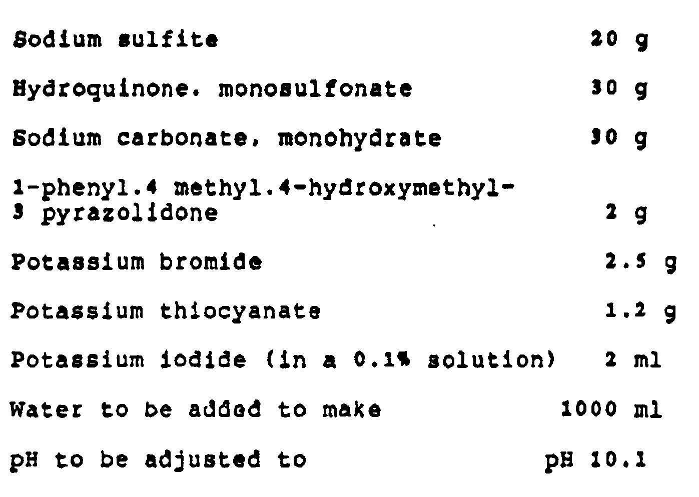

(Reversal bath)

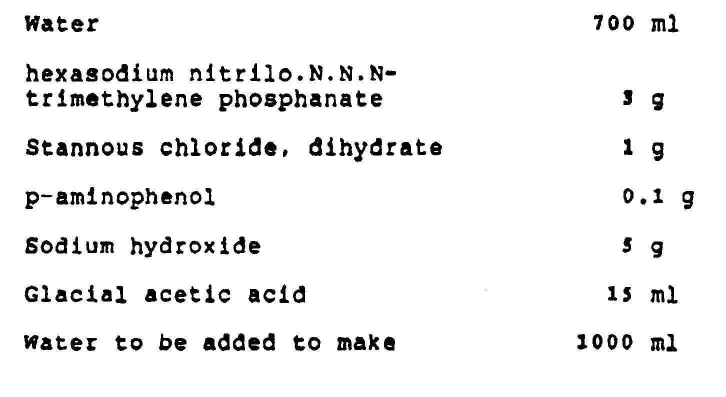

(Color developer C)

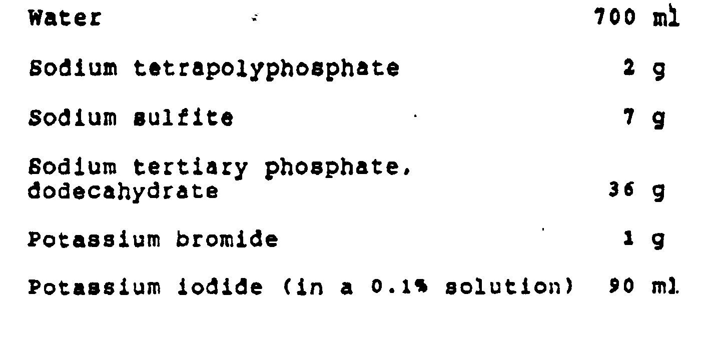

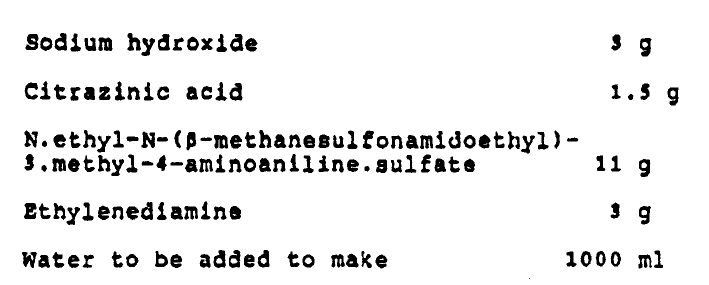

(Adjuster)

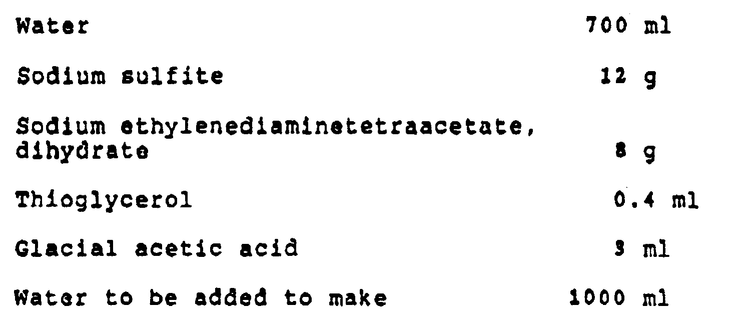

(Bleaching bath)

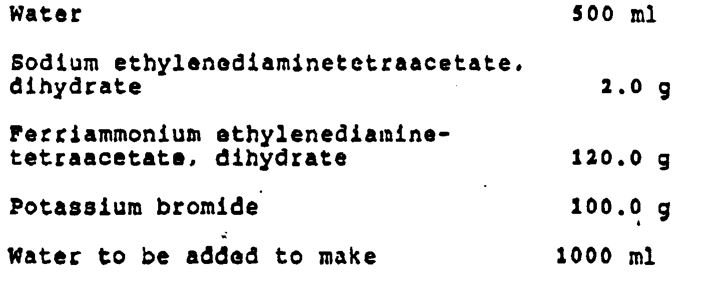

(Fixer)

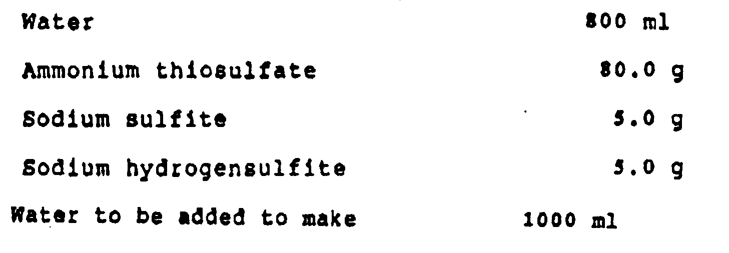

(Stabilizer)

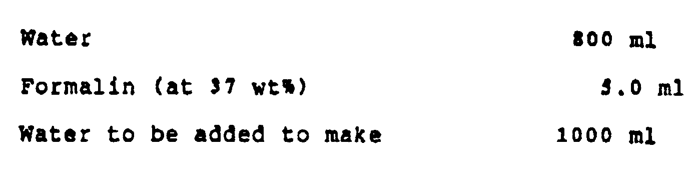

With each of the processed samples No. 6-12 through No. 6-16, the maximum spectral absorption wavelength (Xmax) and the half band width (W1/2).Dλ420 were measure in the same manner as in Example 6-1. The results thereof are shown in Table 6-2.

The measurements of transmission densities relating to the Example 6-2 were made with a densitometer, Model KD-7R.

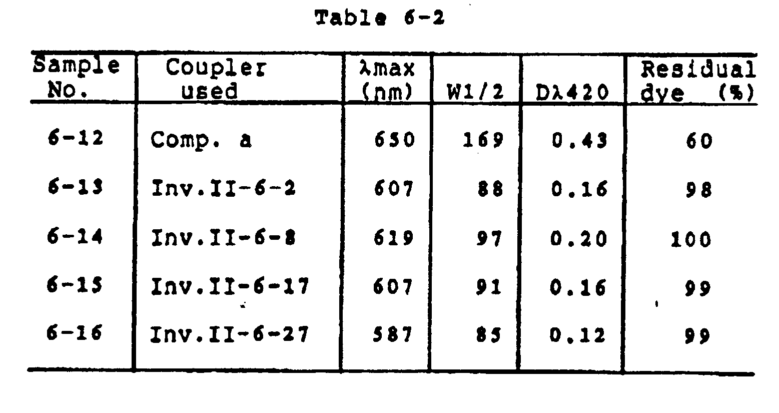

As is obvious from Table 6-2, it is found that every sample having the couplers of the invention has excellent spectral absorption characteristics and excellent color reproducibility, because their half band widths are extremely narrower and their DX420 are also less, as compared with those of the sample having the comparative coupler.

It is also found that the samples used the couplers of the invention were remarkably improved in the percentage of the residual dye-images and were also relatively solid.

### Example 7-1

Sample 7-1 of red-sensitive color light-sensitive material was prepared by coating the following layers over to a paper support laminated with polyethylene on the both sides thereof in order from the support side, respectively. The amounts of the compounds added are expressed in terms of those added persq. meter, unless otherwise particularly stated. (Amounts of silver halides are expressed in terms of the contents of silver.)

### Layer 1: An emulsion layer

A red-sensitive emulsion layer comprising 1.2 g of gelatin, 0.30 g of red-sensitive silver chlorobromide emulsion (containing silver chloride of 96 mol%) and 0.45 g of comparative cyan coupler a dissolved in 1.50 g of trioctyl phosphate.

### Layer 2: A protective layer

A protective layer containing 0.50 g of gelatin, whereto 2,4-dichloro-6-hydroxy-s-triazine sodium salt was added as a hardener in an amount of 0.017 g per g of the gelatin used.

Next, Samples No. 7-2 through No. 7-20 each of the invention were prepared in quite the same manner as in Example 7-1, except that the comparative coupler a was replaced by the couplers of the invention indicated in Table 7-1 (provided that the amounts added were the same molar amounts as in Sample 7-1).

The resulted Samples No. 7-1 through No. 7-20 were exposed to light through a wedge in an ordinary method and were then processed in the following processing steps.

(Processing steps)

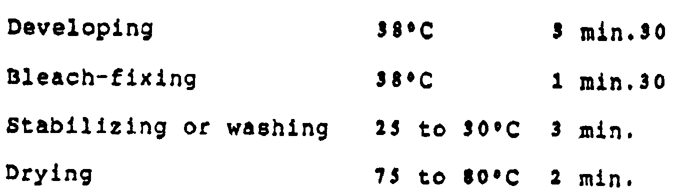

The processing liquids used in each of the steps were as follows.

(Color developer)

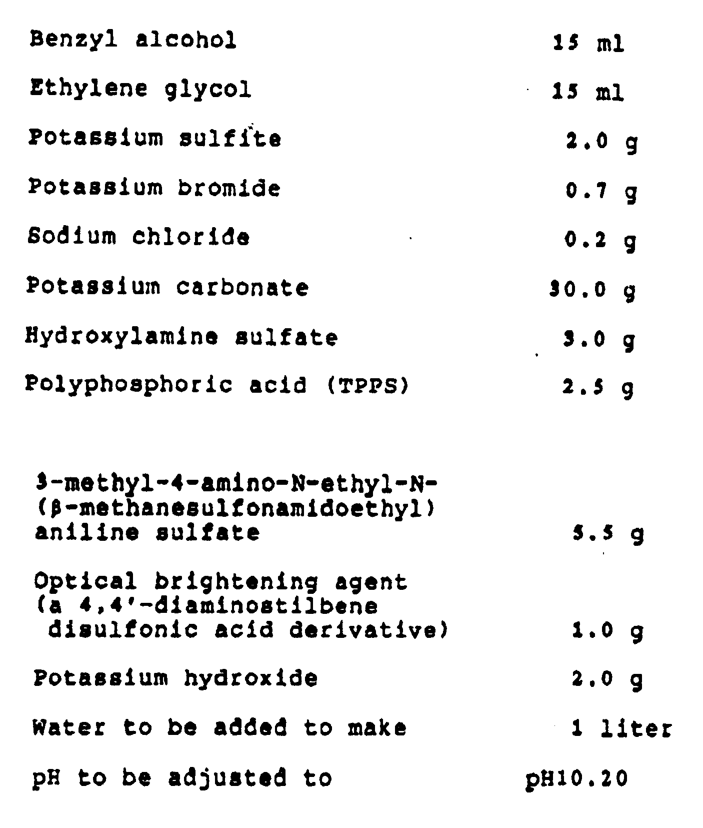

(Bleach-fixer)

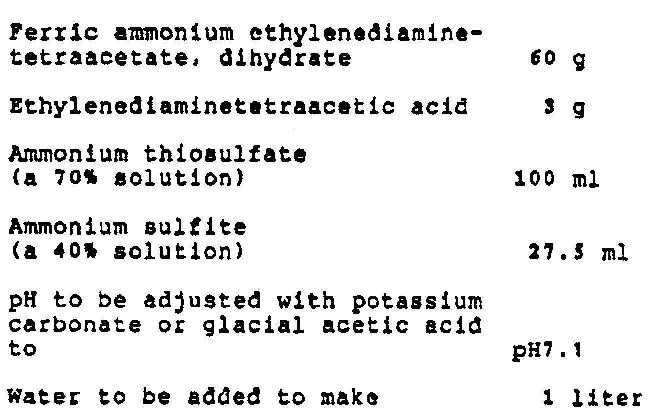

(Stabilizer)

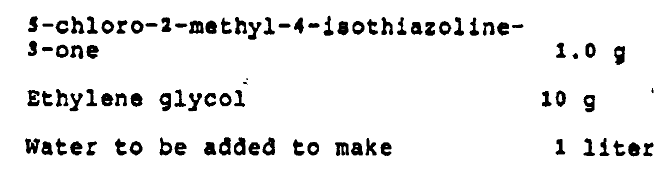

With respect to each sample thus processed, the measurements were made respectively each of the maximum spectral absorption wavelengths (λmax) when the reflection densities of λmax were 1.0, and the reflection densities of 420 nm and the half band widths of spetral absorption (i.e., W1/2) which are the difference between the wavelength of reflection density 0.5 on the side of a wavelength longer that that of λmax and the wavelength of reflection density 0.5 on the side of a wavelength shorter than that of λmax.)

The smaller a value of DX420 is, the less an irregular absorption in blue region is. The smaller a value of half band width is, the sharper an absorption is. The facts mean that a color reproducibility is excellent.

Fig. 7-1 shows the absorption spectra of Sample Nos. 7-1, 7-2 and 7-13.

Further, each of the processed samples was allowed to stand for 14 days under the circumstances of a high temperature and humidity (at 60° C and 80%RH), and the heat and moisture resisting properties of the resulted dye-images were checked up. The results thereof are also shown in Table 7-1, wherein the heat and moisture resisting properties of the dye-images are indicated by the percentage of the residual dyes to the initial density of 1.0 after testing the heat and moisture resistance.

For those measurements, a densitometer, Model KD-7R (manufactured by Konishiroku Photo Ind. Co., Ltd.), was used.

The results thereof are collectively shown in Table 7-1.

Comparative Coupler a

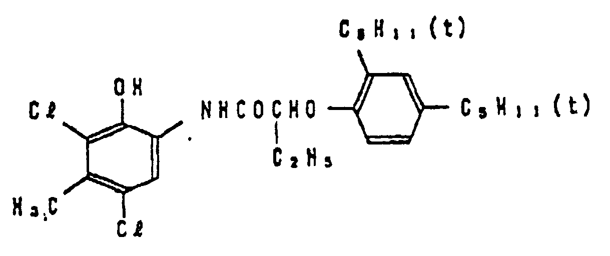

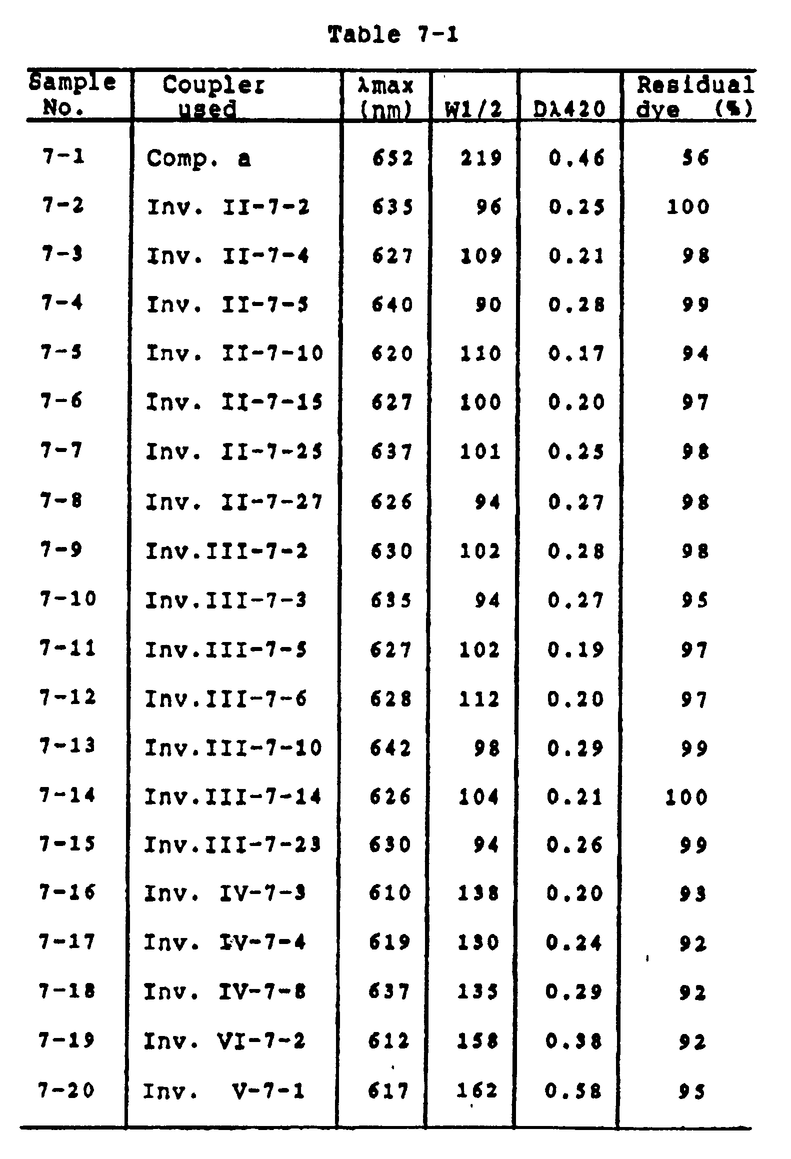

As is obvious from the results shown in Table 7-1, it is found that every sample having the couplers of the invention has extremely excellent color reproducibi lity, because their half band widths (W1/2) are narrower and the irregular absorptions detectable by λmax are also less, as compared with the sample used the comparative coupler. Further, the samples of the invention can provide excellently color-reproduced cyan images as compared with the comparative samples, because their spectral waveforms are excellent, the λmax value thereof are small though.

Still further, it is found that the samples having the couplers of the invention are also high in dye-residual percentage and excellent in heat- and moisture-resistance, as compared with the comparative samples.

In addition, Fig. 7-1 shows the fact that the couplers of the invention are relatively less in undesirable irregular absorption caused in green region e.g., 550 nm) and relatively sharp in absorption in the regions around λmax, as compared with the convention phenol type couplers.

### Example 7-2

Samples No. 7-21 through No. 7-29 of red-sensitive color reversal photographic light-sensitive materials were prepared by coating the following layers over to a triacetyl cellulose fi 1m support, in orderfrom the support side.

The amounts of the compounds added are expressed by the amounts thereof per sq.meter unless otherwise specially stated. (Amounts of silver halides are expressed in terms of the contents of silver.)

### Layer 1: An emulsion layer

A red-sensitive emulsion layer comprising 1.4 g of gelatin, 0.5 g of red-sensitive si Iver chlorobromide emulsion (containing silver chloride of 96 mol%) and the couplers (9.1x10-⁴ moles) shown in Table 7-2 dissolved in 1.65 g of dioctylphenyl phosphate.

### Layer 2: A protective layer

A protective layer containing 0.5 g of gelatin, whereto 2,4-dichloro-6-hydroxy-s-triazine sodium salt was added as a hardener in an amount of 0.017 g per g of the gelatin used.

The resulted Samples were exposed to light through a wedge in an ordinary method and were then processed in the following processing steps.

(Reversal processing steps)

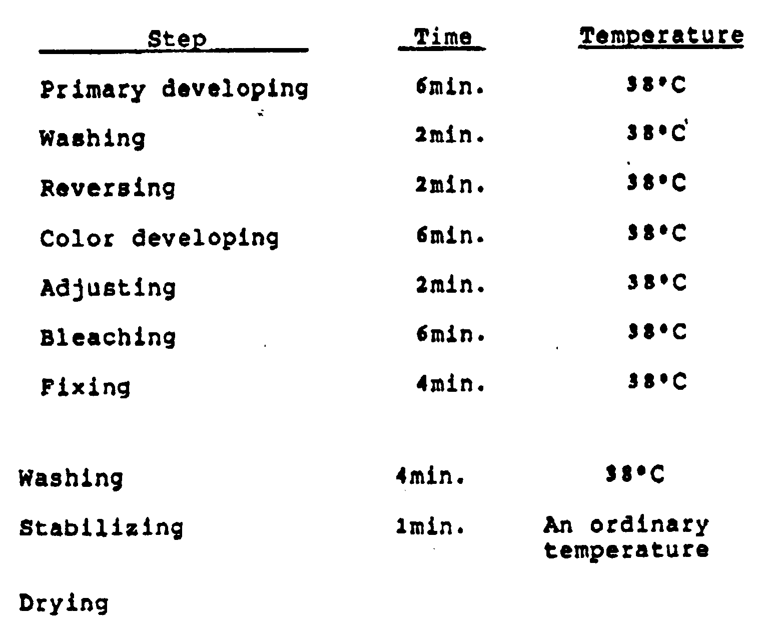

The compositions of the processing liquids were as follows.

(Primary developer)

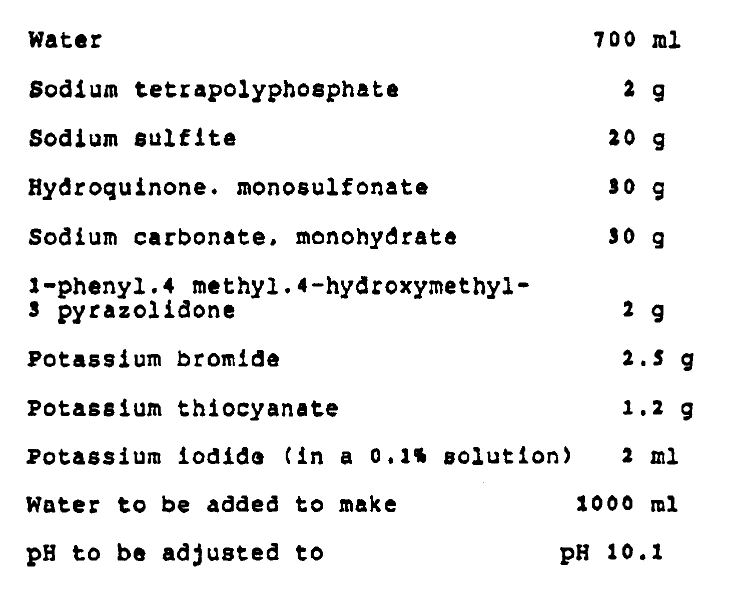

(Reversal bath)

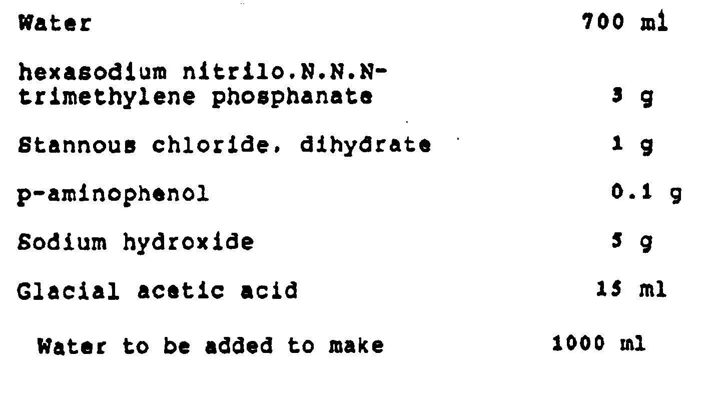

(Color developer C)

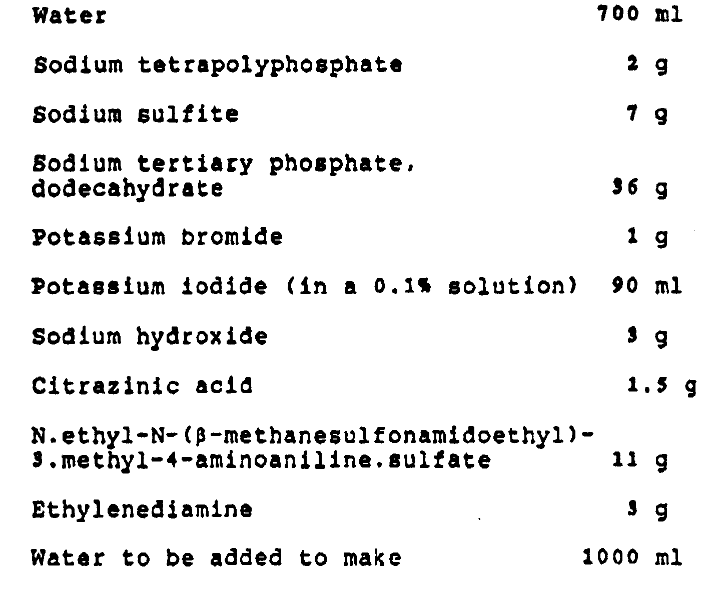

(Adjuster)

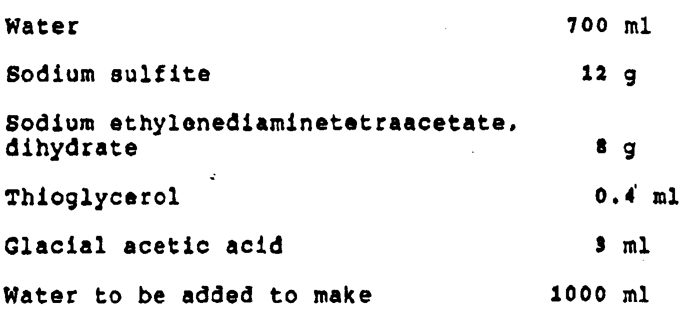

(Bleaching bath)

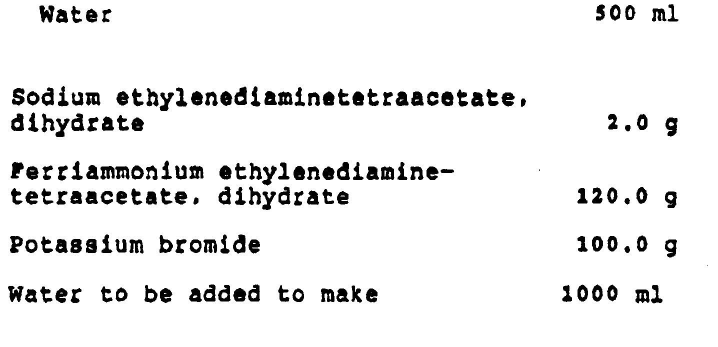

(Fixer)

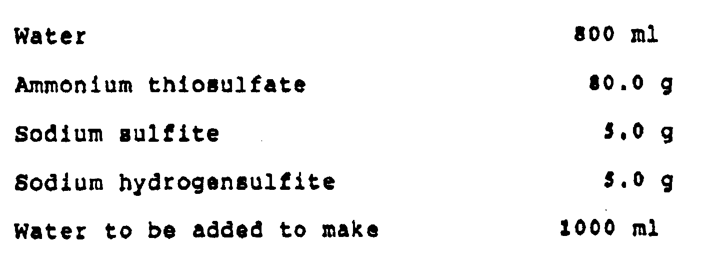

(Stabilizer)

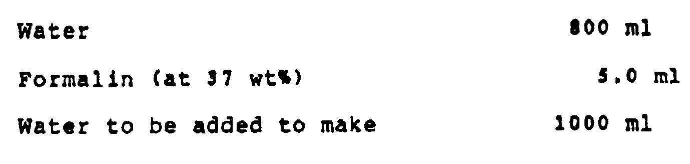

With each of the processed samples, the maximum spectral absorption wavelength (Xmax) and the half band width (W1/2) were measured in the same manner as in Example 7-1. The results thereof are shown in Table 7-2.

The measurements of transmission densities relating to the Example 7-2 were made with a densitometer, Model KD-7R.

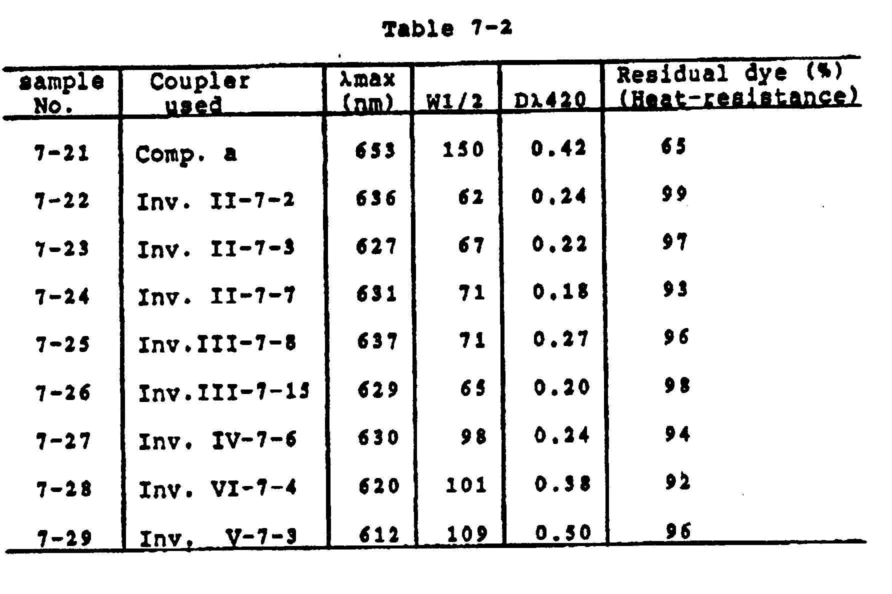

As is obvious from Table 7-2, it is found that every sample having the couplers of the invention has remarkably excellent color reproducibility, because their half band widths are narrower and their irregular absorptions are also less, as compared with the comparative couplers.

It is also found that the samples used the couplers of the invention were high in the percentage of the residual dye-images and excellent in heat- and moisture-resistance.

## Claims

1. A light-sensitive silver halide color photographic material comprising a support and, provided thereon, at least one red light-sensitive silver halide emulsion layer containing a pyrazoloazole type cyan dyeforming coupler having the formula [I]-I, said coupler being such that, following reaction with an oxidized product of a color developing agent, the resultant compound has a spectral absorption maximum within the visible wavelengths of from 600nm to 700nm: 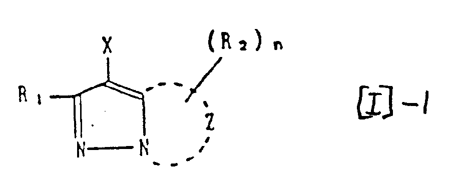
wherein at least one of R₁ and the or each R2 is an electron withdrawing group, and the remainder are hydrogen atoms or substituents; Z is a non-metallic group which completes, together with the atoms to which it is attached, a nitrogen-containing heterocyclic ring; each R₂ is bonded to a carbon atom of the heterocyclic ring; X is a hydrogen atom or a substituent capable of being split off upon reaction with an oxidized product of a color developing agent; and n is 1 or 2.

2. A material according to claim 1, wherein R₁ is an electron withdrawing group.

3. A material according to claim 1 or 2, wherein at least one R₂ group is an electron withdrawing group.

4. A material according to any one of claims 1 to 3, wherein the electron withdrawing group is a sulfonyl group, a sulfinyl group, a sulfonyloxy group, a sulfonylmethyl group, a sulfamoyl group, a phosphoryl group, a tetrazolyl group, a pyrrolyl group, a halogenated alkoxy group, a halogenated aryloxy group, an acyl group, a halogen atom, a nitro group, a carboxy group, a carbamoyl group, an acyloxy group, an oxycarbonyl group, a halogenated alkyl group, a halogenated aryl group, a cyano group, an alkylsulfonylmethyl group or an arylsulfonyl methyl group.

5. A material according to any one of claims 1 to 7, wherein the cyan dye-forming coupler is of Formula [I]-4: 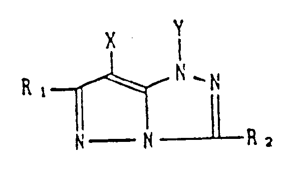
wherein R₁ is a cyano group, an acyloxy group, an oxycarbonyl group or a carbamoyl group; R₂ and Yare each a hydrogen atom or a substituent; and X is a hydrogen atom or a group capable of splitting off upon reaction with an oxidized product of a color developing agent.

6. A material according to any one of claims 1 to 4, wherein the cyan dye-forming coupler is of 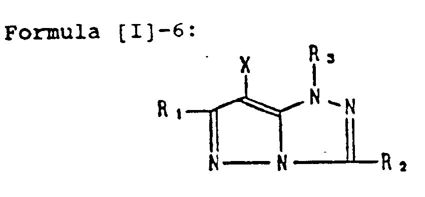
wherein R₂ is a halogen atom or a sulfonyl, sulfonyloxy, sulfinyl, sulfamoyl, phosphoryl, tetrazolyl, pyrrolyl, halogenated alkoxy, halogenated aryloxy, cyano, nitro, acyl or carbamoyl group; R₁ and R₃ are each independently a hydrogen atom or a substituent; and X is a hydrogen atom or a group capable of being split off upon reaction with an oxidized product of a color developing agent.

7. A material according to any one of the preceding claims, wherein the red light-sensitive emulsion layer contains a spectral sensitizing dye of formulae [A], [B], [C], [D], [E] or [F]: 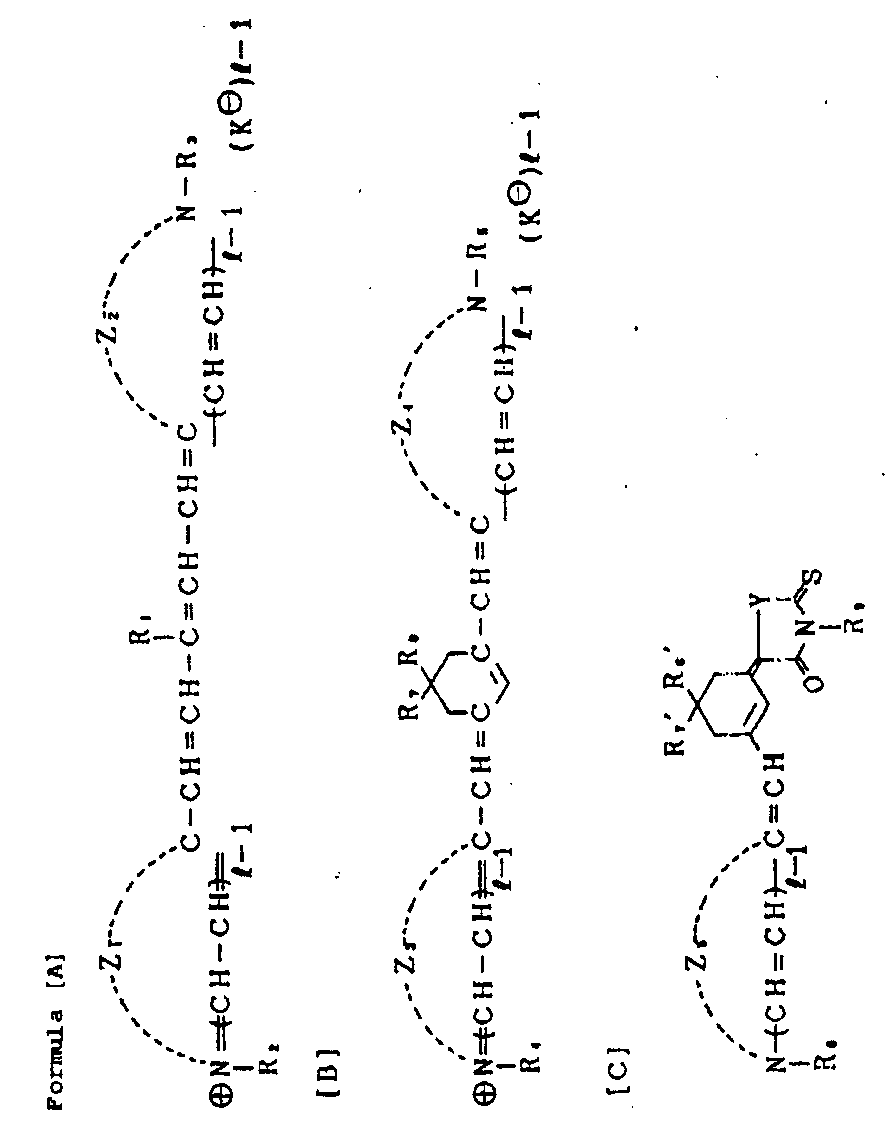 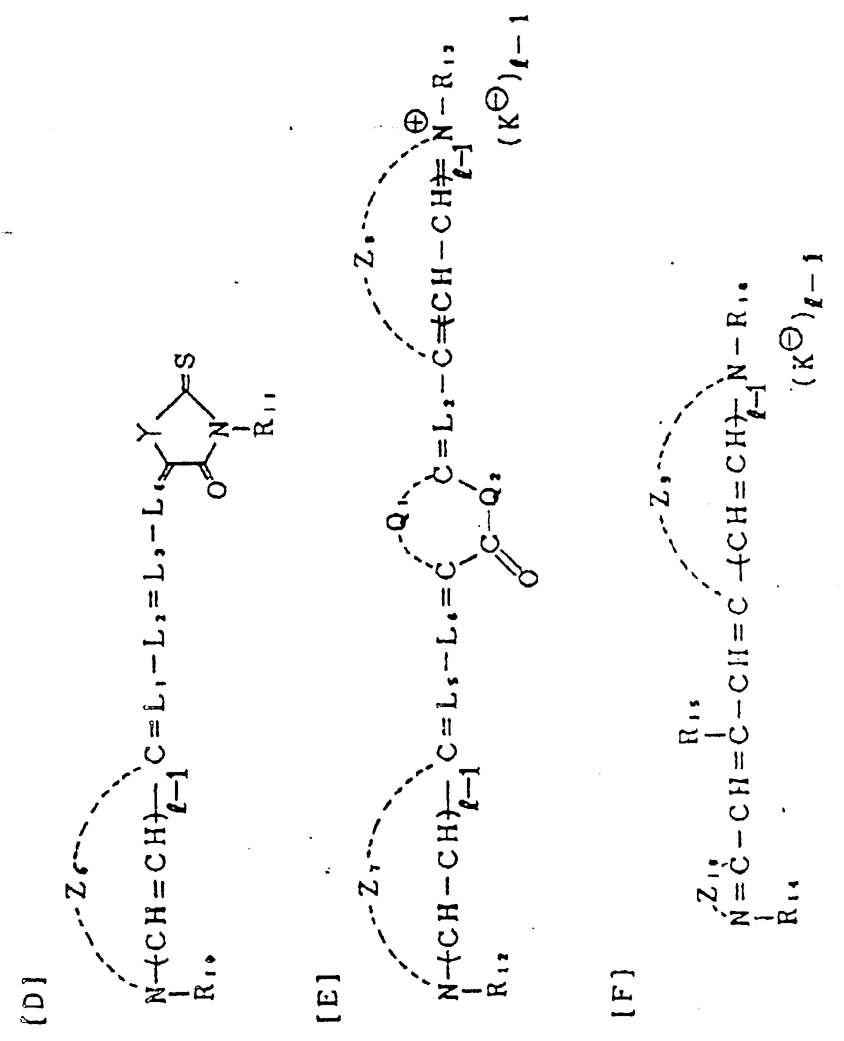
wherein Z₁ through Z₉ each is a group which completes a benzene ring or a naphthalene ring condensed with a pyridine ring, an imidazole ring, a thiazole ring, a selenazole ring, a oxazole ring or a tetrazole ring; Z₁₀ is a group which completes a benzothiazole ring, a benzoselenazole ring, a (β-naphthothiazole ring, a (β-naphthoselenazole ring, a benzimidazole ring or a 2-guinoline ring; Q₁ and Q₂ each is a group which completes a 4-thiazolidinone, 5-thiazolidinone or 4-imidazolidinone nucleus; R₁ and R₁₅ each is a hydrogen atom, an alkyl group or an aryl group; R₇, R₈, R₇' and R₈ each is an alkyl group; R₉ and R₁₁ each is an alkyl group, an aryl group or a heterocyclic group; R₂ , R₃, R₄, R₅, Rₛ, R₁₀, R₁₂, R₁₃, R₁₄ and R₁₆ each is an alkyl group or an aryl group;
f is 1 or2; Y is a sulfuratom or a selenium atom; L₁ through L₆ each is a substituted or unsubstituted methyne group; and K is an acid anion.

8. A cyan dye-forming coupler as defined in any one of claims 1 to 6.

## Patentansprüche

1. Lichtempfindliches farbphotographisches Silberhalogenid-Aufzeichnungsmaterial mit einem Schichtträgerund mindestens einer darauf vorgesehenen, für rotes Licht empfindlichen Silberhalogenidemulsionsschicht mit einem einen blaugrünen Farbstoff bildenden Kuppler vom Pyrazoloazol-Typ der Formel [1]-1 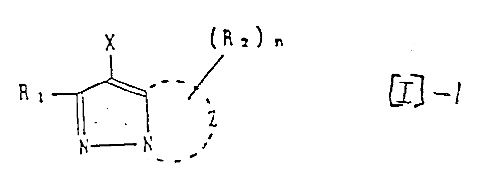
worin mindestens einer der Reste R₁ und R₂ oder jeder Rest R₂ für eine Elektronen abziehende Gruppe steht und der Rest Wasserstoffatome oder Substituenten darstellt; Z eine nicht-metallische Gruppe, die zusammen mit den Atomen, an denen sie hängt, einen stickstoffhaltigen heterozyklischen Ring vervollständigt, bedeutet; jeder Rest R₂ an ein Kohlenstoffatom des heterozyklischen Rings gebunden ist; X ein Wasserstoffatom oder einen bei der Reaktion mit einem Oxidationsprodukt einer Farbentwicklerverbindung abspaltbaren Substituenten darstellt und n = 1 oder 2,
wobei der Kuppler derart ist, daß die nach der Reaktion mit einem Oxidationsprodukt einer Farbentwicklerverbindung gebildete Verbindung ein spektrales Absorptionsmaximum innerhalb der sichtbaren Wellenlängen von 600 bis 700 nm aufweist.

2. Aufzeichnungsmaterial nach Anspruch 1, wobei R₁ für eine Elektronen abziehende Gruppe steht.

3. Aufzeichnungsmaterial nach Anspruch 1 oder 2, wobei mindestens einer der Reste R₂füreine Elektronen abziehende Gruppe steht.

4. Aufzeichnungsmaterial nach einem der Ansprüche 1 bis 3, wobei die Elektronen abziehende Gruppe aus einer Sulfonylgruppe, einer Sulfinylgruppe, einer Sulfonyloxygruppe, einer Sulfonylmethylgruppe, einer Sulfamoylgruppe, einer Phosphorylgruppe, einer Tetrazolylgruppe, einer Pyrrolylgruppe, einer halogenierten Alkoxygruppe, einer halogenierten Aryloxygruppe, einer Acylgruppe, einem Halogenatom, einer Nitrogruppe, einer Carboxygruppe, einer Carbamoylgruppe, einer Acyloxygruppe, einer Oxycarbonylgruppe, einer halogenierten Alkylgruppe, einer halogenierten Arylgruppe, einer Cyanogruppe, einer Alkylsulfonylmethylgruppe oder einer Arylsulfonylmethylgruppe besteht.

5. Aufzeichnungsmaterial nach einem derAnsprüche 1 bis 4, wobei der den blaugrünen Farbstoff bildenden Kuppler der Formel [1]-4 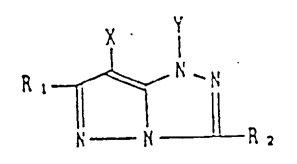 worin bedeuten:
R₁ eine Cyanogruppe, eine Acyloxygruppe, eine Oxycarbonylgruppe oder eine Carbamoylgruppe;
R₂ und Y jeweils ein Wasserstoffatom oder einen Substituenten und
X ein Wasserstoffatom oder eine bei der Reaktion mit einem Oxidationsprodukt einer Farbentwicklerverbindung abspaltbare Gruppe
entspricht.

6. Aufzeichnungsmaterial nach einem der Ansprüche 1 bis 4, wobei der den blaugrünen Farbstoff bildende Kuppler der Formel: 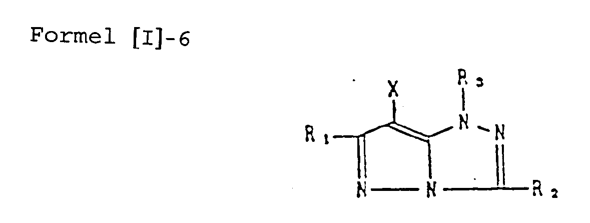
worin bedeuten:
R₂ ein Halogenatom oder eine Sulfonyl-, Sulfonyloxy-, Sulfinyl-, Sulfamoyl-, Phosphoryl-, Tetrazolyl-, Pyrrolyl-, halogenierte Alkoxy-, halogenierte Aryloxy-, Cyano-, Nitro-, Acyl- oder Carbamoylgruppe;
R₁ und R₃ jeweils unabhängig voneinander ein Wasserstoffatom oder einen Substituenten und
X ein Wasserstoffatom oder eine bei der Reaktion mit dem Oxidationsprodukt einer Farbentwicklerverbindung abspaltbare Gruppe
entspricht.

7. Aufzeichnungsmaterial nach einem der vorhergehenden Ansprüche, wobei die für rotes Licht empfindliche Emulsionsschicht einen spektralen Sensibilisierungsfarbstoff der Formeln [A], [B], [C], [D], [E] oder [F]: 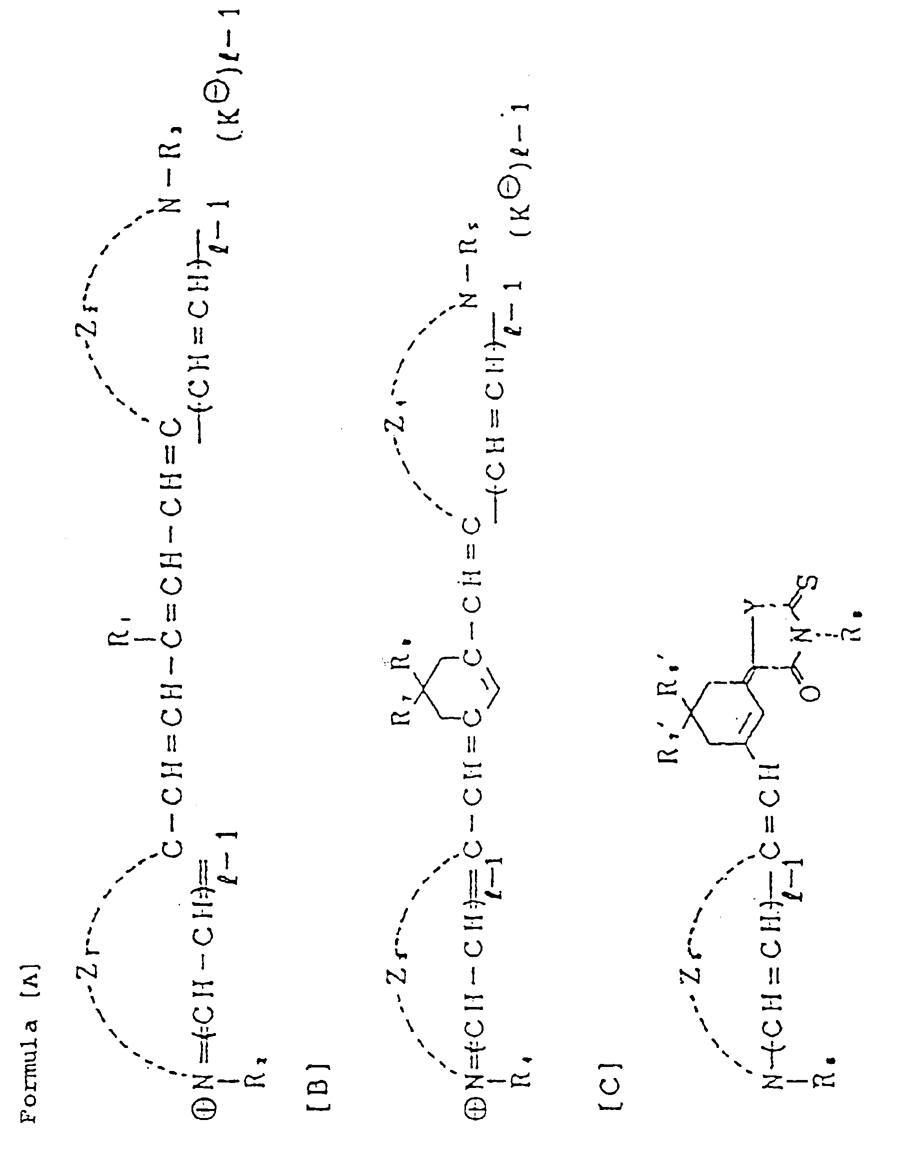 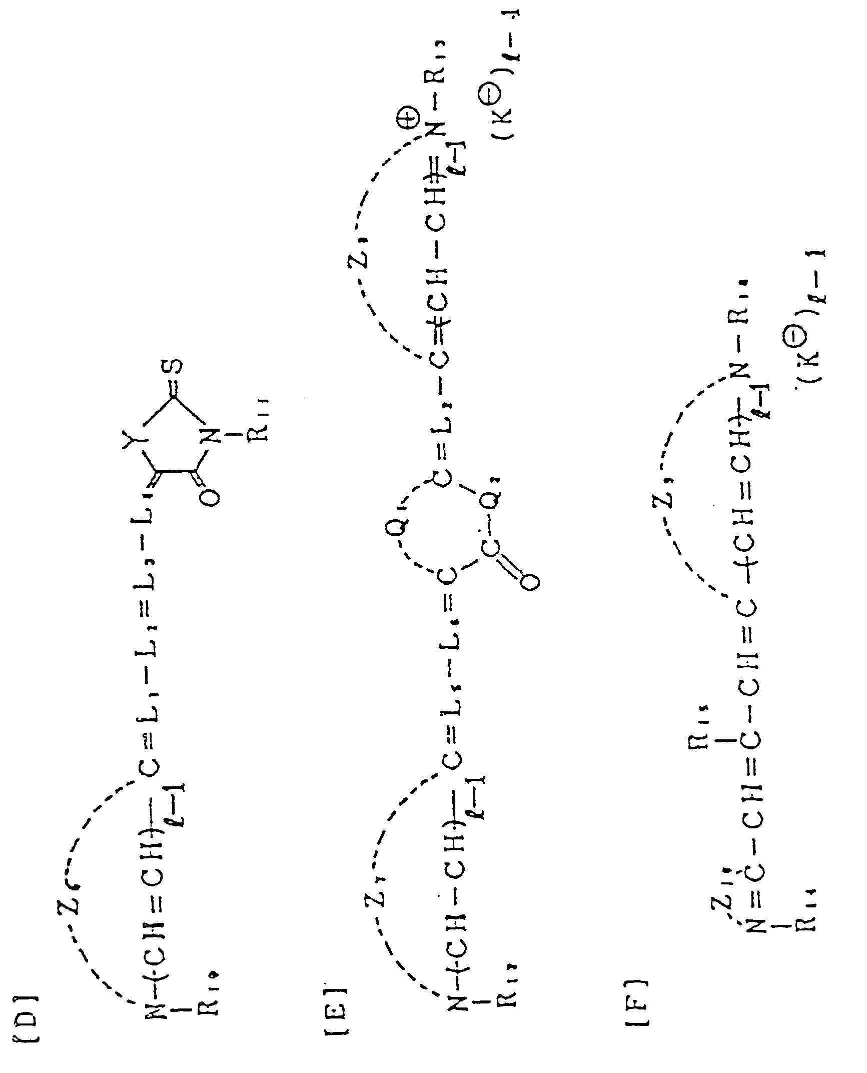
worin bedeuten:
Z₁ bis Z₉jeweils eine Gruppe, die einen mit einem Pyridin-, Imidazol-, Thiazol-, Selenazol-, Oxazol- oder Tetrazolring kondensierten Benzol- oder Naphtalinring vervollständigt;
Z₁₀ eine einen Benzothiazol-, Benzoselenazol-, β-Naphthothiazol-, β-Naphthoselenazol-, Benzimidazol-oder 2-Chinolinring vervollständigende Gruppe;
Q₁ und Q₂ jeweils eine Gruppe, die einen 4-Thiazoiidinon, 5-Thiazoiidinon- oder4-Imidazolidinonkern vervollständigt;
R₁ und R₁₅ jeweils ein Wasserstoffatom, eine Alkylgruppe oder eine Arylgruppe;
R₇, R₈, R₇' und R₈' jeweils eine Alkylgruppe;
R₉ und R₁₁ jeweils eine Alkylgruppe, eine Arylgruppe oder eine heterocyclische Gruppe;
R₂, R₃, R₄, R₅, R₆, R₁₀, R₁₂, R₁₃, R₁₄ und R₁₆jeweils eine Alkylgruppe oder eine Arylgruppe;
= 1 oder 2;
Y ein Schwefel- oder Selenatom;
L₁ bis L₆ jeweils eine substituierte oder unsubstituierte Methingruppe und
K ein Säureanion
enthält.

8. Einen blaugrünen Farbstoff bildender Kuppler gemäß der Definition nach einem der Ansprüche 1 bis 6.

## Revendications

1. Matériau photosensible pour la photographie en couleur, à base d'halogénure d'argent, comprenant un support portant au moins une couche d'émulsion photosensible d'halogénure d'argent sensible au rouge contenant un coupleur formateur de colorant cyan du type pyrazoloazole, de formule [)]-1, ledit coupleur étant tel que par réaction avec un produit oxydé d'un agent de développement couleur, le composé résultant présente un maximum d'absorption spectrale dans le domaine des longueurs d'onde visibles de 600 nm à 700 nm : 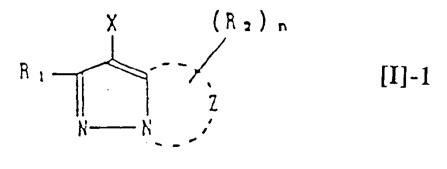 formule dans laquelle au moins l'un de R₁ et de R₂, ou de chaque R₂, représente un groupe accepteur d'électrons, et les autres représentent des atomes d'hydrogène ou des substituants ; Z est un groupe non métallique qui complète, conjointement avec les atomes auxquels il est lié, un hétérocycle azoté ; chaque R₂ est lié à un atome de carbone de l'hétérocycle ; X est un atome d'hydrogène ou un substituant capable de se séparer par réaction avec un produit oxydé d'un agent de développement couleur; et n vaut 1 ou 2.

2. Matériau selon la revendication 1, dans lequel R₁ est un groupe accepteur d'électrons.

3. Matériau selon la revendication 1 ou 2, dans lequel au moins un groupe R₂ est un groupe accepteur d'électrons.

4. Matériau selon l'une quelconque des revendications 1 à 3, dans lequel le groupe accepteur d'électrons est un groupe sulfonyle, un groupe sulfinyle, un groupe sulfonyloxy, un groupe sulfonylméthyle, un groupe sulfamoyle, un groupe phosphoryle, un groupe tétrazolyle,un groupe pyrrolyle, un groupe alcoxy halogéné, un groupe aryloxy halogéné, un groupe acyle, un atome d'halogène, un groupe nitro, un groupe carboxy, un groupe carbamoyle, un groupe acyloxy, un groupe oxycarbonyle, un groupe alkyle halogéné, un groupe aryle halogéné, un groupe cyano, un groupe alkylsulfonylméthyle ou un groupe arylsulfonylmé- thyle.

5. Matériau selon l'une quelconque des revendications 1 à 4, dans lequel le coupleur formateur de colorant cyan répond à la formule [1]-4 : 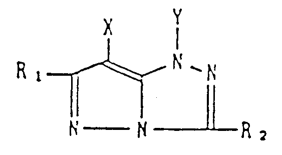 dans laquelle R₁ est un groupe cyano, un groupe acyloxy, un groupe oxycarbonyle ou un groupe carbamoyle ; R₂ et Y sont chacun un atome d'hydrogène ou un substituant ; et X est un atome d'hydrogène ou un groupe capable d'être éliminé par réaction avec un produit oxydé d'un agent de développement couleur.

6. Matériau selon l'une quelconque des revendications 1 à 4, dans lequel le coupleur formateur de colorant cyan répond à la formule [1]-6 : 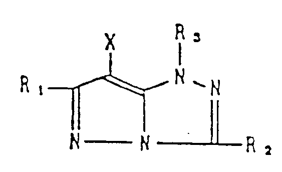 dans laquelle R₂ est un atome d'halogène ou un groupe sulfonyle, sulfonyloxy, sulfinyle, sulfamoyle, phosphoryle, tétrazolyle, pyrrolyle, alcoxy halogéné, aryloxy halogéné, cyano, nitro, acyle ou carbamoyle ; R₁ et R₃ représentent chacun, indépendamment, un atome d'hydrogène ou un substituant; et X est un atome d'hydrogène ou un groupe capable d'être éliminé par réaction avec un produit oxydé d'un agent de développement couleur.

7. Matériau selon l'une quelconque des revendications précédentes, dans lequel la couche d'émulsion photosensible, sensible au rouge, contient un colorant de sensibilisation spectrale répondant aux formules [A], [B], [C], [D], [E] ou [F] : 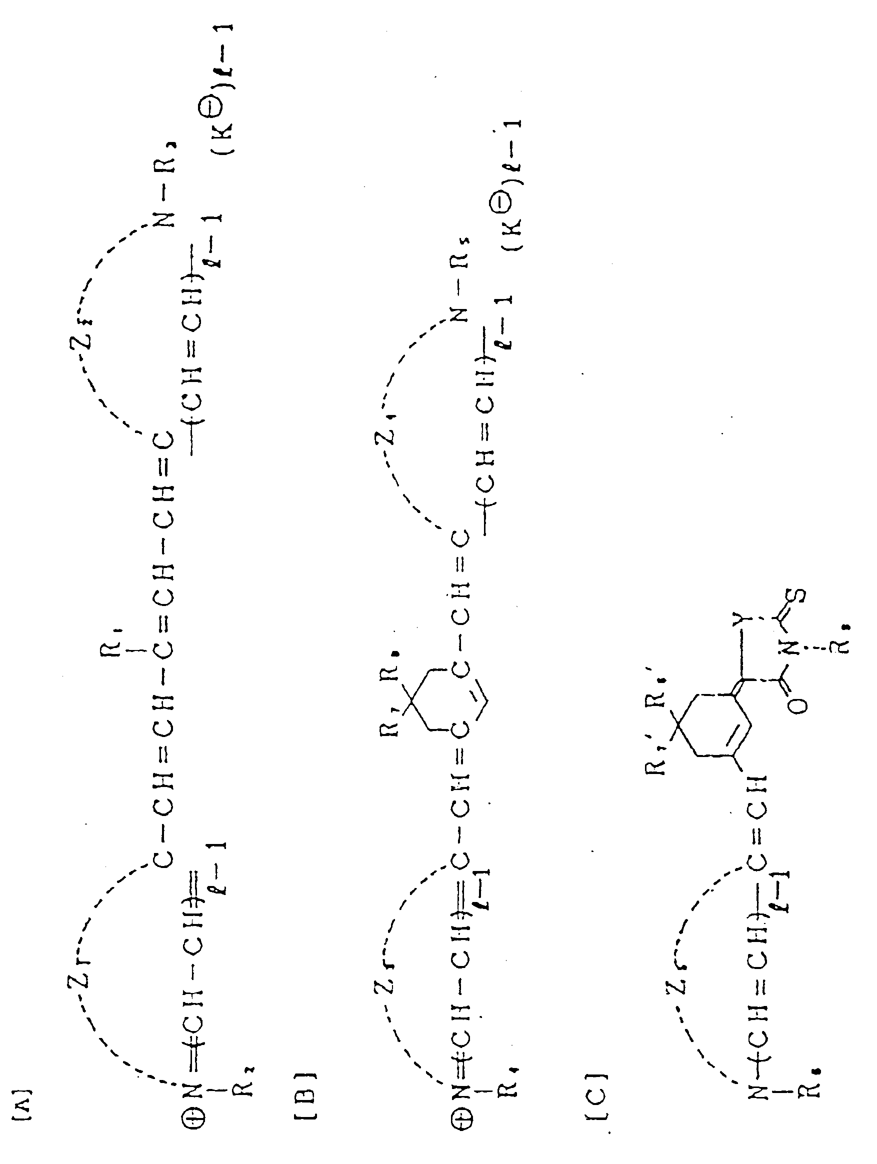 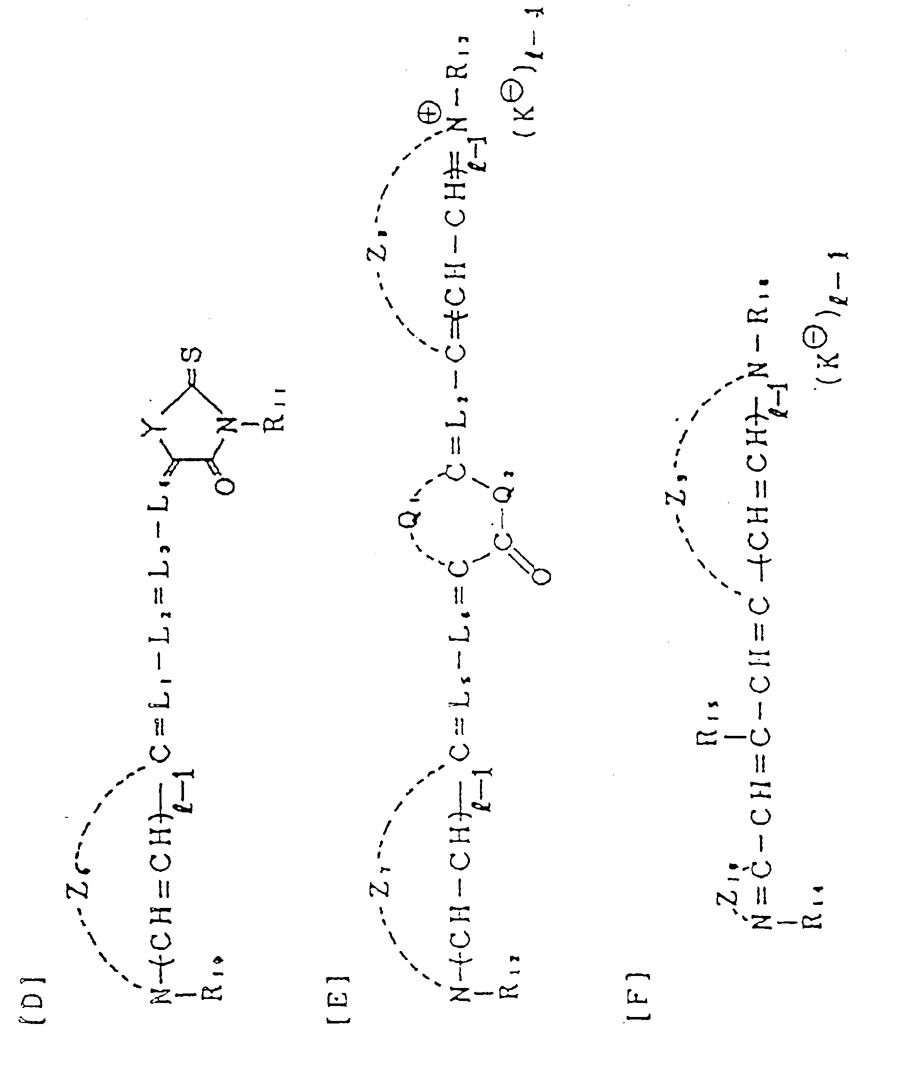 dans lesquelles Z₁ à Z₉ représentent chacun un groupe qui complète un cycle benzénique ou un cycle naphtalénique condensé avec un cycle pyridinique, un cycle imidazole, un cycle thiazole, un cycle sélénazole, un cycle oxazole ou un cycle tétrazole ; Z₁₀ est un groupe qui complète un cycle benzothiazole, un cycle benzosélénazole, un cycle (β-naphtothiazole, un cycle (β-naphtosélénazole, un cycle benzimidazole ou un cycle 2-quinoléine ; Q₁ et Q₂ représentent chacun un groupe qui complète un noyau 4-thiazolidinone, 5-thiazolidinone ou 4-imidazolidinone ; R₁ et R₁₅ représentent chacun un atome d'hydrogène, un groupe alkyle ou un groupe aryle ; R₇, R₈, R₇' et R₈' représentent chacun un groupe alkyle, R₉ et R₁₁ représentent chacun un groupe alkyle, un groupe aryle ou un groupe hétérocyclique ; R₂, R₃, R₄, R₅, R₆, R₁₀, R₁₂, R₁₃, R₁₄ et R₁₅ représentent chacun un groupe alkyle ou un groupe aryle ;
f vaut 1 ou 2; Y est un atome de soufre ou un atome de sélénium ; L₁ à L₈ représentent chacun un groupe méthyne substitué ou non substitué ; et K est un anion acide.

8. Coupleur formateur de colorant cyan, tel que défini dans l'une quelconque des revendications 1 à 6.
